# EUROPEAN PATENT APPLICATION

(11) **EP 4 303 226 A1**
(43) Date of publication of application: **10.01.2024**
(21) Application number: 22183980.6
(22) Date of filing: 08.07.2022
(51) Int. Cl.: C07K 14/34, A61K 48/00, C12N 15/67, C12N 15/85, C07K 14/47, C12N 9/10

(54) **TRANSGENE CASSETTES AND EPIGENETIC SILENCERS FOR THE TREATMENT OF DISORDERS**

(71) Applicant: Ospedale San Raffaele S.r.l., 20132 Milano (IT); Consiglio Nazionale delle Ricerche, 00185 Roma (IT)
(72) Inventor: BROCCOLI, Vania, 20132 Milan (IT); SESSA, Alessandro, 20132 Milan (IT)
(74) Representative: D Young & Co LLP

(57) **Abstract**

A polynucleotide comprising at least one miR-124 target sequence, and/or at least one miR-338-3p target sequence, and/or at least one miR-31 target sequence, wherein the miRNA target sequences are operably linked to a transgene.

## Description

### FIELD OF THE INVENTION

The present invention relates to transgene cassettes and polynucleotides for the treatment of diseases or disorders. Polynucleotides of the invention may comprise epigenetic silencer factors (ESFs) that mediate targeted gene silencing. Further, the polynucleotides of the invention may facilitate cell-specific transgene expression, for improved target specificity and safety.

### BACKGROUND TO THE INVENTION

Gene therapy involves the incorporation of genetic material into a cell to treat or prevent disease. The genetic material may supplement defective genes with functional copies of those genes, inactivate improperly functioning genes, silence genes that may be associated with a disease state (e.g. oncogenic or cancer-associated genes) or introduce new therapeutic genes to a cell.

To date, two main targeting technologies have been used to silence gene expression: RNA interference (RNAi) with single short hairpin RNA (shRNA); and gene targeting with artificial nucleases. Although promising pre-clinical and clinical data have been obtained using these technologies, partial depletion of gene expression with shRNA and the low efficiency by which homozygous disruption occurs in diploid mammalian cells may reduce the efficacy of these treatments. These disadvantages are particularly relevant in those applications where residual levels of gene activity are sufficient for biological function.

In addition, epigenetic mechanisms have been exploited to silence gene expression. Epigenetics refers to mechanisms that convey heritable changes in the function of the genome without altering the primary DNA sequence. These changes can mediate short-term instructions that can be quickly reverted in response to exogenous stimuli (e.g. histone posttranscriptional modifications; HPTMs). Alternatively, they can constitute long-term instructions that stably contribute to cellular identity and memory (e.g. DNA methylation).

Treatment of cancer may involve a range of approaches including surgery, chemotherapy and radiotherapy. However, even if surgical removal of a tumor is as radical as possible, even a small number of remaining cancer cells with tumor-initiating potential may be sufficient to regrow a tumor mass in the short term, leading to the reappearance of the disease. In particular, cancer stem cells (CSCs), defined as cells able to self-renew and to initiate or regrowth the tumor, remain quiescent or at very low proliferative activity and may be capable of resisting certain adjuvant treatments. Thus, there is a significant need to achieve a long-lasting remission, in particular after tumor resection by a more efficient targeting of cancer cells.

Such approaches may be particularly desirable for diseases such as glioblastoma multiforme (GBM), which is the most common and lethal brain cancer in adults with 1-5 cases per 100,000 people per year and 12 - 15 months of median survival. This poor outcome is due to the combination of both the aggressiveness of the disease and the limited efficacy of current therapies that increase the overall survival only marginally. Patients usually undergo surgical resection of the primary tumor mass followed by adjuvant radio- and chemo-(Temozolomide) therapies, although the issues with tumor regrowth described above may lead to recurrence of the cancer.

Attempts have made to restrain cancer (e.g. GBM) development by silencing expression of one or more transcription factors (TFs) with different technologies. For example, TF inactivation has been attempted with several technologies including shRNA, miRNA and TALEN-based epigenetic repressors, but complete and long-term gene silencing has proven challenging. Moreover, cancer cells may rearrange their genetic program to adapt to the silencing of a single gene and, thus, maintain unaltered tumorigenic potential.

Accordingly, there remains a significant need for the development of more effective treatments for cancer, in particular treatments that are effective against aggressive cancers such as GBM, and treatments that enable targeting of CSCs.

A further limitation in gene therapy is the ability to selectively determine whether a transgene is expressed within the cells to which it is delivered. There is an ongoing need for transgene expression cassettes that afford cell type specific transgene expression in order to reduce off-target effects associated with transgene expression, which in turn may improve the specificity and safety of any such gene therapies.

### SUMMARY OF THE INVENTION

Herein, the inventors provide a polynucleotide (e.g. transgene expression cassette) that utilizes miRNA target sequences in order to regulate transgene expression. Said expression cassettes allow transgene expression to be regulated such that unwanted expression is reduced or eliminated, thereby improving safety and reducing off target effects.

The present inventors have engineered oncogenic and cancer-associated transcription factors that function as epigenetic repressors, referred to as epigenetic silencer factors (ESFs), which, for example, enable silencing of downstream tumorigenic networks, thus limiting CSC survival and proliferation and therein reducing the chances of tumor regrowth. Herein, the inventors provide polynucleotides (e.g. transgene expression cassettes), which comprise said ESFs and miRNA target sequences, which may regulate the expression of the ESF in a cell-type specific manner.

In addition, the inventors have engineered oncogenic and cancer-associated transcription factors that function as epigenetic repressors, referred to as epigenetic silencer factors (ESFs), which, for example, enable silencing of downstream tumorigenic networks, thus limiting CSC survival and proliferation and therein reducing the chances of tumor regrowth.

In a first aspect, there is provided a polynucleotide comprising at least one miR-124 target sequence, and/or at least one miR-338-3p target sequence, and/or at least one miR-31 target sequence, wherein the miRNA target sequences are operably linked to a transgene.

In one embodiment, the polynucleotide comprises at least one miR-124 target sequence, wherein the target sequence is operably linked to the transgene.

In one embodiment, the polynucleotide comprises at least one miR-338-3p target sequence, wherein the target sequence is operably linked to the transgene.

In one embodiment, the polynucleotide comprises at least one miR-31 target sequence, wherein the target sequence is operably linked to the transgene.

In one embodiment, the polynucleotide comprises at least one miR-124 target sequence, and at least one miR-338-3p target sequence, wherein the target sequences are operably linked to the transgene.

In one embodiment, the polynucleotide comprises at least one miR-124 target sequence, and at least one miR-31 target sequence, wherein the target sequences are operably linked to the transgene.

In one embodiment, the polynucleotide comprises at least one miR-338-3p target sequence, and at least one miR-31 target sequence, wherein the target sequences are operably linked to the transgene.

In one embodiment, the polynucleotide comprises at least one miR-124 target sequence, at least one miR-338-3p target sequence, and at least one miR-31 target sequence, wherein the target sequences are operably linked to the transgene.

In one aspect, there is provided a polynucleotide comprising at least one miR-124 target sequence, at least one miR-338-3p target sequence and at least one miR-31 target sequence, wherein the miRNA target sequences are operably linked to a transgene.

In one embodiment, the number of copies of each of the miRNA target sequences is independently selected from the group consisting of: one, two, three, and four.

In one embodiment, the polynucleotide comprises four miR-124 target sequences, four miR-338-3p target sequences and four miR-31 target sequences, wherein the miRNA target sequences are operably linked to the transgene.

In one embodiment:
(a) the miR-124 target sequence comprises or consists of a nucleotide sequence that has at least 90% sequence identity, such as at least 91%, 92%, 93%, 94%, 95%, 96%, 97%, 98%, 99%, or 100% sequence identity to SEQ ID NO: 1;
(b) the miR-338-3p target sequence comprises or consists of a nucleotide sequence that has at least 90% sequence identity, such as at least 91%, 92%, 93%, 94%, 95%, 96%, 97%, 98%, 99%, or 100% sequence identity to SEQ ID NO: 2; and/or
(c) the miR-31 target sequence comprises or consists of a nucleotide sequence that has at least 90% sequence identity, such as at least 91%, 92%, 93%, 94%, 95%, 96%, 97%, 98%, 99%, or 100% sequence identity to SEQ ID NO: 3.

In one embodiment, the miR-124 target sequence comprises or consists of a nucleotide sequence that has at least 90% sequence identity, such as at least 91%, 92%, 93%, 94%, 95%, 96%, 97%, 98%, 99%, or 100% sequence identity to SEQ ID NO: 1. In one embodiment, the miR-338-3p target sequence comprises or consists of a nucleotide sequence that has at least 90% sequence identity, such as at least 91%, 92%, 93%, 94%, 95%, 96%, 97%, 98%, 99%, or 100% sequence identity to SEQ ID NO: 2. In one embodiment, the miR-31 target sequence comprises or consists of a nucleotide sequence that has at least 90% sequence identity, such as at least 91%, 92%, 93%, 94%, 95%, 96%, 97%, 98%, 99%, or 100% sequence identity to SEQ ID NO: 3.

In one embodiment:
(a) the miR-124 target sequence comprises or consists of a nucleotide sequence that has at least 90% sequence identity to SEQ ID NO: 1;
(b) the miR-338-3p target sequence comprises or consists of a nucleotide sequence that has at least 90% sequence identity to SEQ ID NO: 2; and/or
(c) the miR-31 target sequence comprises or consists of a nucleotide sequence that has at least 90% sequence identity to SEQ ID NO: 3.

In one embodiment:
(a) the miR-124 target sequence comprises or consists of a nucleotide sequence that has at least 95% sequence identity to SEQ ID NO: 1;
(b) the miR-338-3p target sequence comprises or consists of a nucleotide sequence that has at least 95% sequence identity to SEQ ID NO: 2; and/or
(c) the miR-31 target sequence comprises or consists of a nucleotide sequence that has at least 95% sequence identity to SEQ ID NO: 3.

In one embodiment:
(a) the miR-124 target sequence comprises or consists of a nucleotide sequence that has at least 99% sequence identity to SEQ ID NO: 1;
(b) the miR-338-3p target sequence comprises or consists of a nucleotide sequence that has at least 99% sequence identity to SEQ ID NO: 2; and/or
(c) the miR-31 target sequence comprises or consists of a nucleotide sequence that has at least 99% sequence identity to SEQ ID NO: 3.

In one embodiment:
(a) the miR-124 target sequence comprises or consists of a nucleotide sequence that has 100% sequence identity to SEQ ID NO: 1;
(b) the miR-338-3p target sequence comprises or consists of a nucleotide sequence that has 100% sequence identity to SEQ ID NO: 2; and/or
(c) the miR-31 target sequence comprises or consists of a nucleotide sequence that has 100% sequence identity to SEQ ID NO: 3.

In one embodiment, the miRNA target sequences are located downstream, i.e., 3', of the transgene. In other words, the miRNA target sequences may be located after the transgene in the 5' to 3' direction.

In one embodiment, the miRNA target sequences are located within the 3'-UTR of the transgene.

In one embodiment, the miRNA target sequences or clusters of copies of the miRNA target sequences are, from 5' to 3', arranged in the order: miR-124 target sequence(s), miR-338-3p target sequence(s), and miR-31 target sequence(s). The target sequences, or clusters comprising one or more copy thereof, may be, for example, arranged from 5' to 3' such that they form groups according to their target specificity, for example, in one embodiment the polynucleotide comprises 5' - [miR-124 target sequence] ₄ - [miR-338-3p target sequence] ₄ - [miR-31 target sequence]₄ - 3'.

Both the individual target sequences and the clusters of target sequences may be contiguous with one another, separated by spacer sequences, or any combination of thereof.

Thus, in one embodiment, the miRNA target sequences are separated by spacer sequences.

In one embodiment, there is provided a polynucleotide wherein the polynucleotide comprises a nucleotide sequence that has at least 90% sequence identity to SEQ ID NO: 4. In one embodiment, the polynucleotide comprises a nucleotide sequence that has at least 90%, such as at least 91%, 92%, 93%, 94%, 95%, 96%, 97%, 98%, 99%, or 100% sequence identity to SEQ ID NO: 4.

In one embodiment, the miRNA target sequences comprise a sequence as set forth in SEQ ID NO: 4.

In one embodiment, the miRNA target sequences consist of a sequence as set forth in SEQ ID NO: 4.

In one embodiment, the transgene encodes an epigenetic silencer factor (ESF) comprising a transcription factor DNA-binding domain operably linked to at least one epigenetic effector domain, wherein the transcription factor is an oncogenic transcription factor or a cancer-associated transcription factor.

In some embodiments, the ESF is a fusion protein comprising the transcription factor DNA-binding domain and the at least one epigenetic effector domain.

In preferred embodiments, the transcription factor is an oncogenic transcription factor. In some embodiments, the transcription factor is a cancer-associated transcription factor.

In one embodiment, the transcription factor is selected from the group consisting of SOX2, MYC, MYCN, TEAD1, TEAD2, TEAD3, TEAD4, FOXA1, FOXA2, ELK1, ELK3, ELK4, SRF, FOXM1, FOXC1, FOXC2, TWIST1, SALL4, ELF1, HIF1A, SOX9, SOX12, SOX18, ETS1, PAX3, PAX8, GLI1, GLI2, GLI3, ETV1, ETV2, ETV3, RUNX1, RUNX2, RUNX3, MAFB, TFAP2C and E2F1.

In one embodiment, the transcription factor is SOX2.

In one embodiment, the transcription factor is TEAD1.

In one embodiment, the transcription factor is MYC.

In preferred embodiments, the ESF does not comprise a transcription factor activation domain.

In one embodiment, the epigenetic effector domain is selected from the group consisting of a KRAB domain, a DNMT3A domain, a DNMT3L domain, a ZIM3-KRAB (Z-KRAB) domain, a Chromo Shadow (CS) domain, a YAF2-RYBP (Y-R) domain, an Engrailed Repressor (En-R) domain, a MeCP2 domain, a GLI3RD domain and a MAD1RD domain.

In one embodiment, the epigenetic effector domain is (a) a CS domain; (b) a Y-R domain; (c) a CS domain and a Y-R domain; (d) a KRAB domain; and/or (e) a DNMT3A domain and a DNMT3L domain.

The ESF according to the invention may comprise a combination of a number of the foregoing transcription factor DNA-binding and epigenetic effector domains.

In one embodiment, the epigenetic effector domain is a CS domain.

In one embodiment, the epigenetic effector domain is a Y-R domain.

In one embodiment, the epigenetic effector domain is a CS domain and a Y-R domain.

In one embodiment, the epigenetic effector domain is a KRAB domain.

In one embodiment, the epigenetic effector domain is a DNMT3A domain.

In one embodiment, the epigenetic effector domain is a DNMT3L domain.

In one embodiment, the epigenetic effector domain is a DNMT3A domain and a DNMT3L domain.

In one embodiment, the epigenetic effector domain is a ZIM3-KRAB (Z-KRAB) domain.

In one embodiment, the epigenetic effector domain is an Engrailed Repressor (En-R) domain.

In one embodiment, the epigenetic effector domain is a MeCP2 domain.

In one embodiment, the epigenetic effector domain is a GLI3RD domain.

In one embodiment, the epigenetic effector domain is a MAD1RD domain.

In one embodiment, the ESF comprises:
(a) a KRAB domain, a SOX2 DNA-binding domain, a DNMT3A domain, and a DNMT3L domain;
(b) a Chromo Shadow (CS) domain and a SOX2 DNA-binding domain;
(c) a SOX2 DNA-binding domain, and a YAF2-RYBP (Y-R) domain;
(d) a KRAB domain, a TEAD1 DNA-binding domain, a DNMT3A domain, and a DNMT3L domain;
(e) a KRAB domain, a DNMT3A domain, a DNMT3L domain, and a MYC DNA-binding domain;
(f) a Chromo Shadow (CS) domain, and a TEAD1 DNA-binding domain;
(g) a TEAD1 DNA-binding domain, and a YAF2-RYBP (Y-R) domain;
(h) a Chromo Shadow (CS) domain, a TEAD1 DNA-binding domain, and a YAF2-RYBP (Y-R) domain;
(i) a Chromo Shadow (CS) domain, and a MYC DNA-binding domain;
(j) a YAF2-RYBP (Y-R) domain, and a MYC DNA-binding domain; or
(k) a Chromo Shadow (CS) domain, a YAF2-RYBP (Y-R) domain, and a MYC DNA-binding domain.

In one embodiment, the ESF comprises, from 5' to 3':
(a) a KRAB domain, a SOX2 DNA-binding domain, a DNMT3A domain, and a DNMT3L domain;
(b) a Chromo Shadow (CS) domain and a SOX2 DNA-binding domain;
(c) a SOX2 DNA-binding domain, and a YAF2-RYBP (Y-R) domain;
(d) a KRAB domain, a TEAD1 DNA-binding domain, a DNMT3A domain, and a DNMT3L domain;
(e) a KRAB domain, a DNMT3A domain, a DNMT3L domain, and a MYC DNA-binding domain;
(f) a Chromo Shadow (CS) domain, and a TEAD1 DNA-binding domain;
(g) a TEAD1 DNA-binding domain, and a YAF2-RYBP (Y-R) domain;
(h) a Chromo Shadow (CS) domain, a TEAD1 DNA-binding domain, and a YAF2-RYBP (Y-R) domain;
(i) a Chromo Shadow (CS) domain, and a MYC DNA-binding domain;
(j) a YAF2-RYBP (Y-R) domain, and a MYC DNA-binding domain; or
(k) a Chromo Shadow (CS) domain, a YAF2-RYBP (Y-R) domain, and a MYC DNA-binding domain.

In one embodiment, the polynucleotide comprises a nucleotide sequence that has at least 90% sequence identity, such as at least 91%, 92%, 93%, 94%, 95%, 96%, 97%, 98%, 99%, or 100% sequence identity to any one of SEQ ID NOs: 114 - 119 and 126 - 131.

In one embodiment, the polynucleotide comprises a nucleotide sequence that has at least 90% sequence identity to any one of SEQ ID NOs: 114 - 119 and 126 - 131.

In one embodiment, the polynucleotide comprises a nucleotide sequence that has at least 95% sequence identity to any one of SEQ ID NOs: 114 - 119 and 126 - 131.

In one embodiment, the polynucleotide comprises a nucleotide sequence that has at least 99% sequence identity to any one of SEQ ID NOs: 114 - 119 and 126 - 131.

In one embodiment, the polynucleotide comprises or consists of a nucleotide sequence of any one of SEQ ID NOs: 114 - 119 and 126 - 131.

In one embodiment, the polynucleotide comprises a nucleotide sequence that has at least 90% sequence identity, such as at least 91%, 92%, 93%, 94%, 95%, 96%, 97%, 98%, 99%, or 100% sequence identity to any one of SEQ ID NOs: 158 - 163.

In one embodiment, the polynucleotide comprises a sequence according to any one of SEQ ID NOs: 158 - 163.

In one embodiment, the polynucleotide comprises a nucleotide sequence that has at least 90% sequence identity, such as at least 91%, 92%, 93%, 94%, 95%, 96%, 97%, 98%, 99%, or 100% sequence identity to any one of SEQ ID NOs: 178 - 182.

In one embodiment, the polynucleotide comprises a sequence according to any one of SEQ ID NOs: 178 - 182.

In one embodiment, the polynucleotide comprises a nucleotide sequence that has at least 90% sequence identity, such as at least 91%, 92%, 93%, 94%, 95%, 96%, 97%, 98%, 99%, or 100% sequence identity to any one of SEQ ID NOs: 164 - 174.

In one embodiment, the polynucleotide comprises a sequence according to any one of SEQ ID NOs: 164 - 174. In one embodiment, the polynucleotide comprises a nucleotide sequence that has at least 90% sequence identity, such as at least 91%, 92%, 93%, 94%, 95%, 96%, 97%, 98%, 99%, or 100% sequence identity to any one of SEQ ID NOs: 136 - 141 and 153 - 157.

In one embodiment, the polynucleotide comprises a sequence according to any one of SEQ ID NOs: 136 - 141 and 153 - 157.

In one embodiment, the polynucleotide comprises a nucleotide sequence that has at least 90% sequence identity, such as at least 91%, 92%, 93%, 94%, 95%, 96%, 97%, 98%, 99%, or 100% sequence identity to any one of SEQ ID NOs: 142 - 152.

In one embodiment, the polynucleotide comprises a sequence according to any one of SEQ ID NOs: 142 - 152.

In one embodiment, the polynucleotide comprises a nucleotide sequence that has at least 90% sequence identity, such as at least 91%, 92%, 93%, 94%, 95%, 96%, 97%, 98%, 99%, or 100% sequence identity to any one of SEQ ID NOs: 114 - 119, 126 - 131, and 136 - 174.

In one embodiment, the polynucleotide comprises a sequence according to any one of SEQ ID NOs: 114 - 119, 126 - 131, and 136 - 174 .

In one embodiment, the polynucleotide further comprises a promoter. In one embodiment, the promoter is operably linked to the transgene or nucleotide sequence encoding the ESF.

In one embodiment, the promoter is a constitutive promoter. In some embodiments, the constitutive promoter is an Ef1a promoter.

In one embodiment, the promoter is a tissue-specific promoter, preferably a cancer cell-specific promoter.

In one embodiment, the promoter is a proliferating cell-specific promoter.

In one embodiment, the polynucleotide further comprises a promoter operably linked to the transgene, optionally wherein the promoter is a tissue-specific promoter or a constitutive promoter, optionally a cancer cell-specific promoter.

In one embodiment, the promoter is selected from the group consisting of a Mki67 promoter, a Ccnd1 promoter, a Ccnb2 promoter, a Ccna2 promoter, a Cdc25c promoter, a Cdc2 promoter, a Cks1 promoter, a PCNA promoter, a CDC6 promoter, a POLD1 promoter, a CSK1B promoter, a MCM2 promoter and a PLK1 promoter.

In one embodiment, the promoter is a Mki67 promoter.

In one embodiment, the promoter is an Ef1a promoter.

In one aspect, there is provided a vector comprising the polynucleotide according to the invention.

In one embodiment, the vector is a viral vector.

In one embodiment, the vector is a lentiviral vector.

In one embodiment, the vector is an adeno-associated viral (AAV) vector.

In one embodiment, the AAV vector is of serotype, 2, 5, or 9. In a preferred embodiment, the vector is an AAV2 vector. In another preferred embodiment, the vector is an AAV5 vector.

In one embodiment, the vector is an mRNA vector.

In one aspect, there is provided a protein encoded by a polynucleotide or vector according to the invention.

In one embodiment, there is provided a therapeutic protein encoded by a polynucleotide or vector according to the invention.

In one embodiment, there is provided an ESF encoded by a polynucleotide or vector according to the invention.

In one aspect, there is provided a protein (e.g. an ESF) that comprises or consists of an amino acid sequence that has at least 70% sequence identity, such as at least 75%, 80%, 85%, 90%, 91%, 92%, 93%, 94%, 95%, 96%, 97%, 98%, 99%, or 100% sequence identity to any one of SEQ ID NO: 109 - 113 and 120 - 125.

In one embodiment, the polynucleotide or transgene encodes a protein that has at least 70% sequence identity, such as at least 75%, 80%, 85%, 90%, 91%, 92%, 93%, 94%, 95%, 96%, 97%, 98%, 99%, or 100% sequence identity to any one of SEQ ID NO: 109 - 113 and 120 - 125, or a fragment thereof.

In one embodiment, the polynucleotide or transgene encodes a protein that has at least 90% sequence identity to any one of SEQ ID NO: 109 - 113 and 120 - 125, or a fragment thereof.

In one embodiment, the polynucleotide or transgene encodes a protein that has at least 95% sequence identity to any one of SEQ ID NO: 109 - 113 and 120 - 125, or a fragment thereof.

In one embodiment, the polynucleotide or transgene encodes a protein that has at least 99% sequence identity to any one of SEQ ID NO: 109 - 113 and 120 - 125, or a fragment thereof.

In one embodiment, the polynucleotide or transgene encodes a protein that has 100% sequence identity to any one of SEQ ID NO: 109 - 113 and 120 - 125, or a fragment thereof.

In one aspect, there is provided a polynucleotide comprising a transgene encoding an epigenetic silencer factor (ESF), wherein the transgene is operably linked to at least one micro RNA (miRNA) target sequence, wherein the ESF comprises a transcription factor DNA-binding domain operably linked to at least one epigenetic effector domain, and wherein the transcription factor is an oncogenic transcription factor or a cancer-associated transcription factor.

The miRNA target sequence may be designed to be recognised by any suitable miRNA or selected from any suitable known miRNA target sequences, e.g., an miRNA that is expressed in a cell type in which transgene expression is not desired.

In one embodiment, the at least one miRNA target sequence is a target site for an miRNA selected from the group consisting of miR-124, miR-338-3p, and miR-31.

In one embodiment, the miRNA target sequence is a target site for miR-124 or consists of a nucleotide sequence that has at least 90%, such as 91%, 92%, 93%, 94%, 95%, 96%, 97%, 98%, 99% or 100% sequence identity to SEQ ID NO: 1.

In one embodiment, the miRNA target sequence is a target site for miR-338-3p or consists of a nucleotide sequence that has at least 90%, such as 91%, 92%, 93%, 94%, 95%, 96%, 97%, 98%, 99% or 100% sequence identity to SEQ ID NO: 2.

In one embodiment, the miRNA target sequence is a target site for miR-31 or consists of a nucleotide sequence that has at least 90%, such as 91%, 92%, 93%, 94%, 95%, 96%, 97%, 98%, 99% or 100% sequence identity to SEQ ID NO: 3.

In one embodiment, the at least one miRNA target sequence comprises SEQ ID NOs: 1, 2, and 3, or a sequence that has at least 90%, such as 91%, 92%, 93%, 94%, 95%, 96%, 97%, 98%, 99% or 100% sequence identity thereto.

In a preferred embodiment, the at least one miRNA target sequence comprises a target site for miR-124, miR-338-3p, and miR-31, preferably wherein four copies of each target sequence are present.

The order of the miRNA target sequences may be varied. In one embodiment, the miRNA target sequences or clusters of copies of sequences are, from 5' to 3', arranged in the order: miR-124 target sequence(s), miR-338-3p target sequence(s), and miR-31 target sequence(s).

In one embodiment, the miRNA target sequences or clusters of copies of sequences are, from 5' to 3', arranged in the order: miR-124 target sequence(s), miR-31 target sequence(s), and miR-338-3p target sequence(s).

In another embodiment, the miRNA target sequences or clusters of copies of sequences are, from 5' to 3', arranged in the order: miR-338-3p target sequence(s), miR-124 target sequence(s), and miR-31 target sequence(s).

In another embodiment, the miRNA target sequences or clusters of copies of sequences are, from 5' to 3', arranged in the order: miR-338-3p target sequence(s), miR-31 target sequence(s), and miR-124 target sequence(s).

In another embodiment, the miRNA target sequences or clusters of copies of sequences are, from 5' to 3', arranged in the order: miR-31 target sequence(s), miR-124 target sequence(s), and miR-338-3p target sequence(s).

In another embodiment, the miRNA target sequences or clusters of copies of sequences are, from 5' to 3', arranged in the order: miR-31 target sequence(s), miR-338-3p target sequence(s), and miR-124 target sequence(s).

Both the individual target sequences and clusters of copies of sequences may be contiguous with one another, separated by spacer sequences, or any combination of thereof.

Thus, in one embodiment, the miRNA target sequences are separated by spacer sequences.

In one embodiment, there is provided a polynucleotide wherein the polynucleotide comprises a nucleotide sequence that has at least 90% sequence identity to SEQ ID NO: 4. In one embodiment, the polynucleotide comprises a sequence that has at least 90%, such as at least 91%, 92%, 93%, 94%, 95%, 96%, 97%, 98%, 99%, or 100% sequence identity to SEQ ID NO: 4.

In one aspect, there is provided an epigenetic silencer factor (ESF) comprising a transcription factor DNA-binding domain operably linked to at least one epigenetic effector domain, or a polynucleotide encoding therefor, wherein the transcription factor is an oncogenic transcription factor or a cancer-associated transcription factor, wherein the ESF comprises:
(a) a Chromo Shadow (CS) domain, and a TEAD1 DNA-binding domain;
(b) a TEAD1 DNA-binding domain, and a YAF2-RYBP (Y-R) domain;
(c) a Chromo Shadow (CS) domain, a TEAD1 DNA-binding domain, and a YAF2-RYBP (Y-R) domain;
(d) a Chromo Shadow (CS) domain, and a MYC DNA-binding domain;
(e) a YAF2-RYBP (Y-R) domain, and a MYC DNA-binding domain; or
(f) a Chromo Shadow (CS) domain, a YAF2-RYBP (Y-R) domain, and a MYC DNA-binding domain.

In one embodiment, there is provided an epigenetic silencer factor (ESF) comprising a transcription factor DNA-binding domain operably linked to at least one epigenetic effector domain, wherein the transcription factor is an oncogenic transcription factor or a cancer-associated transcription factor, wherein the ESF consists of:
(a) a Chromo Shadow (CS) domain, and a TEAD1 DNA-binding domain;
(b) a TEAD1 DNA-binding domain, and a YAF2-RYBP (Y-R) domain;
(c) a Chromo Shadow (CS) domain, a TEAD1 DNA-binding domain, and a YAF2-RYBP (Y-R) domain;
(d) a Chromo Shadow (CS) domain, and a MYC DNA-binding domain;
(e) a YAF2-RYBP (Y-R) domain, and a MYC DNA-binding domain; or
(f) a Chromo Shadow (CS) domain, a YAF2-RYBP (Y-R) domain, and a MYC DNA-binding domain.

In one embodiment, the ESF comprises a sequence that has at least 70%, such as at least 75%, 80%, 85%, 90%, 91%, 92%, 93%, 94%, 95%, 96%, 97%, 98%, 99%, or 100% sequence identity to any one of SEQ ID NOs: 120 - 125, or a fragment thereof.

In one embodiment, the ESF comprises a sequence that has at least 90%, sequence identity to any one of SEQ ID NOs: 120 - 125, or a fragment thereof.

In one embodiment, the ESF comprises a sequence that has at least 95%, sequence identity to any one of SEQ ID NOs: 120 - 125, or a fragment thereof.

In one embodiment, the ESF comprises a sequence that has at least 99%, sequence identity to any one of SEQ ID NOs: 120 - 125, or a fragment thereof.

In one embodiment, the ESF comprises a sequence that has 100%, sequence identity to any one of SEQ ID NOs: 120 - 125, or a fragment thereof.

In one embodiment, the ESF comprises or consists of a sequence according to any one of SEQ ID NOs: 120 - 125, or a fragment thereof.

In one embodiment, the polynucleotide encoding the ESF comprises a sequence that has at least 70%, such as at least 75%, 80%, 85%, 90%, 91%, 92%, 93%, 94%, 95%, 96%, 97%, 98%, 99%, or 100% sequence identity to any one of SEQ ID NOs: 126 - 131.

In one embodiment, the polynucleotide encoding the ESF comprises a sequence that has at least 70%, such as at least 75%, 80%, 85%, 90%, 91%, 92%, 93%, 94%, 95%, 96%, 97%, 98%, 99%, or 100% sequence identity to any one of SEQ ID NOs: 158 - 163.

In one embodiment, the polynucleotide encoding the ESF comprises a sequence that has at least 70%, such as at least 75%, 80%, 85%, 90%, 91%, 92%, 93%, 94%, 95%, 96%, 97%, 98%, 99%, or 100% sequence identity to any one of SEQ ID NOs: 178 - 182.

In one embodiment, the polynucleotide encoding the ESF comprises a sequence that has at least 70%, such as at least 75%, 80%, 85%, 90%, 91%, 92%, 93%, 94%, 95%, 96%, 97%, 98%, 99%, or 100% sequence identity to any one of SEQ ID NOs: 168 - 173.

In one embodiment, the polynucleotide encoding the ESF comprises a sequence that has at least 70%, such as at least 75%, 80%, 85%, 90%, 91%, 92%, 93%, 94%, 95%, 96%, 97%, 98%, 99%, or 100% sequence identity to any one of SEQ ID NOs: 136 - 141.

In one embodiment, the polynucleotide encoding the ESF comprises a sequence that has at least 70%, such as at least 75%, 80%, 85%, 90%, 91%, 92%, 93%, 94%, 95%, 96%, 97%, 98%, 99%, or 100% sequence identity to any one of SEQ ID NOs: 146 - 148 and 150 - 152.

The following aspects and embodiments may be applied to any of the foregoing aspects and embodiments of the invention.

In one aspect, there is provided the nanoparticle comprising a polynucleotide, vector, ESF or protein according to the invention.

In one embodiment, the nanoparticle is a polymeric nanoparticle, inorganic nanoparticle or lipid nanoparticle. In some embodiments, the nanoparticle is a liposome

In one aspect, there is provided a cell comprising the polynucleotide, vector, protein, ESF or nanoparticle according to the invention.

In one embodiment, the cell is a eukaryotic cell, such as a mammalian cell. In another embodiment the cell is a human cell.

In one aspect, there is provided a composition comprising the polynucleotide, vector, protein, ESF, nanoparticle, or cell according to the invention.

The composition may be a hydrogel. In some embodiments, the hydrogel is a poly(ethylene glycol) dimethacrylate (PEG-DMA) hydrogel. In some embodiments, the hydrogel further comprises hydroxyapatite nanoparticles.

In one aspect, there is provided the pharmaceutical composition comprising the polynucleotide, vector, protein, ESF, nanoparticle, or cell according to the invention, and a pharmaceutically acceptable diluent, solvent, carrier, salt and/or adjuvant.

In one aspect, there is provided the polynucleotide, vector, protein, ESF, nanoparticle, cell, composition, or pharmaceutical composition according to the invention, for use in therapy.

In one aspect, there is provided the polynucleotide, vector, protein, ESF, nanoparticle, cell, composition, or pharmaceutical composition according to the invention, for use in the treatment of a disease or disorder.

In one aspect, there is provided the polynucleotide, vector, protein, ESF, nanoparticle, cell, composition, or pharmaceutical composition according to the invention, for use in the treatment of cancer.

In some embodiments, the cancer is glioma, gliobastoma, medulloblastoma, astrocytoma, neuroblastomas, ependymoma, meningioma, retinoblastoma, rhabdomyosarcoma, lung cancer, prostate cancer, breast cancer, liver cancer, pancreatic cancer, bladder cancer, oropharyngeal cancer or kidney cancer. In some embodiments, the cancer is a brain tumour. In some embodiments, the cancer is gliobastoma multiforme.

In one embodiment, the cancer is a liver metastasis.

In one embodiment, the cancer is a colon cancer, for example a colon adenocarcinoma.

In some embodiments, the treatment reduces tumour size.

In some embodiments, the treatment is as an adjuvant therapy, optionally in combination with surgery. The treatment may reduce the risk of cancer (e.g. GBM) recurrence.

In some embodiments, the ESF, polynucleotide, vector, cell or composition (e.g. hydrogel) is administered locally.

In one aspect, there is provided use of the polynucleotide, vector, protein, ESF, nanoparticle, cell, composition, or pharmaceutical composition according to the invention, for decreasing transcription and/or expression of at least one target gene in a cell.

In one aspect, there is provided a method of decreasing transcription and/or expression of at least one target gene in a cell, the method comprising introducing the polynucleotide, vector, protein, ESF, nanoparticle, cell, composition, or pharmaceutical composition according to the invention, into said cell.

In one embodiment, the foregoing method is an *in vitro* method.

In one embodiment, the foregoing method is an *in vivo* method.

In one embodiment, the foregoing method is an ex *vivo* method.

In one embodiment, the at least one target gene is silenced, preferably permanently silenced.

In another aspect, the invention provides use of the polynucleotide, vector, protein, ESF, nanoparticle, cell, composition, or pharmaceutical composition according to the invention, for the manufacture of a medicament for therapy.

In another aspect, the invention provides use of the polynucleotide, vector, protein, ESF, nanoparticle, cell, composition, or pharmaceutical composition according to the invention, for the manufacture of a medicament for the treatment of cancer.

In one aspect, there is provided an epigenetic silencer factor (ESF), or polynucleotide encoding therefor, for use in the treatment of cancer, wherein the ESF comprises a transcription factor DNA-binding domain operably linked to at least one epigenetic effector domain, wherein the transcription factor is an oncogenic transcription factor or a cancer-associated transcription factor, and wherein the cancer is selected from the group consisting of: glioma, gliobastoma, medulloblastoma, astrocytoma, neuroblastomas, ependymoma, meningioma, retinoblastoma, rhabdomyosarcoma, lung cancer, prostate cancer, breast cancer, liver cancer, pancreatic cancer (e.g. human pancreatic ductal adenocarcinoma), bladder cancer, oropharyngeal cancer, kidney cancer, colon cancer (e.g. colon adenocarcinoma), or a metastasis of any of the foregoing.

The ESF or polynucleotide may be, for example, in the form of or comprised in a vector, nanoparticle, cell or composition.

In some embodiments, the cancer is glioma, gliobastoma, medulloblastoma, astrocytoma, neuroblastomas, ependymoma, meningioma, retinoblastoma, rhabdomyosarcoma, lung cancer, prostate cancer, breast cancer, pancreatic cancer (e.g. human pancreatic ductal adenocarcinoma), bladder cancer, oropharyngeal cancer, colon cancer (e.g. colon adenocarcinoma) or kidney cancer. In some embodiments, the cancer is a brain tumour. In some embodiments, the cancer is gliobastoma multiforme.

In one embodiment, the cancer is a glioblastoma, prostate cancer, human pancreatic ductal adenocarcinoma, colon cancer or colon adenocarcinoma.

In one embodiment, the cancer is a metastasis of a primary cancer, wherein the primary cancer is glioma, gliobastoma, medulloblastoma, astrocytoma, neuroblastomas, ependymoma, meningioma, retinoblastoma, rhabdomyosarcoma, lung cancer, prostate cancer, breast cancer, pancreatic cancer (e.g. human pancreatic ductal adenocarcinoma), bladder cancer, oropharyngeal cancer, colon cancer (e.g. colon adenocarcinoma) or kidney cancer. In some embodiments, the primary cancer is a brain tumour. In some embodiments, the primary cancer is gliobastoma multiforme.

In one embodiment, the cancer is a human pancreatic ductal adenocarcinoma.

In one embodiment, the cancer is a liver metastasis of a colon cancer.

In one embodiment, the cancer is a colon cancer.

In some embodiments, the metastasis is a liver metastasis (preferably derived from a colon cancer).

In some embodiments, the treatment is as an adjuvant therapy, optionally in combination with surgery. The treatment may reduce the risk of cancer (e.g. GBM) recurrence.

In some embodiments, the ESF, polynucleotide, vector, cell or composition (e.g. hydrogel) is administered locally.

In one embodiment, the vector is a viral vector.

In one embodiment, the vector is a lentiviral vector.

In one embodiment, the vector is an adeno-associated viral (AAV) vector.

In one embodiment, the vector is an mRNA vector.

### DESCRIPTION OF THE DRAWINGS

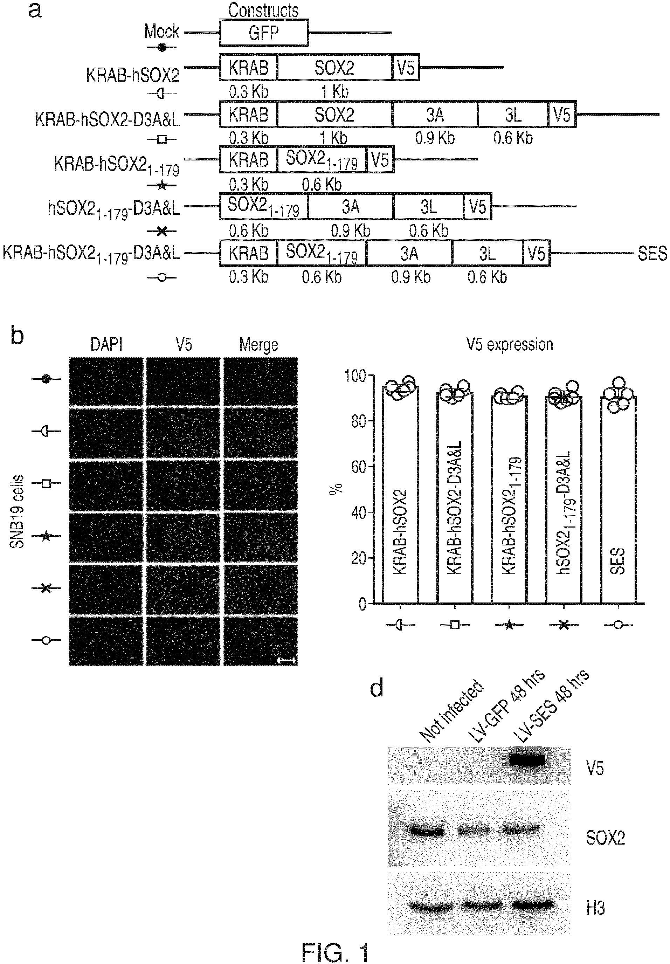

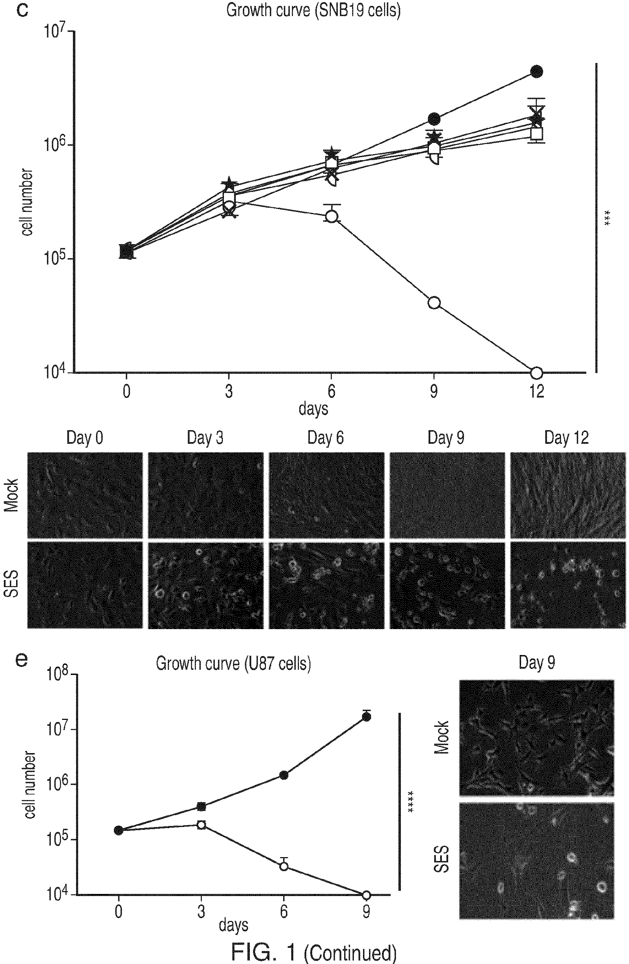

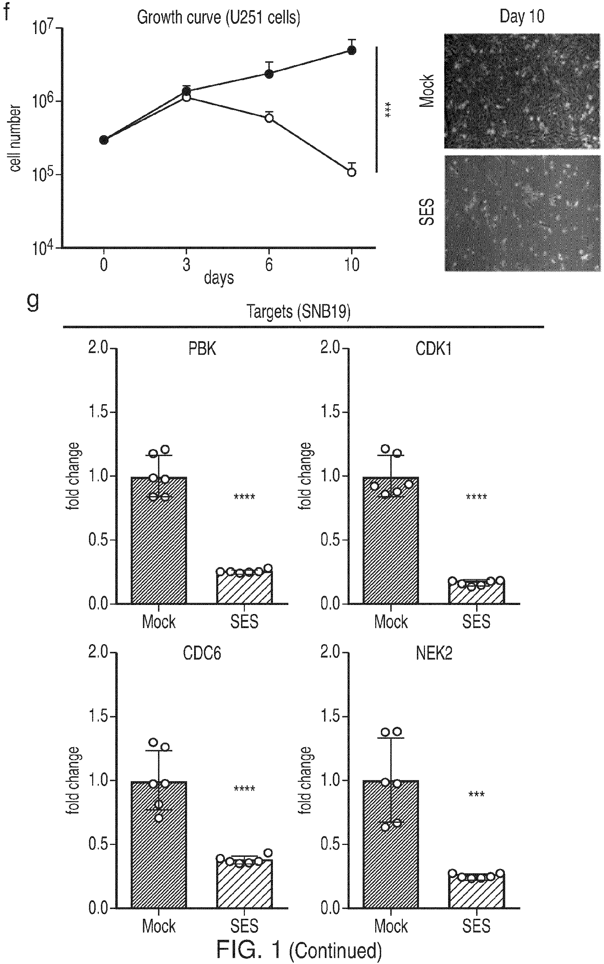

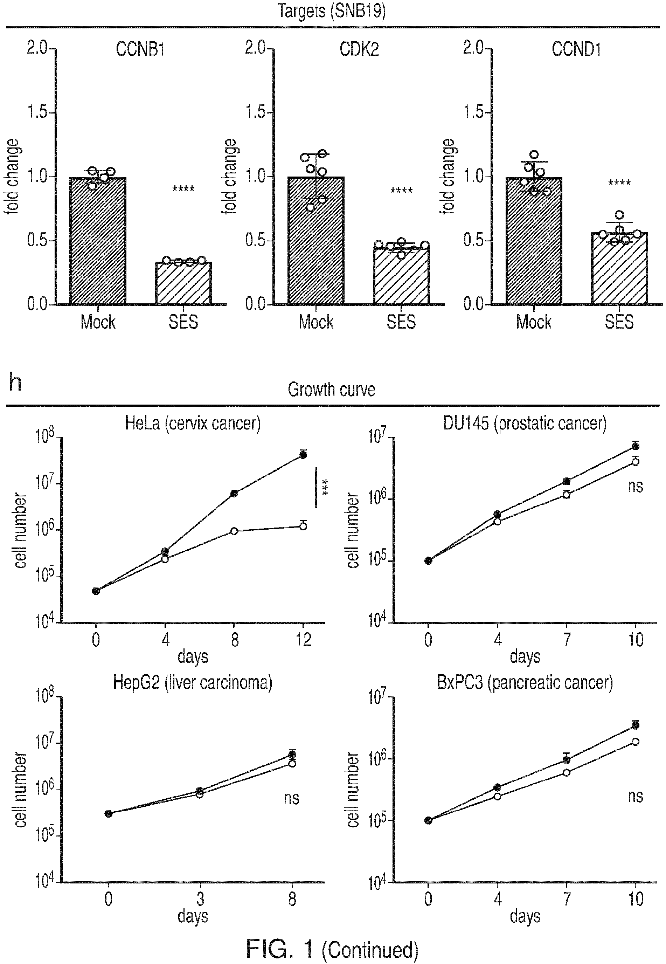

**FIGURE 1****. Generation and testing of SOX2 epigenetic silencer (SES).**
   (a) Constructs generated based on human SOX2 transcription factor and the epigenetic domain KRAB, DNMT3a (3A) and DNMT3L (3L), V5 was added as tag. (b) Infection efficiency of lentivirus carrying the indicated constructs in SNB19 cells. (c) Left, growth curve of SNB19 cells infected with the indicated constructs show that SES was able to kill the cells after 12 days in culture, ****p < 0.0001; statistically compared with two-way ANOVA; right, microphotographs of the cells at the indicated time points from the infection with either mock (GFP) or SES. (d) Western blot (WB) for V5, SOX2 and H3 (as loading control) in SNB19 cells either not infected or infected with lentivirus carrying GFP or SES. (e) Left, growth curve of U87 cells, ****p < 0.0001; statistically compared with two-way ANOVA; right, microphotographs of the cells at pre-treatment and after 9 days from the infection with either mock (GFP) or SES. f) Left, growth curve of U251 cells, ***p < 0.001; statistically compared with two-way ANOVA; right, microphotographs of the cells at pre-treatment and after 10 days from the infection with either mock (GFP) or SES. (g) Quantification of the indicated SOX2 targets in SNB19 cells 3 days after infection with either GFP or SES. ****p < 0.0001, ***p < 0.001; statistically compared using unpaired t test. (h) Growth curves of the indicated cancer cell lines. ***p < 0.001, ns = not significant; statistically compared with two-way ANOVA.

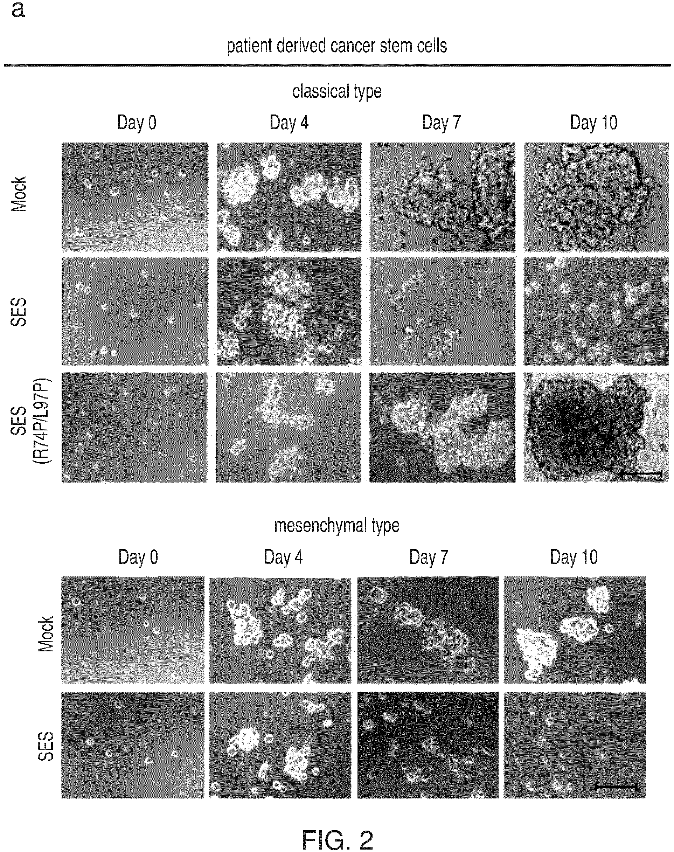

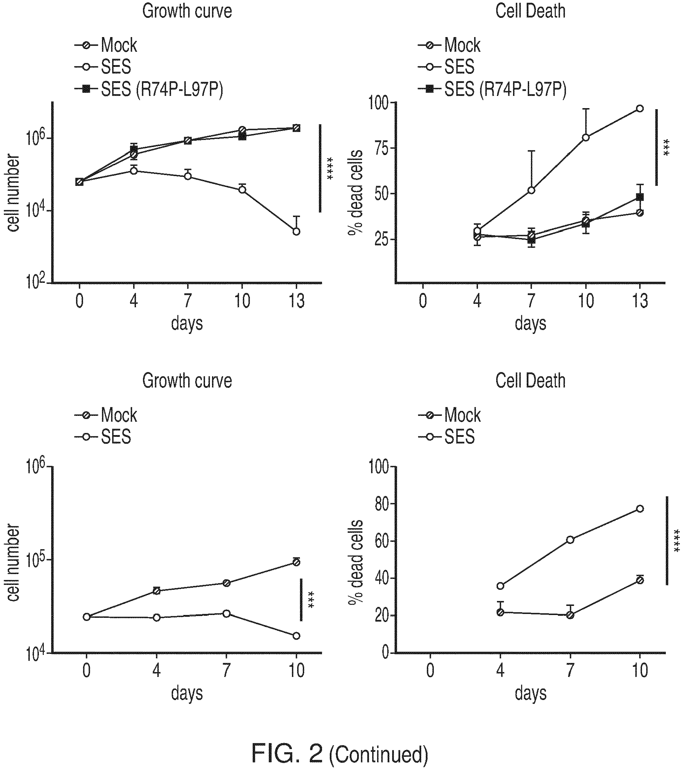

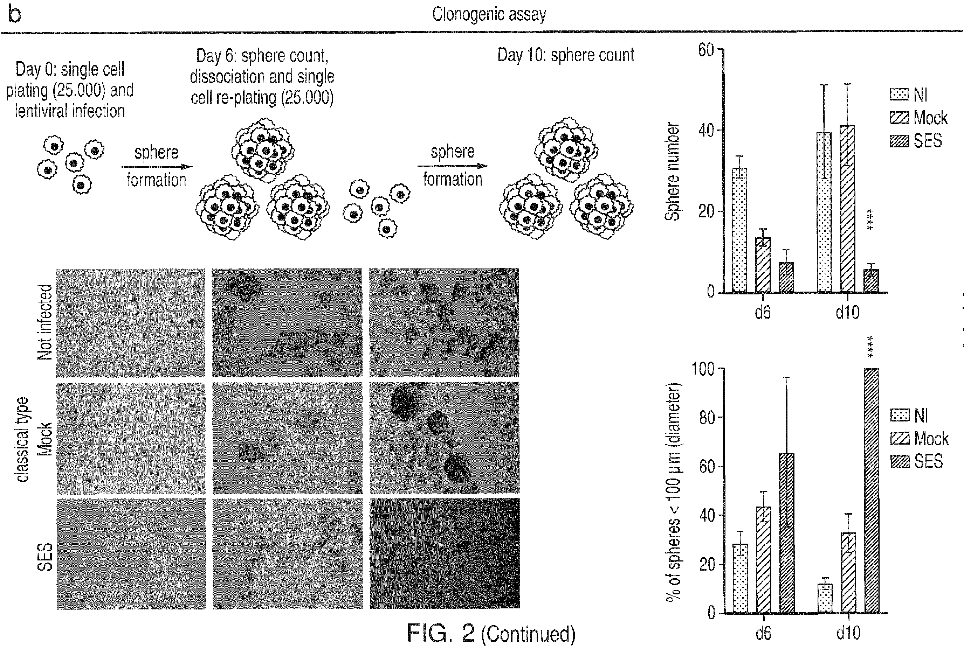

**FIGURE 2****. Efficacy of SOX2 epigenetic silencer on patient derived cancer stem cells in vitro.**
   (a) Microphotographs, growth curve and percentage of death cells of patient derived stem cells (CSCs) of classical (left) and mesenchymal (right) GBM subtype infected with either GFP (both subtypes), SES (both) or binding defective SES(R74P-L97P) (Classical subtype only). ****p < 0.0001; ***p < 0.001; statistically compared with two-way ANOVA. CSCs (classical type) either not infected or infected with lentivirus carrying GFP or SES were assayed for their clonogenic potential using sphere number and dimension (percentage of spheres below 100 um in diameter) at the indicated time points as parameters. ****p < 0.0001; statistically compared with two-way ANOVA.

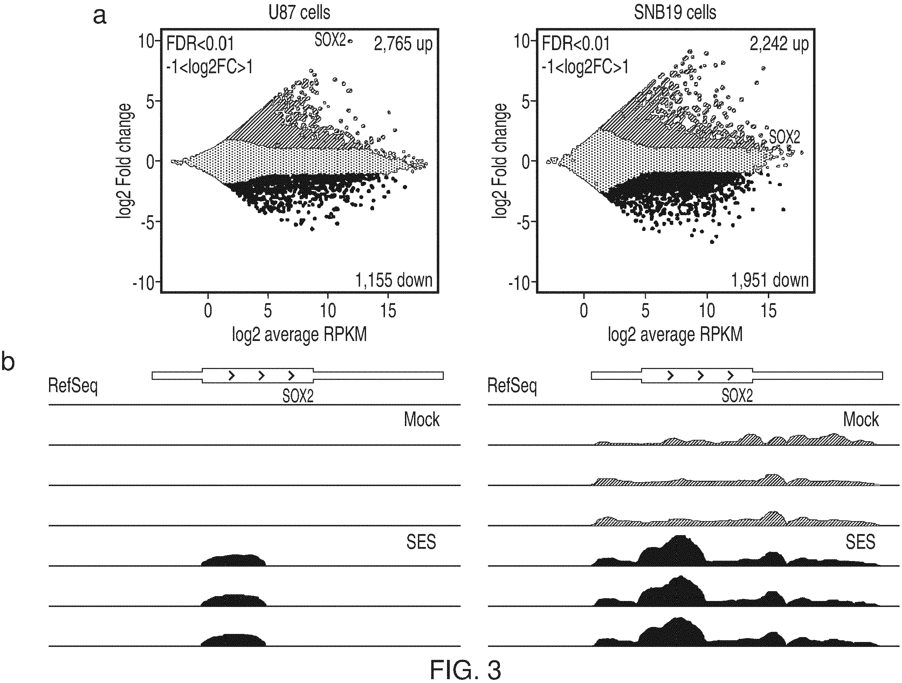

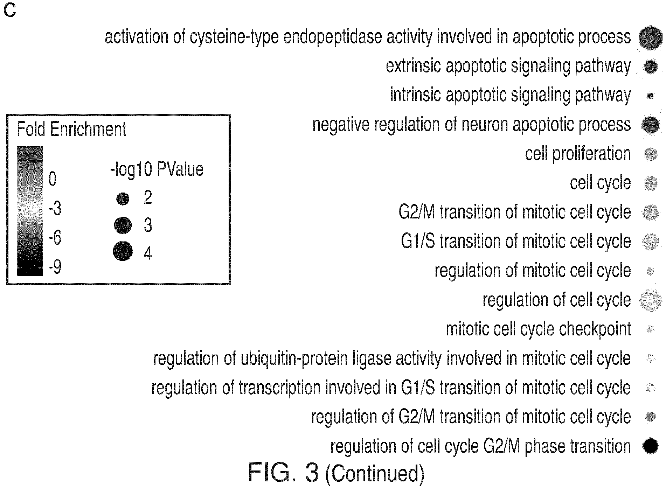

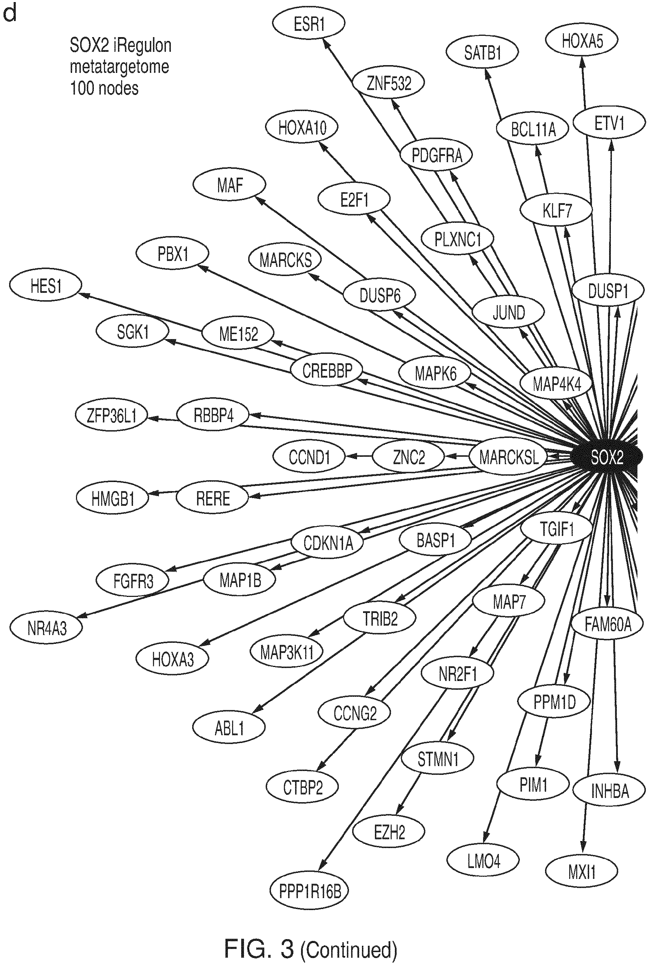

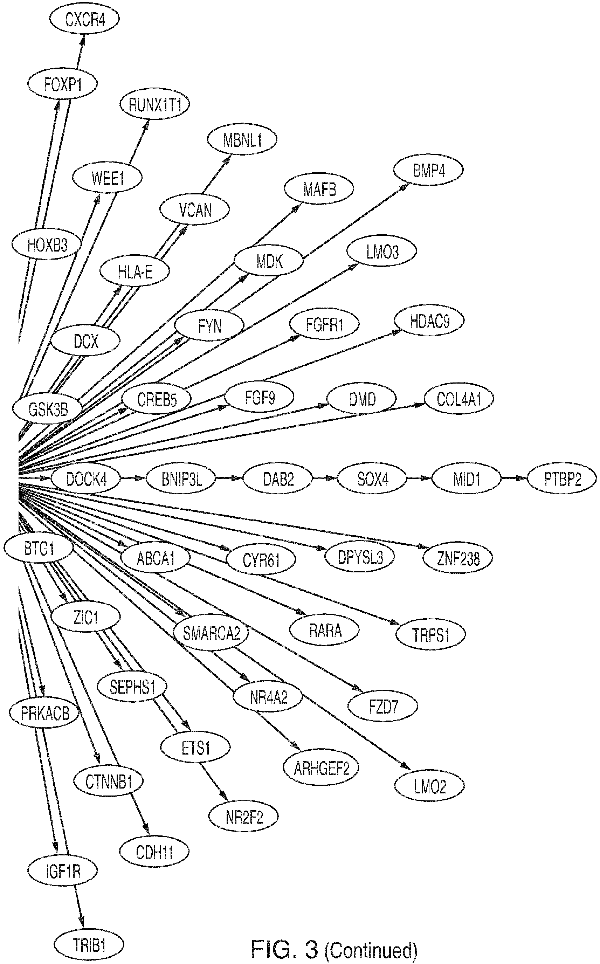

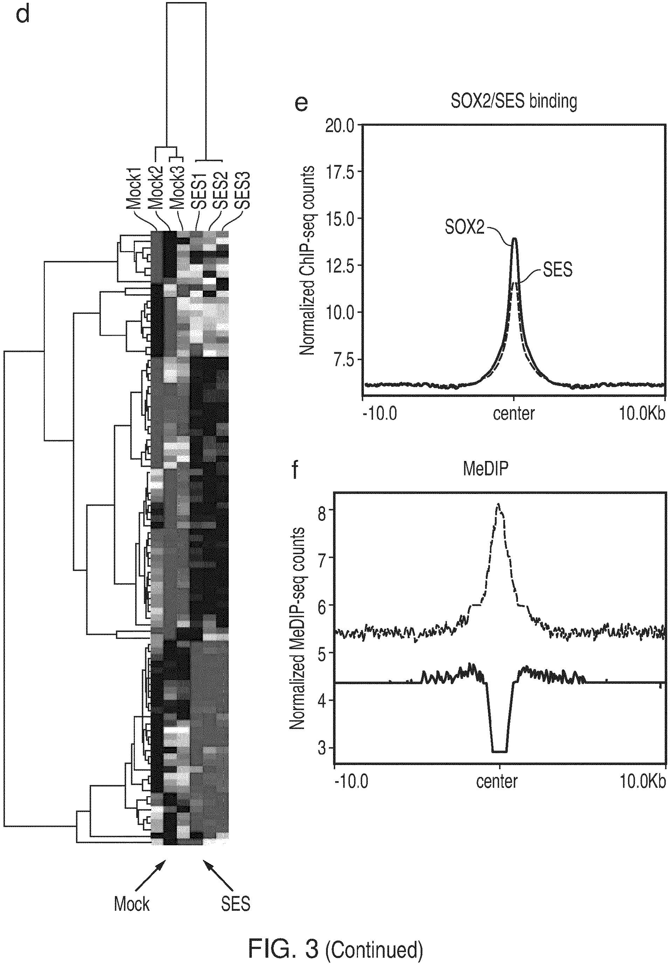

**FIGURE 3****. Molecular consequences of SES.**
   (a) SES caused massive gene deregulation in both U87 and SNB19 cells as assessed by RNA-seq. (b) IGV snapshots of RNAseq tracks within Sox2 locus in both cell lines in each condition (Mock and SES infected) indicate the overexpression of the initial part of SOX2 (included in SES construct, see Fig 1a) both in SOX2 negative (U87) and SOX2 positive (SNB19) cell lines. (c) Gene Ontology analysis indicated that gene associated with apoptosis (upregulation) and cell cycle regulation (downregulation) were impaired by SES expression. (d) Genes predicted to be regulated by SOX2 were affected, with the majority being downregulated. (e) Density plot of ChIP-seq normalized signals (SOX2 and SES) on the SOX2 peaks indicated that SES binds the same regions at genome wide level. (f) Density plot of MeDIP-seq normalized signals (Mock and SES) on the SOX2 peaks indicated that SES is able to increase DNA methylation levels.

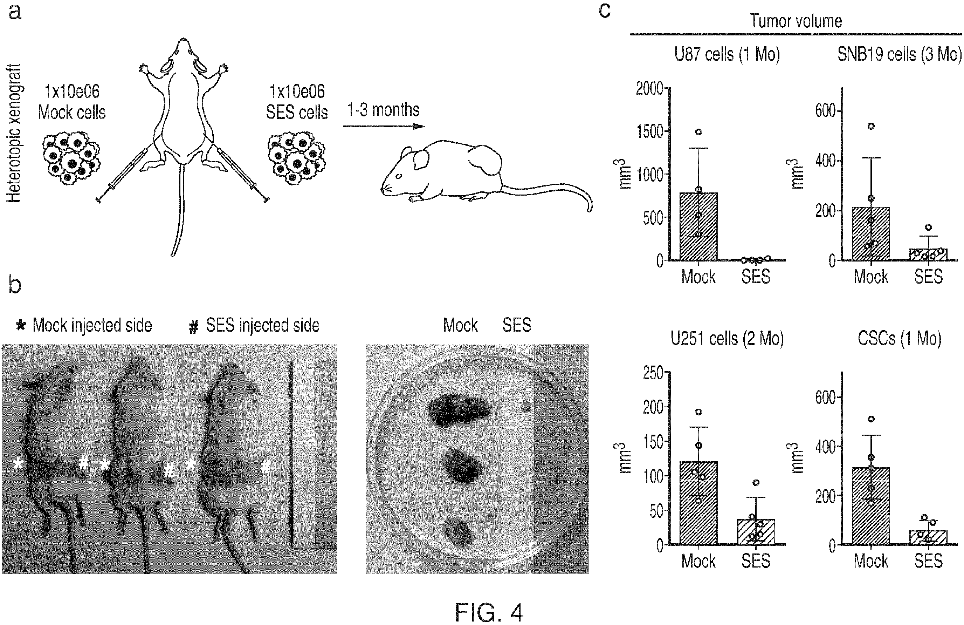

**FIGURE 4****. SES functionality *in vivo:* heterotopic xenograft.**
   (a) Heterotopic xenograft by subcutaneous injection of 1 million of GBM cells, pre-infected with either mock (GFP) or SES in NSG mice. (b) After 4 weeks from the injection, mock U87 cells always generated huge masses while in only one case we retrieved one small nodule from SES infected cells. (c) Evaluation of volume of tumors generated using the indicated cells after the indicated time windows (Mo = months).

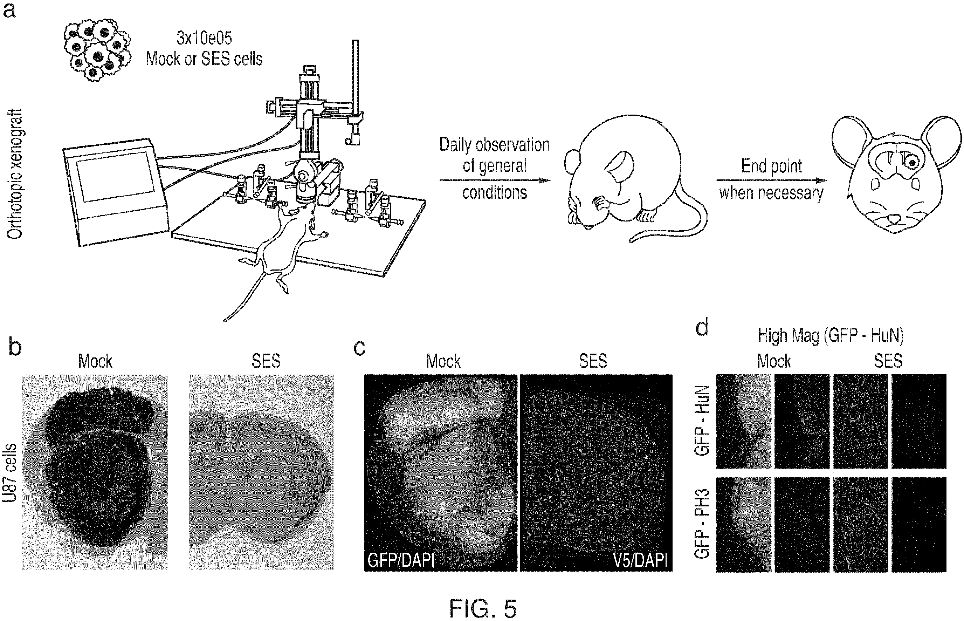

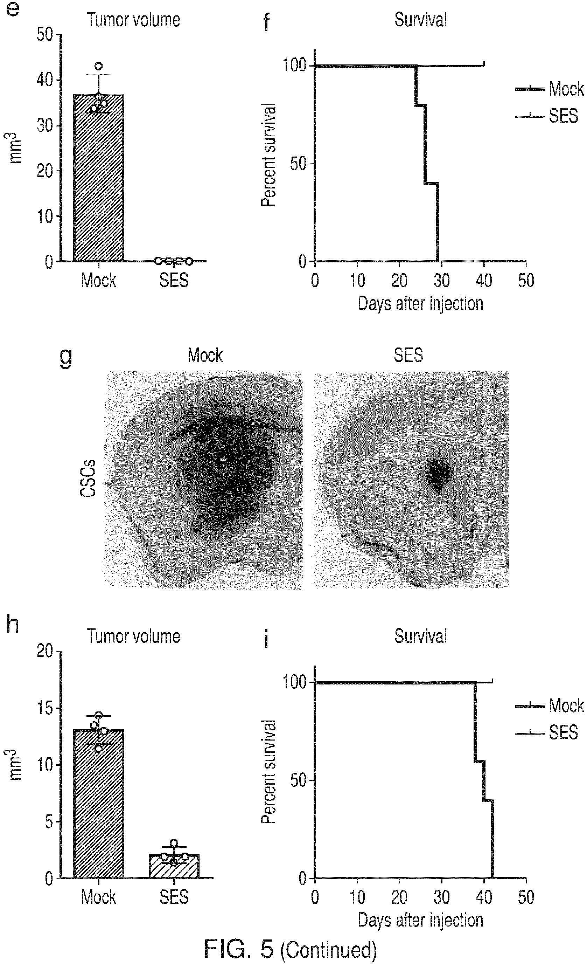

**FIGURE 5****. SES functionality *in vivo:* orthotopic xenograft.**
   (a) Orthotopic xenograft by injection in the striatum of 300000 cells, pre-infected with either mock (GFP) or SES in the brain of NSG mice. (b-c) After 25-30 days from the injection, mock U87 cells always generated huge GFP positive tumors that were also able to invade the cortex, while we never detected any tumors in brains transplanted with SES U87 cells by either Nissl histological staining (b) or V5 antibody (c). (d) Tumors arising from mock cells were formed by human nuclei (HuN) positive, proliferating (PH3 positive) cells while virtually no human cells were retrieved in brain injected with SES cells. Of note, the few PH3 positive cells present, were located at the level of the lateral ventricles, being presumably mouse neural precursors in active division. (e) Estimation of tumor volume. (f) Kaplan-Meier curve shows that mice injected with mock cells died within a month from the surgery while SES receiving animals were in good health and the time of sacrifice (n = 5 animals per group). (g) Xenografts of mock infected CSCs gave rise huge tumors while mice injected with SES CSCs displayed small tumors after 6 weeks from the surgery. (h) Estimation of tumor volume. (i) Kaplan-Meier curve shows that mice injected with mock cells died within 6 weeks from the surgery while SES receiving animals were better condition at the time of sacrifice (n = 5 animals per group).

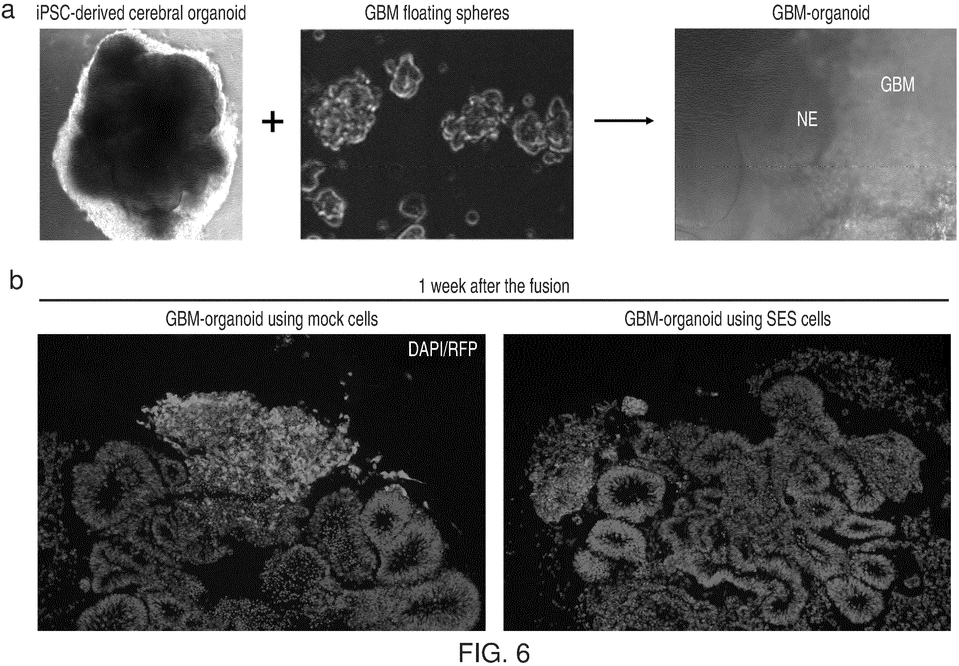

**FIGURE 6****. GBM-cortex organoids.**
   (a) Early patterned cortical organoids were seeded with GBM floating spheres (labelled with RFP) to obtain a fusion. (b) Fused GBM-cortical organoids using either mock or SES-infected spheres (1 week after seeding) were fixed, cut and stained. Notably SES limits the growth as well as the infiltration of GBM cells (RFP positive) in normal cortical parenchyma (DAPI only staining).

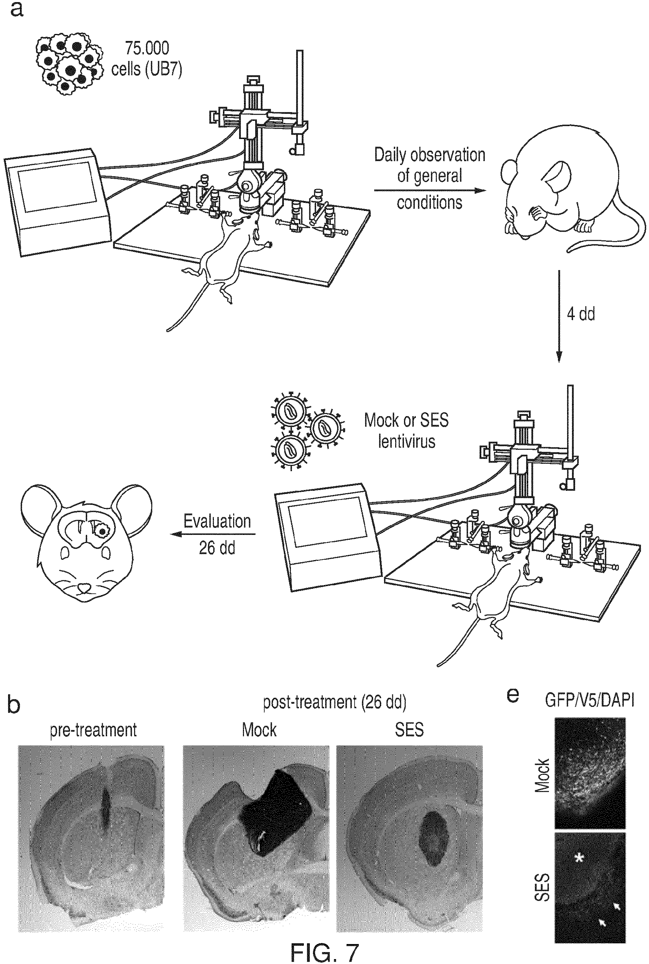

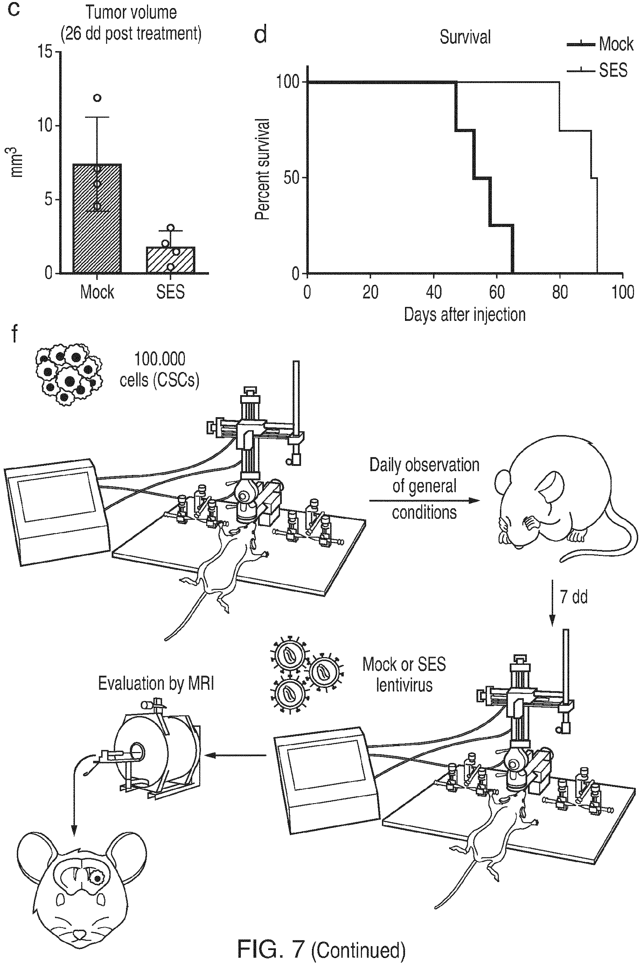

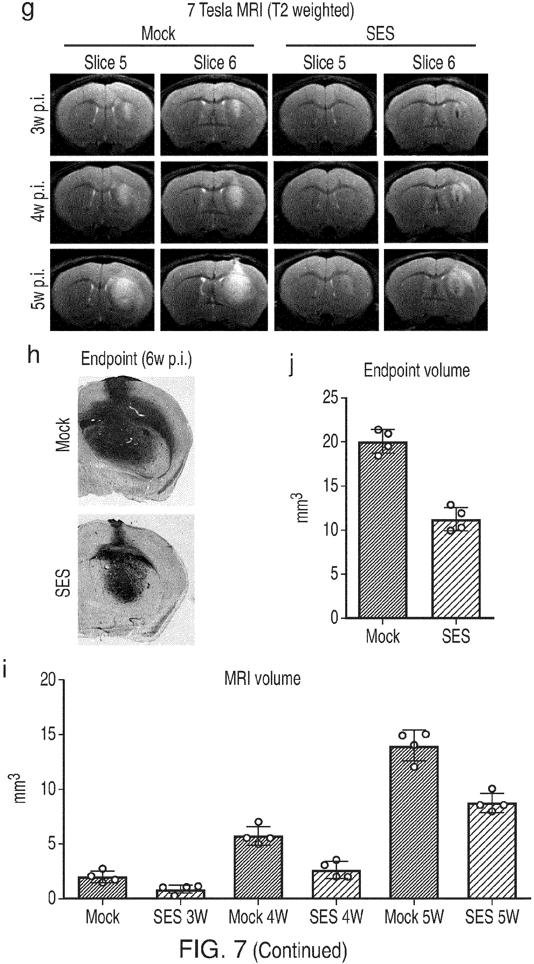

**FIGURE 7****. SES functionality *in vivo:* treatment of preformed orthotopic xenograft.**
   (a) The orthotopic xenograft was generated by injection of 75000 naïve U87 cells in the striatum of NSG mice; after 4 days the animals were reoperated to inject lentivirus carrying either mock (GFP) or SES and evaluated 26 days after (30 days in total). (b) Histological staining at the time of the lentiviral injection (pre-treatment) and at the end of the protocol (post-treatment) showed that the growth of the tumor is limited in SES treated animals. (c) Estimation of tumor volume after 26 days from the lentiviral injection (treatment). (d) Kaplan-Meier curve shows that mice which tumors were injected with mock virus died within two months from the injection of the cells while all but one SES receiving animals reached 3 months (n = 4 animals per group). (e) Immunohistochemistry indicated that the resulting tumors were GFP positive in the case of the mock treatment, and were V5 negative in the case of the SES injections (asterisk) suggesting a negative selection effect for those tumor cells that were infected by the SES virus. Of note V5 labelling (arrowheads) were present in the parenchyma of the mouse brain surrounding the tumor. (f) The orthotopic xenograft was generated by injection of 100000 naïve CSCs in the striatum of NSG mice; after 7 days the animals were reoperated to inject lentivirus carrying either mock (GFP) or SES and evaluated by MRI scanning and sacrificed 6 weeks after the treatment (7 weeks from the grafting of cells). (g) Examples of MRI scanning 3, 4 and 5 weeks post infection (p.i.) (two slices for both one mock and one SES treated mouse). (h) Histological staining at the end of the protocol (endpoint) showed that the growth of the tumor is limited in SES-treated animals. (i) Evaluation of the tumor volume as measured by hyper-intensity of T2-weighted imaging (MIPAV software). (j) Estimation of tumor volume by histological measurements at the endpoint and thus after 6 weeks from the lentiviral injection (treatment), 7 from the CSC injection.

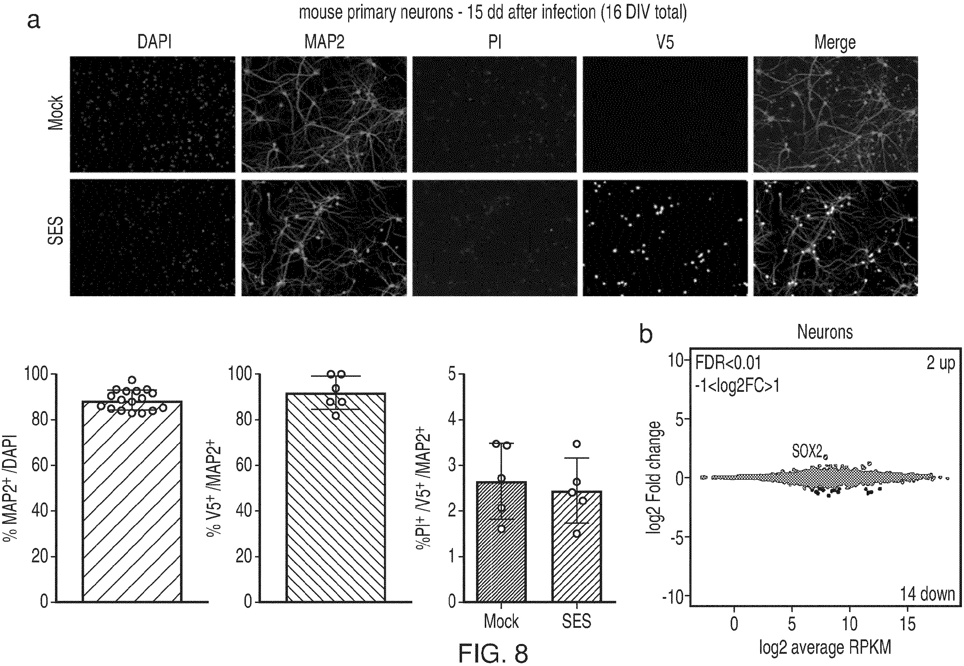

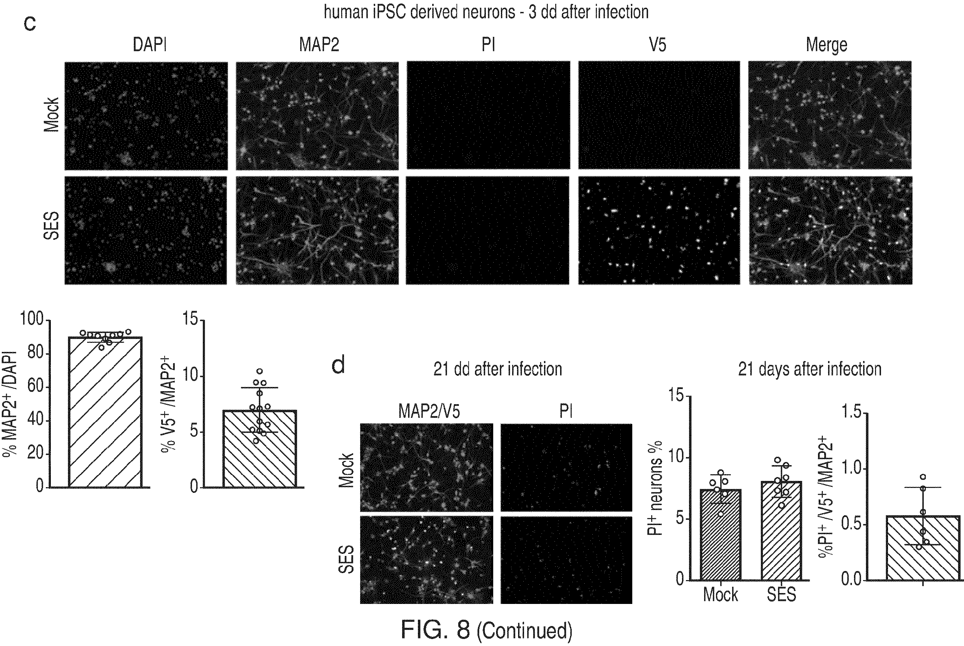

**FIGURE 8****. SES effect on cultured neurons.**
   (a) Infection and death evaluation of primary murine hippocampal neurons infected with either mock or SES indicated no neurodegeneration induced by SES. (b) SES caused only marginal gene deregulation in mouse primary neurons as assessed by RNA-seq. (c) Human iPSC-derived neurons were infected with either mock or SES. (d) The evaluation of neuronal loss through the staining for PI, V5 and MAP2 indicated that the presence of SES was not increasing the neuronal death at least at 21 days after the infection.

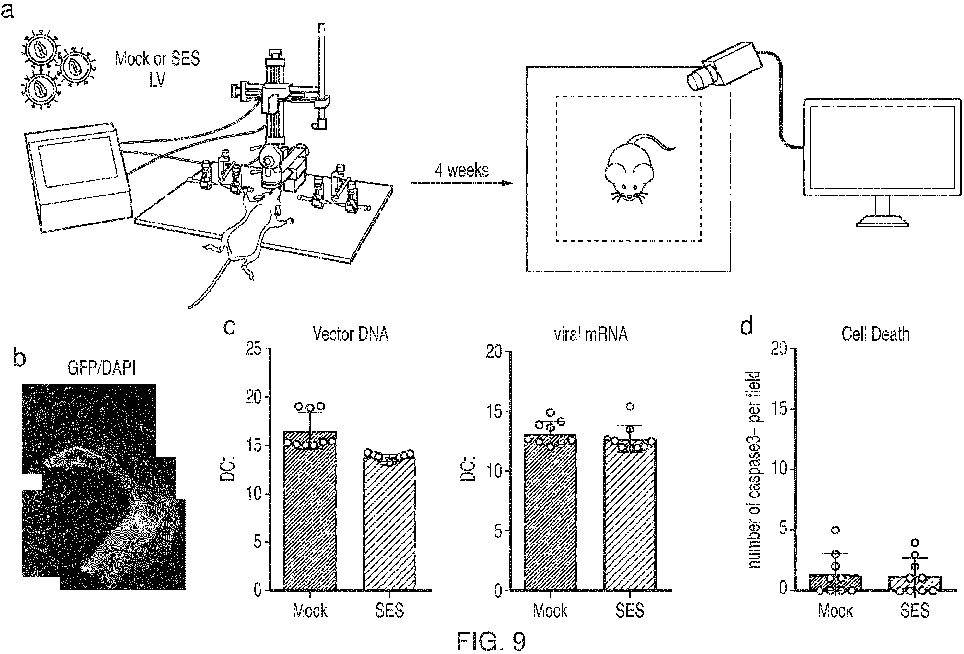

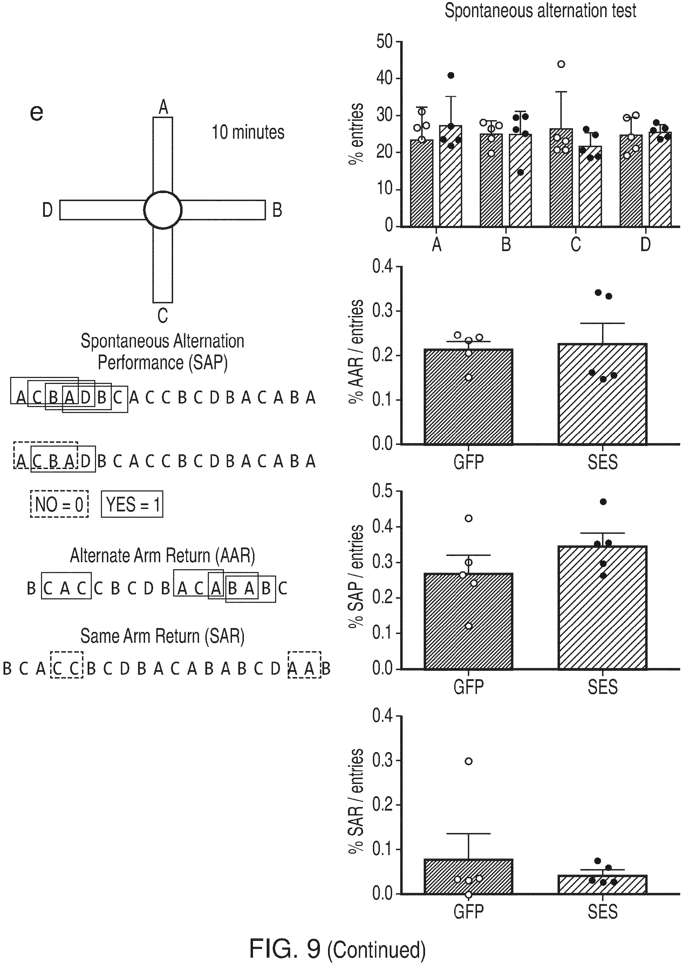

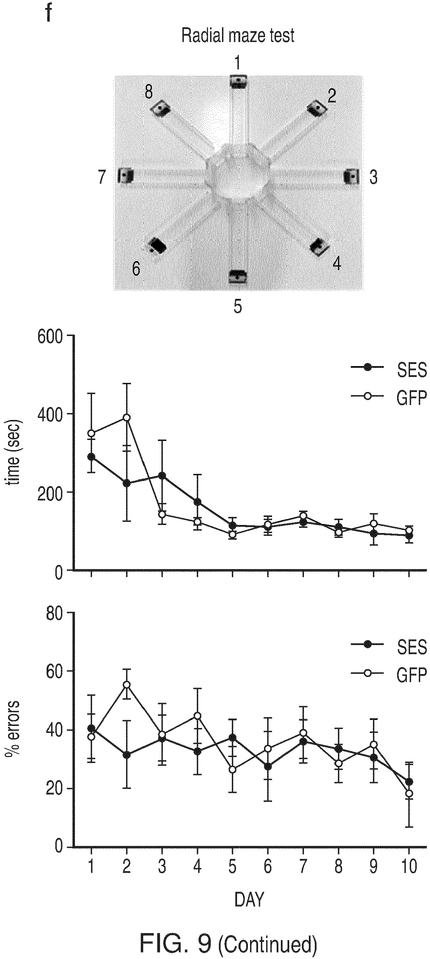

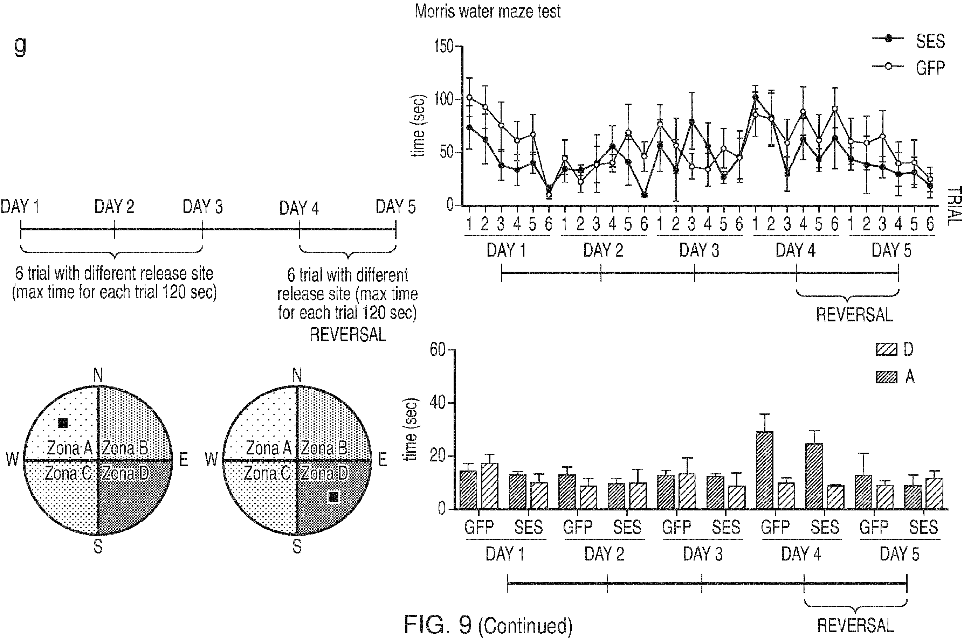

**FIGURE 9****. SES effect on normal mouse brain.**
   (a) Mock or SES lentiviral injections in the hippocampi of WT c57bl/6 mice. (b) Example of viral transduction in the murine hippocampus, using GFP as reporter, after 4 weeks from the injection. (c) Quantification of both viral genome and mRNA of exogenous transgenes in infected hippocampi by qPCR show no difference between the conditions. (d) Quantification of cleaved Caspase 3 positive cells within infected hippocampi shows no difference between the conditions indicating that SES was not toxic for murine neural cells in this setting. (e) Spontaneous alternation test suggested no difference between mock and SES injected mice as assessed by: the percentage of the entries in the different arms, and both the percentage of spontaneous alternation performance (SAP), the percentage of alternate arm return (ARR) and the percentage of same arm return (SAR) on the total entries. Statistically compared with Mann-Whitney test. (f) Radial maze test indicated no difference in the time to accomplish the task or particular tendency of SES treated animals in committing errors during the entire protocol of the test compared to the mock injected animals. Statistically compared with two-way ANOVA. (g) Morris water maze test. On the left the protocol we used and the scheme of the platform (black square) positioning. On the right, the plot of the time used to accomplish the task (up) and the quantification of the time spent in the platform area or in the opposite one (bottom) indicate no differences between conditions. Statistically compared with Mann-Whitney test.

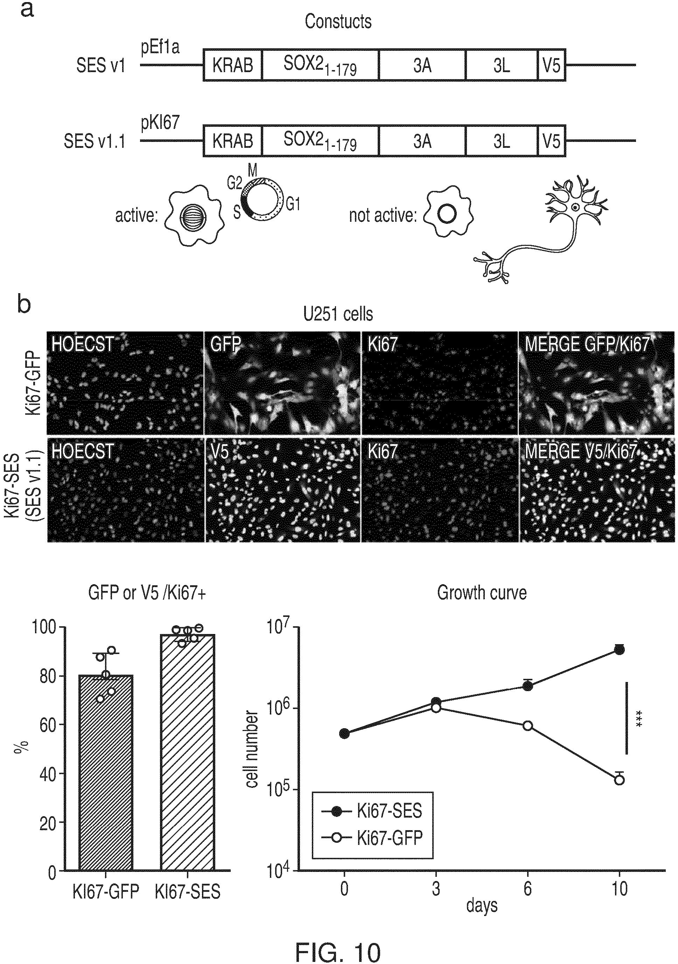

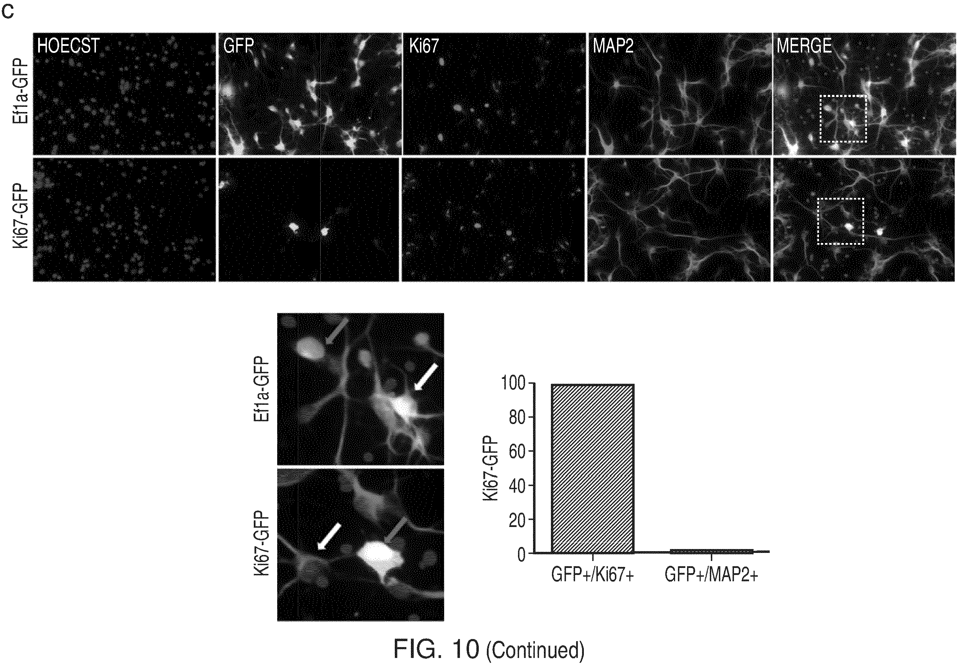

**FIGURE 10****. Vector amelioration.**
   (a) Scheme depicts the original SES (v1) and the further version (v1.1) carrying a different promoter (KI67 promoter expressed in proliferating cells, v1.1). (b) The SES v1.1 test in U251 cells indicated that the KI67 promoter guides the expression of a transgene (either GFP or SES) in a very high percentage of KI67 positive proliferating cells. The SES v1.1 effect on the growth was similar compared to the original version. (c) Both a constitutive GFP and pKI67-GFP were used on mouse primary cortical cultures that contain mainly post-mitotic neurons but also glial types, both proliferating and post-mitotic. Immunofluorescence with the indicated antibodies, indicated that GFP when guided by pKI67 was found only in KI67⁺ proliferating cells (red arrows and quantification) while never in MAP2⁺ neurons (white arrows, and quantification). Constitutive GFP was observed in virtually all cells as expected.

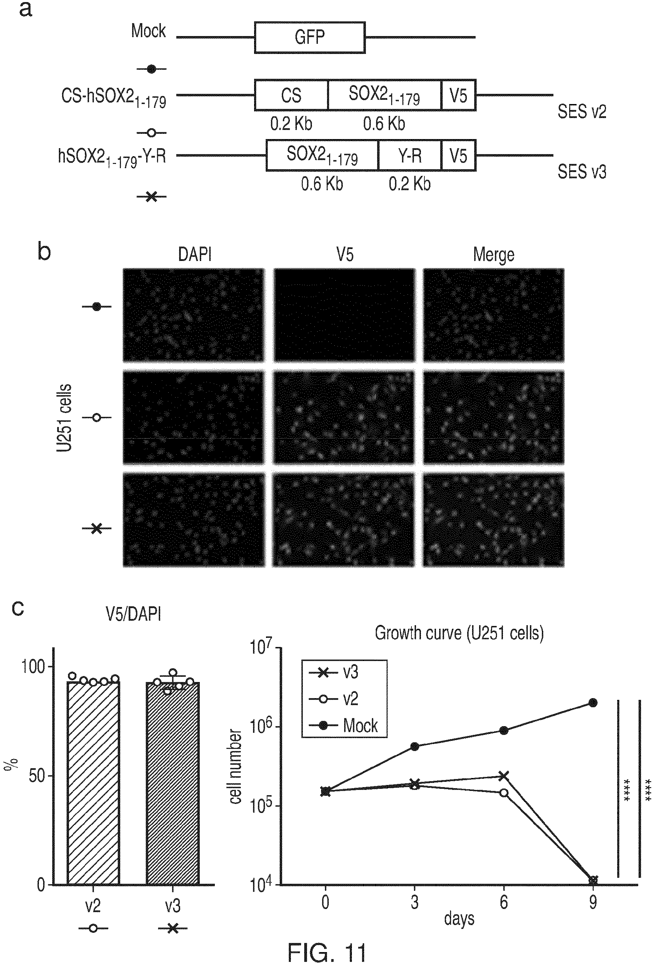

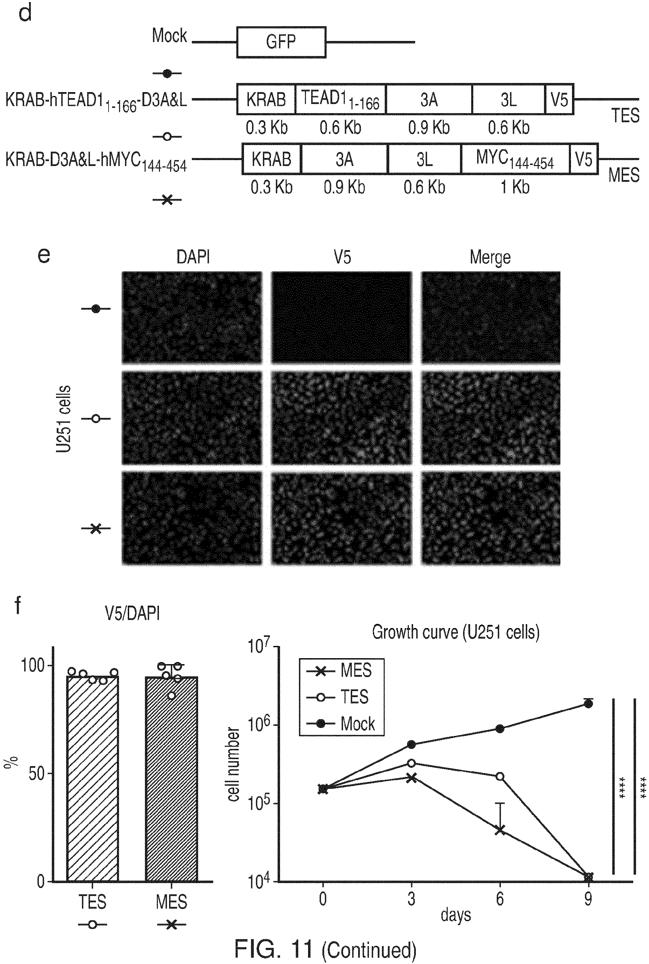

**FIGURE 11****. Generation of other ESFs.**
   (a) Construct generated based on human SOX2 transcription factor fused with the epigenetic domain chromo shadow (CS) and V5 was named SES v2. Construct generated based on human SOX2 transcription factor fused with the epigenetic domain YAF2-RYBP (Y-R) and V5 was named SES v3. (b) Infection efficiency of lentivirus carrying the indicated constructs (see a panel) in U251 cells. (c) Growth curve of U251 cells infected with the indicated constructs show that SESv2 and v3 were able to kill the cells after 9 days in culture, ****p < 0.0001; statistically compared with two-way ANOVA. (d) Construct generated based on human TEAD1 transcription factor and the epigenetic domain KRAB, DNMT3a (3A), DNMT3L (3L) and V5 was named TES, while the construct generated based on human MYC transcription factor and the epigenetic domain KRAB, DNMT3a (3A), DNMT3L (3L) and V5 was named MES. (e) Infection efficiency of lentivirus carrying the indicated constructs (see a panel, right) in U251 cells. (e) Growth curve of U251 cells infected with the indicated constructs show that MES was able to kill the cells after 9 days in culture, ****p < 0.0001.
**FIGURE 12****. Efficacy of SESv3 on patient-derived cancer stem cells *in vitro***
   (a) Microphotographs and (b) growth curve of patient-derived stem cells (CSCs) of classical GBM subtype infected with either GFP or SESv3. ****p < 0.0001; ***p < 0.001; statistically compared with two-way ANOVA.
**FIGURE 13****. Efficacy of TES and MES on patient-derived cancer stem cells in vitro**
   (a-c) Microphotographs (a), growth curve and percentage of death cells (b-c) of patient-derived stem cells (CSCs) of classical (left) and mesenchymal (right) GBM subtype either not infected (NI) or infected with GFP or TES. *p < 0.05; ***p < 0.001; ****p < 0.0001; ns = not significant; statistically compared with two-way ANOVA. (d-e) Growth curve and percentage of death cells of patient-derived stem cells (CSCs) of classical (left, d) and mesenchymal (right, e) GBM subtype either not infected (NI) or infected with GFP or TES. *p < 0.05; ***p < 0.001; ****p < 0.0001; ns = not significant; statistically compared with two-way ANOVA.

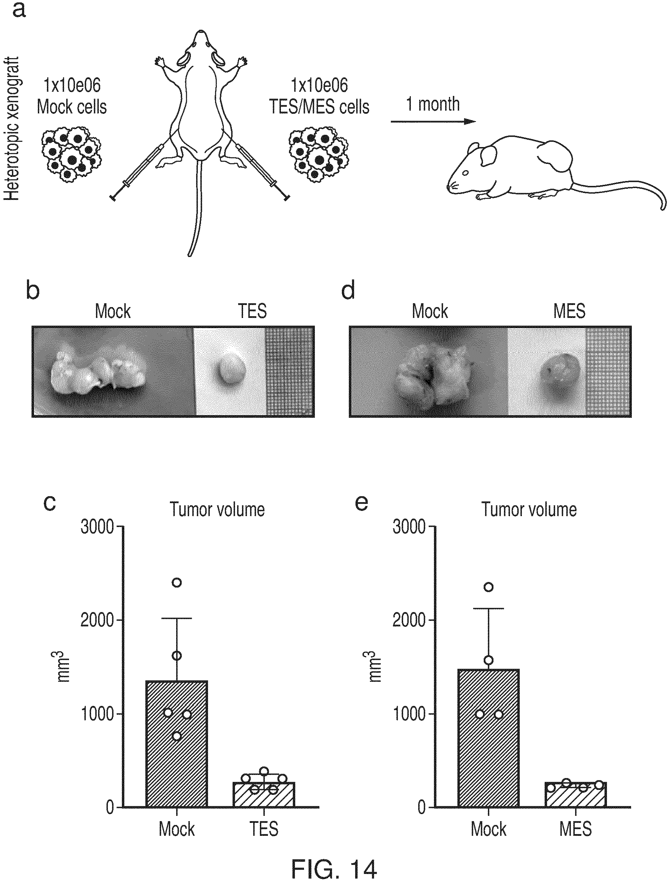

**FIGURE 14****. TES/MES functionality *in vivo:* heterotopic xenograft**
   (a) Heterotopic xenograft by subcutaneous injection of 3,000,000 of classical CSCs, pre-infected with either mock (GFP) or TES or MES in NSG mice. After 4 weeks from the injection, mock CSCs always generated huge masses while smaller tumors emerged from TES (b-c) or MES infected cells (d-e).

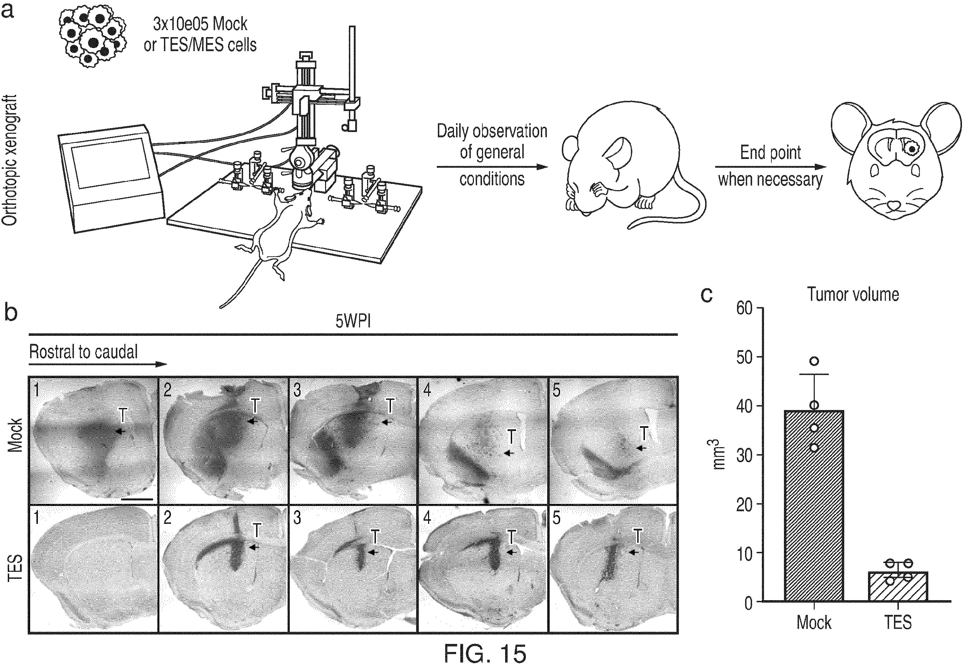

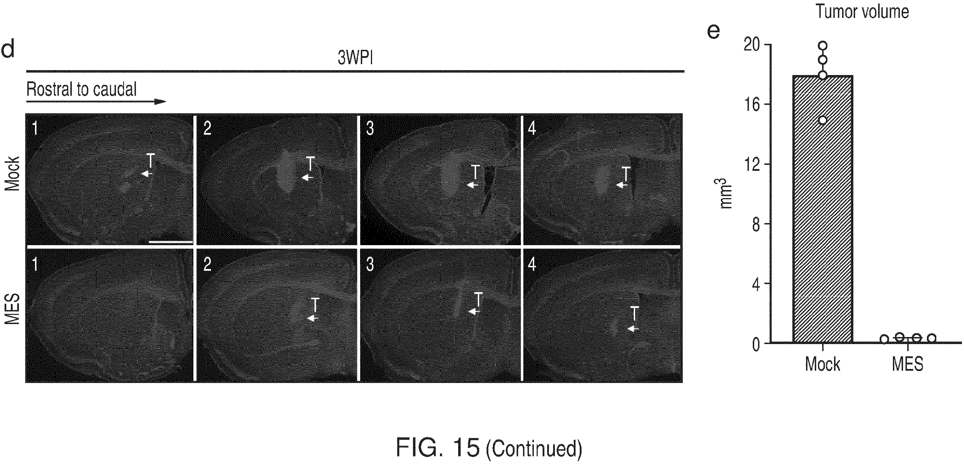

**FIGURE 15****. TES/MES functionality *in vivo:* orthotopic xenograft**
   (a) Orthotopic xenograft by injection in the striatum of 300.000 classical CSCs, pre-infected with either mock (GFP) or TES or MES in brain of NSG mice. (b) After 5 weeks from the injection (WPI), mock CSCs always generated huge tumors able to invade also the cortex while TES cells formed smaller tumors in brains. Evaluation by Nissl histological staining. (c) Estimation of tumor volume (n = 4 animals per group). (d) After 3 weeks from the injection (WPI), mock CSCs already showed important tumor masses while MES cells formed smaller tumors. Evaluation by DAPI staining. (c) Estimation of tumor volume (n = 4 animals per group).

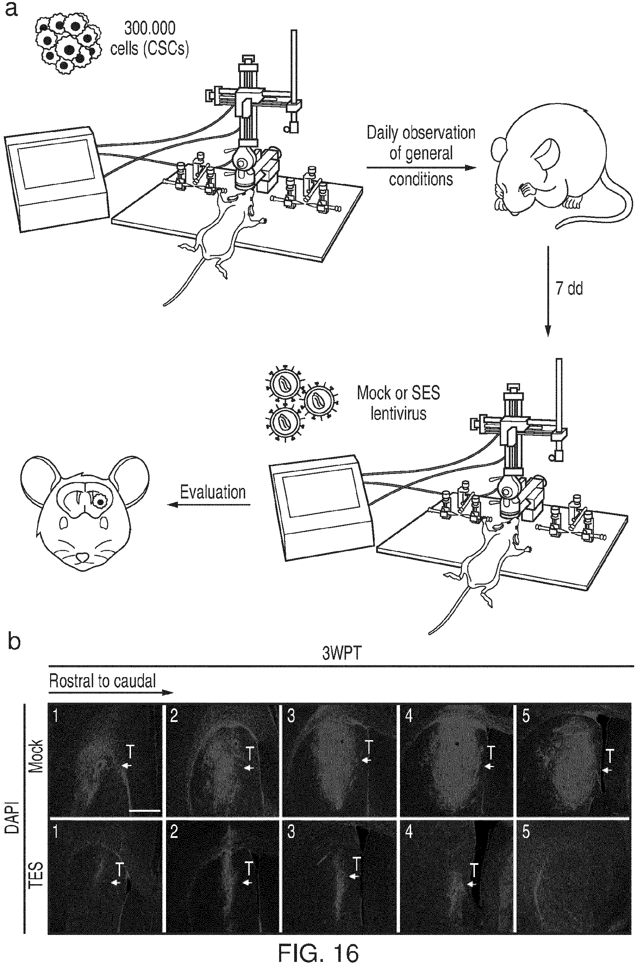

**FIGURE 16****. TES functionality *in vivo:* treatment of preformed orthotopic xenograft**
   (a) The orthotopic xenograft was generated by injection of 300.000 classical CSCs in the striatum of NSG mice; after 7 days the animals were re-operated to inject lentivirus carrying either mock (GFP) or TES and evaluated 3 weeks after (WPT) (4 weeks in total). (b) DAPI staining at the end of the protocol (post-treatment) showed that the growth of the tumor is limited to the site of injection in TES treated animals while huge masses are present in mock treated mice. (c) Estimation of tumor volume after 3 weeks from the lentiviral injection (treatment) (n = 3 animals per group).

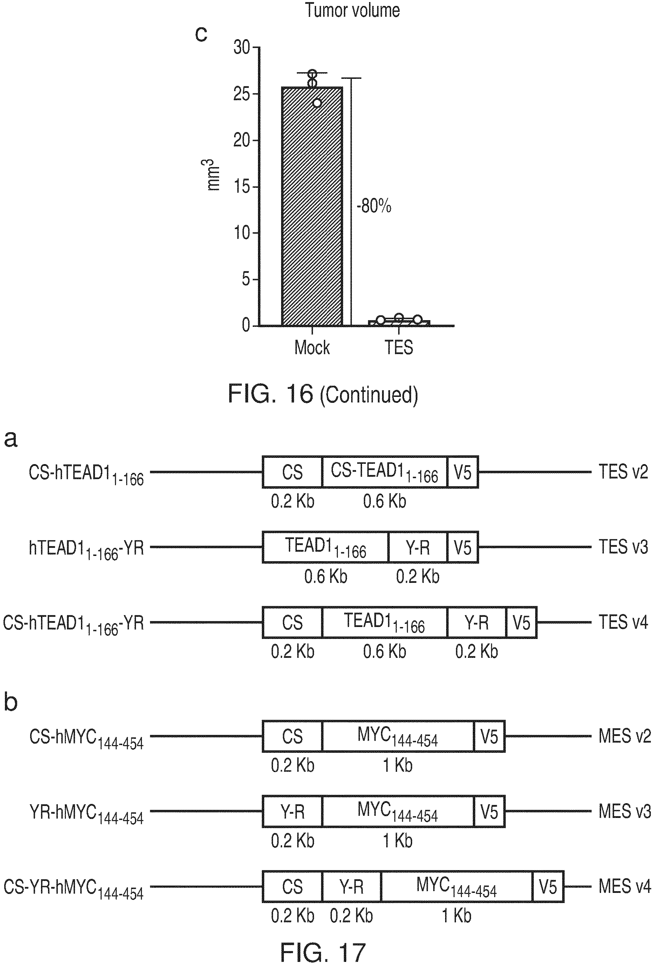

**FIGURE 17****. Epigenetic silencer factor constructs.**
   Schematic representation of ESF constructs. (a) Constructs generated based on human TEAD1 transcription factor and the epigenetic repressor domains Chromo Shadow (CS) (from the gene *CBX5*) and YAF2-RYBP (YR) (from the gene *RYBP*)*,* each variant has the V5 tag at the C-terminus. (b) Constructs generated based on human MYC transcription factor and the epigenetic repressor domains Chromo Shadow (CS) (from the gene *CBX5*) and YAF2-RYBP (YR) (from the gene *RYBP*)*.* Each variant has a 3' V5 tag. Subscript notation denotes the amino acid ranges of each of the protein domains encoded by the construct.

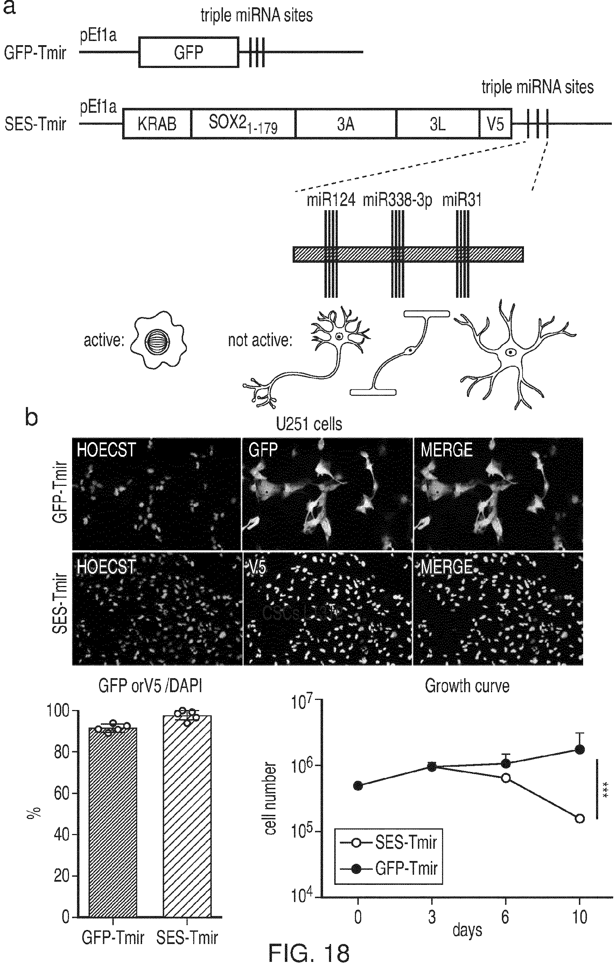

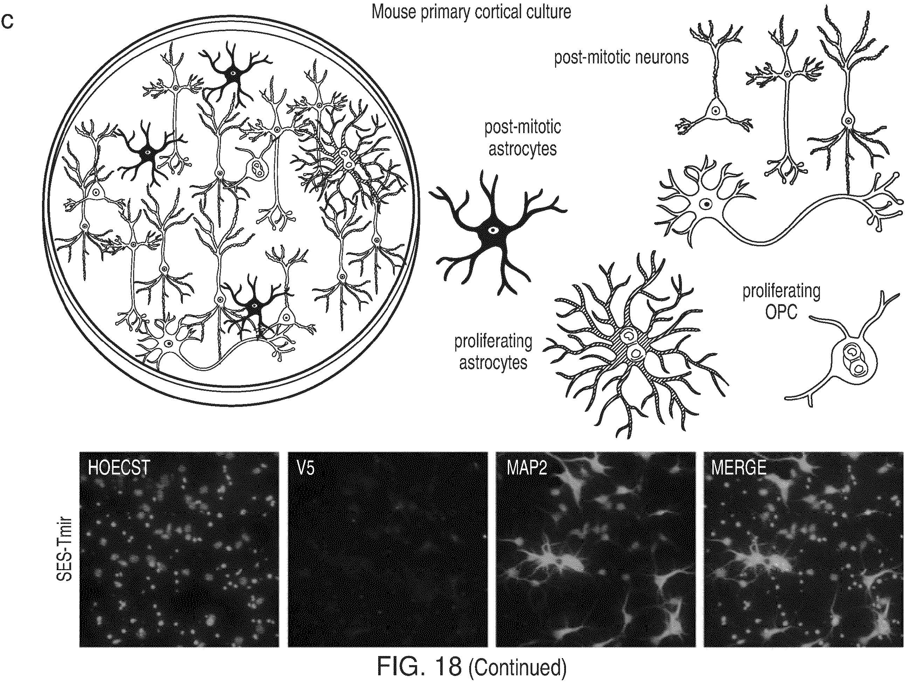

**FIGURE 18****. Enhancing ESF specificity using miRNA-based detargeting silencer factor constructs.**
   (a) Schemeatic depiction of both the control and SES vector containing a Tmir cassette (four copies each of miRNA target sequences for miR124, miR338-3p, and miR31) within the 3' UTR. The Tmir cassette allows the expression of the transgene in cancer cells but not in the brain cells (neurons, oligodendrocytes, and astrocytes). (b) Both GFP-Tmir and SES-Tmir are expressed in U251 GBM cancer cell line as indicated by GFP/V5 expression counterstained with Hoecst. SES-Tmir is able to reduce the proliferation of cancer cell *in vitro.* (c) SES-Tmir is not expressed (detargeted) in mouse primary cortical cultures that contain post-mitotic neurons, post-mitotic and proliferating astrocytes as well as proliferating Oligo Precursor Cells (OPCs).

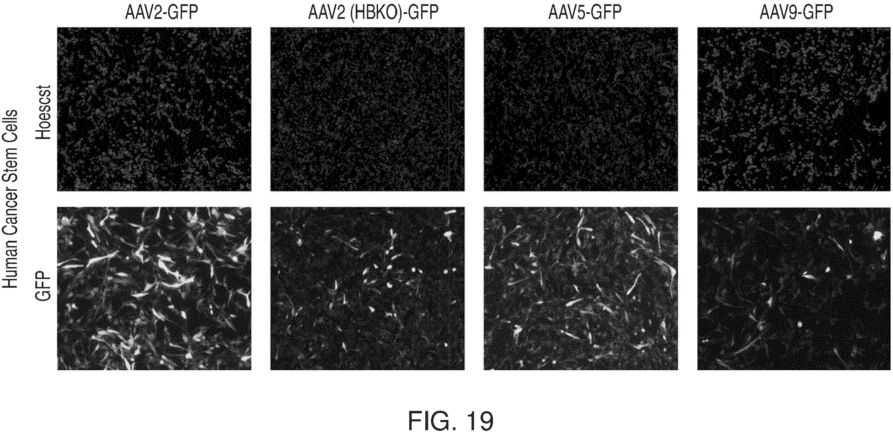

**FIGURE 19****. AAV functionality on patient derived Cancer Stem Cells.**
   Recombinant AAV serotypes containing an Ef1a::GFP construct were used to infect patient derived GBM cancer stem cells *in vitro.* Four days after the infection, cells were fixed and immunofluorescence for GFP performed, and cells were stained with the nuclear marker Hoecst.

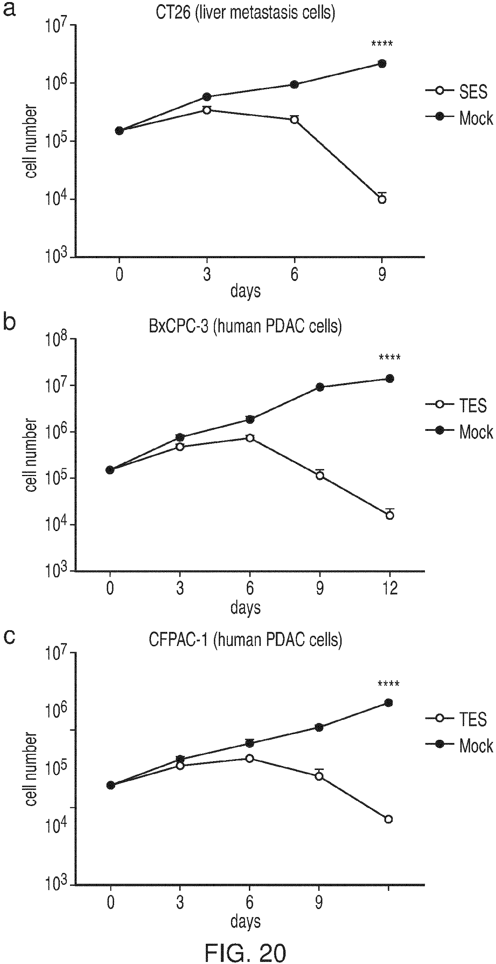

**FIGURE 20****. ESF efficacy in different cancer cell types.**
   (a) Growth curve of CT26 cells, which are derived from liver metastasis of colon adenocarcinoma in mice, treated with lentivirus carrying either GFP or SES (Sox2 Epigenetic Silencer). ****p < 0.0001. (b) Growth curve of BxPC-3 cells, human Pancreatic Ductal Adeno Carcinoma cells, treated with lentivirus carrying either GFP or TES (Tead Epigenetic Silencer). ****p < 0.0001. (c) Growth curve of CFPAC-1 cells, human Pancreatic Ductal Adeno Carcinoma cells, treated with lentivirus carrying either GFP or TES (Tead Epigenetic Silencer). ****p < 0.0001.

### DETAILED DESCRIPTION OF THE INVENTION

The terms "comprising", "comprises" and "comprised of" as used herein are synonymous with "including" or "includes"; or "containing" or "contains", and are inclusive or open-ended and do not exclude additional, non-recited members, elements or steps. The terms "comprising", "comprises" and "comprised of" also include the term "consisting of'.

It will be understood that when referring to a protein or polypeptide herein, the same may equally be applied to a polynucleotide encoding the same, and, where relevant (i.e., when referring to a coding sequence within a polynucleotide) *vice versa.*

It will be understood that any of the following aspects of the invention may be suitably combined in the practice of the invention herein. For example, the miRNA transgene cassettes according to the invention may be used in conjunction with the ESFs according to another aspect of the invention, or may be used independently to deliver any suitable transgene.

### Polynucleotides and transgene cassettes

### Triplet miRNA cassettes

Herein, the inventors provide a transgene expression cassette that comprises target sequences that are recognized by micro RNAs (miRNAs), in order to regulate transgene expression. Said expression cassettes allow the expression of a transgene to be regulated such that unwanted expression is reduced or eliminated in cell types comprising the miRNA, thereby improving safety and reducing off target effects.

In one aspect, there is provided a polynucleotide comprising at least one miR-124 target sequence, at least one miR-338-3p target sequence and at least one miR-31 target sequence, wherein the miRNA target sequences are operably linked to a transgene.

In one embodiment, the number of copies of each of the miRNA target sequences is independently selected from the group consisting of: one, two, three, and four.

In one embodiment, the number of copies of each of the miRNA target sequences is one.

In one embodiment, the number of copies of each of the miRNA target sequences is two.

In one embodiment, the number of copies of each of the miRNA target sequences is three.

In one embodiment, the number of copies of each of the miRNA target sequences is four.

In one embodiment, the number of copies of each of the miRNA target sequences is greater than four, such as five, six, seven, eight, nine, or ten.

A suitable miRNA target sequence for miR-124 is:
attgccttatttc [SEQ ID NO: 1]

In one embodiment, the miRNA target sequence comprises the sequence of SEQ ID NO: 1.

A suitable miRNA target sequence for miR-338-3p is:
caacaaaatcactgatgctgga [SEQ ID NO: 2]

In one embodiment, the miRNA target sequence comprises the sequence of SEQ ID NO: 2.

A suitable miRNA target sequence for miR-31 is:
cagctatgccagcatcttgcc [SEQ ID NO: 3]

In one embodiment, the miRNA target sequence comprises the sequence of SEQ ID NO: 3.

In one embodiment, the miRNA target sequences comprise SEQ ID NOs: 1, 2, and 3.

In one embodiment, the miRNA target sequences are located downstream, i.e., 3', of the transgene.

In one embodiment, the miRNA target sequences are located within the 3'-UTR of the transgene.

In one embodiment, the miRNA target sequences or clusters of copies of sequences are, from 5' to 3', arranged in the order: miR-124, miR-338-3p, and miR-31.

According to the foregoing embodiment, the target sequences, or clusters comprising one or more copy thereof, are arranged from 5' to 3' such that they form groups according to their target specificity, i.e., 5' - [miR-124 target sequence] ₄ - [miR-338-3p target sequence] ₄ - [miR-31 target sequence]₄ - 3'.

Both the individual target sequences and the clusters of sequences may be contiguous with one another, separated by spacer sequences, or any combination thereof.

Thus, in one embodiment, the miRNA target sequences are separated by spacer sequences.

In an embodiment wherein the polynucleotide comprises four target sequences for each of miR-124, miR-338-3p, and miR-31, the polynucleotide may comprise the sequence as set forth in SEQ ID NO: 4.

Triple miRNA Target sequence (Tmir) (SEQ ID NO: 4): 
atattgccttatttccgattattgccttatttcgcattattgccttatttctcactattgccttattt
ccgatcccggggtttaaaccgatcaacaaaatcactgatgctggacgatcaacaaaatcactgatgct
ggacgatcaacaaaatcactgatgctggatcaccaacaaaatcactgatgctggacgatgtttaaacc
ctgcaggcgatcagctatgccagcatcttgcccgatcagctatgccagcatcttgcccgatcagctat
gccagcatcttgcctcaccagctatgccagcatcttgcccgatcctgcaggagatct
miR124 Target sequences
miR338-3p Target sequences
miR31 Target sequences

In one embodiment, the polynucleotide comprises a sequence as set forth in SEQ ID NO: 4.

In one embodiment, the polynucleotide consists of a sequence as set forth in SEQ ID NO: 4.

The polynucleotide and transgene cassette described herein may be used with any transgene, e.g., an ESF, as described herein.

miR-124 may be, for example, also referred to as miRNA-124 or miR124; miR-338-3p, as miR-338-3p, or miRNA338-3p; and miR-31 as miRNA-31 or miR31.

### miRNA cassettes comprising ESFs

miRNA mediated regulation of transgene expression may be exploited for controlling ESFs according to the invention. Thus, in one aspect, there is provided a polynucleotide comprising an epigenetic silencer factor (ESF) sequence operably linked to at least one micro RNA (miRNA) target sequence, wherein the ESF comprises a transcription factor DNA-binding domain operably linked to at least one epigenetic effector domain, and wherein the transcription factor is an oncogenic transcription factor or a cancer-associated transcription factor.

In one embodiment the miRNA target sequence is located downstream, i.e., 3', of the ESF-coding sequence.

In one embodiment, the miRNA target sequence is located within the 3'-UTR of the transgene.

miRNA target sequences may be represented by one of more copies of a sequence of a given identity. The number of copies of each target sequence may be independently selected, i.e., the number of copies of a given sequence is not necessarily dependent on the number of copies of another, different, sequence.

Thus, in one embodiment, the miRNA target sequence(s) comprises more than one copy of said miRNA target sequence.

In one embodiment, the miRNA target sequence(s) comprises one copy of said miRNA target sequence.

In one embodiment, the miRNA target sequence(s) comprises two copies of said miRNA target sequence.

In one embodiment, the miRNA target sequence(s) comprises three copies of said miRNA target sequence.

In one embodiment, the miRNA target sequence(s) comprises four copies of said miRNA target sequence.

In one embodiment, the miRNA target sequence(s) comprises more than four copies, for example five, six, seven, eight, nine, or ten copies of said miRNA target sequence.

The miRNA target sequence may be designed to be recognised by any suitable miRNA or selected from any suitable known miRNA target sequences, e.g., an miRNA that is expressed in a cell type in which transgene expression is not desired.

In one embodiment, the miRNA target sequence is a target for an miRNA selected from the group consisting of miR-124, miR-338-3p, and miR-31.

In one embodiment, the miRNA target sequence is a target site for miR-124.

In one embodiment, the miRNA target sequence is a target site for miR-338-3p.

In one embodiment, the miRNA target sequence is a target site for miR-31.

In one embodiment, the miRNA target sequence(s) comprises a sequence selected from the group consisting of SEQ ID NOs: 1, 2, and 3.

In one embodiment, the miRNA target sequence comprises a sequence according to SEQ ID NO: 1.

In one embodiment, the miRNA target sequence comprises a sequence according to SEQ ID NO: 2.

In one embodiment, the miRNA target sequence comprises a sequence according to SEQ ID NO: 3.

In one embodiment, the miRNA target sequences comprise a target site for miR-124, miR-338-3p, and miR-31.

In one embodiment, the miRNA target sequences comprise SEQ ID NOs: 1, 2, and 3.

In a preferred embodiment, the miRNA target sequences comprise four copies of a target sequence for each of miR-124, miR-338-3p, and miR-31.

The order of the miRNA target sequences may be varied. In one embodiment, the miRNA target sequences or clusters of copies of sequences are, from 5' to 3', arranged in the order: miR-124, miR-338-3p, and miR-31.

In another embodiment, the miRNA target sequences or clusters of copies of sequences are, from 5' to 3', arranged in the order: miR-124, miR-31, and miR-338-3p.

In another embodiment, the miRNA target sequences or clusters of copies of sequences are, from 5' to 3', arranged in the order: miR-338-3p, miR-124, and miR-31.

In another embodiment, the miRNA target sequences or clusters of copies of sequences are, from 5' to 3', arranged in the order: miR-338-3p, miR-31, and miR-124.

In another embodiment, the miRNA target sequences or clusters of copies of sequences are, from 5' to 3', arranged in the order: miR-31, miR-124, and miR-338-3p.

In another embodiment, the miRNA target sequences or clusters of copies of sequences are, from 5' to 3', arranged in the order: miR-31, miR-338-3p, and miR-124.

Both the individual target sequences and the clusters of sequences may be contiguous with one another, separated by spacer sequences, or any combination thereof.

Thus, in one embodiment, the miRNA target sequences are separated by spacer sequences.

In one embodiment, the polynucleotide comprises a sequence according to SEQ ID NO: 4.

### Spacers

As used herein, a "spacer" may be a sequence (e.g. a nucleotide or amino acid sequence) that may be used to separate other sequence elements within a larger polymer.

In one embodiment, one or more spacer sequence separates polynucleotide sequences (e.g. miRNA target sequences).

Individual miRNA target sequences or groups of miRNA target sequences may be separated by one or more spacer sequence. In one embodiment, the miRNA target sequences are separated by one or more spacer sequence. The spacer sequence may comprise, for example, at least one, at least two, at least three, at least four, at least five, at least ten, at least twenty, or at least thirty nucleotide bases.

By way of non-limiting example, in the following Triple miRNA Target sequence, the miRNA target sequences are each separated by sequences which may not be considered part of said miRNA target sequence. Such sequences may be considered to be spacers that separate functional sequence elements.

Triple miRNA Target sequence (Tmir) (SEQ ID NO: 4): 
atattgccttatttccgattattgccttatttcgcattattgccttatttctcactattgccttattt
ccgatcccggggtttaaaccgatcaacaaaatcactgatgctggacgatcaacaaaatcactgatgct
ggacgatcaacaaaatcactgatgctggatcaccaacaaaatcactgatgctggacgatgtttaaacc
ctgcaggcgatcagctatgccagcatcttgcccgatcagctatgccagcatcttgcccgatcagctat
gccagcatcttgcctcaccagctatgccagcatcttgcccgatcctgcaggagatct
 Spacer sequences within SEQ ID NO:4 include: (a) at; (b) cgatt; (c) gcatt; (d) tcact; (e) cgatcccggggtttaaaccgat (SEQ ID NO: 175); (f) cgat; (g) tcac; (h) cgatgtttaaaccctgcaggcgat (SEQ ID NO: 176); (i) cgatcctgcaggagatct (SEQ ID NO: 177).

In one embodiment, the spacer is selected from the group consisting of: (a) - (i).

In one embodiment, the polynucleotide comprises one or more spacer selected from the group consisting of: (a) - (i).

In one embodiment, the spacer sequences separating the clusters of miRNA target sequences are longer than the spacer sequences separating the miRNA target sequences within a cluster.

### Transgenes

The polynucleotide according to the invention may comprise any suitable transgene. A suitable transgene may be operably linked to the miRNA target sequences according to the invention such that the expression of said transgene is dependent upon the presence of the miRNA.

In one embodiment the transgene is an ESF.

### Epigenetic silencer factor (ESF)

The invention provides polynucleotides that encode a polypeptide transcription factor DNA-binding domain operably linked to at least one epigenetic effector domain, preferably wherein the transcription factor is an oncogenic transcription factor or a cancer-associated transcription factor. The polypeptide may be for decreasing transcription and/or expression of one or more target gene. The polypeptide, or transgene encoding said polypeptide, may be referred to as an epigenetic silencer factor (ESF). The polypeptide may be a multimeric polypeptide, for example comprised of two, three or more polypeptide chains. For example, the polypeptide may be a dimer, such as a heterodimer. The polypeptide may be a comprised of a single polypeptide chain. The polypeptide may be a fusion protein.

ESFs are agents that may decrease the transcription and/or expression of one or more target gene (e.g. silence one or more target gene). ESFs of the invention may comprise at least part of a transcription factor that binds to DNA, wherein the part is operably linked to an epigenetic effector domain. The effector domain may have transcriptional repression activity and may enable silencing (e.g. permanent silencing) of one or more target gene of the transcription factor. In particular, when the transcription factor is an oncogenic transcription factor or cancer-associated transcription factor the ESF may block a gene expression cascade involved in tumor growth.

ESFs may be chimeric or fusion proteins that are comprised of a DNA-binding domain operably linked to an effector domain (e.g. a KRAB domain, a Chromo Shadow domain, YAF2-RYBP domain, a DNMT3A domain and/or a DNMT3L domain). The effector domain may harbour a catalytic activity, which represses transcription and/or expression of one or more target gene. Alternatively, or additionally, the effector domain may recruit additional agents within a cell to one or more target gene, which may repress transcription and/or expression of the target gene(s).

By "operably linked", it is to be understood that individual components are linked together in a manner which enables them to carry out their function (e.g. binding to DNA, catalysing a reaction or recruiting additional agents from within a cell) substantially unhindered. For example, a DNA-binding domain may be conjugated to an effector domain, for example to form a fusion protein. Methods for conjugating polypeptides are known in the art, for example through the provision of a linker amino acid sequence connecting the polypeptides (e.g. a linker comprising glycine and/or serine residues). Alternative methods of conjugating polypeptides known in the art include chemical and light-induced conjugation methods (e.g. using chemical cross-linking agents). Preferably, the DNA-binding domain and effector domain of the ESF form a fusion protein.

In some embodiments, the ESF is a fusion protein comprising a transcription factor DNA-binding domain and at least one epigenetic effector domain.

The ESF may be formed by separate polypeptide chains that bind together to form a complex, for example a heterodimeric complex. For example, the binding may be enabled by an epitope (e.g. a Suntag) comprised on a first chain and an epitope-binding molecule, such as a single-chain variable fragment (scFv), comprised on a second chain.

In some embodiments, the ESF comprises two epigenetic effector domains, for example fused to the same DNA-binding domain. In some embodiments, the ESF comprises three epigenetic effector domains, for example fused to the same DNA-binding domain. The ESF may comprise four, five, six or more epigenetic effector domains, for example fused to the same DNA-binding domain.

Where the ESF comprises more than one epigenetic effector domain, the effector domains may be different. Where the ESF comprises more than one epigenetic effector domain, the effector domains may be the same.

In preferred embodiments, the ESF comprises a KRAB domain, a DNMT3A domain and/or a DNMT3L domain.

In other preferred embodiments, the ESF comprises a Chromo Shadow domain, a YAF2-RYBP domain, a DNMT3A domain and/or a DNMT3L domain.

### Epigenetic effector domain

The term "epigenetic effector domain", is to be understood as referring to a part of the ESF which provides for an epigenetic effect on a target gene, for example by catalysing a reaction on DNA or chromatin (e.g. methylation of DNA, methylation or acetylation of a histone, or demethylation or deacetylation of a histone), or by recruiting an additional agent, resulting in the repression of the transcription of the gene.

"Domain" is to be understood in this context as referring to a part of the ESF that harbours a certain function. The domain may be an individual domain (e.g. a catalytic domain) isolated from a natural protein or it may be an entire, full-length natural protein. Put another way, either the full-length protein or a functional fragment thereof can be used as an effector domain. Therefore, for example, "KRAB domain" may refer to a part of the ESF that comprises an amino acid sequence with the function of a KRAB domain.

Chromatin remodelling enzymes that are known to be involved in the permanent epigenetic silencing of endogenous retroviruses (ERVs; Feschotte, C. et al. (2012) Nat. Rev. Genet. 13: 283-96; Leung, D.C. et al. (2012) Trends Biochem. Sci. 37: 127-33) may provide suitable effector domains for exploitation in the invention.

In some embodiments, the epigenetic effector domain represses transcription and/or expression of at least one target gene. In some embodiments, the epigenetic effector domain is a repressor domain.

In some embodiments, the epigenetic effector domain catalyses chemical modification of chromatin and/or chromatin remodelling.

In some embodiments, the epigenetic effector domain catalyses DNA modification, such as DNA methylation. In some embodiments, the epigenetic effector domain is a DNA methyltransferase and/or is capable of recruiting a DNA methyltransferase.

In some embodiments, the epigenetic effector domain catalyses histone modification, such as histone methylation or histone acetylation. In some embodiments, the epigenetic effector domain is a histone methyltransferase or histone acetyltransferase. In some embodiments, the epigenetic effector domain catalyses histone demethylation or histone deacetylation. In some embodiments, the epigenetic effector domain is a histone methylase or histone acetylase.

### Kruppel-associated box (KRAB) domain

The family of the Krüppel-associated box containing zinc finger proteins (KRAB-ZFP; Huntley, S. et al. (2006) Genome Res. 16: 669-77) plays an important role in the silencing of endogenous retroviruses. These transcription factors bind to specific ERV sequences through their ZFP DNA-binding domain, while they recruit the KRAB Associated Protein 1 (KAP1) with their conserved KRAB domain. KAP1 in turn binds a large number of effectors that promote the local formation of repressive chromatin (Iyengar, S. et al. (2011) J. Biol. Chem. 286: 26267-76). For example, they may induce repressive chromatin modification (e.g. H3K9me3) and/or remove active marks (e.g. H3K4ac)

In some embodiments, the ESF comprises a KRAB domain.

Various KRAB domains are known in the family of KRAB-ZFP proteins. For example, an ESF of the invention may comprise the KRAB domain of human zinc finger protein 10 (ZNF10; Szulc, J. et al. (2006) Nat. Methods 3: 109-16). An example sequence of a KRAB domain of human zinc finger protein 10 is:

ALSPQHSAVTQGSIIKNKEGMDAKSLTAWSRTLVTFKDVFVDFTREEWKLLDTAQQIVYRNV
MLENYKNLVSLGYQLTKPDVILRLEKGEEPWLVEREIHQETHPDSETAFEIKSSV

(SEQ ID NO: 5)

Further examples of suitable KRAB domains for use in the invention include: 
MNNSQGRVTFEDVTVNFTQGEWQRLNPEQRNLYRDVMLENYSNLVSVGQGETTKPDVILRLE
QGKEPWLEEEEVLGSGRAEKNGDIGGQIWKPKDVKESL
(the KRAB domain of the ZIM3 protein; SEQ ID NO: 6 
ITLEDVAVDFTWEEWQLLGAAQKDLYRDVMLENYSNLVAVGYQASKPDALFKLEQGEQLWTI
EDGIHSGACS
(the KRAB domain of the ZNF350 protein; SEQ ID NO: 7) 
VMFEEVSVCFTSEEWACLGPIQRALYWDVMLENYGNVTSLEWETMTENEEVTSKPSSSQRAD
SHKGTSKRLQG
(the KRAB domain of the ZNF197 protein; SEQ ID NO: 8) 
VSFKDVAVDFTQEEWQQLDPDEKITYRDVMLENYSHLVSVGYDTTKPNVIIKLEQGEEPWIM
GGEFPCQHSP
(the KRAB domain of the RBAK protein; SEQ ID NO: 9) 
VKIEDMAVSLILEEWGCQNLARRNLSRDNRQENYGSAFPQGGENRNENEESTSKAETSEDSA
SRGETTGRSQKE
(the KRAB domain of the ZKSCAN1 protein; SEQ ID NO: 10) 
LTFKDVFVDFTLEEWQQLDSAQKNLYRDVMLENYSHLVSVGYLVAKPDVIFRLGPGEESWMA
DGGTPVRTCA
(the KRAB domain of the KRBOX4 protein; SEQ ID NO: 11) 
VTFEDVTLGFTPEEWGLLDLKQKSLYREVMLENYRNLVSVEHQLSKPDVVSQLEEAEDFWPV
ERGIPQDTIP
(the KRAB domain of the ZNF274 protein; SEQ ID NO: 12)

An example nucleotide sequence encoding a KRAB domain is:

ATGGATGCTAAGTCACTGACTGCCTGGTCCCGGACACTGGTGACCTTCAAGGATGTGTTTGT
GGACTTCACCAGGGAGGAGTGGAAGCTGCTGGACACTGCTCAGCAGATCCTGTACAGAAATG
TGATGCTGGAGAACTATAAGAACCTGGTTTCCTTGGGTTATCAGCTTACTAAGCCAGATGTG

ATCCTCCGGTTGGAGAAGGGAGAAGAGCCCTGGCTGGTGGAGAGAGAAATTCACCAAGAGAC
CCATCCTGATTCAGAGACTGCATTTGAAATCAAATCATCAGTT

(SEQ ID NO: 13)

### DNA methyltransferase (DNMT) domain

In some embodiments, the ESF comprises a DNA methyltransferase (DNMT) domain. In some embodiments, the ESF comprises a DNMT3A domain, a DNMT3B domain and/or a DNMT1 domain. In some embodiments, the ESF comprises a DNMT3A domain.

An ESF of the invention may, for example, comprise a domain of human DNA methyltransferase 3A (DNMT3A; Law, J.A. et al. (2010) Nat. Rev. Genet. 11: 204-20), preferably the catalytic domain. An example DNMT3A sequence is:

TYGLLRRREDWPSRLQMFFANNHDQEFDPPKVYPPVPAEKRKPIRVLSLFDGIATGLLVLKD
LGIQVDRYIASEVCEDSITVGMVRHQGKIMYVGDVRSVTQKHIQEWGPFDLVIGGSPCNDLS
IVNPARKGLYEGTGRLFFEFYRLLHDARPKEGDDRPFFWLFENVVAMGVSDKRDISRFLESN
PVMIDAKEVSAAHRARYFWGNLPGMNRPLASTVNDKLELQECLEHGRIAKFSKVRTITTRSN
SIKQGKDQHFPVFMNEKEDILWCTEMERVFGFPVHYTDVSNMSRLARQRLLGRSWSVPVIRH
LFAPLKEYFACV

(SEQ ID NO: 14)

DNA methyltransferases 3B and 1 (DNMT3B and DNMT1), similarly to DNMT3A, are also responsible for the deposition and maintenance of DNA methylation, and may also be used in an ESF of the present invention. Example sequences are: 
CHGVLRRRKDWNVRLQAFFTSDTGLEYEAPKLYPAIPAARRRPIRVLSLFDGIATGYLVLKE
LGIKVGKYVASEVCEESIAVGTVKHEGNIKYVNDVRNITKKNIEEWGPFDLVIGGSPCNDLS
NVNPARKGLYEGTGRLFFEFYHLLNYSRPKEGDDRPFFWMFENVVAMKVGDKRDISRFLECN
PVMIDAIKVSAAHRARYFWGNLPGMNRPVIASKNDKLELQDCLEYNRIAKLKKVQTITTKSN
SIKQGKNQLFPVVMNGKEDVLWCTELERIFGFPVHYTDVSNMGRGARQKLLGRSWSVPVIRH
LFAPLKDYFACE
(the catalytic domain of human DNMT3B; SEQ ID NO: 15) 
MVAELISEEDLEFMKGDTRHLNGEEDAGGREDSILVNGACSDQSSDSPPILEAIRTPEIRGR
RSSSRLSKREVSSLLSYTQDLTGDGDGEDGDGSDTPVMPKLFRETRTRSESPAVRTRNNNSV
SSRERHRPSPRSTRGRQGRNHVDESPVEFPATRSLRRRATASAGTPWPSPPSSYLTIDLTDD
TEDTHGTPQSSSTPYARLAQDSQQGGMESPQVEADSGDGDSSEYQDGKEFGIGDLVWGKIKG
FSWWPAMVVSWKATSKRQAMSGMRWVQWFGDGKFSEVSADKLVALGLFSQHFNLATFNKLVS
YRKAMYHALEKARVRAGKTFPSSPGDSLEDQLKPMLEWAHGGFKPTGIEGLKPNNTQPENKT
RRRTADDSATSDYCPAPKRLKTNCYNNGKDRGDEDQSREQMASDVANNKSSLEDGCLSCGRK
NPVSFHPLFEGGLCQTCRDRFLELFYMYDDDGYQSYCTVCCEGRELLLCSNTSCCRCFCVEC
LEVLVGTGTAAEAKLQEPWSCYMCLPQRCHGVLRRRKDWNVRLQAFFTSDTGLEYEAPKLYP
AIPAARRRPIRVLSLFDGIATGYLVLKELGIKVGKYVASEVCEESIAVGTVKHEGNIKYVND
VRNITKKNIEEWGPFDLVIGGSPCNDLSNVNPARKGLYEGTGRLFFEFYHLLNYSRPKEGDD
RPFFWMFENVVAMKVGDKRDISRFLECNPVMIDAIKVSAAHRARYFWGNLPGMNRPVIASKN
DKLELQDCLEYNRIAKLKKVQTITTKSNSIKQGKNQLFPVVMNGKEDVLWCTELERIFGFPV
HYTDVSNMGRGARQKLLGRSWSVPVIRHLFAPLKDYFACE
(DNMT3B: SEQ ID NO: 16) 
LRTLDVFSGCGGLSEGFHQAGISDTLWAIEMWDPAAQAFRLNNPGSTVFTEDCNILLKLVMA
GETTNSRGQRLPQKGDVEMLCGGPPCQGFSGMNRFNSRTYSKFKNSLVVSFLSYCDYYRPRF
FLLENVRNFVSFKRSMVLKLTLRCLVRMGYQCTFGVLQAGQYGVAQTRRRAIILAAAPGEKL
PLFPEPLHVFAPRACQLSVVVDDKKFVSNITRLSSGPFRTITVRDTMSDLPEVRNGASALEI
SYNGEPQSWFQRQLRGAQYQPILRDHICKDMSALVAARMRHIPLAPGSDWRDLPNIEVRLSD
GTMARKLRYTHHDRKNGRSSSGALRGVCSCVEAGKACDPAARQFNTLIPWCLPHTGNRHNHW
AGLYGRLEWDGFFSTTVTNPEPMGKQGRVLHPEQHRVVSVRECARSQGFPDTYRLFGNILDK
HRQVGNAVPPPLAKAIGLEIKLCMLAKARESASAKIKEEEAAKD
(the catalytic domain of human DNMT1; SEQ ID NO: 17)

### DNMT-like domain

In some embodiments, the ESF comprises a DNMT-like domain. A "DNMT-like" domain refers to a protein which is a member of a DNMT family, but which does not possess DNA methylation activity. The DNMT-like protein typically activates or recruits other epigenetic effector domains.

An ESF of the invention may, for example, comprise DNA (cytosine-5)-methyltransferase 3-like (DNMT3L), a catalytically inactive DNA methyltransferase that activates DNMT3A by binding to its catalytic domain. An example DNMT3L sequence is:

MAAIPALDPEAEPSMDVILVGSSELSSSVSPGTGRDLIAYEVKANQRNIEDICICCGSLQVH
TQHPLFEGGICAPCKDKFLDALFLYDDDGYQSYCSICCSGETLLICGNPDCTRCYCFECVDS
LVGPGTSGKVHAMSNWVCYLCLPSSRSGLLQRRRKWRSQLKAFYDRESENPLEMFETVPVWR
RQPVRVLSLFEDIKKELTSLGFLESGSDPGQLKHVVDVTDTVRKDVEEWGPFDLVYGATPPL
GHTCDRPPSWYLFQFHRLLQYARPKPGSPRPFFWMFVDNLVLNKEDLDVASRFLEMEPVTIP
DVHGGSLQNAVRVWSNIPAIRSRHWALVSEEELSLLAQNKQSSKLAAKWPTKLVKNCFLPLR
EYFKYFSTELTSSL

(SEQ ID NO: 18)

A DNMT3A and DNMT3L domain may be used together, and referred to herein as "DNMT3A/3L" or "DNMT3a3L".

An example nucleotide sequence encoding DNMT3A and DNMT3L domains is:

AACCATGATCAAGAGTTCGATCCTCCTAAAGTGTATCCCCCTGTCCCTGCCGAAAAGCGGAA
ACCGATCAGGGTTCTCAGTCTCTTCGATGGAATTGCCACAGGCCTCTTGGTCCTTAAGGACC
TGGGCATCCAAGTgGAcCGCTACATCGCCTCAGAAGTATGTGAgGACAGCATCACAGTCGGC
ATGGTGCGCCACCAGGGGAAAATAATGTACGTCGGCGACGTCAGATCAGTTACTCAGAAACA
TATTCAGGAATGGGGGCCTTTTGATCTCGTAATTGGCGGCAGCCCCTGCAATGATCTGAGTA
TTGTGAACCCCGCCCGGAAAGGTTTGTACGAAGGTACGGGCAGACTGTTCTTCGAGTTTTAC
AGACTGTTGCACGACGCACGACCAAAGGAGGGTGAcGAtcGACCCTTCTTCTGGCTGTTCGA
GAACGTGGTCGCTATGGGCGTGTCTGACAAGCGGGACATTTCtAGATTCCTGGAGAGCAATC
CAGTGATGATTGATGCAAAGGAAGTAtccGCTGCCCACCGCGCCAGATACTTCTGGGGCAAT
CTGCCCGGCATGAATCGACCCTTGGCAAGCACCGTGAATGATAAGTTGGAGTTGCAGGAGTG
TCTgGAGCATGGGCGGATCGCAAAGTTTAGCAAGGTGCGGACCATCACGACCCGAAGCAATT
CAATTAAGCAGGGAAAGGACCAACATTTTCCAGTGTTTATGAACGAGAAAGAGGAcATCCTG

TGGTGcACAGAAATGGAGCGGGTTTTCGGATTCCCCGTACATTATACTGATGTGTCCAATAT
GTCTAGGTTGGCTAGACAGAGGCTGtTGGACGCTCATGGAGTGTTCCTGTCATCCGCCACC
TGTTCGCTCCTCTGAAGGAGTATTTCGCcTGCGTtTCATCCGGgAATTCAAACGCAAACAGC
AGAGGCCCATCCTTTTCTTCCGGCCTGGTgCCACTTAGTCTGCGCGGCTCTCACATGGGACC
TATGGAAATATACAAAACAGTGAGCGCCTGGAAGAGGCAGCCTGTGCGGGTCCTGAGTCTGT
TCAGAAACATTGACAAGGTGCTTAAGTCCCTTGGATTTCTGGAGTCTGGCAGCGGTTCTGGG
GGTGgcACCCTTAAGTATGTGGAGGACGTGACAAACGTCGTGAGGAGAGATGTGGAGAAATG
GGGGCCATTTGACCTGGTATACGGGAGCACTCAACCTCTCGGTTCTTCTTGTGATCGCTGCC
CAGGGTGGTATATGTTTCAGTTCCATCGcATCCTGCAATACGCCCTTCCGCGGCAGGAGAGT
CAGCGACCATTCTTCTGGATATTCATGGACAATCTCCTGCTGACAGAgGACGACCAAGAGAC
TACGACTAGATTTCTTCAGACCGAGGCCGTTACTCTCCAGGACGTTAGAGGTAGGGACTACC
AGAATGCCATGCGAGTGTGGAGTAACATCCCCGGACTCAAGTCAAAGCAcGCACCCCTGACC
CCCAAGGAAGAGGAATACCTcCAGGCGCAGGTGAGGAGCCGCAGTAAGCTCGATGCTCCTAA
AGTCGATTTGTTGGTGAAGAATTGCTTGCTGCCCCTGAGAGAGTACTTTAAGTACTTTAGCC
AGAACAGCTTGCCACTG

(SEQ ID NO: 19)

### Chromo Shadow (CS) domain

In some embodiments, the ESF comprises a Chromo Shadow (CS) domain. The CS domain may be a CS domain of CBX5.

An example CS domain sequence is:
MLEPEKIIGATDSCGDLMFLMKWKDTDEADLVLAKEANVKCPQIVIAFYEERLTWHAYPE
(SEQ ID NO: 20)

An example nucleotide sequence encoding a CS domain is:

ATGCTGGAACCAGAAAAGATCATTGGGGCAACAGATTCCTGTGGTGATTTAATGTTCCTAAT
GAAATGGAAAGACACAGATGAAGCTGACCTGGTTCTTGCAAAAGAAGCTAATGTGAAATGTC
CACAAATTGTGATAGCATTTTATGAAGAGAGACTGACATGGCATGCATATCCTGAG

(SEQ ID NO: 21)

### YAF2-RYBP (Y-R) domain

In some embodiments, the ESF comprises a YAF2-RYBP (Y-R) domain.

An example Y-R domain sequence is:
MRPRLKNVDRSTAQQLAVTVGNVTVIITDFKEKTRSSSTSSSTVTSSAGSEQQNQ
(SEQ ID NO: 22)

An example nucleotide sequence encoding a Y-R domain is:

ATGAGGCCCCGGCTGAAAAACGTGGACAGGAGCACTGCACAGCAGTTGGCAGTAACTGTGGG
CAACGTCACCGTCATTATCACAGACTTTAAGGAAAAGACTCGCTCCTCATCGACATCCTCAT
CCACAGTGACCTCCAGTGCAGGGTCAGAACAGCAGAACCAG

(SEQ ID NO: 23)

### Further epigenetic effector domains

Example sequences of further suitable epigenetic effector domains are:

| **TF** | **Sequence** | **SEQ ID NO** |
|---|---|---|
| EnR | MALEDRCSPQSAPSPITLQMQHLHHQQQQQQQQQQMQHLHQL QQLQQLHQQQLAAGVFHHPAMAFDAAAAAAAAAAAAAAHAHAA ALQQRLSGSGSPASCSTPASSTPLTIKEEESDSVIGDMSFHNQ THTTNEEEEAEEDDDIDVDVDDTSAGGRLPPPAHQQQSTAKPS LAFSISNILSDRFGDVQKPGKSMENQASIFRPFEASRSQTATP SAFTRVDLLEFSRQQQAAAAAATAAMMLERANFLNCFNPAAYP RIHEEIVQSRLRRSAANAVIPPPMSSKMSDANPEKSAL | 24 |
| MECP2 | SPKKKRKVEASVQVKRVLEKSPGKLLVKMPFQASPGGKGEGGG ATTSAQVMVIKRPGRKRKAEADPQAIPKKRGRKPGSVVAAAAA EAKKKAVKESSIRSVQETVLPIKKRKTRETVSIEVKEVVKPLL VSTLGEKSGKGLKTCKSPGRKSKESSPKGRSSSASSPPKKEHH HHHHAESPKAPMPLLPPPPPPEPQSSEDPISPPEPQDLSSSI CKEEKMPRAGSLESDGCPKEPAKTQPMVAAAATTTTTTTTTVA EKYKHRGEGERKDIVSSSMPRPNREEPVDSRTPVTERVS | 25 |
| GLI3RD | GTVFAMDPRNGYMEPHYHPPHLFPAFHPPVPIDARHHEGRYHY DPSPIPPLHMTSALSSSPTYPDLPFIRISPHRNPTAASESPFS PPHPYINPYMDYIRSLHSSPSLSMISATRGLSPTDAPHAGVSP AE | 26 |
| MAD1RD | RMNIQMLLEAADYLER | 27 |

Example nucleotide sequences encoding further suitable epigenetic effector domains are:

| **TF** | **Sequence** | **SEQ ID NO** |
|---|---|---|
| ZIM3-KRAB | ATGAACAATTCCCAGGAAGAGTGACCTTCGAGGATGTCACTG TGAACTTCACCCAGGGGGAGTGGCAGCGGCTGAATCCCGAACA GAGAAACTTGTACAGGGATGTGATGCTGGAGAATTACAGCAAC CTTGTCTCTGTGGGACAAGGGGAAACCACCAAACCCGATGTGA TCTTGAGGTTGGAACAAGGAAAGGAGCCATGGTTGGAGGAAGA GGAAGTGCTGGGAAGTGGCCGTGCAGAAAAAAATGGGGACATT GGAGGGCAGATTTGGAAGCCAAAGGATGTGAAAGAGAGTCTC | 28 |
| EnR | ATGGCCCTGGAGGATCGCTGCAGTCCACAGTCAGCGCCCAGCC CCATTACCCTACAAATGCAGCATCTTCACCACCAGCAACAGCA GCAGCAGCAACAGCAGCAGCAAATGCAGCACCTCCACCAGCTG CAGCAACTGCAGCAGTTGCACCAACAGCAACTGGCCGCCGGTG TCTTCCACCATCCGGCAATGGCCTTCGATGCCGCTGCAGCCGC CGCTGCTGCAGCTGCTGCTGCGGCCGCCCACGCTCATGCTGCT GCACTGCAGCAGCGCCTCAGTGGCAGTGGATCGCCCGCATCCT GCTCCACGCCCGCCTCGTCCACGCCGCTGACCATCAAGGAGGA GGAAAGCGACTCCGTGATCGGTGACATGAGTTTCCACAATCAG ACGCACACCACCAACGAGGAGGAGGAGGCGGAGGAGGATGACG ACATTGATGTGGATGTGGATGATACGTCGGCGGCGGACGCCT GCCACCACCCGCCCACCAGCAGCAGTCGACGGCCAAGCCCTCG CTGGCCTTTTCCATCTCCAACATCCTGAGCGATCGTTTCGGAG ATGTCCAGAAGCCGGGCAAGTCGATGGAGAACCAGGCCAGCAT ATTCCGCCCCTTCGAGGCGAGTCGTTCCCAGACTGCCACGCCC TCCGCCTTTACAAGAGTGGATCTGCTGGAGTTTAGCCGGCAAC AGCAGGCTGCCGCCGCAGCCGCTACTGCGGCCATGATGCTGGA | 29 |
| | ACGGGCCAACTTCCTTAACTGCTTCAATCCGGCTGCCTATCCC AGGATACACGAGGAAATCGTGCAGAGTCGGCTGCGCAGGAGTG CAGCCAATGCCGTCATCCCGCCGCCCATGAGCTCCAAGATGAG CGATGCCAATCCAGAGAAATCTGCTCTG | |
| MECP2 | AGCCCAAAGAAGAAACGGAAGGTGGAAGCCTCAGTGCAGGTGA AAAGGGTGCTGGAAAAATCCCCGGCAAACTCCTCGTGAAGAT GCCCTTCCAGGCTTCCCCTGGCGGAAAAGGTGAAGGGGGTGGC GCAACCACATCTGCCCAGGTCATGGTCATCAAGCGACCTGGAA GGAAAAGAAAGGCCGAGGCTGACCCTCAGGCCATTCCAAAGAA ACGGGGACGCAAGCCAGGGTCCGTGGTCGCAGCTGCAGCAGCT GAGGCTAAGAAAAAGGCAGTGAAGGAAAGCTCCATCCGCAGTG TGCAGGAGACTGTCCTGCCCATCAAGAAGAGGAAGACTAGGGA GACCGTGTCCATCGAGGTCAAAGAAGTGGTCAAGCCCCTGCTC GTGTCCACCCTGGGCGAAAAATCTGGAAAGGGGCTCAAAACAT GCAAGTCACCTGGACGGAAAAGCAAGGAGTCTAGTCCAAAGGG GCGCTCAAGCTCCGCTTCTAGTCCCCCTAAAAAGGAACACCAT CACCATCACCATCACGCCGAGTCTCCTAAGGCTCCTATGCCAC TGCTCCCACCACCTCCACCACCTGAGCCACAGTCAAGCGAAGA CCCCATCAGCCCACCCGAGCCTCAGGATCTGTCCTCTAGTATT TGCAAAGAGGAAAAGATGCCCAGAGCAGGCAGCCTGGAGAGTG ATGGCTGTCCAAAAGAACCCGCCAAGACCCAGCCTATGGTGGC AGCCGCTGCAACTACCACCACAACCACAACTACCACAGTGGCC GAAAAATACAAGCATCGCGGCGAGGGCGAACGAAAGGACATTG TGTCAAGCTCCATGCCCAGACCTAACCGGGAGGAACCAGTCGA TAGTAGGACACCCGTGACTGAGAGAGTCTCA | 30 |
| GLI3RD | GGGACGGTGTTTGCCATGGACCCCAGGAATGGTTACATGGAGC CCCACTACCACCCTCCTCATCTTTTCCCTGCCTTCCATCCTCC TGTACCAATTGATGCCAGACATCATGAGGGCCGTTACCATTAC GATCCATCTCCGATTCCTCCATTGCATATGACTTCCGCCTTAT CTAGTAGCCCTACGTATCCGGACCTGCCCTTCATTAGGATCTC CCCACACCGGAACCCCACTGCTGCTTCCGAGTCTCCCTTCAGC CCTCCACATCCCTACATTAATCCCTACATGGACTATATCCGCT CCTTGCACAGCAGCCCATCGCTCTCCATGATCTCAGCAACCCG TGGGCTGAGCCCTACAGATGCGCCCCATGCAGGAGTCAGCCCA GCAGAA | 31 |
| MAD1RD | CGGATGAACATCCAGATGCTGCTGGAGGCGGCCGACTATCTGG AGCGG | 32 |

The ESF of the invention may, for example, comprise an amino acid sequence that has at least 70%, 80%, 90%, 95%, 96%, 97%, 98%, 99% or 100% identity to any one of SEQ ID NOs: 5-12, 14-18, 20, 22 or 24-27, preferably wherein the amino acid sequence substantially retains the natural function of the protein represented by SEQ ID NO: 5-12, 14-18, 20, 22 or 24-27, respectively.

The ESF of the invention may, for example, be encoded by a polynucleotide comprising a nucleic acid sequence which encodes a protein that has at least 70%, 80%, 90%, 95%, 96%, 97%, 98%, 99% or 100% amino acid identity to any one of SEQ ID NOs: 5-12, 14-18, 20, 22 or 24-27, preferably wherein the amino acid sequence substantially retains the natural function of the protein represented by SEQ ID NO: 5-12, 14-18, 20, 22 or 24-27, respectively.

The polynucleotide of the invention may comprise a nucleic acid sequence that has at least 70%, 80%, 90%, 95%, 96%, 97%, 98%, 99% or 100% sequence identity to any one of SEQ ID NOs: 13, 19, 21, 23 or 28-32, preferably wherein the encoded amino acid sequence substantially retains the natural function of the protein encoded by SEQ ID NO: 13, 19, 21, 23 or 28-32, respectively.

### Transcription factor

A transcription factor (TF) may control the transcription of DNA through binding to certain DNA sequences. Transcription factors typically function to regulate genes to control transcription and/or expression, for example dependent on cell type and timing. Groups of transcription factors may work in a coordinated manner to direct cell division, cell growth and cell death; cell migration and organization during embryonic development; and in response to extracellular signals.

Transcription factors comprise at least one DNA-binding domain, which may target the transcription factor to certain sequences to direct their regulatory function.

The ESFs of the invention comprise at least one transcription factor DNA-binding domain. The skilled person is readily able to identify DNA-binding domains from transcription factors using well known methods, for example using sequence comparison tools and/or databases.

The polynucleotides, polypeptides and ESFs of the invention may comprise a minimum transcription factor sequence that retains its function in binding to DNA. However, it is preferred that as much transcription factor sequence as possible is retained in the polypeptides and ESFs of the invention, without adversely impacting the function of the ESF in decreasing transcription and/or expression. Without wishing to be bound by theory, transcription factor sequence in addition to the DNA-binding domain may enable recruitment of additional factors within a cell.

Unmodified transcription factors may comprise an activation domain (AD; also referred to as a trans-activation domain), which may function to activate gene transcription and/or expression. The polypeptides and ESFs of the invention preferably do not comprise a functional activation domain. The transcription factor sequence may be modified (e.g. mutated or truncated) to disrupt activation domain function. The transcription factor sequence incorporated into the polypeptide or ESF may lack an activation domain.

In preferred embodiments, the ESF does not comprise a functional transcription factor activation domain. The ESF may, for example, not comprise a transcription factor activation domain. The ESF may, for example, comprise a fragment of a transcription factor that lacks a functional transcription activation domain, and which comprises a functional DNA-binding domain.

In preferred embodiments, the transcription factor is an oncogenic transcription factor.

The term "oncogenic transcription factor" as used herein refers to a transcription factor that may transform a healthy cell into a cancer cell, for example through causation of inappropriate gene expression patterns, which can for example promote tumor initiation and progression.

In some embodiments, the transcription factor is a cancer-associated transcription factor.

The term "cancer-associated transcription factor" as used herein refers to a transcription factor that may induce tumorigenic properties, but cannot transform a healthy cell into cancer cell.

In some embodiments, the transcription factor is selected from the group consisting of SOX2, MYC, MYCN, TEAD1, TEAD2, TEAD3, TEAD4, FOXA1, FOXA2, ELK1, ELK3, ELK4, SRF, FOXM1, FOXC1, FOXC2, TWIST1, SALL4, ELF1, HIF1A, SOX9, SOX12, SOX18, ETS1, PAX3, PAX8, GLI1, GLI2, GLI3, ETV1, ETV2, ETV3, RUNX1, RUNX2, RUNX3, MAFB, TFAP2C and E2F1.

In some embodiments, the transcription factor is SOX2. The ESF may comprise a fragment of human SOX2 consisting of amino acids 1-179.

In some embodiments, the transcription factor is MYC. The ESF may comprise a fragment of human MYC consisting of amino acids 144-454.

In some embodiments, the transcription factor is TEAD1. The ESF may comprise a fragment of human TEAD1 consisting of amino acids 1-166.

Example sequences comprising suitable transcription factor DNA-binding domains are:

| **TF** | **Sequence** | **SEQ ID NO** |
|---|---|---|
| SOX2 | MYNMMETELKPPGPQQTSGGGGGNSTAAAAGGNQKNSPDRVKR PMNAFMVWSRGQRRKMAQENPKMHNSEISKRLGAEWKLLSETE KRPFIDEAKRLRALHMKEHPDYKYRPRRKTKTLMKKDKYTLPG GLLAPGGNSMASGVGVGAGLGAGVNQRMDSYAHMNGWSNGSYS MMQDQLG | 33 |
| MYC | IIQDCMWSGFSAAAKLVSEKLASYQAARKDSGSPNPARGHSVC | 34 |
| | STSSLYLQDLSAAASECIDPSVVFPYPLNDSSSPKSCASQDSS AFSPSSDSLLSSTESSPQGSPEPLVLHEETPPTTSSDSEEEQE DEEEIDVVSVEKRQAPGKRSESGSPSAGGHSKPPHSPLVLKRC HVSTHQHNYAAPPSTRKDYPAAKRVKLDSVRVLRQISNNRKCT SPRSSDTEENVKRRTHNVLERQRRNELKRSFFALRDQIPELEN NEKAPKVVILKKATAYILSVQAEEQKLISEEDLLRKRREQLKH KLEQLRNSCA | |
| MYCN | | 35 |
| TEAD1 | IEPSSWSGSESPAENMERMSDSADKPIDNDAEGVWSPDIEQSF QEALAIYPPCGRRKIILSDEGKMYGRNELIARYIKLRTGKTRT RKQVSSHIQVLARRKSRDFHSKLKDQTAKDKALQHMAAMSSAQ IVSATAIHNKLGLPGIPRPTFPGAPGFWPGMIQTGQP | 36 |
| TEAD2 | MGEPRAGAALDDGSGWTGSEEGSEEGTGGSEGAGGDGGPDAEG VWSPDIEQSFQEALAIYPPCGRRKIILSDEGKMYGRNELIARY IKLRTGKTRTRKQVSSHIQVLARRKSREIQSKLKDQVSKDKAF QTMATMSSAQLISAPSLQAKLGPTGPQASELFQFWSGGSGPPW NVPDVKPFSQTPFTLSLTPPSTDLPGYEPPQALSPLPPPTPSP PAWQARGLGTARLQLVEFSAFVEPPDAVDSYQRHLFVHISQHC PSPGAPPLESVDVRQIYDKFPEKKGGLRELYDRGPPHAFFLVK FWADLNWGPSGEEAGAGGSISSGGFYGVSSQYESLEHMTLTCS SKVCSFGKQVVEKVETERAQLEDGRFVYRLLRSPMCEYLVNFL HKLRQLPERYMMNSVLENFTILQVVTNRDTQELLLCTAYVFEV STSERGAQHHIYRLVRD | 37 |
| TEAD3 | IASNSWNASSSPGEAREDGPEGLDKGLDNDAEGVWSPDIEQSF QEALAIYPPCGRRKIILSDEGKMYGRNELIARYIKLRTGKTRT RKQVSSHIQVLARKKVREYQVGIKAMNLDQVSKDKALQSMASM SSAQIVSASVLQNKFSPPSPLPQAVFSTSSRFWSSPPLLGQQP GPSQDIKPFAQPAYPIQPPLPPTLSSYEPLAPLPSAAASVPVW QDRTIASSRLRLLEYSAFMEVQRDPDTYSKHLFVHIGQTNPAF SDPPLEAVDVRQIYDKFPEKKGGLKELYEKGPPNAFFLVKFWA DLNSTIQEGPGAFYGVSSQYSSADSMTISVSTKVCSFGKQVVE KVETEYARLENGRFVYRIHRSPMCEYMINFIHKLKHLPEKYMM NSVLENFTILQVVTSRDSQETLLVIAFVFEVSTSEHGAQHHVY KLVKD | 38 |
| TEAD4 | LEGTAGTITSNEWSSPTSPEGSTASGGSQALDKPIDNDAEGVW SPDIEQSFQEALAIYPPCGRRKIILSDEGKMYGRNELIARYIK LRTGKTRTRKQVSSHIQVLARRKAREIQAKLKFWQGALPGQAG TSHDVKPFSQQTYAVQPPLPLPGFESPAGPAPSPSAPPAPPWQ GRSVASSKLWMLEFSAFLEQQQDPDTYNKHLFVHIGQSSPSYS DPYLEAVDIRQIYDKFPEKKGGLKDLFERGPSNAFFLVKFWAD LNTNIEDEGSSFYGVSSQYESPENMIITCSTKVCSFGKQVVEK VETEYARYENGHYSYRIHRSPLCEYMINFIHKLKHLPEKYMMN SVLENFTILQVVTNRDTQETLLCIAYVFEVSASEHGAQHHIYR LVKE | 39 |
| FOXA1 | MLGTVKMEGHETSDWNSYYADTQEAYSSVPVSNMNSGLGSMNS | 40 |
| | MNTYMTMNTMTTSGNMTPASFNMSYANPGLGAGLSPGAVAGMP GGSAGAMNSMTAAGVTAMGTALSPSGMGAMGAQQAASMNGLGP YAAAMNPCMSPMAYAPSNLGRSRAGGGGDAKTFKRSYPHAKPP YSYISLITMAIQQAPSKMLTLSEIYQWIMDLFPYYRQNQQRWQ NSIRHSLSFNDCFVKVARSPDKPGKGSYWTLHPDSGNMFENGC YLRRQKRFKCEKQPGAGGGGGSGSGGSGAKGGPESRKDPSGAS NPSADSPLHRGVHGKTGQLEGAPAPGPAASPQTLDHSGATATG GASELKTPASSTAPPISSGPGALASVPASHPAHGLAPHESQLH LKGDPHYSFNHPFSINNLMSSSEQQHKLDFKAYEQALQYSPYG STLPASLPLGSASVTTRSPIEPSALEPAYYQGVYSRPVLNTS | |
| FOXA2 | | 41 |
| ELK1 | MDPSVTLWQFLLQLLREQGNGHIISWTSRDGGEFKLVDAEEVA RLWGLRKNKTNMNYDKLSRALRYYYDKNIIRKVSGQKFVYKFV SYPEVAGCSTEDCPPQPEVSVTSTMPNVAPAAIHAAPGDTVSG KPGTPKGAGMAGPGGLARSSRNEYMRSGLYSTFTIQSLQPQPP PHPRPAVVLPSAAPAGAAAPPSGSRSTSPSPLEACLEAEEAGL PLQVILTPPEAPNLKSEELNVEPGLGRALPPEVKVEGPKEELE VAGERGFVPETTKAEPEVPPQEGVPARLPAVVMDTAGQAGGHA ASSPEISQPQKGRKPRDLELPLSPSLLGGPGPERTPGSGSGSG LQAPGPALTPSLLPTHTLTPVLLTPSSLPPSIHFWSTLSPIAP RSPAKLSFQFPSSGSAQVHIPSISVDGLSTPVVLSPGPQKP | 42 |
| ELK3 | MESAITLWQFLLQLLLDQKHEHLICWTSNDGEFKLLKAEEVAK LWGLRKNKTNMNYDKLSRALRYYYDKNIIKKVIGQKFVYKFVS FPEILKMDPHAVEISRESLLLQDSDCKASPEGREAHKHGLAAL RSTSRNEYIHSGLYSSFTINSLQNPPDAFKAIKTEKLEEPPED SPPVEEVRTVIRFVTNKTDKHVTRPVVSLPSTSEAAAASAFLA SSVSAKISSLMLPNAASISSASPFSSRSPSLSPNSPLPSEHRS LFLEAACHDSDSLEPLNLSSGSKTKSPSLPPKAKKPKGLEISA PPLVLSGTDIGSIALNSPALPSGSLTPAFFTAQTPNGLLLTPS PLLSSIHFWSSLSPVAPLSPARLQGPSTLFQFPTLLNGHMPVP IPSLDRAASPVLLSSNSQKS | 43 |
| ELK4 | MDSAITLWQFLLQLLQKPQNKHMICWTSNDGQFKLLQAEEVAR LWGIRKNKPNMNYDKLSRALRYYYVKNIIKKVNGQKFVYKFVS YPEILNMDPMTVGRIEGDCESLNFSEVSSSSKDVENGGKDKPP QPGAKTSSRNDYIHSGLYSSFTLNSLNSSNVKLFKLIKTENPA EKLAEKKSPQEPTPSVIKFVTTPSKKPPVEPVAATISIGPSIS PSSEETIQALETLVSPKLPSLEAPTSASNVMTAFATTPPISSI PPLQEPPRTPSPPLSSHPDIDTDIDSVASQPMELPENLSLEPK DQDSVLLEKDKVNNSSRSKKPKGLELAPTLVITSSDPSPLGIL SPSLPTASLTPAFFSQTPIILTPSPLLSSIHFWSTLSPVAPLS PARLQGANTLFQFPSVLNSHGPFTLSGLDGPSTPGPFSPDLQK T | 44 |
| SRF | MLPTQAGAAAALGRGSALGGSLNRTPTGRPGGGGGTRGANGGR VPGNGAGLGPGRLEREAAAAAATTPAPTAGALYSGSEGDSESG EEEELGAERRGLKRSLSEMEIGMVVGGPEASAAATGGYGPVSG | 45 |
| | AVSGAKPGKKTRGRVKIKMEFIDNKLRRYTTFSKRKTGIMKKA YELSTLTGTQVLLLVASETGHVYTFATRKLQPMITSETGKALI QTCLNSPDSPPRSDPTTDQRMSATGFEETDLTYQVSESDSSGE TKDTLKPAFTVTNLPGTTSTIQTAPSTSTTMQVSSGPSFPITN YLAPVSASVSPSAVSSANGTVLKSTGSGPVSSGGLMQLPTSFT LMPGGAVAQQVPVQAIQVHQAPQQASPSRDSSTDLTQTSSSGT VTLPATIMTSSVPTTVGGHMMYPSPHAVMYAPTSGLGDGSLTV LNAFSQAPSTMQVSHSQVQEPGGVPQVFLTASSGTVQIPVSAV QLHQMAVIGQQAGSSSNLTELQVVNLDTAHSTKSE | |
| FOXM1 | MKTSPRRPLILKRRRLPLPVQNAPSETSEEEPKRSPAQQESNQ AEASKEVAESNSCKFPAGIKIINHPTMPNTQVVAIPNNANIHS IITALTAKGKESGSSGPNKFILISCGGAPTQPPGLRPQTQTSY DAKRTEVTLETLGPKPAARDVNLPRPPGALCEQKRETCADGEA AGCTINNSLSNIQWLRKMSSDGLGSRSIKQEMEEKENCHLEQR QVKVEEPSRPSASWQNSVSERPPYSYMAMIQFAINSTERKRMT LKDIYTWIEDHFPYFKHIAKPGWKNSIRHNLSLHDMFVRETSA NGKVSFWTIHPSANRYLTLDQVFKPLDPGSPQLPEHLESQQKR PNPELRRNMTIKTELPLGARRKMKPLLPRVSSYLVPIQFPVNQ SLVLQPSVKVPLPLAASLMSSELARHSKRVRIAPKVFGEQVVF GYMSKFFSGDLRDFGTPITSLFNFIFLCLSVLLAEEGIAPLSS AGPGKEEKLLFGEGFSPLLPVQTIKEEEIQPGEEMPHLARPIK VESPPLEEWPSPAPSFKEESSHSWEDSSQSPTPRPKKSYSGLR SPTRCVSEMLVIQHRERRERSRSRRKQHLLPPCVDEPELLFSE GPSTSRWAAELPFPADSSDPASQLSYSQEVGGPFKTPIKETLP ISSTPSKSVLPRTPESWRLTPPAKVGGLDFSPVQTSQGASDPL PDPLGLMDLSTTPLQSAPPLESPQRLLSSEPLDLISVPFGNSS PSDIDVPKPGSPEPQVSGLAANRSLTEGLVLDTMNDSLSKILL DISFPGLDEDPLGPDNINWSQFIPELQ | 46 |
| FOXC1 | MQARYSVSSPNSLGVVPYLGGEQSYYRAAAAAAGGGYTAMPAP MSVYSHPAHAEQYPGGMARAYGPYTPQPQPKDMVKPPYSYIAL ITMAIQNAPDKKITLNGIYQFIMDRFPFYRDNKQGWQNSIRHN LSLNECFVKVPRDDKKPGKGSYWTLDPDSYNMFENGSFLRRRR RFKKKDAVKDKEEKDRLHLKEPPPPGRQPPPAPPEQADGNAPG PQPPPVRIQDIKTENGTCPSPPQPLSPAAALGSGSAAAVPKIE SPDSSSSSLSSGSSPPGSLPSARPLSLDGADSAPPPPAPSAPP PHHSQGFSVDNIMTSLRGSPQSAAAELSSGLLASAAASSRAGI APPLALGAYSPGQSSLYSSPCSQTSSAGSSGGGGGGAGAAGGA GGAGTYHCNLQAMSLYAAGERGGHLQGAPGGAGGSAVDDPLPD YSLPPVTSSSSSSSLSHGGGGGGGGGGQEAGHHPAAHQGRLTSW YLNQAGGDLGHLASAAAAAAAAGYPGQQQNFHSVREMFESQRI GLNNSPVNGNSSCQMAFPSSQSLYRTSGAFVYDCSKF | 47 |
| FOXC2 | | 48 |
| TWIST1 | | 49 |
| | | |
| HIF1A | MEGAGGANDKKKISSERRKEKSRDAARSRRSKESEVFYELAHQ LPLPHNVSSHLDKASVMRLTISYLRVRKLLDAGDLDIEDDMKA QMNCFYLKALDGFVMVLTDDGDMIYISDNVNKYMGLTQFELTG HSVFDFTHPCDHEEMREMLTHRNGLVKKGKEQNTQRSFFLRMK CTLTSRGRTMNIKSATWKVLHCTGHIHVYDTNSNQPQCGYKKP PMTCLVLICEPIPHPSNIEIPLDSKTFLSRHSLDMKFSYCDER ITELMGYEPEELLGRSIYEYYHALDSDHLTKTHHDMFTKGQVT TGQYRMLAKRGGYVWVETQATVIYNTKNSQPQCIVCVNYVVSG IIQHDLIFSLQQTECVLKPVESSDMKMTQLFTKVESEDTSSLF DKLKKEPDALTLLAPAAGDTIISLDFGSNDTETDDQQLEEVPL YNDVMLPSPNEKLQNINLAMSPLPTAETPKPLRSSADPALNQE VALKLEPNPESLELSFTMPQIQDQTPSPSDGSTRQSSPEPNSP SEYCFYVDSDMVNEFKLELVEKLFAEDTEAKNPFSTQDTDLDL EMLAPYIPMDDDFQLRSFDQLSPLESSSASPESASPQSTVTVF QQTQIQEPTANATTTTATTDELKTVTKDRMEDIKILIASPSPT HIHKETTSATSSPYRDTQSRTASPNRAGKGVIEQTEKSHPRSP NVLSVALSQRTTVPEEELNPKILALQNAQRKRKMEHDGSLFQA VGII | 50 |
| SALL4 | MSRRKQAKPQHINSEEDQGEQQPQQQTPEFADAAPAAPAAGEL GAPVNHPGNDEVASEDEATVKRLRREETHVCEKCCAEFFSISE FLEHKKNCTKNPPVLIMNDSEGPVPSEDFSGAVLSHQPTSPGS KDCHRENGGSSEDMKEKPDAESVVYLKTETALPPTPQDISYLA KGKVANTNVTLQALRGTKVAVNQRSADALPAPVPGANSIPWVL EQILCLQQQLQQIQLTEQIRIQVNMWASHALHSSGAGADTLK TLGSHMSQQVSAAVALLSQKAGSQGLSLDALKQAKLPHANIPS ATSSLSPGLAPFTLKPDGTRVLPNVMSRLPSALLPQAPGSVLF QSPFSTVALDTSKKGKGKPPNISAVDVKPKDEAALYKHKCKYC SKVFGTDSSLQIHLRSHTGERPFVCSVCGHRFTTKGNLKVHFH RHPQVKANPQLFAEFQDKVAAGNGIPYALSVPDPIDEPSLSLD SKPVLVTTSVGLPQNLSSGTNPKDLTGGSLPGDLQPGPSPESE GGPTLPGVGPNYNSPRAGGFQGSGTPEPGSETLKLQQLVENID KATTDPNECLICHRVLSCQSSLKMHYRTHTGERPFQCKICGRA FSTKGNLKTHLGVHRTNTSIKTQHSCPICQKKFTNAVMLQQHI RMHMGGQIPNTPLPENPCDFTGSEPMTVGENGSTGAICHDDVI ESIDVEEVSSQEAPSSSSKVPTPLPSIHSASPTLGFAMMASLD APGKVGPAPFNLQRQGSRENGSVESDGLTNDSSSLMGDQEYQS RSPDILETTSFQALSPANSQAESIKSKSPDAGSKAESSENSRT EMEGRSSLPSTFIRAPPTYVKVEVPGTFVGPSTLSPGMTPLLA AQPRRQAKQHGCTRCGKNFSSASALQIHERTHTGEKPFVCNIC GRAFTTKGNLKVHYMTHGANNNSARRGRKLAIENTMALLGTDG KRVSEIFPKEILAPSVNVDPVVWNQYTSMLNGGLAVKTNEISV IQSGGVPTLPVSLGATSVVNNATVSKMDGSQSGISADVEKPSA TDGVPKHQFPHFLEENKIAVS | 51 |
| ELF1 | MAAVVQQNDLVFEFASNVMEDERQLGDPAIFPAVIVEHVPGAD ILNSYAGLACVEEPNDMITESSLDVAEEEIIDDDDDDITLTVE ASCHDGDETIETIEAAEALLNMDSPGPMLDEKRINNNIFSSPE DDMVVAPVTHVSVTLDGIPEVMETQQVQEKYADSPGASSPEQP KRKKGRKTKPPRPDSPATTPNISVKKKNKDGKGNTIYLWEFLL ALLQDKATCPKYIKWTQREKGIFKLVDSKAVSRLWGKHKNKPD MNYETMGRALRYYYQRGILAKVEGQRLVYQFKEMPKDLIYIND EDPSSSIESSDPSLSSSATSNRNQTSRSRVSSSPGVKGGATTV LKPGNSKAAKPKDPVEVAQPSEVLRTVQPTQSPYPTQLFRTVH | 52 |
| | VVQPVQAVPEGEAARTSTMQDETLNSSVQSIRTIQAPTQVPVV VSPRNQQLHTVTLQTVPLTTVIASTDPSAGTGSQKFILQAIPS SQPMTVLKENVMLQSQKAGSPPSIVLGPAQVQQVLTSNVQTIC NGTVSVASSPSFSATAPVVTFSPRSSQLVAHPPGTVITSVIKT QETKTLTQEVEKKESEDHLKENTEKTEQQPQPYVMVVSSSNGF TSQVAMKQNELLEPNSF | |
| SOX9 | | 53 |
| SOX12 | MVQQRGARAKRDGGPPPPGPGPAEEGAREPGWCKTPSGHIKRP MNAFMVWSQHERRKIMDQWPDMHNAEISKRLGRRWQLLQDSEK IPFVREAERLRLKHMADYPDYKYRPRKKSKGAPAKARPRPPGG SGGGSRLKPGPQLPGRGGRRAAGGPLGGGAAAPEDDDEDDDEE LLEVRLVETPGRELWRMVPAGRAARGQAERAQGPSGEGAAAAA AASPTPSEDEEPEEEEEEAAAAEEGEEETVASGEESLGFLSRL PPGPAGLDCSALDRDPDLQPPSGTSHFEFPDYCTPEVTEMIAG DWRPSSIADLVFTY | 54 |
| SOX18 | | 55 |
| ETS1 | MKAAVDLKPTLTIIKTEKVDLELFPSPDMECADVPLLTPSSKE MMSQALKATFSGFTKEQQRLGIPKDPRQWTETHVRDWVMWAVN EFSLKGVDFQKFCMNGAALCALGKDCFLELAPDFVGDILWEHL EILQKEDVKPYQVNGVNPAYPESRYTSDYFISYGIEHAQCVPP SEFSEPSFITESYQTLHPISSEELLSLKYENDYPSVILRDPLQ TDTLQNDYFAIKQEVVTPDNMCMGRTSRGKLGGQDSFESIESY DSCDRLTQSWSSQSSFNSLQRVPSYDSFDSEDYPAALPNHKPK GTFKDYVRDRADLNKDKPVIPAAALAGYTGSGPIQLWQFLLEL LTDKSCQSFISWTGDGWEFKLSDPDEVARRWGKRKNKPKMNYE KLSRGLRYYYDKNIIHKTAGKRYVYRFVCDLQSLLGYTPEELH AMLDVKPDADE | 56 |
| PAX3 | MTTLAGAVPRMMRPGPGQNYPRSGFPLEVSTPLGQGRVNQLGG VFINGRPLPNHIRHKIVEMAHHGIRPCVISRQLRVSHGCVSKI LCRYQETGSIRPGAIGGSKPKQVTTPDVEKKIEEYKRENPGMF SWEIRDKLLKDAVCDRNTVPSVSSISRILRSKFGKGEEEEADL ERKEAEESEKKAKHSIDGILSERGKALVSGVSSH | 57 |
| PAX8 | | 58 |
| | | |
| GLI1 | MFNSMTPPPISSYGEPCCLRPLPSQGAPSVGTEGLSGPPFCHQ ANLMSGPHSYGPARETNSCTEGPLFSSPRSAVKLTKKRALSIS PLSDASLDLQTVIRTSPSSLVAFINSRCTSPGGSYGHLSIGTM SPSLGFPAQMNHQKGPSPSFGVQPCGPHDSARGGMIPHPQSRG PFPTCQLKSELDMLVGKCREEPLEGDMSSPNSTGIQDPLLGML DGREDLEREEKREPESVYETDCRWDGCSQEFDSQEQLVHHINS EHIHGERKEFVCHWGGCSRELRPFKAQYMLVVHMRRHTGEKPH KCTFEGCRKSYSRLENLKTHLRSHTGEKPYMCEHEGCSKAFSN ASDRAKHQNRTHSNEKPYVCKLPGCTKRYTDPSSLRKHVKTVH GPDAHVTKRHRGDGPLPRAPSISTVEPKREREGGPIREESRLT VPEGAMKPQPSPGAQSSCSSDHSPAGSAANTDSGVEMTGNAGG STEDLSSLDEGPCIAGTGLSTLRRLENLRLDQLHQLRPIGTRG LKLPSLSHTGTTVSRRVGPPVSLERRSSSSSSISSAYTVSRRS SLASPFPPGSPPENGASSLPGLMPAQHYLLRARYASARGGGTS PTAASSLDRIGGLPMPPWRSRAEYPGYNPNAGVTRRASDPAQA ADRPAPARVQRFKSLGCVHTPPTVAGGGQNFDPYLPTSVYSPQ PPSITENAAMDARGLQEEPEVGTSMVGSGLNPYMDFPPTDTLG YGGPEGAAAEPYGARGPGSLPLGPGPPTNYGPNPCPQQASYPD PTQETWGEFPSHSGLYPGPKALGGTYSQCPRLEHYGQVQVKPE QGCPVGSDSTGLAPCLNAHPSEGPPHPQPLFSHYPQPSPPQYL QSGPYTQPPPDYLPSEPRPCLDFDSPTHSTGQLKAQLVCNYVQ SQQELLWEGGGREDAPAQEPSYQSPKFLGGSQVSPSRAKAPVN TYGPGFGPNLPNHKSGSYPTPSPCHENFVVGANRASHRAAAPP RLLPPLPTCYGPLKVGGTNPSCGHPEVGRLGGGPALYPPPEGQ VCNPLDSLDLDNTQLDFVAILDEPQGLSPPPSHDQRGSSGHTP PPSGPPNMAVGNMSVLLRSLPGETEFLNSSA | 59 |
| GLI2 | METSASATASEKQEAKSGILEAAGFPDPGKKASPLVVAAAAAA AVAAQGVPQHLLPPFHAPLPIDMRHQEGRYHYEPHSVHGVHGP PALSGSPVISDISLIRLSPHPAGPGESPFNAPHPYVNPHMEHY LRSVHSSPTLSMISAARGLSPADVAQEHLKERGLFGLPAPGTT PSDYYHQMTLVAGHPAPYGDLLMQSGGAASAPHLHDYLNPVDV SRFSSPRVTPRLSRKRALSISPLSDASLDLQRMIRTSPNSLVA YINNSRSSSAASGSYGHLSAGALSPAFTFPHPINPVAYQQILS QQRGLGSAFGHTPPLIQPSPTFLAQQPMALTSINATPTQLSSS SNCLSDTNQNKQSSESAVSSTVNPVAIHKRSKVKTEPEGLRPA SPLALTQGQVSGHGSCGCALPLSQEQLADLKEDLDRDDCKQEA EVVIYETNCHWEDCTKEYDTQEQLVHHINNEHIHGEKKEFVCR WQACTREQKPFKAQYMLVVHMRRHTGEKPHKCTFEGCSKAYSR LENLKTHLRSHTGEKPYVCEHEGCNKAFSNASDRAKHQNRTHS NEKPYICKIPGCTKRYTDPSSLRKHVKTVHGPDAHVTKKQRND VHLRTPLLKENGDSEAGTEPGGPESTEASSTSQAVEDCLHVRA IKTESSGLCQSSPGAQSSCSSEPSPLGSAPNNDSGVEMPGTGP GSLGDLTALDDTPPGADTSALAAPSAGGLQLRKHMTTMHRFEQ LKKEKLKSLKDSCSWAGPTPHTRNTKLPPLPGSGSILENFSGS GGGGPAGLLPNPRLSELSASEVTMLSQLQERRDSSTSTVSSAY TVSRRSSGISPYFSSRRSSEASPLGAGRPHNASSADSYDPIST DASRRSSEASQCSGGSGLLNLTPAQQYSLRAKYAAATGGPPPT PLPGLERMSLRTRLALLDAPERTLPAGCPRPLGPRRGSDGPTY | 60 |
| | GHGHAGAAPAFPHEAPGGARRASDPVRRPDALSLPRVQRFHS THNVNPGPLPPCADRRGLRLQSHPSTDGGLARGAYSPRPPSIS ENVAMEAVAAGVDGAGPEADLGLPEDDLVLPDDVVQYIKAHAS GALDEGTGQVYPTESTGFSDNPRLPSPGLHGQRRMVAADSNVG PSAPMLGGCQLGFGAPSSLNKNNMPVQWNEVSSGTVDALASQV KPPPFPQGNLAVVQQKPAFGQYPGYSPQGLQASPGGLDSTQPH LQPRSGAPSQGIPRVNYMQQLRQPVAGSQCPGMTTTMSPHACY GQVHPQLSPSTISGALNQFPQSCSNMPAKPGHLGHPQQTEVAP DPTTMGNRHRELGVPDSALAGVPPPHPVQSYPQQSHHLAASMS QEGYHQVPSLLPARQPGFMEPQTGPMGVATAGFGLVQPRPPLE PSPTGRHRGVRAVQQQLAYARATGHAMAAMPSSQETAEAVPKG AMGNMGSVPPQPPPQDAGGAPDHSMLYYYGQIHMYEQDGGLEN LGSCQVMRSQPPQPQACQDSIQPQPLPSPGVNQVSSTVDSQLL EAPQIDFDAIMDDGDHSSLFSGALSPSLLHSLSQNSSRLTTPR NSLTLPSIPAGISNMAVGDMSSMLTSLAEESKFLNMMT | |
| GLI3 | | 61 |
| ETV1 | MDGFYDQQVPYMVTNSQRGRNCNEKPTNVRKRKFINRDLAHDS EELFQDLSQLQETWLAEVAFHGLPLKIKKEPHSPCSEISSACS QEQPFKFSYGEKCLYNVSAYDQKPQVGMRPSNPPTPSSTPVSP | 62 |
| | LHHASPNSTHTPKPDRAFPAHLPPSQSIPDSSYPMDHRFRRQL SEPCNSFPPLPTMPREGRPMYQRQMSEPNIPFPPQGFKQEYHD PVYEHNTMVGSAASQSFPPPLMIKQEPRDFAYDSEVPSCHSIY MRQEGFLAHPSRTEGCMFEKGPRQFYDDTCVVPEKFDGDIKQE PGMYREGPTYQRRGSLQLWQFLVALLDDPSNSHFIAWTGRGME FKLIEPEEVARRWGIQKNRPAMNYDKLSRSLRYYYEKGIMQKV AGERYVYKFVCDPEALFSMAFPDNQRPLLKTDMERHINEEDTV PLSHFDESMAYMPEGGCCNPHPYNEGYVY | |
| ETV2 | MDLWNWDEASPQEVPPGNKLAGLEGAKLGFCFPDLALQGDTPT ATAETCWKGPIQLWQFLLELLHDGARSSCIRWTGNSREFQLCD PKEVARLWGERKRKPGMNYEKLSRGLRYYYRRDIVRKSGGRKY TYRFGGRVPSLAYPDCAGGGRGAETQ | 63 |
| ETV3 | MKAGCSIVEKPEGGGGYQFPDWAYKTESSPGSRQIQLWHFILE LLQKEEFRHVIAWQQGEYGEFVIKDPDEVARLWGRRKCKPQMN YDKLSRALRYYYNKRILHKTKGKRFTYKFNFNKLVMPNYPFIN IRSSGKIQTLLVGN | 64 |
| RUNX1 | | 65 |
| RUNX2 | MASNSLFSTVTPCQQNFFWDPSTSRRFSPPSSSLQPGKMSDVS PVVAAQQQQQQQQQQQQQQQQQQQQQEAAAAAAAAAAAAA AAAVPRLRPPHDNRTMVEIIADHPAELVRTDSPNFLCSVLPSH WRCNKTLPVAFKVVALGEVPDGTVVTVMAGNDENYSAELRNAS AVMKNQVARFNDLRFVGRSGRGKSFTLTITVFTNPPQVATYHR AIKVTVDGPREPRRHRQKLDDSKPSLFSDRLSDLGRIPHPSMR VGVPPQNPRPSLNSAPSPFNPQGQSQITDPRQAQSSPPWSYDQ SYPSYLSQMTSPSIHSTTPLSSTRGTGLPAITDVPRRISGASE LGPFSDPRQFPSISSLTESRFSNPRMHYPATFTYTPPVTSGMS LGMSATTHYHTYLPPPYPGSSQSQSGPFQTSSTPYLYYGTSSG SYQFPMVPGGDRSPSRMLPPCTTTSNGSTLLNPNLPNQNDGVD ADGSHSSSPTVLNSSGRMDESVWRPY | 66 |
| RUNX3 | MRIPVDPSTSRRFTPPSPAFPCGGGGGKMGENSGALSAQAAVG PGGRARPEVRSMVDVLADHAGELVRTDSPNFLCSVLPSHWRCN KTLPVAFKVVALGDVPDGTVVTVMAGNDENYSAELRNASAVMK NQVARFNDLRFVGRSGRGKSFTLTITVFTNPTQVATYHRAIKV TVDGPREPRRHRQKLEDQTKPFPDRFGDLERLRMRVTPSTPSP RGSLSTTSHFSSQPQTPIQGTSELNPFSDPRQFDRSFPTLPTL TESRFPDPRMHYPGAMSAAFPYSATPSGTSISSLSVAGMPATS RFHHTYLPPPYPGAPQNQSGPFQANPSPYHLYYGTSSGSYQFS MVAGSSSGGDRSPTRMLASCTSSAASVAAGNLMNPSLGGQSDG VEADGSHSNSPTALSTPGRMDEAVWRPY | 67 |
| MAFB | | 68 |
| | | |
| TFAP2C | MLWKITDNVKYEEDCEDRHDGSSNGNPRVPHLSSAGQHLYSPA PPLSHTGVAEYQPPPYFPPPYQQLAYSQSADPYSHLGEAYAAA INPLHQPAPTGSQQQAWPGRQSQEGAGLPSHHGRPAGLLPHLS GLEAGAVSARRDAYRRSDLLLPHAHALDAAGLAENLGLHDMPH QMDEVQNVDDQHLLLHDQTVIRKGPISMTKNPLNLPCQKELVG AVMNPTEVFCSVPGRLSLLSSTSKYKVTVAEVQRRLSPPECLN ASLLGGVLRRAKSKNGGRSLREKLDKIGLNLPAGRRKAAHVTL LTSLVEGEAVHLARDFAYVCEAEFPSKPVAEYLTRPHLGGRNE MAARKNMLLAAQQLCKEFTELLSQDRTPHGTSRLAPVLETNIQ NCLSHFSLITHGFGSQAICAAVSALQNYIKEALIVIDKSYMNP GDQSPADSNKTLEKMEKHRK | 69 |
| E2F1 | MALAGAPAGGPCAPALEALLGAGALRLLDSSQIVIISAAQDAS APPAPTGPAAPAAGPCDPDLLLFATPQAPRPTPSAPRPALGRP PVKRRLDLETDHQYLAESSGPARGRGRHPGKGVKSPGEKSRYE TSLNLTTKRFLELLSHSADGVVDLNWAAEVLKVQKRRIYDITN VLEGIQLIAKKSKNHIQWLGSHTTVGVGGRLEGLTQDLRQLQE SEQQLDHLMNICTTQLRLLSEDTDSQRLAYVTCQDLRSIADPA EQMVMVIKAPPETQLQAVDSSENFQISLKSKQGPIDVFLCPEE TVGGISPGKTPSQEVTSEEENRATDSATIVSPPPSSPPSSLTT DPSQSLLSLEQEPLLSRMGSLRAPVDEDRLSPLVAADSLLEHV REDFSGLLPEEFISLSPPHEALDYHFGLEEGEGIRDLFDCDFG DLTPLDF | 70 |

Example nucleotide sequences encoding polypeptides comprising transcription factor DNA-binding domains are:

| | | |
|---|---|---|
| **TF** | **Sequence** | **SEQ ID NO** |
| SOX2 | ATGTACAACATGATGGAGACGGAGCTGAAGCCGCCGGGCCCGC AGCAAACTTCGGGGGGCGGCGGCGGCAACTCCACCGCGGCGGC GGCCGGCGGCAACCAGAAAAACAGCCCGGACCGCGTCAAGCGG CCCATGAATGCCTTCATGGTGTGGTCCCGCGGGCAGCGGCGCA AGATGGCCCAGGAGAACCCCAAGATGCACAACTCGGAGATCAG CAAGCGCCTGGGCGCCGAGTGGAAACTTTTGTCGGAGACGGAG AAGCGGCCGTTCATCGACGAGGCTAAGCGGCTGCGAGCGCTGC ACATGAAGGAGCACCCGGATTATAAATACCGGCCCCGGCGGAA AACCAAGACGCTCATGAAGAAGGATAAGTACACGCTGCCCGGC GGGCTGCTGGCCCCCGGCGGCAATAGCATGGCGAGCGGGGTCG GGGTGGGCGCCGGCCTGGGCGCGGGCGTGAACCAGCGCATGGA CAGTTACGCGCACATGAACGGCTGGAGCAACGGCAGCTACAGC ATGATGCAGGACCAGCTGGGC | 71 |
| MYC | ATCATCCAGGACTGTATGTGGAGCGGCTTCTCGGCCGCCGCCA AGCTCGTCTCAGAGAAGCTGGCCTCCTACCAGGCTGCGCGCAA AGACAGCGGCAGCCCGAACCCCGCCCGCGGCCACAGCGTCTGC TCCACCTCCAGCTTGTACCTGCAGGATCTGAGCGCCGCCGCCT CAGAGTGCATCGACCCCTCGGTGGTCTTCCCCTACCCTCTCAA CGACAGCAGCTCGCCCAAGTCCTGCGCCTCGCAAGACTCCAGC GCCTTCTCTCCGTCCTCGGATTCTCTGCTCTCCTCGACGGAGT CCTCCCCGCAGGGCAGCCCCGAGCCCCTGGTGCTCCATGAGGA GACACCGCCCACCACCAGCAGCGACTCTGAGGAGGAACAAGAA GATGAGGAAGAAATCGATGTTGTTTCTGTGGAAAAGAGGCAGG CTCCTGGCAAAAGGTCAGAGTCTGGATCACCTTCTGCTGGAGG | 72 |
| | CCACAGCAAACCTCCTCACAGCCCACTGGTCCTCAAGAGGTGC CACGTCTCCACACATCAGCACAACTACGCAGCGCCTCCCTCCA CTCGGAAGGACTATCCTGCTGCCAAGAGGGTCAAGTTGGACAG TGTCAGAGTCCTGAGACAGATCAGCAACAACCGAAAATGCACC AGCCCCAGGTCCTCGGACACCGAGGAGAATGTCAAGAGGCGAA CACACAACGTCTTGGAGCGCCAGAGGAGGAACGAGCTAAAACG GAGCTTTTTTGCCCTGCGTGACCAGATCCCGGAGTTGGAAAAC AATGAAAAGGCCCCCAAGGTAGTTATCCTTAAAAAAGCCACAG CATACATCCTGTCCGTCCAAGCAGAGGAGCAAAAGCTCATTTC TGAAGAGGACTTGTTGCGGAAACGACGAGAACAGTTGAAACAC AAACTTGAACAGCTACGGAACTCTTGTGCG | |
| MYCN | ATGCCGAGCTGCTCCACGTCCACCATGCCGGGCATGATCTGCA AGAACCCAGACCTCGAGTTTGACTCGCTACAGCCCTGCTTCTA CCCGGACGAAGATGACTTCTACTTCGGCGGCCCCGACTCGACC CCCCCGGGGGAGGACATCTGGAAGAAGTTTGAGCTGCTGCCCA CGCCCCCGCTGTCGCCCAGCCGTGGCTTCGCGGAGCACAGCTC CGAGCCCCCGAGCTGGGTCACGGAGATGCTGCTTGAGAACGAG CTGTGGGGCAGCCCGGCCGAGGAGGACGCGTTCGGCCTGGGGG GACTGGGTGGCCTCACCCCCAACCCGGTCATCCTCCAGGACTG CATGTGGAGCGGCTTCTCCGCCCGCGAGAAGCTGGAGCGCGCC GTGAGCGAGAAGCTGCAGCACGGCCGCGGGCCGCCAACCGCCG GTTCCACCGCCCAGTCCCCGGGAGCCGGCGCCGCCAGCCCTGC GGGTCGCGGGCACGGCGGGGCTGCGGGAGCCGGCCGCGCCGGG GCCGCCCTGCCCGCCGAGCTCGCCCACCCGGCCGCCGAGTGCG TGGATCCCGCCGTGGTCTTCCCCTTTCCCGTGAACAAGCGCGA GCCAGCGCCCGTGCCCGCAGCCCCGGCCAGTGCCCCGGCGGCG GGCCCTGCGGTCGCCTCGGGGGCGGGTATTGCCGCCCCAGCCG GGGCCCCGGGGGTCGCCCCTCCGCGCCCAGGCGGCCGCCAGAC CAGCGGCGGCGACCACAAGGCCCTCAGTACCTCCGGAGAGGAC ACCCTGAGCGATTCAGATGATGAAGATGATGAAGAGGAAGATG AAGAGGAAGAAATCGACGTGGTCACTGTGGAGAAGCGGCGTTC CTCCTCCAACACCAAGGCTGTCACCACATTCACCATCACTGTG CGTCCCAAGAACGCAGCCCTGGGTCCCGGGAGGGCTCAGTCCA GCGAGCTGATCCTCAAACGATGCCTTCCCATCCACCAGCAGCA CAACTATGCCGCCCCCTCTCCCTACGTGGAGAGTGAGGATGCA CCCCCACAGAAGAAGATAAAGAGCGAGGCGTCCCCACGTCCGC TCAAGAGTGTCATCCCCCCAAAGGCTAAGAGCTTGAGCCCCCG AAACTCTGACTCGGAGGACAGTGAGCGTCGCAGAAACCACAAC ATCCTGGAGCGCCAGCGCCGCAACGACCTTCGGTCCAGCTTTC TCACGCTCAGGGACCACGTGCCGGAGTTGGTAAAGAATGAGAA GGCCGCCAAGGTGGTCATTTTGAAAAAGGCCACTGAGTATGTC CACTCCCTCCAGGCCGAGGAGCACCAGCTTTTGCTGGAAAAGG AAAAATTGCAGGCAAGACAGCAGCAGTTGCTAAAGAAAATTGA ACACGCTCGGACTTGC | 73 |
| TEAD1 | ATTGAGCCCAGCAGCTGGAGCGGCAGTGAGAGCCCTGCCGAAA ACATGGAAAGGATGAGTGACTCTGCAGATAAGCCAATTGACAA TGATGCAGAAGGGGTCTGGAGCCCCGACATCGAGCAAAGCTTT CAGGAGGCCCTGGCTATCTATCCACCATGTGGGAGGAGGAAAA TCATCTTATCAGACGAAGGCAAAATGTATGGTAGGAATGAATT GATAGCCAGATACATCAAACTCAGGACAGGCAAGACGAGGACC AGAAAACAGGTGTCTAGTCACATTCAGGTTCTTGCCAGAAGGA AATCTCGTGATTTTCATTCCAAGCTAAAGGATCAGACTGCAAA GGATAAGGCCCTGCAGCACATGGCGGCCATGTCCTCAGCCCAG ATCGTCTCGGCCACTGCCATTCATAACAAGCTGGGGCTGCCTG GGATTCCACGCCCGACCTTCCCAGGGGCGCCGGGTTCTGGCC | 74 |
| | GGGAATGATTCAAACAGGGCAGCCA | |
| TEAD2 | ATGGGGGAACCCCGGGCTGGGGCCGCCCTGGACGATGGCAGCG GCTGGACGGGCAGTGAGGAAGGCAGTGAGGAGGGTACCGGCGG CAGTGAGGGGGCTGGGGGTGACGGGGGCCCGGATGCAGAGGGG GTGTGGAGCCCAGACATTGAGCAGAGCTTCCAGGAGGCCCTGG CCATCTATCCACCCTGCGGCCGCCGGAAAATAATTTTGTCTGA TGAAGGCAAGATGTATGGTCGGAATGAACTGATCGCCCGCTAC ATCAAGCTGAGAACGGGGAAGACCCGAACTCGAAAACAGGTTT CTAGTCACATCCAGGTTTTGGCCCGAAGGAAATCAAGGGAAAT CCAGTCCAAGTTGAAGGACCAGGTTTCCAAGGACAAGGCTTTC CAGACAATGGCAACCATGTCCTCTGCCCAGCTCATCTCCGCGC CTTCTCTGCAGGCCAAACTGGGTCCCACTGGTCCTCAGGCCTC TGAGCTTTTCCAGTTTTGGTCTGGAGGATCTGGCCCCCCTGG AATGTTCCAGATGTGAAGCCATTCTCACAGACACCGTTCACCT TGTCACTGACTCCCCCATCTACTGACCTCCCAGGGTACGAGCC CCCCCAAGCCCTCTCACCCCTGCCCCCACCTACCCCATCGCCC CCAGCCTGGCAGGCTCGGGGCCTGGGCACCGCCCGGTTGCAGC TGGTAGAGTTCTCAGCCTTCGTGGAACCGCCAGATGCAGTTGA TTCTTACCAGAGGCACCTGTTCGTGCACATCAGCCAGCACTGC CCCAGCCCCGGAGCGCCGCCGCTCGAGAGTGTGGACGTCCGGC AGATCTACGACAAATTCCCTGAGAAAAAGGGTGGCCTCCGAGA GCTATATGATCGTGGCCCCCCCCATGCCTTCTTCCTGGTCAAG TTCTGGGCGGACCTGAACTGGGGCCCAAGTGGTGAGGAGGCAG GGGCCGGTGGCAGCATCAGCAGTGGTGGCTTCTACGGAGTGAG CAGCCAGTATGAGAGCCTGGAACACATGACCCTCACCTGTTCC TCCAAGGTCTGCTCTTTTGGCAAGCAGGTGGTGGAGAAGGTGG AGACGGAACGGGCCCAGCTGGAGGACGGCAGATTTGTGTACCG CCTGCTGCGCTCGCCCATGTGCGAGTACCTGGTGAATTTCTTG CACAAGTTGCGGCAGCTGCCTGAGCGATACATGATGAACAGCG TCCTGGAAAACTTCACCATCCTCCAGGTGGTGACAAACAGAGA CACCCAGGAACTGCTGCTCTGCACCGCCTATGTCTTCGAGGTC TCCACCAGCGAGCGTGGGGCCCAGCATCACATTTACCGCCTGG TCAGGGAC | 75 |
| TEAD3 | ATAGCGTCCAACAGCTGGAACGCCAGCAGCAGCCCCGGGGAGG CCCGGGAGGATGGGCCCGAGGGCCTGGACAAGGGGCTGGACAA CGATGCGGAGGGCGTGTGGAGCCCGGACATCGAGCAGAGCTTC CAGGAGGCCCTGGCCATCTACCCGCCCTGCGGCCGGCGGAAGA TCATCCTGTCAGACGAGGGCAAGATGTACGGCCGAAATGAGTT GATTGCACGCTATATTAAACTGAGGACGGGGAAGACTCGGACG AGAAAACAGGTGTCCAGCCACATACAGGTTCTAGCTCGGAAGA AGGTGCGGGAGTACCAGGTTGGCATCAAGGCCATGAACCTGGA CCAGGTCTCCAAGGACAAAGCCCTTCAGAGCATGGCGTCCATG TCCTCTGCCCAGATCGTCTCTGCCAGTGTCCTGCAGAACAAGT TCAGCCCACCTTCCCCTCTGCCCCAGGCCGTCTTCTCCACTTC CTCGCGGTTCTGGAGCAGCCCCCCTCTCCTGGGACAGCAGCCT GGACCCTCTCAGGACATCAAGCCCTTTGCACAGCCAGCCTACC CCATCCAGCCGCCCCTGCCGCCGACGCTCAGCAGTTATGAGCC CCTGGCCCCGCTCCCCTCAGCTGCTGCCTCTGTGCCTGTGTGG CAGGACCGTACCATTGCCTCCTCCCGGCTGCGGCTCCTGGAGT ATTCAGCCTTCATGGAGGTGCAGCGAGACCCTGACACGTACAG CAAACACCTGTTTGTGCACATCGGCCAGACGAACCCCGCCTTC TCAGACCCACCCCTGGAGGCAGTAGATGTGCGCCAGATCTATG ACAAATTCCCCGAGAAAAAGGGAGGATTGAAGGAGCTCTATGA GAAGGGGCCCCCTAATGCCTTCTTCCTTGTCAAGTTCTGGGCC GACCTCAACAGCACCATCCAGGAGGGCCCGGGAGCCTTCTATG | 76 |
| | GGGTCAGCTCTCAGTACAGCTCTGCTGATAGCATGACCATCAG CGTCTCCACCAAGGTGTGCTCCTTTGGCAAACAGGTGGTAGAG AAGGTGGAGACTGAGTATGCCAGGCTGGAGAACGGCGCTTTG TGTACCGTATCCACCGCTCGCCCATGTGCGAGTACATGATCAA CTTCATCCACAAGCTGAAGCACCTGCCCGAGAAGTACATGATG AACAGCGTGCTGGAGAACTTCACCATCCTGCAGGTGGTCACGA GCCGGGACTCCCAGGAGACCCTGCTTGTCATTGCTTTTGTCTT CGAAGTCTCCACCAGTGAGCACGGGGCCCAGCACCATGTCTAC AAGCTCGTCAAAGAC | |
| TEAD4 | TTGGAGGGCACGGCCGGCACCATTACCTCCAACGAGTGGAGCT CTCCCACCTCCCCTGAGGGAGCACCGCCTCTGGGGCAGTCA GGCACTGGACAAGCCCATCGACAATGACGCAGAGGGCGTGTGG AGCCCGGATATTGAGCAGAGTTTCCAGGAGGCCCTCGCCATCT ACCCGCCCTGTGGCAGGCGCAAAATCATCCTGTCGGACGAGGG CAAGATGTATGGTCGGAACGAGCTGATTGCCCGCTACATCAAG CTCCGGACAGGGAAGACCCGCACCAGGAAGCAGGTCTCCAGCC ACATCCAGGTGCTGGCTCGTCGCAAAGCTCGCGAGATCCAGGC CAAGCTAAAGTTTTGGCAAGGAGCTTTGCCAGGCCAAGCCGGA ACGTCCCATGATGTGAAGCCTTTCTCTCAGCAAACCTATGCTG TCCAGCCTCCGCTGCCTCTGCCAGGGTTTGAGTCTCCTGCAGG GCCCGCCCCATCGCCCTCTGCGCCCCGGCACCCCCATGGCAG GGCCGCAGCGTGGCCAGCTCCAAGCTCTGGATGTTGGAGTTCT CTGCCTTCCTGGAGCAGCAGCAGGACCCGGACACGTACAACAA GCACCTGTTCGTGCACATTGGCCAGTCCAGCCCAAGCTACAGC GACCCCTACCTCGAAGCCGTGGACATCCGCCAAATCTATGACA AATTCCCGGAGAAAAAGGGTGGACTCAAGGATCTCTTCGAACG GGGACCCTCCAATGCCTTTTTTCTTGTGAAGTTCTGGGCAGAC CTCAACACCAACATCGAGGATGAAGGCAGCTCCTTCTATGGGG TCTCCAGCCAGTATGAGAGCCCCGAGAACATGATCATCACCTG CTCCACGAAGGTCTGCTCTTTCGGCAAGCAGGTGGTGGAGAAA GTTGAGACAGAGTATGCTCGCTATGAGAATGGACACTACTCTT ACCGCATCCACCGGTCCCCGCTCTGTGAGTACATGATCAACTT CATCCACAAGCTCAAGCACCTCCCTGAGAAGTACATGATGAAC AGCGTGCTGGAGAACTTCACCATCCTGCAGGTGGTCACCAACA GAGACACACAGGAGACCTTGCTGTGCATTGCCTATGTCTTTGA GGTGTCAGCCAGTGAGCACGGGGCTCAGCACCACATCTACAGG CTGGTGAAAGAA | 77 |
| FOXA1 | ATGTTAGGAACTGTGAAGATGGAAGGGCATGAAACCAGCGACT GGAACAGCTACTACGCAGACACGCAGGAGGCCTACTCCTCCGT CCCGGTCAGCAACATGAACTCAGGCCTGGGCTCCATGAACTCC ATGAACACCTACATGACCATGAACACCATGACTACGAGCGGCA ACATGACCCCGGCGTCCTTCAACATGTCCTATGCCAACCCGGG CCTAGGGGCCGGCCTGAGTCCCGGCGCAGTAGCCGGCATGCCG GGGGGCTCGGCGGGCGCCATGAACAGCATGACTGCGGCCGGCG TGACGGCCATGGGTACGGCGCTGAGCCCGAGCGGCATGGGCGC CATGGGTGCGCAGCAGGCGGCCTCCATGAATGGCCTGGGCCCC TACGCGGCCGCCATGAACCCGTGCATGAGCCCCATGGCGTACG CGCCGTCCAACCTGGGCCGCAGCCGCGCGGGCGGCGGCGGCGA CGCCAAGACGTTCAAGCGCAGCTACCCGCACGCCAAGCCGCCC TACTCGTACATCTCGCTCATCACCATGGCCATCCAGCAGGCGC CCAGCAAGATGCTCACGCTGAGCGAGATCTACCAGTGGATCAT GGACCTCTTCCCCTATTACCGGCAGAACCAGCAGCGCTGGCAG AACTCCATCCGCCACTCGCTGTCCTTCAATGACTGCTTCGTCA AGGTGGCACGCTCCCCGGACAAGCCGGGCAAGGGCTCCTACTG GACGCTGCACCCGGACTCCGGCAACATGTTCGAGAACGGCTGC | 78 |
| | TACTTGCGCCGCCAGAAGCGCTTCAAGTGCGAGAAGCAGCCGG GGGCCGGCGGCGGGGGCGGAGCGGAAGCGGGGGCAGCGGCGC CAAGGGCGGCCCTGAGAGCCGCAAGGACCCCTCTGGCGCCTCT AACCCCAGCGCCGACTCGCCCCTCCATCGGGGTGTGCACGGGA AGACCGGCCAGCTAGAGGGCGCGCCGGCCCCCGGGCCCGCCGC CAGCCCCCAGACTCTGGACCACAGTGGGGCGACGGCGACAGGG GGCGCCTCGGAGTTGAAGACTCCAGCCTCCTCAACTGCGCCCC CCATAAGCTCCGGGCCCGGGGCGCTGGCCTCTGTGCCCGCCTC TCACCCGGCACACGGCTTGGCACCCCACGAGTCCCAGCTGCAC CTGAAAGGGGACCCCCACTACTCCTTCAACCACCCGTTCTCCA TCAACAACCTCATGTCCTCCTCGGAGCAGCAGCATAAGCTGGA CTTCAAGGCATACGAACAGGCACTGCAATACTCGCCTTACGGC TCTACGTTGCCCGCCAGCCTGCCTCTAGGCAGCGCCTCGGTGA CCACCAGGAGCCCCATCGAGCCCTCAGCCCTGGAGCCGGCGTA CTACCAAGGTGTGTATTCCAGACCCGTCCTAAACACTTCCTAG | |
| FOXA2 | ATGCACTCGGCTTCCAGTATGCTGGGAGCGGTGAAGATGGAAG GGCACGAGCCGTCCGACTGGAGCAGCTACTATGCAGAGCCCGA GGGCTACTCCTCCGTGAGCAACATGAACGCCGGCCTGGGGATG AACGGCATGAACACGTACATGAGCATGTCGGCGGCCGCCATGG GCAGCGGCTCGGGCAACATGAGCGCGGGCTCCATGAACATGTC GTCGTACGTGGGCGCTGGCATGAGCCCGTCCCTGGCGGGGATG TCCCCCGGCGCGGGCGCCATGGCGGGCATGGGCGGCTCGGCCG GGGCGGCCGGCGTGGCGGGCATGGGGCCGCACTTGAGTCCCAG CCTGAGCCCGCTCGGGGGGCAGGCGGCCGGGGCCATGGGCGGC CTGGCCCCCTACGCCAACATGAACTCCATGAGCCCCATGTACG GGCAGGCGGGCCTGAGCCGCGCCCGCGACCCCAAGACCTACAG GCGCAGCTACACGCACGCAAAGCCGCCCTACTCGTACATCTCG CTCATCACCATGGCCATCCAGCAGAGCCCCAACAAGATGCTGA CGCTGAGCGAGATCTACCAGTGGATCATGGACCTCTTCCCCTT CTACCGGCAGAACCAGCAGCGCTGGCAGAACTCCATCCGCCAC TCGCTCTCCTTCAACGACTGTTTCCTGAAGGTGCCCCGCTCGC CCGACAAGCCCGGCAAGGGCTCCTTCTGGACCCTGCACCCTGA CTCGGGCAACATGTTCGAGAACGGCTGCTACCTGCGCCGCCAG AAGCGCTTCAAGTGCGAGAAGCAGCTGGCGCTGAAGGAGGCCG CAGGCGCCGCCGGCAGCGGCAAGAAGGCGGCCGCCGGAGCCCA GGCCTCACAGGCTCAACTCGGGGAGGCCGCCGGGCCGGCCTCC GAGACTCCGGCGGGCACCGAGTCGCCTCACTCGAGCGCCTCCC CGTGCCAGGAGCACAAGCGAGGGGGCCTGGGAGAGCTGAAGGG GACGCCGGCTGCGGCGCTGAGCCCCCCAGAGCCGGCGCCCTCT CCCGGGCAGCAGCAGCAGGCCGCGGCCCACCTGCTGGGCCCGC CCCACCACCCGGGCCTGCCGCCTGAGGCCCACCTGAAGCCGGA ACACCACTACGCCTTCAACCACCCGTTCTCCATCAACAACCTC ATGTCCTCGGAGCAGCAGCACCACCACAGCCACCACCACCACC AACCCCACAAAATGGACCTCAAGGCCTACGAACAGGTGATGCA CTACCCCGGCTACGGTTCCCCCATGCCTGGCAGCTTGGCCATG GGCCCGGTCACGAACAAAACGGGCCTGGACGCCTCGCCCCTGG CCGCAGATACCTCCTACTACCAGGGGGTGTACTCCCGGCCCAT TATGAACTCCTCT | 79 |
| ELK1 | ATGGACCCATCTGTGACGCTGTGGCAGTTTCTGCTGCAGCTGC TGAGAGAGCAAGGCAATGGCCACATCATCTCCTGGACTTCACG GGATGGTGGTGAATTCAAGCTGGTGGATGCAGAGGAGGTGGCC CGGCTGTGGGGGCTACGCAAGAACAAGACCAACATGAATTACG ACAAGCTCAGCCGGGCCTTGCGGTACTACTATGACAAGAACAT CATCCGCAAGGTGAGCGGCCAGAAGTTCGTCTACAAGTTTGTG TCCTACCCTGAGGTCGCAGGGTGCTCCACTGAGGACTGCCCGC | 80 |
| | CCCAGCCAGAGGTGTCTGTTACCTCCACCATGCCAAATGTGGC CCCTGCTGCTATACATGCCGCCCCAGGGACACTGTCTCTGGA AAGCCAGGCACACCCAAGGGTGCAGGAATGGCAGGCCCAGGCG GTTTGGCACGCAGCAGCCGGAACGAGTACATGCGCTCGGGCCT CTATTCCACCTTCACCATCCAGTCTCTGCAGCCGCAGCCACCC CCTCATCCTCGGCCTGCTGTGGTGCTCCCCAGTGCAGCTCCTG CAGGGGCAGCAGCGCCCCCCTCGGGGAGCAGGAGCACCAGTCC AAGCCCCTTGGAGGCCTGTCTGGAGGCTGAAGAGGCCGGCTTG CCTCTGCAGGTCATCCTGACCCCGCCCGAGGCCCCAAACCTGA AATCGGAAGAGCTTAATGTGGAGCCGGGTTTGGCCGGGCTTT GCCCCCAGAAGTGAAAGTAGAAGGGCCCAAGGAAGAGTTGGAA GTTGCGGGGGAGAGAGGGTTTGTGCCAGAAACCACCAAGGCCG AGCCAGAAGTCCCTCCACAGGAGGGCGTGCCAGCCCGGCTGCC CGCGGTTGTTATGGACACCGCAGGGCAGGCGGCGGCCATGCG GCTTCCAGCCCTGAGATCTCCCAGCCGCAGAAGGCCGGAAGC CCCGGGACCTAGAGCTTCCACTCAGCCCGAGCCTGCTAGGTGG GCCGGGACCCGAACGGACCCCAGGATCGGAAGTGGCTCCGGC CTCCAGGCTCCGGGGCCGGCGCTGACCCCATCCCTGCTTCCTA CGCATACATTGACCCCGGTGCTGCTGACACCCAGCTCGCTGCC TCCTAGCATTCACTTCTGGAGCACCCTGAGTCCCATTGCGCCC CGTAGCCCGGCCAAGCTCTCCTTCCAGTTTCCATCCAGTGGCA GCGCCCAGGTGCACATCCCTTCTATCAGCGTGGATGGCCTCTC GACCCCCGTGGTGCTCTCCCCAGGGCCCCAGAAGCCA | |
| ELK3 | ATGGAGAGTGCAATCACGCTGTGGCAGTTCCTGTTGCAGTTGC TGCTGGATCAGAAACATGAGCATTTGATCTGCTGGACCTCGAA CGATGGTGAATTCAAGCTCCTCAAAGCAGAAGAAGTGGCCAAG CTGTGGGGACTCCGAAAAAACAAAACAAATATGAACTATGATA AGCTGAGCAGAGCCCTGCGATACTATTATGACAAGAACATCAT CAAGAAGGTGATCGGGCAGAAGTTTGTGTACAAGTTTGTCTCT TTCCCGGAGATCCTGAAGATGGATCCTCACGCGGTGGAGATCA GCCGGGAGAGCCTTCTGCTGCAGGACAGCGACTGCAAGGCGTC TCCGGAGGGCCGCGAGGCCCACAAACACGGCCTGGCCGCCCTC AGAAGCACGAGCCGCAACGAATACATCCACTCAGGCCTGTACT CGTCCTTCACCATTAATTCCCTGCAGAACCCACCAGACGCCTT CAAGGCCATCAAGACGGAGAAGCTGGAGGAGCCGCCCGAAGAC AGCCCCCCCGTGGAAGAAGTCAGGACTGTGATCAGGTTTGTGA CCAATAAAACCGACAAGCACGTCACCAGGCCGGTGGTGTCCCT GCCTTCCACGTCAGAGGCTGCGGCGGCGTCCGCCTTCCTGGCC TCGTCCGTCTCGGCCAAGATCTCCTCTTTAATGTTGCCAAACG CTGCCAGTATTTCATCCGCCTCACCCTTCTCATCTCGGTCCCC GTCCCTGTCCCCCAACTCACCCCTCCCTTCTGAACACAGAAGC CTCTTCCTGGAGGCCGCCTGCCATGACTCCGATTCCCTGGAGC CCTTGAACCTGTCATCGGGCTCCAAGACCAAGTCTCCATCTCT TCCCCCAAAGGCCAAAAAACCCAAAGGCTTGGAAATCTCAGCG CCCCCGCTGGTGCTCTCCGGCACCGACATCGGCTCCATCGCCC TCAACAGCCCAGCCCTCCCCTCGGGATCCCTCACCCCAGCCTT CTTCACCGCACAGACACCAAATGGATTGCTTCTGACTCCGAGT CCACTGCTCTCCAGCATACATTTCTGGAGCAGCCTTAGTCCAG TTGCTCCGCTGAGTCCTGCCAGGCTGCAAGGCCAAGCACGCT GTTCCAGTTCCCCACACTGCTTAATGGCCACATGCCAGTGCCA ATCCCCAGTCTGGACAGAGCTGCTTCTCCAGTACTGCTTTCTT CAAACTCTCAGAAATCC | 81 |
| ELK4 | ATGGACAGTGCTATCACCCTGTGGCAGTTCCTTCTTCAGCTCC TGCAGAAGCCTCAGAACAAGCACATGATCTGTTGGACCTCTAA TGATGGGCAGTTTAAGCTTTTGCAGGCAGAAGAGGTGGCTCGT | 82 |
| | CTCTGGGGGATTCGCAAGAACAAGCCTAACATGAATTATGACA AACTCAGCCGAGCCCTCAGATACTATTATGTAAAGAATATCAT CAAAAAAGTGAATGGTCAGAAGTTTGTGTACAAGTTTGTCTCT TATCCAGAGATTTTGAACATGGATCCAATGACAGTGGGCAGGA TTGAGGGTGACTGTGAAAGTTTAAACTTCAGTGAAGTCAGCAG CAGTTCCAAAGATGTGGAGAATGGAGGGAAAGATAAACCACCT CAGCCTGGTGCCAAGACCTCTAGCCGCAATGACTACATACACT CTGGCTTATATTCTTCATTTACTCTCAACTCTTTGAACTCCTC CAATGTAAAGCTTTTCAAATTGATAAAGACTGAGAATCCAGCC GAGAAACTGGCAGAGAAAAAATCTCCTCAGGAGCCCACACCAT CTGTCATCAAATTTGTCACGACACCTTCCAAAAAGCCACCGGT TGAACCTGTTGCTGCCACCATTTCAATTGGCCCAAGTATTTCT CCATCTTCAGAAGAAACTATCCAAGCTTTGGAGACATTGGTTT CCCCAAAACTGCCTTCCCTGGAAGCCCCAACCTCTGCCTCTAA CGTAATGACTGCTTTTGCCACCACACCACCCATTTCGTCCATA CCCCCTTTGCAGGAACCTCCCAGAACACCTTCACCACCACTGA GTTCTCACCCAGACATCGACACAGACATTGATTCAGTGGCTTC TCAGCCAATGGAACTTCCAGAGAATTTGTCACTGGAGCCTAAA GACCAGGATTCAGTCTTGCTAGAAAAGGACAAAGTAAATAATT CATCAAGATCCAAGAAACCCAAAGGGTTAGAACTGGCACCCAC CCTTGTGATCACGAGCAGTGATCCAAGCCCACTGGGAATACTG AGCCCATCTCTCCCTACAGCTTCTCTTACACCAGCATTTTTT CACAGACACCCATCATACTGACTCCAAGCCCCTTGCTCTCCAG TATCCACTTCTGGAGTACTCTCAGTCCTGTTGCTCCCCTAAGT CCAGCCAGACTGCAAGGTGCTAACACACTTTTCCAGTTTCCTT CTGTACTGAACAGTCATGGGCCATTCACTCTGTCTGGGCTGGA TGGACCTTCCACCCCTGGCCCATTTTCCCCAGACCTACAGAAG ACA | |
| SRF | ATGTTACCGACCCAAGCTGGGGCCGCGGCGGCTCTGGCCGGG GCTCGGCCCTGGGGGGCAGCCTGAACCGGACCCCGACGGGGCG GCCGGGCGGCGGCGGCGGACACGCGGGGCTAACGGGGCCGG GTCCCCGGGAATGGCGCGGGGCTCGGGCCCGGCCGCCTGGAGC GGGAGGCTGCGGCAGCGGCGGCAACCACCCCGGCGCCCACCGC GGGGGCCCTCTACAGCGGCAGCGAGGGCGACTCGGAGTCGGGC GAGGAGGAGGAGCTGGGCGCCGAGCGGCGCGGCCTGAAGCGGA GCCTGAGCGAGATGGAGATCGGTATGGTGGTCGGTGGGCCCGA GGCGTCGGCAGCGGCCACCGGGGGCTACGGCCGGTGAGCGGC GCGGTGAGCGGGGCCAAGCCGGGTAAGAAGACCCGGGGCCGCG TGAAGATCAAGATGGAGTTCATCGACAACAAGCTGCGGCGCTA CACGACCTTCAGCAAGAGGAAGACGGGCATCATGAAGAAGGCC TATGAGCTGTCCACGCTGACAGGGACACAGGTGCTGTTGCTGG TGGCCAGTGAGACAGGCCATGTGTATACCTTTGCCACCCGAAA ACTGCAGCCCATGATCACCAGTGAGACCGGCAAGGCACTGATT CAGACCTGCCTCAACTCGCCAGACTCTCCACCCCGTTCAGACC CCACAACAGACCAGAGAATGAGTGCCACTGGCTTTGAAGAGAC AGATCTCACCTACCAGGTGTCGGAGTCTGACAGCAGTGGGGAG ACCAAGGACACACTGAAGCCGGCGTTCACAGTCACCAACCTGC CGGGTACAACCTCCACCATCCAAACAGCACCTAGCACCTCTAC CACCATGCAAGTCAGCAGCGGCCCCTCCTTTCCCATCACCAAC TACCTGGCACCAGTGTCTGCTAGTGTCAGCCCCAGTGCTGTCA GCAGTGCCAATGGGACTGTGCTGAAGAGTACAGGCAGCGGCCC TGTCTCCTCTGGGGGCCTTATGCAGCTGCCTACCAGCTTCACC CTCATGCCTGGTGGGGCAGTGGCCCAGCAGGTCCCAGTGCAGG CCATTCAAGTGCACCAGGCCCCACAGCAAGCGTCTCCCTCCCG TGACAGCAGCACAGACCTCACGCAGACCTCCTCCAGCGGGACA | 83 |
| | GTGACGCTGCCCGCCACCATCATGACGTCATCCGTGCCCACAA CTGTGGGTGGCCACATGATGTACCCTAGCCCGCATGCGGTGAT GTATGCCCCCACCTCGGCCTGGGTGATGGCAGCCTCACCGTG CTGAATGCCTTCTCCCAGGCACCATCCACCATGCAGGTGTCAC ACAGCCAGGTCCAGGAGCCAGGTGGCGTCCCCCAGGTGTTCCT GACAGCATCATCTGGGACAGTGCAGATCCCTGTTTCAGCAGTT CAGCTCCACCAGATGGCTGTGATAGGGCAGCAGGCCGGGAGCA GCAGCAACCTCACCGAGCTACAGGTGGTGAACCTGGACACCGC CCACAGCACCAAGAGTGAA | |
| FOXM1 | ATGAAAACTAGCCCCCGTCGGCCACTGATTCTCAAAAGACGGA GGCTGCCCCTTCCTGTTCAAAATGCCCAAGTGAAACATCAGA GGAGGAACCTAAGAGATCCCCTGCCCAACAGGAGTCTAATCAA GCAGAGGCCTCCAAGGAAGTGGCAGAGTCCAACTCTTGCAAGT TTCCAGCTGGGATCAAGATTATTAACCACCCCACCATGCCCAA CACGCAAGTAGTGGCCATCCCCAACAATGCTAATATTCACAGC ATCATCACAGCACTGACTGCCAAGGGAAAAGAGAGTGGCAGTA GTGGGCCCAACAAATTCATCCTCATCAGCTGTGGGGGAGCCCC AACTCAGCCTCCAGGACTCCGGCCTCAAACCCAAACCAGCTAT GATGCCAAAAGGACAGAAGTGACCCTGGAGACCTTGGACCAA AACCTGCAGCTAGGGATGTGAATCTTCCTAGACCACCTGGAGC CCTTTGCGAGCAGAAACGGAGACCTGTGCAGATGGTGAGGCA GCAGGCTGCACTATCAACAATAGCCTATCCAACATCCAGTGGC TTCGAAAGATGAGTTCTGATGGACTGGGCTCCCGCAGCATCAA GCAAGAGATGGAGGAAAAGGAGAATTGTCACCTGGAGCAGCGA CAGGTTAAGGTTGAGGAGCCTTCGAGACCATCAGCGTCCTGGC AGAACTCTGTGTCTGAGCGGCCACCCTACTCTTACATGGCCAT GATACAATTCGCCATCAACAGCACTGAGAGGAAGCGCATGACT TTGAAAGACATCTATACGTGGATTGAGGACCACTTTCCCTACT TTAAGCACATTGCCAAGCCAGGCTGGAAGAACTCCATCCGCCA CAACCTTTCCCTGCACGACATGTTTGTCCGGGAGACGTCTGCC AATGGCAAGGTCTCCTTCTGGACCATTCACCCCAGTGCCAACC GCTACTTGACATTGGACCAGGTGTTTAAGCCACTGGACCCAGG GTCTCCACAATTGCCCGAGCACTTGGAATCACAGCAGAAACGA CCGAATCCAGAGCTCCGCCGGAACATGACCATCAAAACCGAAC TCCCCCTGGGCGCACGGCGGAAGATGAAGCCACTGCTACCACG GGTCAGCTCATACCTGGTACCTATCCAGTTCCCGGTGAACCAG TCACTGGTGTTGCAGCCCTCGGTGAAGGTGCCATTGCCCCTGG CGGCTTCCCTCATGAGCTCAGAGCTTGCCCGCCATAGCAAGCG AGTCCGCATTGCCCCCAAGGTTTTGGGGAACAGGTGGTGTTT GGTTACATGAGTAAGTTCTTTAGTGGCGATCTGCGAGATTTTG GTACACCCATCACCAGCTTGTTTAATTTATCTTTCTTTGTTT ATCAGTGCTGCTAGCTGAGGAGGGGATAGCTCCTCTTTCTTCT GCAGGACCAGGGAAAGAGGAGAAACTCCTGTTTGGAGAAGGGT TTTCTCCTTTGCTTCCAGTTCAGACTATCAAGGAGGAAGAAAT CCAGCCTGGGGAGGAAATGCCACACTTAGCGAGACCCATCAAA GTGGAGAGCCCTCCCTTGGAAGAGTGGCCCTCCCCGGCCCCAT CTTTCAAAGAGGAATCATCTCACTCCTGGGAGGATTCGTCCCA ATCTCCCACCCCAAGACCCAAGAAGTCCTACAGTGGGCTTAGG TCCCCAACCCGGTGTGTCTCGGAAATGCTTGTGATTCAACACA GGGAGAGGAGGGAGAGGAGCCGGTCTCGGAGAAAACAGCATCT ACTGCCTCCCTGTGTGGATGAGCCGGAGCTGCTCTTCTCAGAG GGGCCCAGTACTTCCCGCTGGGCCGCAGAGCTCCCGTTCCCAG CAGACTCCTCTGACCCTGCCTCCCAGCTCAGCTACTCCCAGGA AGTGGGAGGACCTTTTAAGACACCCATTAAGGAAACGCTGCCC ATCTCCTCCACCCCGAGCAAATCTGTCCTCCCCAGAACCCCTG | 84 |
| | AATCCTGGAGGCTCACGCCCCCAGCCAAAGTAGGGGACTGGA TTTCAGCCCAGTACAAACCTCCCAGGGTGCCTCTGACCCCTTG CCTGACCCCCTGGGGCTGATGGATCTCAGCACCACTCCCTTGC AAAGTGCTCCCCCCCTTGAATCACCGCAAAGGCTCCTCAGTTC AGAACCCTTAGACCTCATCTCCGTCCCCTTTGGCAACTCTTCT CCCTCAGATATAGACGTCCCCAAGCCAGGCTCCCCGGAGCCAC AGGTTTCTGGCCTTGCAGCCAATCGTTCTCTGACAGAAGGCCT GGTCCTGGACACAATGAATGACAGCCTCAGCAAGATCCTGCTG GACATCAGCTTTCCTGGCCTGGACGAGGACCCACTGGGCCCTG ACAACATCAACTGGTCCCAGTTTATTCCTGAGCTACAG | |
| FOXC1 | ATGCAGGCGCGCTACTCCGTGTCCAGCCCCAACTCCCTGGGAG TGGTGCCCTACCTCGGCGGCGAGCAGAGCTACTACCGCGCGGC GGCCGCGGCGGCCGGGGGCGGCTACACCGCCATGCCGGCCCCC ATGAGCGTGTACTCGCACCCTGCGCACGCCGAGCAGTACCCGG GCGGCATGGCCCGCGCCTACGGGCCCTACACGCCGCAGCCGCA GCCCAAGGACATGGTGAAGCCGCCCTATAGCTACATCGCGCTC ATCACCATGGCCATCCAGAACGCCCCGGACAAGAAGATCACCC TGAACGGCATCTACCAGTTCATCATGGACCGCTTCCCCTTCTA CCGGGACAACAAGCAGGGCTGGCAGAACAGCATCCGCCACAAC CTCTCGCTCAACGAGTGCTTCGTCAAGGTGCCGCGCGACGACA AGAAGCCGGGCAAGGGCAGCTACTGGACGCTGGACCCGGACTC CTACAACATGTTCGAGAACGGCAGCTTCCTGCGGCGGCGGCGG CGCTTCAAGAAGAAGGACGCGGTGAAGGACAAGGAGGAGAAGG ACAGGCTGCACCTCAAGGAGCCGCCCCCGCCCGGCCGCCAGCC CCCGCCCGCGCCGCCGGAGCAGGCCGACGGCAACGCGCCCGGT CCGCAGCCGCCGCCCGTGCGCATCCAGGACATCAAGACCGAGA ACGGTACGTGCCCCTCGCCGCCCCAGCCCCTGTCCCCGGCCGC CGCCCTGGGCAGCGGCAGCGCCGCCGCGGTGCCCAAGATCGAG AGCCCCGACAGCAGCAGCAGCAGCCTGTCCAGCGGGAGCAGCC CCCCGGGCAGCCTGCCGTCGGCGCGGCCGCTCAGCCTGGACGG TGCGGATTCCGCGCCGCCGCCGCCCGCGCCCTCCGCCCCGCCG CCGCACCATAGCCAGGGCTTCAGCGTGGACAACATCATGACGT CGCTGCGGGGGTCGCCGCAGAGCGCGGCCGCGGAGCTCAGCTC CGGCCTTCTGGCCTCGGCGGCCGCGTCCTCGCGCGCGGGGATC GCACCCCCGCTGGCGCTCGGCGCCTACTCGCCCGGCCAGAGCT CCCTCTACAGCTCCCCCTGCAGCCAGACCTCCAGCGCGGGCAG CTCGGGCGGCGGCGGCGGCGGCGCGGGGGCCGCGGGGGGCGCG GGCGGCGCCGGGACCTACCACTGCAACCTGCAAGCCATGAGCC TGTACGCGGCCGGCGAGCGCGGGGGCCACTTGCAGGCGCGCCC CGGGGGCGCGGGCGGCTCGGCCGTGGACGACCCCCTGCCCGAC TACTCTCTGCCTCCGGTCACCAGCAGCAGCTCGTCGTCCCTGA GTCACGGCGGCGGCGGCGGCGGCGGCGGGGGAGGCCAGGAGGC CGGCCACCACCCTGCGGCCCACCAAGGCCGCCTCACCTCGTGG TACCTGAACCAGGCGGGCGGAGACCTGGGCCACTTGGCGAGCG CGGCGGCGGCGGCGGCGGCCGCAGGCTACCCGGGCCAGCAGCA GAACTTCCACTCGGTGCGGGAGATGTTCGAGTCACAGAGGATC GGCTTGAACAACTCTCCAGTGAACGGAATAGTAGCTGTCAAA TGGCCTTCCCTTCCAGCCAGTCTCTGTACCGCACGTCCGGAGC TTTCGTCTACGACTGTAGCAAGTTT | 85 |
| FOXC2 | ATGCAGGCGCTACTCCGTGTCCGACCCCAACGCCCTGGGAG TGGTGCCCTACCTGAGCGAGCAGAATTACTACCGGCTGCGGG CAGCTACGGCGGCATGGCCAGCCCCATGGGCGTCTATTCCGGC CACCCGGAGCAGTACAGCGCGGGATGGCCGCTCCTACGCGC CCTACCACCACCACCAGCCCGCGGCGCCTAAGGACCTGGTGAA GCCGCCCTACAGCTACATCGCGCTCATCACCATGGCCATCCAG | 86 |
| | AACGCGCCCGAGAAGAAGATCACCTTGAACGGCATCTACCAGT TCATCATGGACCGCTTCCCCTTCTACCGGGAGAACAAGCAGGG CTGGCAGAACAGCATCCGCCACAACCTCTCGCTCAACGAGTGC TTCGTCAAGGTGCCCCGCGACGACAAGAAGCCCGGCAAGGGCA GTTACTGGACCCTGGACCCGGACTCCTACAACATGTTCGAGAA CGGCAGCTTCCTGCGGCGCCGGCGGCGCTTCAAAAAGAAGGAC GTGTCCAAGGAGAAGGAGGAGCGGGCCCACCTCAAGGAGCCGC CCCCGGCGGCGTCCAAGGGCGCCCCGGCCACCCCCCACCTAGC GGACGCCCCCAAGGAGGCCGAGAAGAAGGTGGTGATCAAGAGC GAGGCGGCGTCCCCGGCGCTGCCGGTCATCACCAAGGTGGAGA CGCTGAGCCCCGAGAGCGCGCTGCAGGGCAGCCCGCGCAGCGC GGCCTCCACGCCCGCCGGCTCCCCCGACGGCTCGCTGCCGGAG CACCACGCCGCGGCGCCCAACGGGCTGCCTGGCTTCAGCGTGG AGAACATCATGACCCTGCGAACGTCGCCGCCGGGCGGAGAGCT GAGCCCGGGGGCCGGACGCGCGGGCCTGGTGGTGCCGCCGCTG GCGCTGCCCTACGCCGCCGCGCCGCCCGCCGCCTACGGCCAGC CGTGCGCTCAGGGCCTGGAGGCCGGGGCCGCCGGGGGCTACCA GTGCAGCATGCGAGCGATGAGCCTGTACACCGGGGCCGAGCGG CCGGCGCACATGTGCGTCCCGCCCGCCCTGGACGAGGCCCTCT CGGACCACCCGAGCGGCCCCACGTCGCCCCTGAGCGCTCTCAA CCTCGCCGCCGGCCAGGAGGGCGCGCTCGCCGCCACGGCCAC CACCACCAGCACCACGGCCACCACCACCCGCAGGCGCCGCCGC CCCCGCCGGCTCCCCAGCCCCAGCCGACGCCGCAGCCCGGGGC CGCCGCGGCGCAGGCGGCCTCCTGGTATCTCAACCACAGCGGG GACCTGAACCACCTCCCCGGCCACACGTTCGCGGCCCAGCAGC AAACTTTCCCCAACGTGCGGGAGATGTTCAACTCCCACCGGCT GGGGATTGAGAACTCGACCCTCGGGGAGTCCCAGGTGAGTGGC AATGCCAGCTGCCAGCTGCCCTACAGATCCACGCCGCCTCTCT ATCGCCACGCAGCCCCCTACTCCTACGACTGCACGAAATAC | |
| TWIST1 | ATGATGCAGGACGTGTCCAGCTCGCCAGTCTCGCCGGCCGACG ACAGCCTGAGCAACAGCGAGGAAGAGCCAGACCGGCAGCAGCC GCCGAGCGGCAAGCGCGGGGACGCAAGCGGCGCAGCAGCAGG CGCAGCGCGGGCGGCGGCGCGGGCCCGGCGGAGCCGCGGGTG GGGGCGTCGGAGGCGGCGACGAGCCGGGCAGCCCGGCCCAGGG CAAGCGCGGCAAGAAGTCTGCGGCTGTGGCGGCGGCGGCGGC GCGGGCGGCGGCGGCGGCAGCAGCAGCGGCGGCGGGAGTCCGC AGTCTTACGAGGAGCTGCAGACGCAGCGGGTCATGGCCAACGT GCGGGAGCGCCAGCGCACCCAGTCGCTGAACGAGGCGTTCGCC GCGCTGCGGAAGATCATCCCCACGCTGCCCTCGGACAAGCTGA GCAAGATTCAGACCCTCAAGCTGGCGGCCAGGTACATCGACTT CCTCTACCAGGTCCTCCAGAGCGACGAGCTGGACTCCAAGATG GCAAGCTGCAGCTATGTGGCTCACGAGCGGCTCAGCTACGCCT TCTCGGTCTGGAGGATGGAGGGGGCCTGGTCCATGTCCGCGTC CCAC | 87 |
| HIF1A | ATGGAGGGCGCCGGCGGCGCGAACGACAAGAAAAAGATAAGTT CTGAACGTCGAAAAGAAAAGTCTCGAGATGCAGCCAGATCTCG GCGAAGTAAAGAATCTGAAGTTTTTTATGAGCTTGCTCATCAG TTGCCACTTCCACATAATGTGAGTTCGCATCTTGATAAGGCCT CTGTGATGAGGCTTACCATCAGCTATTTGCGTGTGAGGAAACT TCTGGATGCTGGTGATTTGGATATTGAAGATGACATGAAAGCA CAGATGAATTGCTTTTATTTGAAAGCCTTGGATGGTTTTGTTA TGGTTCTCACAGATGATGGTGACATGATTTACATTTCTGATAA TGTGAACAAATACATGGGATTAACTCAGTTTGAACTAACTGGA CACAGTGTGTTTGATTTTACTCATCCATGTGACCATGAGGAAA TGAGAGAAATGCTTACACACAGAAATGGCCTTGTGAAAAAGGG | 88 |
| | TAAAGAACAAAACACACAGCGAAGCTTTTTCTCAGAATGAAG TGTACCCTAACTAGCCGAGGAAGAACTATGAACATAAAGTCTG CAACATGGAAGGTATTGCACTGCACAGGCCACATTCACGTATA TGATACCAACAGTAACCAACCTCAGTGTGGGTATAAGAAACCA CCTATGACCTGCTTGGTGCTGATTTGTGAACCCATTCCTCACC CATCAAATATTGAAATTCCTTTAGATAGCAAGACTTTCCTCAG TCGACACAGCCTGGATATGAAATTTCTTATTGTGATGAAAGA ATTACCGAATTGATGGGATATGAGCCAGAAGAACTTTTAGGCC GCTCAATTTATGAATATTATCATGCTTTGGACTCTGATCATCT GACCAAAACTCATCATGATATGTTTACTAAAGGACAAGTCACC ACAGGACAGTACAGGATGCTTGCCAAAAGAGGTGGATATGTCT GGGTTGAAACTCAAGCAACTGTCATATATAACACCAAGAATTC TCAACCACAGTGCATTGTATGTGTGAATTACGTTGTGAGTGGT ATTATTCAGCACGACTTGATTTTCTCCCTTCAACAAACAGAAT GTGTCCTTAAACCGGTTGAATCTTCAGATATGAAAATGACTCA GCTATTCACCAAAGTTGAATCAGAAGATACAAGTAGCCTCTTT GACAAACTTAAGAAGGAACCTGATGCTTTAACTTTGCTGGCCC CAGCCGCTGGAGACACAATCATATCTTTAGATTTTGGCAGCAA CGACACAGAAACTGATGACCAGCAACTTGAGGAAGTACCATTA TATAATGATGTAATGCTCCCCTCACCCAACGAAAAATTACAGA ATATAAATTTGGCAATGTCTCCATTACCCACCGCTGAAACGCC AAAGCCACTTCGAAGTAGTGCTGACCCTGCACTCAATCAAGAA GTTGCATTAAAATTAGAACCAAATCCAGAGTCACTGGAACTTT CTTTTACCATGCCCCAGATTCAGGATCAGACACCTAGTCCTTC CGATGGAAGCACTAGACAAAGTTCACCTGAGCCTAATAGTCCC AGTGAATATTGTTTTTATGTGGATAGTGATATGGTCAATGAAT TCAAGTTGGAATTGGTAGAAAAACTTTTTGCTGAAGACACAGA AGCAAAGAACCCATTTTCTACTCAGGACACAGATTTAGACTTG GAGATGTTAGCTCCCTATATCCCAATGGATGATGACTTCCAGT TACGTTCCTTCGATCAGTTGTCACCATTAGAAAGCAGTTCCGC AAGCCCTGAAAGCGCAAGTCCTCAAAGCACAGTTACAGTATTC CAGCAGACTCAAATACAAGAACCTACTGCTAATGCCACCACTA CCACTGCCACCACTGATGAATTAAAAACAGTGACAAAAGACCG TATGGAAGACATTAAAATATTGATTGCATCTCCATCTCCTACC CACATACATAAAGAAACTACTAGTGCCACATCATCACCATATA GAGATACTCAAAGTCGGACAGCCTCACCAAACAGAGCAGGAAA AGGAGTCATAGAACAGACAGAAAAATCTCATCCAAGAAGCCCT AACGTGTTATCTGTCGCTTTGAGTCAAAGAACTACAGTTCCTG AGGAAGAACTAAATCCAAAGATACTAGCTTTGCAGAATGCTCA GAGAAAGCGAAAAATGGAACATGATGGTTCACTTTTTCAAGCA GTAGGAATTATT | |
| SALL4 | ATGTCGAGGCGCAAGCAGGCGAAACCCCAGCACATCAACTCGG AGGAGGACCAGGGCGAGCAGCAGCCGCAGCAGCAGACCCCGGA GTTTGCAGATGCGGCCCCAGCGGCGCCCGCGGCGGGGGAGCTG GGTGCTCCAGTGAACCACCCAGGGAATGACGAGGTGGCGAGTG AGGATGAAGCCACAGTAAAGCGGCTTCGTCGGGAGGAGACGCA CGTCTGTGAGAAATGCTGTGCGGAGTTCTTCAGCATCTCTGAG TTCCTGGAACATAAGAAAAATTGCACTAAAAATCCACCTGTCC TCATCATGAATGACAGCGAGGGGCCTGTGCCTTCAGAAGACTT CTCCGGAGCTGTACTGAGCCACCAGCCCACCAGTCCCGGCAGT AAGGACTGTCACAGGGAGAATGGCGGCAGCTCAGAGGACATGA AGGAGAAGCCGGATGCGGAGTCTGTGGTGTACCTAAAGACAGA GACAGCCCTGCCACCCACCCCCCAGGACATAAGCTATTTAGCC AAAGGCAAAGTGGCCAACACTAATGTGACCTTGCAGGCACTAC GGGGCACCAAGGTGGCGGTGAATCAGCGGAGCGCGGATGCACT | 89 |
| | CCCTGCCCCCGTGCCTGGTGCCAACAGCATCCCGTGGGTCCTC GAGCAGATCTTGTGTCTGCAGCAGCAGCAGCTACAGCAGATCC AGCTCACCGAGCAGATCCGCATCCAGGTGAACATGTGGGCCTC CCACGCCCTCCACTCAAGCGGGGCAGGGGCCGACACTCTGAAG ACCTTGGGCAGCCACATGTCTCAGCAGGTTTCTGCAGCTGTGG CTTTGCTCAGCCAGAAAGCTGGAAGCCAAGGTCTGTCTCTGGA TGCCTTGAAACAAGCCAAGCTACCTCACGCCAACATCCCTTCT GCCACCAGCTCCCTGTCCCCAGGGCTGGCACCCTTCACTCTGA AGCCGGATGGGACCCGGGTGCTCCCGAACGTCATGTCCCGCCT CCCGAGCGCTTTGCTTCCTCAGGCCCCGGGCTCGGTGCTCTTC CAGAGCCCTTTCTCCACTGTGGCGCTAGACACATCCAAGAAAG GGAAGGGGAAGCCACCGAACATCTCCGCGGTGGATGTCAAACC CAAAGACGAGGCGGCCCTCTACAAGCACAAGTGTAAGTACTGT AGCAAGGTTTTTGGGACTGATAGCTCCTTGCAGATCCACCTCC GCTCCCACACTGGAGAGAGACCCTTCGTGTGCTCTGTCTGTGG TCATCGCTTCACCACCAAGGGCAACCTCAAGGTGCACTTTCAC CGACATCCCCAGGTGAAGGCAAACCCCCAGCTGTTTGCCGAGT TCCAGGACAAAGTGGCGGCCGGCAATGGCATCCCCTATGCACT CTCTGTACCTGACCCCATAGATGAACCGAGTCTTTCTTTAGAC AGCAAACCTGTCCTTGTAACCACCTCTGTAGGGCTACCTCAGA ATCTTTCTTCGGGGACTAATCCCAAGGACCTCACGGGTGGCTC CTTGCCCGGTGACCTGCAGCCTGGGCCTTCTCCAGAAAGTGAG GGTGGACCCACACTCCCTGGGGTGGACCAAACTATAATTCCC CAAGGGCTGGTGGCTTCCAAGGGAGTGGACCCCTGAGCCAGG GTCAGAGACCCTGAAATTGCAGCAGTTGGTGGAGAACATTGAC AAGGCCACCACTGATCCCAACGAATGTCTCATTTGCCACCGAG TCTTAAGCTGTCAGAGCTCCCTCAAGATGCATTATCGCACCCA CACCGGGGAGAGACCGTTCCAGTGTAAGATCTGTGGCCGAGCC TTTTCTACCAAAGGTAACCTGAAGACACACCTTGGGGTTCACC GAACCAACACATCCATTAAGACGCAGCATTCGTGCCCCATCTG CCAGAAGAAGTTCACTAATGCCGTGATGCTGCAGCAACATATT CGGATGCACATGGGCGGTCAGATTCCCAACACGCCCCTGCCAG AGAATCCCTGTGACTTTACGGGTTCTGAGCCAATGACCGTGGG TGAGAACGGCAGCACCGGCGCTATCTGCCATGATGATGTCATC GAAAGCATCGATGTAGAGGAAGTCAGCTCCCAGGAGGCTCCCA GCAGCTCCTCCAAGGTCCCCACGCCTCTTCCCAGCATCCACTC GGCATCACCCACGCTAGGGTTTGCCATGATGGCTTCCTTAGAT GCCCCAGGGAAAGTGGGTCCTGCCCCTTTTAACCTGCAGCGCC AGGGCAGCAGAGAAAACGGTTCCGTGGAGAGCGATGGCTTGAC CAACGACTCATCCTCGCTGATGGGAGACCAGGAGTATCAGAGC CGAAGCCCAGATATCCTGGAAACCACATCCTTCCAGGCACTCT CCCCGGCCAATAGTCAAGCCGAAAGCATCAAGTCAAAGTCTCC CGATGCTGGGAGCAAAGCAGAGAGCTCCGAGAACAGCCGCACT GAGATGGAAGGTCGGAGCAGTCTCCCTTCCACGTTTATCCGAG CCCCGCCGACCTATGTCAAGGTTGAAGTTCCTGGCACATTTGT GGGACCCTCGACATTGTCCCCAGGGATGACCCCTTTGTTAGCA GCCCAGCCACGCCGACAGGCCAAGCAACATGGCTGCACACGGT GTGGGAAGAACTTCTCGTCTGCTAGCGCTCTTCAGATCCACGA GCGGACTCACACTGGAGAGAAGCCTTTTGTGTGCAACATTTGT GGGCGAGCTTTTACCACCAAAGGCAACTTAAAGGTTCACTACA TGACACACGGGGCGAACAATAACTCAGCCCGCCGTGGAAGGAA GTTGGCCATCGAGAACACCATGGCTCTGTTAGGTACGGACGGA AAAAGAGTCTCAGAAATCTTTCCCAAGGAAATCCTGGCCCCTT CAGTGAATGTGGACCCTGTTGTGTGGAACCAGTACACCAGCAT GCTCAATGGCGGTCTGGCCGTGAAGACCAATGAGATCTCTGTG | |
| | ATCCAGAGTGGGGGGTTCCTACCCTCCCGGTTTCCTTGGGGG CCACCTCCGTTGTGAATAACGCCACTGTCTCCAAGATGGATGG CTCCCAGTCGGGTATCAGTGCAGATGTGGAAAAACCAAGTGCT ACTGACGGCGTTCCCAAACACCAGTTTCCTCACTTCCTGGAAG AAAACAAGATTGCGGTCAGC | |
| ELF1 | ATGGCTGCTGTTGTCCAACAGAACGACCTAGTATTTGAATTTG CTAGTAACGTCATGGAGGATGAACGACAGCTTGGTGATCCAGC TATTTTTCCTGCCGTAATTGTGGAACATGTTCCTGGTGCTGAT ATTCTCAATAGTTATGCCGGTCTAGCCTGTGTGGAAGAGCCCA ATGACATGATTACTGAGAGTTCACTGGATGTTGCTGAAGAAGA AATCATAGACGATGATGATGATGACATCACCCTTACAGTTGAA GCTTCTTGTCATGACGGGGATGAAACAATTGAAACTATTGAGG CTGCTGAGGCACTCCTCAATATGGATTCCCCTGGCCCTATGCT GGATGAAAAACGAATAAATAATAATATATTTAGTTCACCTGAA GATGACATGGTTGTTGCCCCAGTCACCCATGTGTCCGTCACAT TAGATGGGATTCCTGAAGTGATGGAAACACAGCAGGTGCAAGA AAAATATGCAGACTCACCGGGAGCCTCATCACCAGAACAGCCT AAGAGGAAAAAAGGAAGAAAAACTAAACCACCACGACCAGATT CCCCAGCCACTACGCCAAATATATCTGTGAAGAAGAAAAACAA AGATGGAAAGGAAACACAATTTATCTTTGGGAGTTTTTACTG GCACTGCTCCAGGACAAGGCTACTTGTCCTAAATACATCAAGT GGACCCAGCGAGAGAAAGGCATTTTTAAATTGGTGGATTCTAA AGCAGTGTCCAGGTTGTGGGGGAAGCACAAAAACAAACCTGAT ATGAATTATGAGACCATGGGAAGAGCACTCAGGTACTATTACC AAAGGGGTATTCTGGCAAAAGTGGAAGGTCAGCGCTTGGTGTA TCAGTTTAAAGAAATGCCAAAAGATCTTATATATATAAATGAT GAGGATCCAAGTTCCAGCATAGAGTCTTCAGATCCATCGCTAT CTTCATCAGCCACTTCAAATAGGAATCAAACCAGCCGGTCGAG AGTATCTTCAAGTCCAGGGGTAAAAGGAGGAGCCACTACAGTT CTAAAACCAGGGAATTCTAAAGCTGCAAAACCCAAAGATCCTG TGGAAGTTGCACAACCATCAGAAGTTTTGAGGACAGTGCAGCC CACGCAGTCTCCATATCCTACCCAGCTCTTCCGGACTGTTCAT GTAGTACAGCCAGTACAGGCTGTCCCAGAGGGAGAAGCAGCTA GAACCAGTACCATGCAGGATGAAACATTAAATTCTTCCGTTCA GAGTATTAGGACTATACAGGCTCCAACCCAAGTTCCAGTGGTT GTGTCTCCTAGGAATCAGCAGTTGCATACAGTAACACTCCAAA CAGTGCCACTCACAACAGTTATAGCCAGCACAGATCCATCAGC AGGTACTGGATCTCAGAAGTTTATTTTACAAGCCATTCCATCA TCACAGCCCATGACAGTACTGAAAGAAAATGTCATGCTGCAGT CACAAAAGGCGGGCTCTCCTCCTTCAATTGTCTTGGGCCCTGC CCAGGTTCAGCAGGTCCTTACTAGCAATGTTCAGACCATTTGC AATGGAACCGTCAGTGTGGCTTCCTCTCCATCCTTCAGTGCTA CTGCACCTGTGGTGACCTTTTCTCCTCGCAGTTCACAGCTGGT TGCTCACCCACCTGGCACTGTAATCACTTCAGTTATCAAAACT CAAGAAACAAAAACTCTTACACAGGAAGTAGAGAAAAAGGAAT CTGAAGATCATTTGAAAGAGAACACTGAGAAAACGGAGCAGCA GCCACAGCCTTATGTGATGGTAGTGTCCAGTTCCAATGGATTT ACTTCTCAGGTAGCTATGAAACAAAACGAACTGCTGGAACCCA ACTCTTTT | 90 |
| SOX9 | ATGAATCTCCTGGACCCCTTCATGAAGATGACCGACGAGCAGG AGAAGGGCCTGTCCGGCGCCCCCAGCCCCACCATGTCCGAGGA CTCCGCGGGCTCGCCCTGCCCGTCGGGCTCCGGCTCGGACACC GAGAACACGCGGCCCCAGGAGAACACGTTCCCCAAGGGCGAGC CCGATCTGAAGAAGGAGAGCGAGGAGGACAAGTTCCCCGTGTG CATCCGCGAGGCGGTCAGCCAGGTGCTCAAAGGCTACGACTGG | 91 |
| | ACGCTGGTGCCCATGCCGGTGCGCGTCAACGGCTCCAGCAAGA ACAAGCCGCACGTCAAGCGGCCCATGAACGCCTTCATGGTGTG GGCGCAGGCGGCGCGCAGGAAGCTCGCGGACCAGTACCCGCAC TTGCACAACGCCGAGCTCAGCAAGACGCTGGGCAAGCTCTGGA GACTTCTGAACGAGAGCGAGAAGCGGCCCTTCGTGGAGGAGGC GGAGCGGCTGCGCGTGCAGCACAAGAAGGACCACCCGGATTAC AAGTACCAGCCGCGGCGGAGGAAGTCGGTGAAGAACGGGCAGG CGGAGGCAGAGGAGGCCACGGAGCAGACGCACATCTCCCCCAA CGCCATCTTCAAGGCGCTGCAGGCCGACTCGCCACACTCCTCC TCCGGCATGAGCGAGGTGCACTCCCCCGGCGAGCACTCGGGGC AATCCCAGGGCCCACCGACCCCACCCACCACCCCCAAAACCGA CGTGCAGCCGGGCAAGGCTGACCTGAAGCGAGAGGGGCGCCCC TTGCCAGAGGGGGGCAGACAGCCCCCTATCGACTTCCGCGACG TGGACATCGGCGAGCTGAGCAGCGACGTCATCTCCAACATCGA GACCTTCGATGTCAACGAGTTTGACCAGTACCTGCCGCCCAAC GGCCACCCGGGGGTGCCGGCCACGCACGGCCAGGTCACCTACA CGGGCAGCTACGGCATCAGCAGCACCGCGGCCACCCCGGCGAG CGCGGGCCACGTGTGGATGTCCAAGCAGCAGGCGCCGCCGCCA CCCCCGCAGCAGCCCCCACAGGCCCCGCCGGCCCCGCAGGCGC CCCCGCAGCCGCAGGCGGCGCCCCCACAGCAGCCGGCGGCACC CCCGCAGCAGCCACAGGCGCACACGCTGACCACGCTGAGCAGC GAGCCGGGCCAGTCCCAGCGAACGCACATCAAGACGGAGCAGC TGAGCCCCAGCCACTACAGCGAGCAGCAGCAGCACTCGCCCCA ACAGATCGCCTACAGCCCCTTCAACCTCCCACACTACAGCCCC TCCTACCCGCCCATCACCCGCTCACAGTACGACTACACCGACC ACCAGAACTCCAGCTCCTACTACAGCCACGCGGCAGGCCAGGG CACCGGCCTCTACTCCACCTTCACCTACATGAACCCCGCTCAG CGCCCCATGTACACCCCCATCGCCGACACCTCTGGGGTCCCTT CCATCCCGCAGACCCACAGCCCCCAGCACTGGGAACAACCCGT CTACACACAGCTCACTCGACCT | |
| SOX12 | ATGGTGCAGCAGCGGGGCGCGAGGGCCAAGCGGACGGCGGGC CGCCGCCCCCGGGACCCGGGCCGGCCGAGGAGGGGCGCGCGA GCCCGGCTGGTGCAAGACCCCGAGCGGCCACATCAAGAGGCCG ATGAACGCATTCATGGTGTGGTCGCAGCACGAACGGCGGAAGA TCATGGACCAGTGGCCCGACATGCACAACGCCGAGATCTCCAA GCGCCTGGGCCGCCGCTGGCAGCTGCTGCAGGACTCGGAGAAG ATCCCGTTCGTGCGGGAGGCGGAGCGGCTGCGGCTCAAGCACA TGGCGGATTACCCGGACTACAAGTACCGGCCGCGCAAAAAGAG CAAGGGGGCGCCCGCCAAGGCGCGGCCCCGCCCCCCCGGTGGT AGCGGTGGCGGCAGCCGGCTCAAGCCCGGGCCGCAGCTGCCTG GCCGCGGGGGCCGCCGAGCAGCGGAGGGCCTTTGGGGGCGG GGCGGCGGCGCCCGAGGACGACGATGAAGACGACGACGAGGAG CTGCTGGAAGTGCGCCTGGTCGAGACCCCGGGGCGGGAGCTGT GGAGGATGGTCCCGGCGGGACGGGCCGCTCGGGGACAAGCGGA GCGCGCCCAAGGGCCGTCGGGCGAGGGGGCGGCCGCCGCCGCC GCCGCCTCCCCGACACCGTCGGAGGACGAGGAGCCGGAGGAAG AGGAGGAGGAGGCGGCAGCGGCTGAGGAAGGTGAAGAGGAGAC GGTGGCGTCGGGGGAGGAGTCGCTGGGCTTTCTGTCCAGGCTG CCCCCTGGCCCGGCCGGCCTGGACTGCAGCGCCCTGGATCGCG ACCCGGACCTGCAGCCTCCCTCGGGCACGTCGCACTTCGAGTT CCCGGACTACTGCACCCCCGAGGTTACCGAGATGATCGCGGGG GACTGGCGCCCGTCTAGCATCGCAGACCTGGTTTTCACCTAC | 92 |
| SOX18 | ATGCAGAGATCGCCGCCCGGCTACGGCGCACAGGACGACCCGC CCGCCCGCCGCGACTGTGCATGGGCCCCGGACACGGGGCCGC CGCTGACACGCGCGGCCTCGCCGCCGGCCCCGCCGCCCTCGCC | 93 |
| | GCGCCCGCCGCGCCCGCCTCGCCGCCCAGCCCGCAGCGCAGTC CCCCGCGCAGCCCCGAGCCGGGGCGCTATGGCCTCAGCCCGGC CGGCCGCGGGGAACGCCAGGCGGCAGACGAGTCGCGCATCCGG CGGCCCATGAACGCCTTCATGGTGTGGGCAAAGGACGAGCGCA AGCGGCTGGCTCAGCAGAACCCGGACCTGCACAACGCGGTGCT CAGCAAGATGCTGGGCAAAGCGTGGAAGGAGCTGAACGCGGCG GAGAAGCGGCCCTTCGTGGAGGAAGCCGAACGGCTGCGCGTGC AGCACTTGCGCGACCACCCCAACTACAAGTACCGGCCGCGCCG CAAGAAGCAGGCGCGCAAGGCCCGGCGGCTGGAGCCCGGCCTC CTGCTCCCGGGATTAGCGCCCCCGCAGCCACCGCCCGAGCCTT TCCCCGCGGCGTCTGGCTCGGCTCGCGCCTTCCGCGAGCTGCC CCCGCTGGGCGCCGAGTTCGACGGCCTGGGGCTGCCCACGCCC GAGCGCTCGCCTCTGGACGGCCTGGAGCCCGGCGAGGCTGCCT TCTTCCCACCGCCCGCGGCGCCCGAGGACTGCGCGCTGCGGCC CTTCCGCGCGCCCTACGCGCCCACCGAGTTGTCGCGGGACCCC GGCGGTTGCTACGGGGCTCCCCTGGCGGAGGCGCTCAGGACCG CGCCCCCCGCGGCGCCGCTCGCTGGCCTGTACTACGGCACCCT GGGCACGCCCGGCCCGTACCCCGGCCCGCTGTCGCCGCCGCCC GAGGCCCCGCCGCTGGAGAGCGCCGAGCCGCTGGGGCCCGCCG CCGATCTGTGGGCCGACGTGGACCTCACCGAGTTCGACCAGTA CCTCAACTGCAGCCGGACTCGGCCCGACGCCCCCGGGCTCCCG TACCACGTGGCACTGGCCAAACTGGGCCCGCGCGCCATGTCCT GCCCAGAGGAGAGCAGCCTGATCTCCGCGCTGTCGGACGCCAG CAGCGCGGTCTATTACAGCGCGTGCATCTCCGGC | |
| ETS1 | ATGAAGGCGGCCGTCGATCTCAAGCCGACTCTCACCATCATCA AGACGGAAAAAGTCGATCTGGAGCTTTTCCCCTCCCCGGATAT GGAATGTGCAGATGTCCCACTATTAACTCCAAGCAGCAAAGAA ATGATGTCTCAAGCATTAAAAGCTACTTTCAGTGGTTTCACTA AAGAACAGCAACGACTGGGGATCCCAAAAGACCCCCGGCAGTG GACAGAAACCCATGTTCGGGACTGGGTGATGTGGGCTGTGAAT GAATTCAGCCTGAAAGGTGTAGACTTCCAGAAGTTCTGTATGA ATGGAGCAGCCCTCTGCGCCCTGGGTAAAGACTGCTTTCTCGA GCTGGCCCCAGACTTTGTTGGGGACATCTTATGGAACATCTA GAGATCCTGCAGAAAGAGGATGTGAAACCATATCAAGTTAATG GAGTCAACCCAGCCTATCCAGAATCCCGCTATACCTCGGATTA CTTCATTAGCTATGGTATTGAGCATGCCCAGTGTGTTCCACCA TCGGAGTTCTCAGAGCCCAGCTTCATCACAGAGTCCTATCAGA CGCTCCATCCCATCAGCTCGGAAGAGCTCCTCTCCCTCAAGTA TGAGAATGACTACCCCTCGGTCATTCTCCGAGACCCTCTCCAG ACAGACACCTTGCAGAATGACTACTTTGCTATCAAACAAGAAG TCGTCACCCCAGACAACATGTGCATGGGGAGGACCAGTCGTGG TAAACTCGGGGGCCAGGACTCTTTTGAAAGCATAGAGAGCTAC GATAGTTGTGATCGCCTCACCCAGTCCTGGAGCAGCCAGTCAT CTTTCAACAGCCTGCAGCGTGTTCCCTCCTATGACAGCTTCGA CTCAGAGGACTATCCGGCTGCCCTGCCCAACCACAAGCCCAAG GGCACCTTCAAGGACTATGTGCGGGACCGTGCTGACCTCAATA AGGACAAGCCTGTCATTCCTGCTGCTGCCCTAGCTGGCTACAC AGGCAGTGGACCAATCCAGCTATGGCAGTTTCTTCTGGAATTA CTCACTGATAAATCCTGTCAGTCTTTTATCAGCTGGACAGGAG ATGGCTGGGAATTCAAACTTTCTGACCCAGATGAGGTGGCCAG GAGATGGGGAAAGAGGAAAAACAAACCTAAGATGAATTATGAG AAACTGAGCCGTGGCCTACGCTACTATTACGACAAAAACATCA TCCACAAGACAGCGGGGAAACGCTACGTGTACCGCTTTGTGTG TGACCTGCAGAGCCTGCTGGGGTACACCCCTGAGGAGCTGCAC GCCATGCTGGACGTCAAGCCAGATGCCGACGAG | 94 |
| PAX3 | ATGACCACGCTGGCCGGCGCTGTGCCCAGGATGATGCGGCCGG GCCCGGGGCAGAACTACCCGCGTAGCGGTTCCCGCTGGAAGT GTCCACTCCCCTCGGCCAGGGCCGCGTCAACCAGCTCGGCGGT GTTTTTATCAACGGCAGGCCGCTGCCCAACCACATCCGCCACA AGATCGTGGAGATGGCCCACCACGGCATCCGGCCCTGCGTCAT CTCGCGCCAGCTGCGCGTGTCCCACGGCTGCGTCTCCAAGATC CTGTGCAGGTACCAGGAGACTGGCTCCATACGTCCTGGTGCCA TCGGCGGCAGCAAGCCCAAGCAGGTGACAACGCCTGACGTGGA GAAGAAAATTGAGGAATACAAAAGAGAGAACCCGGGCATGTTC AGCTGGGAAATCCGAGACAAATTACTCAAGGACGCGGTCTGTG ATCGAAACACCGTGCCGTCAGTGAGTTCCATCAGCCGCATCCT GAGAAGTAAATTCGGGAAAGGTGAAGAGGAGGAGGCCGACTTG GAGAGGAAGGAGGCAGAGGAAAGCGAGAAGAAGGCCAAACACA GCATCGACGGCATCCTGAGCGAGCGAGGAAAGGCCCTGGTCTC CGGAGTTTCCTCGCAT | 95 |
| PAX8 | ATGCCTCACAACTCCATCAGATCTGGCCATGGAGGGCTGAACC AGCTGGGAGGGGCCTTTGTGAATGGCAGACCTCTGCCGGAAGT GGTCCGCCAGCGCATCGTAGACCTGGCCCACCAGGGTGTAAGG CCCTGCGACATCTCTCGCCAGCTCCGCGTCAGCCATGGCTGCG TCAGCAAGATCCTTGGCAGGTACTACGAGACTGGCAGCATCCG GCCTGGAGTGATAGGGGGCTCCAAGCCCAAGGTGGCCACCCCC AAGGTGGTGGAGAAGATTGGGGACTACAAACGCCAGAACCCTA CCATGTTTGCCTGGGAGATCCGAGACCGGCTCCTGGCTGAGGG CGTCTGTGACAATGACACTGTGCCCAGTGTCAGCTCCATTAAT AGAATCATCCGGACCAAAGTGCAGCAACCATTCAACCTCCCTA TGGACAGCTGCGTGGCCACCAAGTCCCTGAGTCCCGGACACAC GCTGATCCCCAGCTCAGCTGTAACTCCCCCGGAGTCACCCCAG TCGGATTCCCTGGGCTCCACCTACTCCATCAATGGGCTCCTGG GCATCGCTCAGCCTGGCAGCGACAAGAGGAAAATGGATGACAG TGATCAGGATAGCTGCCGACTAAGCATTGACTCACAGAGCAGC AGCAGCGGACCCCGAAAGCACCTTCGCACGGATGCCTTCAGCC AGCACCACCTCGAGCCGCTCGAGTGCCCATTTGAGCGGCAGCA CTACCCAGAGGCCTATGCCTCCCCCAGCCACACCAAAGGCGAG CAGGGCCTCTACCCGCTGCCCTTGCTCAACAGCACCCTGGACG ACGGGAAGGCCACCCTGACCCCTTCCAACACGCCACTGGGGCG CAACCTCTCGACTCACCAGACCTACCCCGTGGTGGCAGATCCT CACTCACCCTTCGCCATAAAGCAGGAAACCCCCGAGGTGTCCA GTTCTAGCTCCACCCCTTCCTCTTTATCTAGCTCCGCCTTTTT GGATCTGCAGCAAGTCGGCTCCGGGGTCCCGCCCTTCAATGCC TTTCCCCATGCTGCCTCCGTGTACGGGCAGTTCACGGGCCAGG CCCTCCTCTCAGGGCGAGAGATGGTGGGGCCCACGCTGCCCGG ATACCCACCCCACATCCCCACCAGCGGACAGGGCAGCTATGCC TCCTCTGCCATCGCAGGCATGGTGGCAGGAAGTGAATACTCTG GCAATGCCTATGGCCACACCCCCTACTCCTCCTACAGCGAGGC CTGGCGCTTCCCCAACTCCAGCTTGCTGAGTTCCCCATATTAT TACAGTTCCACATCAAGGCCGAGTGCACCGCCCACCACTGCCA CGGCCTTTGACCATCTG | 96 |
| GLI1 | ATGTTCAACTCGATGACCCCACCACCAATCAGTAGCTATGGCG AGCCCTGCTGTCTCCGGCCCCTCCCCAGTCAGGGGCCCCCAG TGTGGGGACAGAAGGACTGTCTGGCCCGCCCTTCTGCCACCAA GCTAACCTCATGTCCGGCCCCCACAGTTATGGGCCAGCCAGAG AGACCAACAGCTGCACCGAGGGCCCACTCTTTTCTTCTCCCCG GAGTGCAGTCAAGTTGACCAAGAAGCGGGCACTGTCCATCTCA CCTCTGTCGGATGCCAGCCTGGACCTGCAGACGGTTATCCGCA CCTCACCCAGCTCCCTCGTAGCTTTCATCAACTCGCGATGCAC | 97 |
| | ATCTCCAGGAGGCTCCTACGGTCATCTCTCCATTGGCACCATG AGCCCATCTCTGGGATTCCCAGCCCAGATGAATCACCAAAAAG GGCCCTCGCCTTCCTTTGGGGTCCAGCCTTGTGGTCCCCATGA CTCTGCCCGGGGTGGATGATCCCACATCCTCAGTCCCGGGGA CCCTTCCCAACTTGCCAGCTGAAGTCTGAGCTGGACATGCTGG TTGGCAAGTGCCGGGAGGAACCCTTGGAAGGTGATATGTCCAG CCCCAACTCCACAGGCATACAGGATCCCCTGTTGGGGATGCTG GATGGGCGGGAGGACCTCGAGAGAGAGGAGAAGCGTGAGCCTG AATCTGTGTATGAAACTGACTGCCGTTGGGATGGCTGCAGCCA GGAATTTGACTCCCAAGAGCAGCTGGTGCACCACATCAACAGC GAGCACATCCACGGGGAGCGGAAGGAGTTCGTGTGCCACTGGG GGGGCTGCTCCAGGGAGCTGAGGCCCTTCAAAGCCCAGTACAT GCTGGTGGTTCACATGCGCAGACACACTGGCGAGAAGCCACAC AAGTGCACGTTTGAAGGGTGCCGGAAGTCATACTCACGCCTCG AAAACCTGAAGACGCACCTGCGGTCACACACGGGTGAGAAGCC ATACATGTGTGAGCACGAGGGCTGCAGTAAAGCCTTCAGCAAT GCCAGTGACCGAGCCAAGCACCAGAATCGGACCCATTCCAATG AGAAGCCGTATGTATGTAAGCTCCCTGGCTGCACCAAACGCTA TACAGATCCTAGCTCGCTGCGAAAACATGTCAAGACAGTGCAT GGTCCTGACGCCCATGTGACCAAACGGCACCGTGGGGATGGCC CCCTGCCTCGGGCACCATCCATTTCTACAGTGGAGCCCAAGAG GGAGCGGGAAGGAGGTCCCATCAGGGAGGAAAGCAGACTGACT GTGCCAGAGGGTGCCATGAAGCCACAGCCAAGCCCTGGGGCCC AGTCATCCTGCAGCAGTGACCACTCCCCGGCAGGGAGTGCAGC CAATACAGACAGTGGTGTGGAAATGACTGGCAATGCAGGGGGC AGCACTGAAGACCTCTCCAGCTTGGACGAGGGACCTTGCATTG CTGGCACTGGTCTGTCCACTCTTCGCCGCCTTGAGAACCTCAG GCTGGACCAGCTACATCAACTCCGGCCAATAGGGACCCGGGGT CTCAAACTGCCCAGCTTGTCCCACACCGGTACCACTGTGTCCC GCCGCGTGGGCCCCCCAGTCTCTCTTGAACGCCGCAGCAGCAG CTCCAGCAGCATCAGCTCTGCCTATACTGTCAGCCGCCGCTCC TCCCTGGCCTCTCCTTTCCCCCCTGGCTCCCCACCAGAGAATG GAGCATCCTCCCTGCCTGGCCTTATGCCTGCCCAGCACTACCT GCTTCGGGCAAGATATGCTTCAGCCAGAGGGGGTGGTACTTCG CCCACTGCAGCATCCAGCCTGGATCGGATAGGTGGTCTTCCCA TGCCTCCTTGGAGAAGCCGAGCCGAGTATCCAGGATACAACCC CAATGCAGGGGTCACCCGGAGGGCCAGTGACCCAGCCCAGGCT GCTGACCGTCCTGCTCCAGCTAGAGTCCAGAGGTTCAAGAGCC TGGGCTGTGTCCATACCCCACCCACTGTGGCAGGGGGAGGACA GAACTTTGATCCTTACCTCCCAACCTCTGTCTACTCACCACAG CCCCCCAGCATCACTGAGAATGCTGCCATGGATGCTAGAGGGC TACAGGAAGAGCCAGAAGTTGGGACCTCCATGGTGGGCAGTGG TCTGAACCCCTATATGGACTTCCCACCTACTGATACTCTGGGA TATGGGGGACCTGAAGGGGCAGCAGCTGAGCCTTATGGAGCGA GGGGTCCAGGCTCTCTGCCTCTTGGGCCTGGTCCACCCACCAA CTATGGCCCCAACCCCTGTCCCCAGCAGGCCTCATATCCTGAC CCCACCCAAGAAACATGGGGTGAGTTCCCTTCCCACTCTGGGC TGTACCCAGGCCCCAAGGCTCTAGGTGGAACCTACAGCCAGTG TCCTCGACTTGAACATTATGGACAAGTGCAAGTCAAGCCAGAA CAGGGGTGCCCAGTGGGGTCTGACTCCACAGGACTGGCACCCT GCCTCAATGCCCACCCCAGTGAGGGGCCCCCACATCCACAGCC TCTCTTTTCCCATTACCCCCAGCCCTCTCCTCCCCAATATCTC CAGTCAGGCCCCTATACCCAGCCACCCCCTGATTATCTTCCTT CAGAACCCAGGCCTTGCCTGGACTTTGATTCCCCCACCCATTC CACAGGGCAGCTCAAGGCTCAGCTTGTGTGTAATTATGTTCAA | |
| | TCTCAACAGGAGCTACTGTGGAGGGTGGGGGCAGGGAAGATG CCCCCGCCCAGGAACCTTCCTACCAGAGTCCCAAGTTTCTGGG GGGTTCCCAGGTTAGCCCAAGCCGTGCTAAAGCTCCAGTGAAC ACATATGGACCTGGCTTTGGACCCAACTTGCCCAATCACAAGT CAGGTTCCTATCCCACCCCTTCACCATGCCATGAAAATTTTGT AGTGGGGGCAAATAGGGCTTCACATAGGGCAGCAGCACCACCT CGACTTCTGCCCCCATTGCCCACTTGCTATGGGCCTCTCAAAG TGGGAGGCACAAACCCCAGCTGTGGTCATCCTGAGGTGGGCAG GCTAGGAGGGGGTCCTGCCTTGTACCCTCCTCCCGAAGGACAG GTATGTAACCCCCTGGACTCTCTTGATCTTGACAACACTCAGC TGGACTTTGTGGCTATTCTGGATGAGCCCCAGGGGCTGAGTCC TCCTCCTTCCCATGATCAGCGGGGCAGCTCTGGACATACCCCA CCTCCCTCTGGGCCCCCCAACATGGCTGTGGGCAACATGAGTG TCTTACTGAGATCCCTACCTGGGGAAACAGAATTCCTCAACTC TAGTGCCTAA | |
| GLI2 | ATGGAGACGTCTGCCTCAGCCACTGCCTCCGAGAAGCAAGAAG CCAAAAGTGGGATCCTGGAGGCCGCTGGCTTCCCCGACCCGGG TAAAAAGGCCTCTCCTTTGGTGGTGGCTGCAGCGGCAGCAGCA GCGGTAGCTGCCCAAGGAGTGCCGCAGCATCTCTTGCCACCAT TCCATGCGCCCCTACCGATTGACATGCGACACCAGGAAGGAAG GTACCATTACGAGCCTCATTCTGTCCACGGTGTGCACGGGCCC CCTGCCCTCAGCGGCAGCCCTGTCATCTCTGACATCTCCTTGA TCCGGCTTTCCCCGCACCCGGCTGGCCCTGGGGAGTCCCCCTT CAACGCCCCCACCCGTACGTGAACCCCCACATGGAGCACTAC CTCCGTTCTGTGCACAGCAGCCCCACGCTCTCCATGATCTCTG CAGCCAGGGGCCTCAGCCCCGCTGATGTGGCCCAGGAGCACCT TAAGGAGAGGGGACTGTTTGGCCTTCCTGCTCCAGGCACCACC CCCTCAGACTATTACCACCAGATGACCCTCGTGGCAGGCCACC CCGCGCCCTACGGGGACCTGCTGATGCAGAGCGGGGGCGCTGC CAGCGCACCCCATCTCCACGACTACCTCAACCCCGTGGACGTG TCCCGTTTCTCCAGCCCGCGGGTGACGCCCCGCCTGAGCCGCA AGCGGGCGCTGTCCATCTCCCCACTCTCAGACGCCAGCCTGGA CCTGCAGCGGATGATCCGCACCTCACCCAACTCGCTAGTGGCC TACATCAACAACTCCCGAAGCAGCTCGGCGGCCAGCGGTTCCT ACGGGCATCTGTCAGCGGGTGCCCTCAGCCCAGCCTTCACCTT CCCCCACCCCATCAACCCCGTGGCCTACCAGCAGATTCTGAGC CAGCAGAGGGGTCTGGGGTCAGCCTTTGGACACACACCACCCC TGATCCAGCCCTCACCCACCTTCCTGGCCCAGCAGCCCATGGC CCTCACCTCCATCAATGCCACGCCCACCCAGCTCAGCAGCAGC AGCAACTGTCTGAGTGACACCAACCAGAACAAGCAGAGCAGTG AGTCGGCCGTCAGCAGCACCGTCAACCCTGTCGCCATTCACAA GCGCAGCAAGGTCAAGACCGAGCCTGAGGGCCTGCGGCCGGCC TCCCCTCTGGCGCTGACGCAGGGCCAGGTGTCTGGACACGGCT CATGTGGGTGTGCCCTTCCCCTCTCCCAGGAGCAGCTGGCTGA CCTCAAGGAAGATCTGGACAGGGATGACTGTAAGCAGGAGGCT GAGGTGGTCATCTATGAGACCAACTGCCACTGGGAAGACTGCA CCAAGGAGTACGACACCCAGGAGCAGCTGGTGCATCACATCAA CAACGAGCACATCCACGGGGAGAAGAAGGAGTTTGTGTGCCGC TGGCAGGCCTGCACGCGGGAGCAGAAGCCCTTCAAGGCGCAGT ACATGCTGGTGGTGCACATGCGGCGACACACGGGCGAGAAGCC CCACAAGTGCACGTTCGAGGGCTGCTCGAAGGCCTACTCCCGC CTGGAGAACCTGAAGACACACCTGCGGTCCCACACCGGGGAGA AGCCATATGTGTGTGAGCACGAGGGCTGCAACAAAGCCTTCTC CAACGCCTCGGACCGCGCCAAGCACCAGAATCGCACCCACTCC AACGAGAAACCCTACATCTGCAAGATCCCAGGCTGCACCAAGA | 98 |
| | GATACACAGACCCCAGCTCTCTCCGGAAGCATGTGAAAACGGT CCACGGCCCAGATGCCCACGTCACCAAGAAGCAGCGCAATGAC GTGCACCTCCGCACACCGCTGCTCAAAGAGAATGGGGACAGTG AGGCCGGCACGGAGCCTGGCGGCCCAGAGAGCACCGAGGCCAG CAGCACCAGCCAGGCCGTGGAGGACTGCCTGCACGTCAGAGCC ATCAAGACCGAGAGCTCCGGGCTGTGTCAGTCCAGCCCCGGGG CCCAGTCGTCCTGCAGCAGCGAGCCCTCTCCTCTGGGCAGTGC CCCCAACAATGACAGTGGCGTGGAGATGCCGGGGACGGGGCCC GGGAGCCTGGGAGACCTGACGGCACTGGATGACACACCCCCAG GGGCCGACACCTCAGCCCTGGCTGCCCCCTCCGCTGGTGGCCT CCAGCTGCGCAAACACATGACCACCATGCACCGGTTCGAGCAG CTCAAGAAGGAGAAGCTCAAGTCACTCAAGGATTCCTGCTCAT GGGCCGGGCCGACTCCACACACGCGGAACACCAAGCTGCCTCC CCTCCCGGGAAGTGGCTCCATCCTGGAAAACTTCAGTGGCAGT GGGGGCGGCGGGCCCGCGGGGCTGCTGCCGAACCCGCGGCTGT CGGAGCTGTCCGCGAGCGAGGTGACCATGCTGAGCCAGCTGCA GGAGCGCCGCGACAGCTCCACCAGCACGGTCAGCTCGGCCTAC ACCGTGAGCCGCCGCTCCTCCGGCATCTCCCCCTACTTCTCCA GCCGCCGCTCCAGCGAGGCCTCGCCCCTGGGCGCCGGCCGCCC GCACAACGCGAGCTCCGCTGACTCCTACGACCCCATCTCCACG GACGCGTCGCGGCGCTCGAGCGAGGCCAGCCAGTGCAGCGGCG GCTCCGGGCTGCTCAACCTCACGCCGGCGCAGCAGTACAGCCT GCGGGCCAAGTACGCGGCAGCCACTGGCGGCCCCCCGCCCACT CCGCTGCCGGGCCTGGAGCGCATGAGCCTGCGGACCAGGCTGG CGCTGCTGGACGCGCCCGAGCGCACGCTGCCCGCCGGCTGCCC ACGCCCACTGGGGCCGCGGCGTGGCAGCGACGGGCCGACCTAT GGCCACGGCCACGCGGGGGCTGCGCCCGCCTTCCCCCACGAGG CTCCAGGCGGCGGAGCCAGGCGGGCCAGCGACCCTGTGCGGCG GCCCGATGCCCTGTCCCTGCCGCGGGTGCAGCGCTTCCACAGC ACCCACAACGTGAACCCCGGCCCGCTGCCGCCCTGTGCCGACA GGCGAGGCCTCCGCCTGCAGAGCCACCCGAGCACCGACGGCGG CCTGGCCCGCGGCGCCTACTCGCCCCGGCCGCCTAGCATCAGC GAGAACGTGGCGATGGAGGCCGTGGCGGCAGGAGTGGACGGCG CGGGGCCCGAGGCCGACCTGGGGCTGCCGGAGGACGACCTGGT GCTTCCAGACGACGTGGTGCAGTACATCAAGGCGCACGCCAGT GGCGCTCTGGACGAGGGCACCGGGCAGGTGTATCCCACGGAAA GCACTGGCTTCTCTGACAACCCCAGACTACCCAGCCCGGGGCT GCACGGCCAGCGCAGGATGGTGGCTGCGGACTCCAACGTGGGC CCCTCCGCCCCTATGCTGGGAGGATGCCAGTTAGGCTTTGGGG CGCCCTCCAGCCTGAACAAAAATAACATGCCTGTGCAGTGGAA TGAGGTGAGCTCCGGCACCGTAGACGCCCTGGCCAGCCAGGTG AAGCCTCCACCCTTTCCTCAGGGCAACCTGGCGGTGGTGCAGC AGAAGCCTGCCTTTGGCCAGTACCCGGGCTACAGTCCGCAAGG CCTACAGGCTAGCCCTGGGGGCCTGGACAGCACGCAGCCACAC CTGCAGCCCCGCAGCGGAGCCCCCTCCCAGGGCATCCCCAGGG TAAACTACATGCAGCAGCTGCGACAGCCAGTGGCAGGCAGCCA GTGTCCTGGCATGACTACCACTATGAGCCCCCATGCCTGCTAT GGCCAAGTCCACCCCCAGCTGAGCCCCAGCACCATCAGTGGGG CCCTCAACCAGTTCCCCCAATCCTGCAGCAACATGCCAGCCAA GCCAGGGCATCTGGGGCACCCTCAGCAGACAGAAGTGGCACCT GACCCCACCACGATGGGCAATCGCCACAGGGAACTTGGGGTCC CCGATTCAGCCCTGGCTGGAGTGCCACCACCTCACCCAGTCCA GAGCTACCCACAGCAGAGCCATCACCTGGCAGCCTCCATGAGC CAGGAGGGCTACCACCAGGTCCCCAGCCTTCTGCCTGCCCGCC AGCCTGGCTTCATGGAGCCCCAAACAGGCCCGATGGGGGTGGC | |
| | TACAGCAGGCTTTGGCCTAGTGCAGCCCCGGCCTCCCCTCGAG CCCAGCCCCACTGGCCGCCACCGTGGGTACGTGCTGTGCAGC AGCAGCTGGCCTACGCCAGGGCCACAGGCCATGCCATGGCTGC CATGCCGTCCAGTCAGGAAACAGCAGAGGCTGTGCCCAAGGGA GCGATGGGCAACATGGGGTCGGTGCCTCCCCAGCCGCCTCCGC AGGACGCAGGTGGGGCCCCGGACCACAGCATGCTCTACTACTA CGGCCAGATCCACATGTACGAACAGGATGGAGGCCTGGAGAAC CTCGGGAGCTGCCAGGTCATGCGGTCCCAGCCACCACAGCCAC AGGCCTGTCAGGACAGCATCCAGCCCCAGCCCTTGCCCTCACC AGGGGTCAACCAGGTGTCCAGCACTGTGGACTCCCAGCTCCTG GAGGCCCCCCAGATTGACTTCGATGCCATCATGGATGATGGCG ATCACTCGAGTTTGTTCTCGGGTGCTCTGAGCCCCAGCCTCCT CCACAGCCTCTCCCAGAACTCCTCCCGCCTCACCACCCCCCGA AACTCCTTGACCCTGCCCTCCATCCCCGCAGGCATCAGCAACA TGGCTGTCGGGGACATGAGCTCCATGCTCACCAGCCTCGCCGA GGAGAGCAAGTTCCTGAACATGATGACC | |
| GLI3 | ATGGAGGCCCAGTCCCACAGCTCCACGACCACTGAAAAGAAAA AAGTTGAGAATTCCATAGTGAAGTGCTCCACTCGAACAGATGT GAGCGAGAAAGCCGTTGCCTCCAGCACCACTTCTAATGAGGAT GAAAGTCCTGGACAGACTTATCACAGAGAGAGAAGAAACGCAA TCACTATGCAGCCACAGAATGTCCAGGGGCTCAGCAAAGTCAG TGAGGAACCTTCAACATCGAGTGACGAGAGGGCCTCATTGATC AAGAAAGAGATCCATGGGTCCCTGCCACACGTGGCGGAGCCCT CTGTGCCGTACCGCGGGACGGTGTTTGCCATGGACCCCAGGAA TGGTTACATGGAGCCCCACTACCACCCTCCTCATCTTTTCCCT GCCTTCCATCCTCCTGTACCAATTGATGCCAGACATCATGAGG GCCGTTACCATTACGATCCATCTCCGATTCCTCCATTGCATAT GACTTCCGCCTTATCTAGTAGCCCTACGTATCCGGACCTGCCC TTCATTAGGATCTCCCCACACCGGAACCCCACTGCTGCTTCCG AGTCTCCCTTCAGCCCTCCACATCCCTACATTAATCCCTACAT GGACTATATCCGCTCCTTGCACAGCAGCCCATCGCTCTCCATG ATCTCAGCAACCCGTGGGCTGAGCCCTACAGATGCGCCCCATG CAGGAGTCAGCCCAGCAGAATACTATCATCAGATGGCCCTGCT AACTGGCCAGCGCAGCCCCTATGCAGACATTATTCCCTCAGCT GCCACCGCCGGCACGGGGGCCATCCACATGGAATATCTTCATG CTATGGATAGCACCAGATTCTCCAGCCCCAGGCTGTCAGCCAG GCCGAGCCGAAAACGTACACTGTCCATATCACCACTCTCCGAT CATAGCTTTGACCTTCAGACCATGATAAGGACGTCTCCCAACT CCTTGGTCACGATTCTCAATAATTCCCGTAGCAGCTCTTCAGC AAGTGGCTCCTATGGTCACTTATCTGCAAGTGCAATCAGCCCT GCCTTGAGCTTCACCTACTCTTCCGCGCCCGTCTCTCTCCACA TGCATCAGCAGATCCTAAGCCGACAACAGAGCTTAGGTTCAGC CTTTGGACACAGCCCTCCACTCATCCACCCTGCCCCAACTTTT CCAACACAGAGGCCTATTCCAGGGATCCCTACGGTTCTGAACC CCGTCCAGGTCAGCTCCGGCCCTTCTGAGTCCTCACAGAACAA GCCCACGAGTGAGTCTGCAGTGAGCAGCACTGGTGACCCGATG CACAACAAGAGGTCCAAGATCAAACCCGATGAAGACCTCCCCA GCCCAGGGGCTCGGGGGCAGCAGGAACAGCCCGAAGGAACAAC CCTTGTCAAGGAGGAAGGGGACAAAGATGAAAGCAAACAGGAG CCTGAAGTCATCTATGAGACAAACTGCCACTGGGAAGGCTGCG CGAGGGAGTTCGACACCCAAGAGCAGCTTGTGCACCATATAAA TAACGACCATATTCATGGAGAGAAGAAGGAGTTCGTGTGCAGG TGGCTGGACTGCTCAAGAGAGCAGAAACCCTTCAAAGCCCAGT ATATGTTGGTAGTGCATATGAGAAGACACACGGGCGAGAAGCC TCACAAATGCACTTTTGAAGGTTGCACAAAGGCCTACTCGAGA | 99 |
| | CTAGAAAACTTGAAAACACACTTGAGATCTCACACTGGAGAGA AACCATACGTCTGTGAGCACGAAGGTTGCAACAAGGCTTTCTC AAATGCCTCTGATCGCGCCAAACACCAAAACAGAACGCATTCC AATGAGAAACCATATGTGTGCAAAATCCCAGGCTGCACTAAGC GTTACACAGACCCAAGCTCCCTCCGGAAACATGTGAAGACAGT GCATGGCCCAGAGGCTCATGTCACCAAGAAGCAGCGAGGGGAC ATCCATCCTCGGCCGCCACCCCCGAGAGATTCCGGCAGCCATT CACAGTCCAGGTCGCCTGGCCGACCGACTCAGGGAGCCCTTGG TGAGCAGCAGGACCTCAGCAACACTACCTCAAAGCGGAAGAA TGCCTCCAGGTGAAAACCGTCAAGGCAGAGAAGCCAATGACAT CTCAGCCAAGCCCTGGTGGTCAGTCTTCATGCAGCAGCCAACA GTCCCCCATCAGCAACTATTCCAACAGTGGGCTCGAGCTTCCT CTGACCGATGGAGGTAGTATAGGAGACCTCAGTGCCATCGATG AAACCCCAATCATGGACTCAACCATTTCCACTGCAACCACAGC CCTTGCTTTGCAAGCCAGGAGAAACCCGGCAGGACCAAATGG ATGGAGCACGTAAAACTAGAAAGGCTAAAACAAGTGAATGGAA TGTTTCCGCGACTGAACCCCATTCTACCCCCTAAAGCCCCTGC GGTCTCTCCTCTCATAGGAAATGGCACACAGTCCAACAACACC TGCAGCTTGGGTGGCCCATGACGCTTCTCCCGGCAGAAGCG ACCTCTCTGGGGTGGACGTCACTATGCTGAACATGCTCAACAG AAGGGACAGCAGCGCCAGCACCATCAGCTCGGCCTACCTGAGC AGCCGCCGCTCCTCAGGGATCTCGCCCTGCTTCTCCAGCCGCC GCTCCAGCGAGGCGTCACAGGCCGAGGGCCGGCCGCAGAACGT GAGCGTGGCCGACTCCTACGACCCCATCTCCACCGACGCCTCG CGCCGCTCCAGCGAAGCCAGCCAGAGCGACGGCCTGCCCAGCC TGCTCAGCCTCACGCCCGCCCAGCAGTACCGCCTCAAGGCCAA GTACGCGGCTGCCACAGGAGGGCCGCCGCCGACGCCCCTGCCC AACATGGAGAGGATGAGCCTGAAGACGCGCCTGGCGCTGCTCG GGGATGCCCTCGAGCCTGGCGTGGCCCTGCCTCCAGTTCATGC CCCGAGGAGGTGCAGCGACGGGGGAGCCCACGGCTACGGGCGG CGCCACCTGCAGCCGCACGATGCGCCGGGCCACGGCGTGAGGA GGGCCAGCGACCCGGTGCGGACAGGCTCCGAGGGCCTGGCCCT GCCTCGTGTGCCGCGCTTCAGCAGCCTCAGCAGCTGCAACCCC CCGGCGATGGCCACGTCCGCGGAGAAGCGCAGTCTCGTGCTTC AGAATTACACGCGGCCCGAGGGCGGCCAGTCCCGAAACTTCCA CTCGTCCCCCTGTCCTCCCAGCATCACCGAGAACGTCACCCTG GAGTCCCTGACCATGGACGCTGATGCCAACCTGAACGATGAGG ATTTCCTGCCGGACGACGTGGTGCAGTATTTAAATTCCCAGAA CCAAGCAGGGTACGAGCAGCACTTCCCCAGCGCCCTCCCGGAC GACAGCAAAGTGCCCCACGGGCCCGGTGACTTTGACGCGCCCG GGCTGCCAGACAGCCACGCTGGCCAGCAGTTCCATGCCCTCGA GCAGCCCTGCCCCGAGGGCAGCAAAACCGACCTGCCCATTCAG TGGAACGAAGTCAGCTCCGGAAGCGCCGACCTGTCCTCCTCCA AGCTCAAGTGTGGGCCGCGGCCCGCTGTGCCGCAGACTCGCGC CTTTGGGTTCTGCAACGGCATGGTCGTCCACCCGCAGAACCCC TTGAGGAGCGGGCCTGCTGGGGGCTATCAGACCCTCGGGGAGA ACAGCAACCCCTACGGTGGCCCAGAGCACTTGATGCTCCACAA CAGCCCCGGAAGTGGCACCAGTGGAAACGCCTTCCATGAACAG CCCTGTAAGGCCCCGCAGTATGGGAACTGTCTCAACAGGCAGC CAGTGGCCCCTGGTGCACTCGACGGTGCCTGTGGTGCCGGGAT TCAAGCCTCAAAGCTGAAGAGCACCCCCATGCAAGGGAGCGGG GGCCAGCTGAATTTCGGCCTGCCGGTAGCGCCAAATGAGTCAG CTGGCAGCATGGTGAATGGCATGCAGAACCAGGACCCAGTGGG ACAGGGGTACCTGGCTCACCAGCTCCTCGGCGACAGCATGCAG CACCCGGGGGCAGGCCGCCCCGGTCAGCAGATGCTTGGGCAGA | |
| | TTAGTGCTACCTCACACATCAACATCTACCAAGGGCCAGAGAG CTGCCTGCCAGGGGCTCACGGCATGGGCAGCCAGCCGTCAAGC TTGGCAGTTGTCAGGGGCTACCAGCCATGTGCCAGCTTTGGGG GCAGCAGGCGCCAGGCTATGCCGAGGGACAGCCTTGCTCTGCA GTCAGGACAGCTCAGTGACACAAGTCAGACCTGCAGGGTGAAT GGTATCAAGATGGAGATGAAAGGGCAGCCCCATCCGCTGTGCT CTAATCTGCAGAATTACTCTGGTCAGTTCTATGACCAAACCGT GGGCTTCAGTCAGCAAGACACGAAAGCTGGTTCATTCTCTATT TCAGACGCCAGCTGCCTGCTACAGGGGACCAGCGCCAAAAACT CTGAGTTACTTTCCCCAGGTGCTAATCAGGTGACAAGCACAGT GGACAGCCTCGACAGCCATGACCTGGAAGGGGTACAGATTGAC TTCGATGCCATCATAGACGATGGGGACCACTCCAGCCTGATGT CGGGGGCCCTGAGCCCAAGTATCATTCAGAACCTTTCCCATAG CTCCTCCCGCCTCACCACGCCTCGGGCGTCCCTCCCATTCCCA GCGCTGTCCATGAGCACCACCAACATGGCTATCGGGGACATGA GTTCTTTGCTGACCTCCCTAGCGGAAGAAAGCAAATTCCTTGC AGTTATGCAA | |
| ETV1 | ATGGATGGATTTTATGACCAGCAAGTGCCTTACATGGTCACCA ATAGTCAGCGTGGGAGAAATTGTAACGAGAAACCAACAAATGT CAGGAAAAGAAAATTCATTAACAGAGATCTGGCTCATGATTCA GAAGAACTCTTTCAAGATCTAAGTCAATTACAGGAAACATGGC TTGCAGAAGTGGCTTTTCATGGCCTGCCACTGAAAATCAAGAA AGAACCCCACAGTCCATGTTCAGAAATCAGCTCTGCCTGCAGT CAAGAACAGCCCTTTAAATTCAGCTATGGAGAAAAGTGCCTGT ACAATGTCAGTGCCTATGATCAGAAGCCACAAGTGGGAATGAG GCCCTCCAACCCCCCCACACCATCCAGCACGCCAGTGTCCCCA CTGCATCATGCATCTCCAAACTCAACTCATACACCGAAACCTG ACCGGGCCTTCCCAGCTCACCTCCCTCCATCGCAGTCCATACC AGATAGCAGCTACCCCATGGACCACAGATTTCGCCGCCAGCTT TCTGAACCCTGTAACTCCTTTCCTCCTTTGCCGACGATGCCAA GGGAAGGACGTCCTATGTACCAACGCCAGATGTCTGAGCCAAA CATCCCCTTCCCACCACAAGGCTTTAAGCAGGAGTACCACGAC CCAGTGTATGAACACAACACCATGGTTGGCAGTGCGGCCAGCC AAAGCTTTCCCCCTCCTCTGATGATTAAACAGGAACCCAGAGA TTTTGCATATGACTCAGAAGTGCCTAGCTGCCACTCCATTTAT ATGAGGCAAGAAGGCTTCCTGGCTCATCCCAGCAGAACAGAAG GCTGTATGTTTGAAAAGGGCCCCAGGCAGTTTTATGATGACAC CTGTGTTGTCCCAGAAAAATTCGATGGAGACATCAAACAAGAG CCAGGAATGTATCGGGAAGGACCCACATACCAACGGCGAGGAT CACTTCAGCTCTGGCAGTTTTTGGTAGCTCTTCTGGATGACCC TTCAAATTCTCATTTTATTGCCTGGACTGGTCGAGGCATGGAA TTTAAACTGATTGAGCCTGAAGAGGTGGCCCGACGTTGGGGCA TTCAGAAAAACAGGCCAGCTATGAACTATGATAAACTTAGCCG TTCACTCCGCTATTACTATGAGAAAGGAATTATGCAAAAGGTG GCTGGAGAGAGATATGTCTACAAGTTTGTGTGTGATCCAGAAG CCCTTTTCTCCATGGCCTTTCCAGATAATCAGCGTCCACTGCT GAAGACAGACATGGAACGTCACATCAACGAGGAGGACACAGTG CCTCTTTCTCACTTTGATGAGAGCATGGCCTACATGCCGGAAG GGGGCTGCTGCAACCCCCACCCCTACAACGAAGGCTACGTGTA T | 100 |
| ETV2 | ATGGACCTGTGGAACTGGGATGAGGCATCCCCACAGGAAGTGC CTCCAGGGAACAAGCTGGCAGGGCTTGAAGGAGCCAAATTAGG CTTCTGTTTCCCTGATCTGGCACTCCAAGGGGACACGCCGACA GCGACAGCAGAGACATGCTGGAAAGGTCCCATTCAGCTGTGGC AGTTCCTCCTGGAGCTGCTCCACGACGGGGCGCGTAGCAGCTG | 101 |
| | CATCCGTTGGACTGGCAACAGCCGCGAGTTCCAGCTGTGCGAC CCCAAAGAGGTGGCTCGGCTGTGGGGCGAGCGCAAGAGAAAGC CGGGCATGAATTACGAGAAGCTGAGCCGGGGCCTTCGCTACTA CTATCGCCGCGACATCGTGCGCAAGAGCGGGGGGCGAAAGTAC ACGTACCGCTTCGGGGGCCGCGTGCCCAGCCTAGCCTATCCGG ACTGTGCGGGAGGCGGACGGGGAGCAGAGACACAA | |
| ETV3 | ATGAAAGCCGGCTGTAGCATCGTGGAAAAGCCAGAAGGAGGTG GAGGGTATCAGTTTCCTGACTGGGCCTACAAAACAGAGTCATC CCCAGGCTCCCGGCAGATCCAGCTGTGGCACTTCATCCTGGAG CTGCTGCAGAAGGAAGAGTTCCGCCATGTCATCGCCTGGCAGC AGGGAGAGTACGGGGAATTTGTCATCAAGGATCCAGATGAGGT GGCCCGCCTCTGGGGCCGCAGGAAATGCAAACCACAGATGAAT TATGACAAGCTGAGCCGGGCCCTCAGATACTATTACAACAAGA GGATCCTTCATAAAACAAAAGGGAAAAGATTTACCTATAAATT TAACTTCAACAAGCTGGTGATGCCCAACTACCCATTCATCAAC ATTCGGTCAAGTGGTAAGATACAAACTCTTTTGGTAGGGAAT | 102 |
| RUNX1 | ATGGCTTCAGACAGCATATTTGAGTCATTTCCTTCGTACCCAC AGTGCTTCATGAGAGAATGCATACTTGGAATGAATCCTTCTAG AGACGTCCACGATGCCAGCACGAGCCGCCGCTTCACGCCGCCT TCCACCGCGCTGAGCCCAGGCAAGATGAGCGAGGCGTTGCCGC TGGGCGCCCCGGACGCCGGCGCTGCCCTGGCCGGCAAGCTGAG GAGCGGCGACCGCAGCATGGTGGAGGTGCTGGCCGACCACCCG GGCGAGCTGGTGCGCACCGACAGCCCCAACTTCCTCTGCTCCG TGCTGCCTACGCACTGGCGCTGCAACAAGACCCTGCCCATCGC TTTCAAGGTGGTGGCCCTAGGGGATGTTCCAGATGGCACTCTG GTCACTGTGATGGCTGGCAATGATGAAAACTACTCGGCTGAGC TGAGAAATGCTACCGCAGCCATGAAGAACCAGGTTGCAAGATT TAATGACCTCAGGTTTGTCGGTCGAAGTGGAAGAGGGAAAAGC TTCACTCTGACCATCACTGTCTTCACAAACCCACCGCAAGTCG CCACCTACCACAGAGCCATCAAAATCACAGTGGATGGCCCCG AGAACCTCGAAGACATCGGCAGAAACTAGATGATCAGACCAAG CCCGGGAGCTTGTCCTTTTCCGAGCGGCTCAGTGAACTGGAGC AGCTGCGGCGCACAGCCATGAGGGTCAGCCCACACCACCCAGC CCCCACGCCCAACCCTCGTGCCTCCCTGAACCACTCCACTGCC TTTAACCCTCAGCCTCAGAGTCAGATGCAGGATACAAGGCAGA TCCAACCATCCCCACCGTGGTCCTACGATCAGTCCTACCAATA CCTGGGATCCATTGCCTCTCCTTCTGTGCACCCAGCAACGCCC ATTTCACCTGGACGTGCCAGCGGCATGACAACCCTCTCTGCAG AACTTTCCAGTCGACTCTCAACGGCACCCGACCTGACAGCGTT CAGCGACCCGCGCCAGTTCCCCGCGCTGCCCTCCATCTCCGAC CCCCGCATGCACTATCCAGGCGCCTTCACCTACTCCCCGACGC CGGTCACCTCGGGCATCGGCATCGGCATGTCGGCCATGGGCTC GGCCACGCGCTACCACACCTACCTGCCGCCGCCCTACCCCGGC TCGTCGCAAGCGCAGGGAGGCCCGTTCCAAGCCAGCTCGCCCT CCTACCACCTGTACTACGGCGCCTCGGCCGGCTCCTACCAGTT CTCCATGGTGGGCGGCGAGCGCTCGCCGCCGCGCATCCTGCCG CCCTGCACCAACGCCTCCACCGGCTCCGCGCTGCTCAACCCCA GCCTCCCGAACCAGAGCGACGTGGTGGAGGCCGAGGGCAGCCA CAGCAACTCCCCCACCAACATGGCGCCCTCCGCGCGCCTGGAG GAGGCCGTGTGGAGGCCCTAC | 103 |
| RUNX2 | ATGGCATCAAACAGCCTCTTCAGCACAGTGACACCATGTCAGC AAAACTTCTTTTGGGATCCGAGCACCAGCCGGCGCTTCAGCCC CCCCTCCAGCAGCCTGCAGCCCGGCAAAATGAGCGACGTGAGC CCGGTGGTGGCTGCGCAACAGCAGCAGCAACAGCAGCAGCAGC AACAGCAGCAGCAGCAGCAGCAACAGCAGCAGCAGCAGCAGGA | 104 |
| | GGCGGCGGCGGCGGCTGCGGCGGCGGCGGCTGCGGCGGCG GCAGCTGCAGTGCCCCGGTTGCGGCCGCCCCACGACAACCGCA CCATGGTGGAGATCATCGCCGACCACCCGGCCGAACTCGTCCG CACCGACAGCCCCAACTTCCTGTGCTCGGTGCTGCCCTCGCAC TGGCGCTGCAACAAGACCCTGCCCGTGGCCTTCAAGGTGGTAG CCCTCGGAGAGGTACCAGATGGGACTGTGGTTACTGTCATGGC GGGTAACGATGAAAATTATTCTGCTGAGCTCCGGAATGCCTCT GCTGTTATGAAAAACCAAGTAGCAAGGTTCAACGATCTGAGAT TTGTGGGCCGGAGTGGACGAGGCAAGAGTTTCACCTTGACCAT AACCGTCTTCACAAATCCTCCCCAAGTAGCTACCTATCACAGA GCAATTAAAGTTACAGTAGATGGACCTCGGGAACCCAGAAGGC ACAGACAGAAGCTTGATGACTCTAAACCTAGTTTGTTCTCTGA CCGCCTCAGTGATTTAGGGCGCATTCCTCATCCCAGTATGAGA GTAGGTGTCCCGCCTCAGAACCCACGGCCCTCCCTGAACTCTG CACCAAGTCCTTTTAATCCACAAGGACAGAGTCAGATTACAGA CCCCAGGCAGGCACAGTCTTCCCCGCCGTGGTCCTATGACCAG TCTTACCCCTCCTACCTGAGCCAGATGACGTCCCCGTCCATCC ACTCTACCACCCCGCTGTCTTCCACACGGGGCACTGGGCTTCC TGCCATCACCGATGTGCCTAGGCGCATTTCAGGTGCTTCAGAA CTGGGCCCTTTTTCAGACCCCAGGCAGTTCCCAAGCATTTCAT CCCTCACTGAGAGCCGCTTCTCCAACCCACGAATGCACTATCC AGCCACCTTTACTTACACCCCGCCAGTCACCTCAGGCATGTCC CTCGGTATGTCCGCCACCACTCACTACCACACCTACCTGCCAC CACCCTACCCCGGCTCTTCCCAAAGCCAGAGTGGACCCTTCCA GACCAGCAGCACTCCATATCTCTACTATGGCACTTCGTCAGGA TCCTATCAGTTTCCCATGGTGCCGGGGGAGACCGGTCTCCTT CCAGAATGCTTCCGCCATGCACCACCACCTCGAATGGCAGCAC GCTATTAAATCCAAATTTGCCTAACCAGAATGATGGTGTTGAC GCTGATGGAAGCCACAGCAGTTCCCCAACTGTTTTGAATTCTA GTGGCAGAATGGATGAATCTGTTTGGCGACCATAT | |
| RUNX3 | ATGCGTATTCCCGTAGACCCAAGCACCAGCCGCCGCTTCACAC CTCCCTCCCCGGCCTTCCCCTGCGGCGGCGGCGGCAAGAT GGGCGAGAACAGCGGCGCGCTGAGCGCGCAGGCGGCCGTGGGG CCCGGAGGGCGCGCCCGGCCCGAGGTGCGCTCGATGGTGGACG TGCTGGCGGACCACGCAGGCGAGCTCGTGCGCACCGACAGCCC CAACTTCCTCTGCTCCGTGCTGCCCTCGCACTGGCGCTGCAAC AAGACGCTGCCCGTCGCCTTCAAGGTGGTGGCATTGGGGGACG TGCCGGATGGTACGGTGGTGACTGTGATGGCAGGCAATGACGA GAACTACTCCGCTGAGCTGCGCAATGCCTCGGCCGTCATGAAG AACCAGGTGGCCAGGTTCAACGACCTTCGCTTCGTGGCCGCA GTGGGCGAGGGAAGAGTTTCACCCTGACCATCACTGTGTTCAC CAACCCCACCCAAGTGGCGACCTACCACCGAGCCATCAAGGTG ACCGTGGACGGACCCCGGGAGCCCAGACGGCACCGGCAGAAGC TGGAGGACCAGACCAAGCCGTTCCCTGACCGCTTTGGGGACCT GGAACGGCTGCGCATGCGGGTGACACCGAGCACACCCAGCCCC CGAGGCTCACTCAGCACCACAAGCCACTTCAGCAGCCAGCCCC AGACCCCAATCCAAGGCACCTCGGAACTGAACCCATTCTCCGA CCCCCGCCAGTTTGACCGCTCCTTCCCCACGCTGCCAACCCTC ACGGAGAGCCGCTTCCCAGACCCCAGGATGCATTATCCCGGGG CCATGTCAGCTGCCTTCCCCTACAGCGCCACGCCCTCGGGCAC GAGCATCAGCAGCCTCAGCGTGGCGGGCATGCCGGCCACCAGC CGCTTCCACCATACCTACCTCCCGCCACCCTACCCGGGGGCCC CGCAGAACCAGAGCGGGCCCTTCCAGGCCAACCCGTCCCCCTA CCACCTCTACTACGGGACATCCTCTGGCTCCTACCAGTTCTCC ATGGTGGCCGGCAGCAGCAGTGGGGGCGACCGCTCACCTACCC | 105 |
| | GCATGCTGGCCTCTTGCACCAGCAGCGCTGCCTCTGTCGCCGC CGGCAACCTCATGAACCCCAGCCTGGGCGGCCAGAGTGATGGC GTGGAGGCCGACGGCAGCCACAGCAACTCACCCACGGCCCTGA GCACGCCAGGCCGCATGGATGAGGCCGTGTGGCGGCCCTAC | |
| MAFB | ATGGCCGCGGAGCTGAGCATGGGGCCAGAGCTGCCCACCAGCC CGCTGGCCATGGAGTATGTCAACGACTTCGACCTGCTCAAGTT CGACGTGAAGAAGGAGCCACTGGGGCGCGCGGAGCGTCCGGGC AGGCCCTGCACACGCCTGCAGCCAGCCGGCTCGGTGTCCTCCA CACCGCTCAGCACTCCGTGTAGCTCCGTGCCCTCGTCGCCCAG CTTCAGCCCGACCGAACAGAAGACACACCTCGAGGATCTGTAC TGGATGGCGAGCAACTACCAGCAGATGAACCCCGAGGCGCTCA ACCTGACGCCCGAGGACGCGGTGGAAGCGCTCATCGGCTCGCA CCCAGTGCCACAGCCGCTGCAAAGCTTCGACAGCTTTCGCGGC GCTCACCACCACCACCATCACCACCACCCTCACCCGCACCACG CGTACCCGGGCGCCGGCGTGGCCCACGACGAGCTGGGCCCGCA CGCTCACCCGCACCATCACCATCATCACCAAGCGTCGCCGCCG CCGTCCAGCGCCGCTAGCCCGGCGCAACAGCTGCCCACTAGCC ACCCCGGGCCCGGGCCGCACGCGACGGCCTCGGCGACGGCGGC GGGCGGCAACGGCAGCGTGGAGGACCGCTTCTCCGACGACCAG CTCGTGTCCATGTCCGTGCGCGAGCTGAACCGCCACCTGCGGG GCTTCACCAAGGACGAGGTGATCCGCCTGAAGCAGAAGCGGCG GACCCTGAAGAACCGGGGCTACGCCCAGTCTTGCAGGTATAAA CGCGTCCAGCAGAAGCACCACCTGGAGAATGAGAAGACGCAGC TCATTCAGCAGGTGGAGCAGCTTAAGCAGGAGGTGTCCCGGCT GGCCCGCGAGAGAGACGCCTACAAGGTCAAGTGCGAGAAACTC GCCAACTCCGGCTTCAGGGAGGCGGGCTCCACCAGCGACAGCC CCTCCTCTCCCGAGTTCTTTCTG | 106 |
| TFAP2C | ATGTTGTGGAAAATAACCGATAATGTCAAGTACGAAGAGGACT GCGAGGATCGCCACGACGGAGCAGCAATGGGAATCCGCGGGT CCCCCACCTCTCCTCCGCCGGGCAGCACCTCTACAGCCCCGCG CCACCCCTCTCCCACACTGGAGTCGCCGAATATCAGCCGCCAC CCTACTTTCCCCCTCCCTACCAGCAGCTGGCCTACTCCCAGTC GGCCGACCCCTACTCGCATCTGGGGGAAGCGTACGCCGCCGCC ATCAACCCCCTGCACCAGCCGGCGCCCACAGGCAGCCAGCAGC AGGCCTGGCCCGGCCGCCAGAGCCAGGAGGGAGCGGGGCTGCC CTCGCACCACGGGCGCCCGGCCGGCCTACTGCCCCACCTCTCC GGGCTGGAGGCGGGCGCGGTGAGCGCCCGCAGGGATGCCTACC GCCGCTCCGACCTGCTGCTGCCCCACGCACACGCCCTGGATGC CGCGGGCCTGGCCGAGAACCTGGGGCTCCACGACATGCCTCAC CAGATGGACGAGGTGCAGAATGTCGACGACCAGCACCTGTTGC TGCACGATCAGACAGTCATTCGCAAAGGTCCCATTTCCATGAC CAAGAACCCTCTGAACCTCCCCTGTCAGAAGGAGCTGGTGGGG GCCGTAATGAACCCCACTGAGGTCTTCTGCTCAGTCCCTGGAA GATTGTCGCTCCTCAGCTCTACGTCTAAATACAAAGTGACAGT GGCTGAAGTACAGAGGCGACTGTCCCCACCTGAATGCTTAAAT GCCTCGTTACTGGGAGGTGTTCTCAGAAGAGCCAAATCGAAAA ATGGAGGCCGGTCCTTGCGGGAGAAGTTGGACAAGATTGGGTT GAATCTTCCGGCCGGGAGGCGGAAAGCCGCTCATGTGACTCTC CTGACATCCTTAGTAGAAGGTGAAGCTGTTCATTTGGCTAGGG ACTTTGCCTATGTCTGTGAAGCCGAATTTCCTAGTAAACCAGT GGCAGAATATTTAACCAGACCTCATCTTGGAGGACGAAATGAG ATGGCAGCTAGGAAGAACATGCTATTGGCGGCCCAGCAACTGT GTAAAGAATTCACAGAACTTCTCAGCCAAGACCGGACACCCCA TGGGACCAGCAGGCTCGCCCCAGTCTTGGAGACGAACATACAG AACTGCTTGTCTCATTTCAGCCTGATTACCCACGGGTTTGGCA | 107 |
| | GCCAGGCCATCTGTGCCGCGGTGTCTGCCCTGCAGAACTACAT CAAAGAAGCCCTGATTGTCATAGACAAATCCTACATGAACCCT GGAGACCAGAGTCCAGCTGATTCTAACAAAACCCTGGAGAAAA TGGAGAAACACAGGAAA | |
| E2F1 | ATGGCCTTGGCCGGGGCCCCTGCGGGCGGCCCATGCGCGCCGG CGCTGGAGGCCCTGCTCGGGGCCGGCGCGCTGCGGCTGCTCGA CTCCTCGCAGATCGTCATCATCTCCGCCGCGCAGGACGCCAGC GCCCCGCCGGCTCCCACCGGCCCCGCGGCGCCCGCCGCCGGCC CCTGCGACCCTGACCTGCTGCTCTTCGCCACACCGCAGGCGCC CCGGCCCACACCCAGTGCGCCGCGGCCCGCGCTCGGCCGCCCG CCGGTGAAGCGGAGGCTGGACCTGGAAACTGACCATCAGTACC TGGCCGAGAGCAGTGGGCCAGCTCGGGGCAGAGGCCGCCATCC AGGAAAAGGTGTGAAATCCCCGGGGGAGAAGTCACGCTATGAG ACCTCACTGAATCTGACCACCAAGCGCTTCCTGGAGCTGCTGA GCCACTCGGCTGACGGTGTCGTCGACCTGAACTGGGCTGCCGA GGTGCTGAAGGTGCAGAAGCGGCGCATCTATGACATCACCAAC GTCCTTGAGGGCATCCAGCTCATTGCCAAGAAGTCCAAGAACC ACATCCAGTGGCTGGGCAGCCACACCACAGTGGGCGTCGGCGG ACGGCTTGAGGGGTTGACCCAGGACCTCCGACAGCTGCAGGAG AGCGAGCAGCAGCTGGACCACCTGATGAATATCTGTACTACGC AGCTGCGCCTGCTCTCCGAGGACACTGACAGCCAGCGCCTGGC CTACGTGACGTGTCAGGACCTTCGTAGCATTGCAGACCCTGCA GAGCAGATGGTTATGGTGATCAAAGCCCCTCCTGAGACCCAGC TCCAAGCCGTGGACTCTTCGGAGAACTTTCAGATCTCCCTTAA GAGCAAACAAGGCCCGATCGATGTTTTCCTGTGCCCTGAGGAG ACCGTAGGTGGGATCAGCCCTGGGAAGACCCCATCCCAGGAGG TCACTTCTGAGGAGGAGAACAGGGCCACTGACTCTGCCACCAT AGTGTCACCACCACCATCATCTCCCCCCTCATCCCTCACCACA GATCCCAGCCAGTCTCTACTCAGCCTGGAGCAAGAACCGCTGT TGTCCCGGATGGGCAGCCTGCGGGCTCCCGTGGACGAGGACCG CCTGTCCCCGCTGGTGGCGGCCGACTCGCTCCTGGAGCATGTG CGGGAGGACTTCTCCGGCCTCCTCCCTGAGGAGTTCATCAGCC TTTCCCCACCCCACGAGGCCCTCGACTACCACTTCGGCCTCGA GGAGGGCGAGGGCATCAGAGACCTCTTCGACTGTGACTTTGGG GACCTCACCCCCCTGGATTTC | 108 |

The transcription factor DNA-binding domain may, for example, comprise an amino acid sequence that has at least 70%, 80%, 90%, 95%, 96%, 97%, 98%, 99% or 100% identity to any one of SEQ ID NOs: 33-70, preferably wherein the amino acid sequence substantially retains the natural function of the protein represented by SEQ ID NO: 33-70, respectively.

The transcription factor DNA-binding domain may, for example, be encoded by a polynucleotide comprising a nucleic acid sequence which encodes a protein that has at least 70%, 80%, 90%, 95%, 96%, 97%, 98%, 99% or 100% amino acid identity to any one of SEQ ID NOs: 33-70, preferably wherein the amino acid sequence substantially retains the natural function of the protein represented by SEQ ID NO: 33-70, respectively.

The transcription factor DNA-binding domain may, for example, be encoded by a polynucleotide comprising a nucleic acid sequence that has at least 70%, 80%, 90%, 95%, 96%, 97%, 98%, 99% or 100% sequence identity to any one of SEQ ID NOs: 71-108, preferably wherein the encoded amino acid sequence substantially retains the natural function of the protein encoded by SEQ ID NO: 71-108, respectively.

### Exemplary ESFs

In some embodiments, the ESF comprises or consists of a KRAB domain, a SOX2 DNA-binding domain, a DNMT3A domain and a DNMT3L domain.

In some embodiments, the ESF comprises or consists of a CS domain and a SOX2 DNA-binding domain.

In some embodiments, the ESF comprises or consists of a SOX2 DNA-binding domain and a Y-R domain.

In some embodiments, the ESF comprises or consists of a KRAB domain, a TEAD1 DNA-binding domain, a DNMT3A domain and a DNMT3L domain.

In some embodiments, the ESF comprises or consists of a KRAB domain, a DNMT3A domain, a DNMT3L domain and a MYC DNA-binding domain.

Example sequences of ESFs of the invention are:
KRAB-hSOX2₁₋₁₇₉-DNMT3a3L 
MDAKSLTAWSRTLVTFKDVFVDFTREEWKLLDTAQQILYRNVMLENYKNLVSLGYQLTKPDV
ILRLEKGEEPWLVEREIHQETHPDSETAFEIKSSVGSGACMYNMMETELKPPGPQQTSGGGG
GNSTAAAAGGNQKNSPDRVKRPMNAFMVWSRGQRRKMAQENPKMHNSEISKRLGAEWKLLSE
TEKRPFIDEAKRLRALHMKEHPDYKYRPRRKTKTLMKKDKYTLPGGLLAPGGNSMASGVGVG
AGLGAGVNQRMDSYAHMNGWSNGSYSMMQDQLGASGSGNHDQEFDPPKVYPPVPAEKRKPIR
VLSLFDGIATGLLVLKDLGIQVDRYIASEVCEDSITVGMVRHQGKIMYVGDVRSVTQKHIQE
WGPFDLVIGGSPCNDLSIVNPARKGLYEGTGRLFFEFYRLLHDARPKEGDDRPFFWLFENVV
AMGVSDKRDISRFLESNPVMIDAKEVSAAHRARYFWGNLPGMNRPLASTVNDKLELQECLEH
GRIAKFSKVRTITTRSNSIKQGKDQHFPVFMNEKEDILWCTEMERVFGFPVHYTDVSNMSRL
ARQRLLGRSWSVPVIRHLFAPLKEYFACVSSGNSNANSRGPSFSSGLVPLSLRGSHMGPMEI
YKTVSAWKRQPVRVLSLFRNIDKVLKSLGFLESGSGSGGGTLKYVEDVTNVVRRDVEKWGPF
DLVYGSTQPLGSSCDRCPGWYMFQFHRILQYALPRQESQRPFFWIFMDNLLLTEDDQETTTR
FLQTEAVTLQDVRGRDYQNAMRVWSNIPGLKSKHAPLTPKEEEYLQAQVRSRSKLDAPKVDL
LVKNCLLPLREYFKYFSQNSLPL

(SEQ ID NO: 109)
   **KRAB**
   hSOX2₁₋₁₇₉
   *DNMT3a3L*
CS-hSOX2₁₋₁₇₉ 
MLEPEKIIGATDSCGDLMFLMKWKDTDEADLVLAKEANVKCPQIVIAFYEERLTWHAYPEAC
MYNMMETELKPPGPQQTSGGGGGNSTAAAAGGNQKNSPDRVKRPMNAFMVWSRGQRRKMAQE
NPKMHNSEISKRLGAEWKLLSETEKRPFIDEAKRLRALHMKEHPDYKYRPRRKTKTLMKKDK
YTLPGGLLAPGGNSMASGVGVGAGLGAGVNQRMDSYAHMNGWSNGSYSMMQDQLG

(SEQ ID NO: 110)
   **CS**
   hSOX2₁₋₁₇₉
SOX2₁₋₁₇₉-Y-R 
MYNMMETELKPPGPQQTSGGGGGNSTAAAAGGNQKNSPDRVKRPMNAFMVWSRGQRRKMAQE
NPKMHNSEISKRLGAEWKLLSETEKRPFIDEAKRLRALHMKEHPDYKYRPRRKTKTLMKKDK
YTLPGGLLAPGGNSMASGVGVGAGLGAGVNQRMDSYAHMNGWSNGSYSMMQDQLGASGSNMR
PRLKNVDRSTAQQLAVTVGNVTVIITDFKEKTRSSSTSSSTVTSSAGSEQQNQ

(SEQ ID NO: 111)
   hSOX2₁₋₁₇₉
   **Y-R**
KRAB-hTEAD₁₋₁₆₆-DNMT3a3L 
MDAKSLTAWSRTLVTFKDVFVDFTREEWKLLDTAQQILYRNVMLENYKNLVSLGYQLTKPDV
ILRLEKGEEPWLVEREIHQETHPDSETAFEIKSSVGSGACIEPSSWSGSESPAENMERMSDS
ADKPIDNDAEGVWSPDIEQSFQEALAIYPPCGRRKIILSDEGKMYGRNELIARYIKLRTGKT
RTRKQVSSHIQVLARRKSRDFHSKLKDQTAKDKALQHMAAMSSAQIVSATAIHNKLGLPGIP
RPTFPGAPGFWPGMIQTGQPASGSGNHDQEFDPPKVYPPVPAEKRKPIRVLSLFDGIATGLL
VLKDLGIQVDRYIASEVCEDSITVGMVRHQGKIMYVGDVRSVTQKHIQEWGPFDLVIGGSPC
NDLSIVNPARKGLYEGTGRLFFEFYRLLHDARPKEGDDRPFFWLFENVVAMGVSDKRDISRF
LESNPVMIDAKEVSAAHRARYFWGNLPGMNRPLASTVNDKLELQECLEHGRIAKFSKVRTIT
TRSNSIKQGKDQHFPVFMNEKEDILWCTEMERVFGFPVHYTDVSNMSRLARQRLLGRSWSVP
VIRHLFAPLKEYFACVSSGNSNANSRGPSFSSGLVPLSLRGSHMGPMEIYKTVSAWKRQPVR
VLSLFRNIDKVLKSLGFLESGSGSGGGTLKYVEDVTNVVRRDVEKWGPFDLVYGSTQPLGSS
CDRCPGWYMFQFHRILQYALPRQESQRPFFWIFMDNLLLTEDDQETTTRFLQTEAVTLQDVR
GRDYQNAMRVWSNIPGLKSKHAPLTPKEEEYLQAQVRSRSKLDAPKVDLLVKNCLLPLREYF
KYFSQNSLPL

(SEQ ID NO: 112)
   **KRAB**
   hTEAD1₁₋₁₆₆
   *DNMT3a3L*
KRAB-DNMT3a3L-hMYC₁₄₄₋₄₅₄ 
MDAKSLTAWSRTLVTFKDVFVDFTREEWKLLDTAQQILYRNVMLENYKNLVSLGYQLTKPDV
ILRLEKGEEPWLVEREIHQETHPDSETAFEIKSSVASGSGNHDQEFDPPKVYPPVPAEKRKP
 
IRVLSLFDGIATGLLVLKDLGIQVDRYIASEVCEDSITVGMVRHQGKIMYVGDVRSVTQKHI
QEWGPFDLVIGGSPCNDLSIVNPARKGLYEGTGRLFFEFYRLLHDARPKEGDDRPFFWLFEN
VVAMGVSDKRDISRFLESNPVMIDAKEVSAAHRARYFWGNLPGMNRPLASTVNDKLELQECL
EHGRIAKFSKVRTITTRSNSIKQGKDQHFPVFMNEKEDILWCTEMERVFGFPVHYTDVSNMS
RLARQRLLGRSWSVPVIRHLFAPLKEYFACVSSGNSNANSRGPSFSSGLVPLSLRGSHMGPM
EIYKTVSAWKRQPVRVLSLFRNIDKVLKSLGFLESGSGSGGGTLKYVEDVTNVVRRDVEKWG
PFDLVYGSTQPLGSSCDRCPGWYMFQFHRILQYALPRQESQRPFFWIFMDNLLLTEDDQETT
TRFLQTEAVTLQDVRGRDYQNAMRVWSNIPGLKSKHAPLTPKEEEYLQAQVRSRSKLDAPKV
DLLVKNCLLPLREYFKYFSQNSLPLGAPAAAIIQDCMWSGFSAAAKLVSEKLASYQAARKDS
GSPNPARGHSVCSTSSLYLQDLSAAASECIDPSVVFPYPLNDSSSPKSCASQDSSAFSPSSD
SLLSSTESSPQGSPEPLVLHEETPPTTSSDSEEEQEDEEEIDVVSVEKRQAPGKRSESGSPS
AGGHSKPPHSPLVLKRCHVSTHQHNYAAPPSTRKDYPAAKRVKLDSVRVLRQISNNRKCTSP
RSSDTEENVKRRTHNVLERQRRNELKRSFFALRDQIPELENNEKAPKVVILKKATAYILSVQ
AEEQKLISEEDLLRKRREQLKHKLEQLRNSCA

(SEQ ID NO: 113)
   **KRAB**
   *DNMT3a3L*
   hMYC₁₄₄₋₄₅₄

Example nucleotide sequences encoding ESFs of the invention are
KRAB-hSOX2₁₋₁₇₉-DNMT3a3L 
ATGGATGCTAAGTCACTGACTGCCTGGTCCCGGACACTGGTGACCTTCAAGGATGTGTTTGT
GGACTTCACCAGGGAGGAGTGGAAGCTGCTGGACACTGCTCAGCAGATCCTGTACAGAAATG
TGATGCTGGAGAACTATAAGAACCTGGTTTCCTTGGGTTATCAGCTTACTAAGCCAGATGTG
ATCCTCCGGTTGGAGAAGGGAGAAGAGCCCTGGCTGGTGGAGAGAGAAATTCACCAAGAGAC
CCATCCTGATTCAGAGACTGCATTTGAAATCAAATCATCAGTTGGCAGCGGCGCATGCatgt
acaacatgatggagacggagctgaagccgccgggcccgcagcaaacttcgggggcggcggc
ggcaactccaccgcggcggcggccggcggcaaccagaaaaacagcccggaccgcgtcaagcg
gcccatgaatgccttcatggtgtggtcccgcgggcagcggcgcaagatggcccaggagaacc
ccaagatgcacaactcggagatcagcaagcgcctgggcgccgagtggaaacttttgtcggag
acggagaagcggccgttcatcgacgaggctaagcggctgcgagcgctgcacatgaaggagca
cccggattataaataccggccccggcggaaaaccaagacgctcatgaagaaggataagtaca
cgctgcccggcgggctgctggcccccggcggcaatagcatggcgagcggggtcggggtgggc
gccggcctgggcgcgggcgtgaaccagcgcatggacagttacgcgcacatgaacggctggag
caacggcagctacagcatgatgcaggaccagctgggcGCTAGCGGCAGCGGCAACCATGATC
AAGAGTTCGATCCTCCTAAAGTGTATCCCCCTGTCCCTGCCGAAAAGCGGAAACCGATCAGG
GTTCTCAGTCTCTTCGATGGAATTGCCACAGGCCTCTTGGTCCTTAAGGACCTGGGCATCCA
AGTgGAcCGCTACATCGCCTCAGAAGTATGTGAgGACAGCATCACAGTCGGCATGGTGCGCC
ACCAGGGGAAAATAATGTACGTCGGCGACGTCAGATCAGTTACTCAGAAACATATTCAGGAA
TGGGGGCCTTTTGATCTCGTAATTGGCGGCAGCCCCTGCAATGATCTGAGTATTGTGAACCC
CGCCCGGAAAGGTTTGTACGAAGGTACGGCAGACTGTTCTTCGAGTTTTACAGACTGTTGC
ACGACGCACGACCAAAGGAGGGTGAcGAtcGACCCTTCTTCTGGCTGTTCGAGAACGTGGTC
GCTATGGGCGTGTCTGACAAGCGGGACATTTCtAGATTCCTGGAGAGCAATCCAGTGATGAT
TGATGCAAAGGAAGTAtccGCTGCCCACCGCGCCAGATACTTCTGGGGCAATCTGCCCGGCA
 
TGAATCGACCCTTGGCAAGCACCGTGAATGATAAGTTGGAGTTGCAGGAGTGTCTgGAGCAT
GGGCGGATCGCAAAGTTTAGCAAGGTGCGGACCATCACGACCCGAAGCAATTCAATTAAGCA
GGGAAAGGACCAACATTTTCCAGTGTTTATGAACGAGAAAGAGGAcATCCTGTGGTGcACAG
AAATGGAGCGGGTTTTCGGATTCCCCGTACATTATACTGATGTGTCCAATATGTCTAGGTTG
GCTAGACAGAGGCTGtTGGGACGCTCATGGAGTGTTCCTGTCATCCGCCACCTGTTCGCTCC
TCTGAAGGAGTATTTCGCcTGCGTtTCATCCGGgAATTCAAACGCAAACAGCAGAGGCCCAT
CCTTTTCTTCCGGCCTGGTgCCACTTAGTCTGCGCGGCTCTCACATGGACCTATGGAAATA
TACAAAACAGTGAGCGCCTGGAAGAGGCAGCCTGTGCGGGTCCTGAGTCTGTTCAGAAACAT
TGACAAGGTGCTTAAGTCCCTTGGATTTCTGGAGTCTGGCAGCGGTTCTGGGGGTGgcACCC
TTAAGTATGTGGAGGACGTGACAAACGTCGTGAGGAGAGATGTGGAGAAATGGGGGCCATTT
GACCTGGTATACGGGAGCACTCAACCTCTCGGTTCTTCTTGTGATCGCTGCCCAGGGTGGTA
TATGTTTCAGTTCCATCGcATCCTGCAATACGCCCTTCCGCGGCAGGAGAGTCAGCGACCAT
TCTTCTGGATATTCATGGACAATCTCCTGCTGACAGAgGACGACCAAGAGACTACGACTAGA
TTTCTTCAGACCGAGGCCGTTACTCTCCAGGACGTTAGAGGTAGGGACTACCAGAATGCCAT
GCGAGTGTGGAGTAACATCCCCGGACTCAAGTCAAAGCAcGCACCCCTGACCCCCAAGGAAG
AGGAATACCTcCAGGCGCAGGTGAGGAGCCGCAGTAAGCTCGATGCTCCTAAAGTCGATTTG
TTGGTGAAGAATTGCTTGCTGCCCCTGAGAGAGTACTTTAAGTACTTTAGCCAGAACAGCTT
GCCACTG

(SEQ ID NO: 114)
   KRAB
   *hSOX2₁₋₁₇₉*
   **DNMT3a3L**
KRAB-hSOX2₁₋₁₇₉-DNMT3a3L 
ATGGATGCTAAGTCACTGACTGCCTGGTCCCGGACACTGGTGACCTTCAAGGATGTGTTTGT
GGACTTCACCAGGGAGGAGTGGAAGCTGCTGGACACTGCTCAGCAGATCCTGTACAGAAATG
TGATGCTGGAGAACTATAAGAACCTGGTTTCCTTGGGTTATCAGCTTACTAAGCCAGATGTG
ATCCTCCGGTTGGAGAAGGGAGAAGAGCCCTGGCTGGTGGAGAGAGAAATTCACCAAGAGAC
CCATCCTGATTCAGAGACTGCATTTGAAATCAAATCATCAGTTGGCAGCGGCGCATGCatgt
acaacatgatggagacggagctgaagccgccgggcccgcagcaaacttcggggggcggcggc
ggcaactccaccgcggcggcggccggcggcaaccagaaaaacagcccggaccgcgtcaagcg
gcccatgaatgccttcatggtgtggtcccgcgggcagcggcgcaagatggcccaggagaacc
ccaagatgcacaactcggagatcagcaagcgcctgggcgccgagtggaaacttttgtcggag
acggagaagcggccgttcatcgacgaggctaagcggctgcgagcgctgcacatgaaggagca
cccggattataaataccggccccggcggaaaaccaagacgctcatgaagaaggataagtaca
cgctgcccggcgggctgctggcccccggcggcaatagcatggcgagcggggtcggggtgggc
gccggcctgggcgcgggcgtgaaccagcgcatggacagttacgcgcacatgaacggctggag
caacggcagctacagcatgatgcaggaccagctgggcGCTAGCGGCAGCGGCAACCATGATC
AAGAGTTCGATCCTCCTAAAGTGTATCCCCCTGTCCCTGCCGAAAAGCGGAAACCGATCAGG
GTTCTCAGTCTCTTCGATGGAATTGCCACAGGCCTCTTGGTCCTTAAGGACCTGGGCATCCA
AGTgGAcCGCTACATCGCCTCAGAAGTATGTGAgGACAGCATCACAGTCGGCATGGTGCGCC
ACCAGGGGAAAATAATGTACGTCGGCGACGTCAGATCAGTTACTCAGAAACATATTCAGGAA
TGGGGGCCTTTTGATCTCGTAATTGGCGGCAGCCCCTGCAATGATCTGAGTATTGTGAACCC
CGCCCGGAAAGGTTTGTACGAAGGTACGGCAGACTGTTCTTCGAGTTTTACAGACTGTTGC
ACGACGCACGACCAAAGGAGGGTGAcGAtcGACCCTTCTTCTGGCTGTTCGAGAACGTGGTC
 
GCTATGGGCGTGTCTGACAAGCGGGACATTTCtAGATTCCTGGAGAGCAATCCAGTGATGAT
TGATGCAAAGGAAGTAtccGCTGCCCACCGCGCCAGATACTTCTGGGGCAATCTGCCCGGCA
TGAATCGACCCTTGGCAAGCACCGTGAATGATAAGTTGGAGTTGCAGGAGTGTCTgGAGCAT
GGGCGGATCGCAAAGTTTAGCAAGGTGCGGACCATCACGACCCGAAGCAATTCAATTAAGCA
GGGAAAGGACCAACATTTTCCAGTGTTTATGAACGAGAAAGAGGAcATCCTGTGGTGcACAG
AAATGGAGCGGGTTTTCGGATTCCCCGTACATTATACTGATGTGTCCAATATGTCTAGGTTG
GCTAGACAGAGGCTGtTGGGACGCTCATGGAGTGTTCCTGTCATCCGCCACCTGTTCGCTCC
TCTGAAGGAGTATTTCGCcTGCGTtTCATCCGGgAATTCAAACGCAAACAGCAGAGGCCCAT
CCTTTTCTTCCGGCCTGGTgCCACTTAGTCTGCGCGGCTCTCACATGGGACCTATGGAAATA
TACAAAACAGTGAGCGCCTGGAAGAGGCAGCCTGTGCGGGTCCTGAGTCTGTTCAGAAACAT
TGACAAGGTGCTTAAGTCCCTTGGATTTCTGGAGTCTGGCAGCGGTTCTGGGGGTGgcACCC
TTAAGTATGTGGAGGACGTGACAAACGTCGTGAGGAGAGATGTGGAGAAATGGGGGCCATTT
GACCTGGTATACGGGAGCACTCAACCTCTCGGTTCTTCTTGTGATCGCTGCCCAGGGTGGTA
TATGTTTCAGTTCCATCGcATCCTGCAATACGCCCTTCCGCGGCAGGAGAGTCAGCGACCAT
TCTTCTGGATATTCATGGACAATCTCCTGCTGACAGAgGACGACCAAGAGACTACGACTAGA
TTTCTTCAGACCGAGGCCGTTACTCTCCAGGACGTTAGAGGTAGGGACTACCAGAATGCCAT
GCGAGTGTGGAGTAACATCCCCGGACTCAAGTCAAAGCAcGCACCCCTGACCCCCAAGGAAG
AGGAATACCTcCAGGCGCAGGTGAGGAGCCGCAGTAAGCTCGATGCTCCTAAAGTCGATTTG
TTGGTGAAGAATTGCTTGCTGCCCCTGAGAGAGTACTTTAAGTACTTTAGCCAGAACAGCTT
GCCACTG

(SEQ ID NO: 115)
   KRAB
   *hSOX2₁₋₁₇₉*
   **DNMT3a3L**
CS-hSOX2₁₋₁₇₉ 
ATGCTGGAACCAGAAAAGATCATTGGGCAACAGATTCCTGTGGTGATTTAATGTTCCTAAT
GAAATGGAAAGACACAGATGAAGCTGACCTGGTTCTTGCAAAAGAAGCTAATGTGAAATGTC
CACAAATTGTGATAGCATTTTATGAAGAGAGACTGACATGGCATGCATATCCTGAGGCATGC
atgtacaacatgatggagacggagctgaagccgccgggcccgcagcaaacttcgggggcgg
cggcggcaactccaccgcggcggcggccggcggcaaccagaaaaacagcccggaccgcgtca
agcggcccatgaatgccttcatggtgtggtcccgcgggcagcggcgcaagatggcccaggag
aaccccaagatgcacaactcggagatcagcaagcgcctgggcgccgagtggaaacttttgtc
ggagacggagaagcggccgttcatcgacgaggctaagcggctgcgagcgctgcacatgaagg
agcacccggattataaataccggccccggcggaaaaccaagacgctcatgaagaaggataag
tacacgctgcccggcgggctgctggcccccggcggcaatagcatggcgagcggggtcggggt
gggcgccggcctgggcgcgggcgtgaaccagcgcatggacagttacgcgcacatgaacggct
ggagcaacggcagctacagcatgatgcaggaccagctgggc

(SEQ ID NO: 116)
   CS
   *hSOX2₁₋₁₇₉*
SOX2₁₋₁₇₉-Y-R 
atgtacaacatgatggagacggagctgaagccgccgggcccgcagcaaacttcgggggcgg
cggcggcaactccaccgcggcggcggccggcggcaaccagaaaaacagcccggaccgcgtca
agcggcccatgaatgccttcatggtgtggtcccgcgggcagcggcgcaagatggcccaggag
aaccccaagatgcacaactcggagatcagcaagcgcctgggcgccgagtggaaacttttgtc
ggagacggagaagcggccgttcatcgacgaggctaagcggctgcgagcgctgcacatgaagg
agcacccggattataaataccggccccggcggaaaaccaagacgctcatgaagaaggataag
tacacgctgcccggcgggctgctggcccccggcggcaatagcatggcgagcggggtcggggt
gggcgccggcctgggcgcgggcgtgaaccagcgcatggacagttacgcgcacatgaacggct
ggagcaacggcagctacagcatgatgcaggaccagctgggcgctagcGGATCCaacATGAGG
CCCCGGCTGAAAAACGTGGACAGGAGCACTGCACAGCAGTTGGCAGTAACTGTGGGCAACGT
CACCGTCATTATCACAGACTTTAAGGAAAAGACTCGCTCCTCATCGACATCCTCATCCACAG
TGACCTCCAGTGCAGGGTCAGAACAGCAGAACCAG

(SEQ ID NO: 117)
   *hSOX2₁₋₁₇₉*
   Y-R
KRAB-hTEAD₁₋₁₆₆-DNMT3a3L 
ATGGATGCTAAGTCACTGACTGCCTGGTCCCGGACACTGGTGACCTTCAAGGATGTGTTTGT
GGACTTCACCAGGGAGGAGTGGAAGCTGCTGGACACTGCTCAGCAGATCCTGTACAGAAATG
TGATGCTGGAGAACTATAAGAACCTGGTTTCCTTGGGTTATCAGCTTACTAAGCCAGATGTG
ATCCTCCGGTTGGAGAAGGGAGAAGAGCCCTGGCTGGTGGAGAGAGAAATTCACCAAGAGAC
CCATCCTGATTCAGAGACTGCATTTGAAATCAAATCATCAGTTGGCAGCGGCGCATGCATTG
AGCCCAGCAGCTGGAGCGGCAGTGAGAGCCCTGCCGAAAACATGGAAAGGATGAGTGACTCT
GCAGATAAGCCAATTGACAATGATGCAGAAGGGGTCTGGAGCCCCGACATCGAGCAAAGCTT
TCAGGAGGCCCTGGCTATCTATCCACCATGTGGGAGGAGGAAAATCATCTTATCAGACGAAG
GCAAAATGTATGGTAGGAATGAATTGATAGCCAGATACATCAAACTCAGGACAGGCAAGACG
AGGACCAGAAAACAGGTGTCTAGTCACATTCAGGTTCTTGCCAGAAGGAAATCTCGTGATTT
TCATTCCAAGCTAAAGGATCAGACTGCAAAGGATAAGGCCCTGCAGCACATGGCGGCCATGT
CCTCAGCCCAGATCGTCTCGGCCACTGCCATTCATAACAAGCTGGGGCTGCCTGGGATTCCA
CGCCCGACCTTCCCAGGGGCGCCGGGGTTCTGGCCGGGAATGATTCAAACAGGGCAGCCAGC
TAGCGGCAGCGGCAACCATGATCAAGAGTTCGATCCTCCTAAAGTGTATCCCCCTGTCCCTG
CCGAAAAGCGGAAACCGATCAGGGTTCTCAGTCTCTTCGATGGAATTGCCACAGGCCTCTTG
GTCCTTAAGGACCTGGGCATCCAAGTgGAcCGCTACATCGCCTCAGAAGTATGTGAgGACAG
CATCACAGTCGGCATGGTGCGCCACCAGGGGAAAATAATGTACGTCGGCGACGTCAGATCAG
TTACTCAGAAACATATTCAGGAATGGGGCCTTTTGATCTCGTAATTGGCGGCAGCCCCTGC
AATGATCTGAGTATTGTGAACCCCGCCCGGAAAGGTTTGTACGAAGGTACGGCAGACTGTT
CTTCGAGTTTTACAGACTGTTGCACGACGCACGACCAAAGGAGGGTGAcGAtcGACCCTTCT
TCTGGCTGTTCGAGAACGTGGTCGCTATGGGCGTGTCTGACAAGCGGGACATTTCtAGATTC
CTGGAGAGCAATCCAGTGATGATTGATGCAAAGGAAGTAtccGCTGCCCACCGCGCCAGATA
CTTCTGGGGCAATCTGCCCGGCATGAATCGACCCTTGGCAAGCACCGTGAATGATAAGTTGG
AGTTGCAGGAGTGTCTgGAGCATGGGCGGATCGCAAAGTTTAGCAAGGTGCGGACCATCACG
ACCCGAAGCAATTCAATTAAGCAGGGAAAGGACCAACATTTTCCAGTGTTTATGAACGAGAA
AGAGGAcATCCTGTGGTGcACAGAAATGGAGCGGGTTTTCGGATTCCCCGTACATTATACTG
ATGTGTCCAATATGTCTAGGTTGGCTAGACAGAGGCTGtTGGACGCTCATGGAGTGTTCCT
GTCATCCGCCACCTGTTCGCTCCTCTGAAGGAGTATTTCGCcTGCGTtTCATCCGGgAATTC
AAACGCAAACAGCAGAGGCCCATCCTTTTCTTCCGGCCTGGTgCCACTTAGTCTGCGCGGCT
 
CTCACATGGGACCTATGGAAATATACAAAACAGTGAGCGCCTGGAAGAGGCAGCCTGTGCGG
GTCCTGAGTCTGTTCAGAAACATTGACAAGGTGCTTAAGTCCCTTGGATTTCTGGAGTCTGG
CAGCGGTTCTGGGGGTGgcACCCTTAAGTATGTGGAGGACGTGACAAACGTCGTGAGGAGAG
ATGTGGAGAAATGGGGGCCATTTGACCTGGTATACGGAGCACTCAACCTCTCGGTTCTTCT
TGTGATCGCTGCCCAGGGTGGTATATGTTTCAGTTCCATCGcATCCTGCAATACGCCCTTCC
GCGGCAGGAGAGTCAGCGACCATTCTTCTGGATATTCATGGACAATCTCCTGCTGACAGAgG
ACGACCAAGAGACTACGACTAGATTTCTTCAGACCGAGGCCGTTACTCTCCAGGACGTTAGA
GGTAGGGACTACCAGAATGCCATGCGAGTGTGGAGTAACATCCCCGGACTCAAGTCAAAGCA
cGCACCCCTGACCCCCAAGGAAGAGGAATACCTcCAGGCGCAGGTGAGGAGCCGCAGTAAGC
TCGATGCTCCTAAAGTCGATTTGTTGGTGAAGAATTGCTTGCTGCCCCTGAGAGAGTACTTT
AAGTACTTTAGCCAGAACAGCTTGCCACTG

(SEQ ID NO: 118)
   KRAB
   *hTEAD₁₋₁₆₆*
   **DNMT3a3L**
KRAB-DNMT3a3L-hMYC₁₄₄₋₄₅₄ 
ATGGATGCTAAGTCACTGACTGCCTGGTCCCGGACACTGGTGACCTTCAAGGATGTGTTTGT
GGACTTCACCAGGGAGGAGTGGAAGCTGCTGGACACTGCTCAGCAGATCCTGTACAGAAATG
TGATGCTGGAGAACTATAAGAACCTGGTTTCCTTGGGTTATCAGCTTACTAAGCCAGATGTG
ATCCTCCGGTTGGAGAAGGGAGAAGAGCCCTGGCTGGTGGAGAGAGAAATTCACCAAGAGAC
CCATCCTGATTCAGAGACTGCATTTGAAATCAAATCATCAGTTGCTAGCGGCAGCGGCAACC
ATGATCAAGAGTTCGATCCTCCTAAAGTGTATCCCCCTGTCCCTGCCGAAAAGCGGAAACCG
ATCAGGGTTCTCAGTCTCTTCGATGGAATTGCCACAGGCCTCTTGGTCCTTAAGGACCTGGG
CATCCAAGTgGAcCGCTACATCGCCTCAGAAGTATGTGAgGACAGCATCACAGTCGGCATGG
TGCGCCACCAGGGGAAAATAATGTACGTCGGCGACGTCAGATCAGTTACTCAGAAACATATT
CAGGAATGGGGGCCTTTTGATCTCGTAATTGGCGGCAGCCCCTGCAATGATCTGAGTATTGT
GAACCCCGCCCGGAAAGGTTTGTACGAAGGTACGGGCAGACTGTTCTTCGAGTTTTACAGAC
TGTTGCACGACGCACGACCAAAGGAGGGTGAcGAtcGACCCTTCTTCTGGCTGTTCGAGAAC
GTGGTCGCTATGGGCGTGTCTGACAAGCGGGACATTTCtAGATTCCTGGAGAGCAATCCAGT
GATGATTGATGCAAAGGAAGTAtccGCTGCCCACCGCGCCAGATACTTCTGGGGCAATCTGC
CCGGCATGAATCGACCCTTGGCAAGCACCGTGAATGATAAGTTGGAGTTGCAGGAGTGTCTg
GAGCATGGGCGGATCGCAAAGTTTAGCAAGGTGCGGACCATCACGACCCGAAGCAATTCAAT
TAAGCAGGGAAAGGACCAACATTTTCCAGTGTTTATGAACGAGAAAGAGGAcATCCTGTGGT
GcACAGAAATGGAGCGGGTTTTCGGATTCCCCGTACATTATACTGATGTGTCCAATATGTCT
AGGTTGGCTAGACAGAGGCTGtTGGGACGCTCATGGAGTGTTCCTGTCATCCGCCACCTGTT
CGCTCCTCTGAAGGAGTATTTCGCcTGCGTtTCATCCGGgAATTCAAACGCAAACAGCAGAG
GCCCATCCTTTTCTTCCGGCCTGGTgCCACTTAGTCTGCGCGGCTCTCACATGGACCTATG
GAAATATACAAAACAGTGAGCGCCTGGAAGAGGCAGCCTGTGCGGTCCTGAGTCTGTTCAG
AAACATTGACAAGGTGCTTAAGTCCCTTGGATTTCTGGAGTCTGGCAGCGGTTCTGGGGGTG
gcACCCTTAAGTATGTGGAGGACGTGACAAACGTCGTGAGGAGAGATGTGGAGAAATGGGGG
CCATTTGACCTGGTATACGGGAGCACTCAACCTCTCGGTTCTTCTTGTGATCGCTGCCCAGG
GTGGTATATGTTTCAGTTCCATCGcATCCTGCAATACGCCCTTCCGCGGCAGGAGAGTCAGC
GACCATTCTTCTGGATATTCATGGACAATCTCCTGCTGACAGAgGACGACCAAGAGACTACG
ACTAGATTTCTTCAGACCGAGGCCGTTACTCTCCAGGACGTTAGAGGTAGGGACTACCAGAA
 
TGCCATGCGAGTGTGGAGTAACATCCCCGGACTCAAGTCAAAGCAcGCACCCCTGACCCCCA
AGGAAGAGGAATACCTcCAGGCGCAGGTGAGGAGCCGCAGTAAGCTCGATGCTCCTAAAGTC
GATTTGTTGGTGAAGAATTGCTTGCTGCCCCTGAGAGAGTACTTTAAGTACTTTAGCCAGAA
CAGCTTGCCACTGGGCGCGCCCGCCGCTGCCATCATCCAGGACTGTATGTGGAGCGGCTTCT
CGGCCGCCGCCAAGCTCGTCTCAGAGAAGCTGGCCTCCTACCAGGCTGCGCGCAAAGACAGC
GGCAGCCCGAACCCCGCCCGCGGCCACAGCGTCTGCTCCACCTCCAGCTTGTACCTGCAGGA
TCTGAGCGCCGCCGCCTCAGAGTGCATCGACCCCTCGGTGGTCTTCCCCTACCCTCTCAACG
ACAGCAGCTCGCCCAAGTCCTGCGCCTCGCAAGACTCCAGCGCCTTCTCTCCGTCCTCGGAT
TCTCTGCTCTCCTCGACGGAGTCCTCCCCGCAGGGCAGCCCCGAGCCCCTGGTGCTCCATGA
GGAGACACCGCCCACCACCAGCAGCGACTCTGAGGAGGAACAAGAAGATGAGGAAGAAATCG
ATGTTGTTTCTGTGGAAAAGAGGCAGGCTCCTGGCAAAAGGTCAGAGTCTGGATCACCTTCT
GCTGGAGGCCACAGCAAACCTCCTCACAGCCCACTGGTCCTCAAGAGGTGCCACGTCTCCAC
ACATCAGCACAACTACGCAGCGCCTCCCTCCACTCGGAAGGACTATCCTGCTGCCAAGAGGG
TCAAGTTGGACAGTGTCAGAGTCCTGAGACAGATCAGCAACAACCGAAAATGCACCAGCCCC
AGGTCCTCGGACACCGAGGAGAATGTCAAGAGGCGAACACACAACGTCTTGGAGCGCCAGAG
GAGGAACGAGCTAAAACGGAGCTTTTTTGCCCTGCGTGACCAGATCCCGGAGTTGGAAAACA
ATGAAAAGGCCCCCAAGGTAGTTATCCTTAAAAAAGCCACAGCATACATCCTGTCCGTCCAA
GCAGAGGAGCAAAAGCTCATTTCTGAAGAGGACTTGTTGCGGAAACGACGAGAACAGTTGAA
ACACAAACTTGAACAGCTACGGAACTCTTGTGCG

(SEQ ID NO: 119)
   KRAB
   **DNMT3a3L**
   *hMYC₁₄₄₋₄₅₄*

The ESF may, for example, comprise or consist of an amino acid sequence that has at least 70%, 80%, 90%, 95%, 96%, 97%, 98%, 99% or 100% identity to any one of SEQ ID NOs: 109-113, preferably wherein the amino acid sequence substantially retains the natural function of the protein represented by SEQ ID NO: 109-113, respectively.

The ESF may, for example, be encoded by a polynucleotide comprising or consisting of a nucleic acid sequence which encodes a protein that has at least 70%, 80%, 90%, 95%, 96%, 97%, 98%, 99% or 100% amino acid identity to any one of SEQ ID NOs: 109-113, preferably wherein the amino acid sequence substantially retains the natural function of the protein represented by SEQ ID NO: 109-113, respectively.

The ESF may, for example, be encoded by a polynucleotide comprising a nucleic acid sequence that has at least 70%, 80%, 90%, 95%, 96%, 97%, 98%, 99% or 100% nucleotide identity to any one of SEQ ID NOs: 114-119, or a fragment thereof, preferably wherein the encoded amino acid sequence substantially retains the natural function of the protein encoded by SEQ ID NO: 114-119, respectively.

Further example sequences of ESFs of the invention are:
CS- *hTEAD₁₋₁₆₆* **[TESv2]** 
MLEPEKIIGATDSCGDLMFLMKWKDTDEADLVLAKEANVKCPQIVIAFYEERLTWHAYPENH
DQEFDPPKVYPPVPAEKRKPIRVLSLFDGIATGLLVLKDLGIQVDRYIASEVCEDSITVGMV
RHQGKIMYVGDVRSVTQKHIQEWGPFDLVIGGSPCNDLSIVNPARKGLYEGTGRLFFEFYRL
LHDARPKEGDDRPFFWLFENVVAMGVSDKRDISRFLESNPVMIDAKEVSAAHRARYFWGNLP
GMNRPLASTVNDKLELQECLEHGRIAKFSKVRTITTRSNSIKQGKDQHFPVFMNEKEDILWC
TEMERVFGFPVHYTDVSNMSRLARQRLLGRSWSVPVIRHLFAPLKEYFACVSSGNSNANSRG
PSFSSGLVPLSLRGSHMGPMEIYKTVSAWKRQPVRVLSLFRNIDKVLKSLGFLESGSGSGGG
TLKYVEDVTNVVRRDVEKWGPFDLVYGSTQPLGSSCDRCPGWYMFQFHRILQYALPRQESQR
PFFWIFMDNLLLTEDDQETTTRFLQTEAVTLQDVRGRDYQNAMRVWSNIPGLKSKHAPLTPK
EEEYLQAQVRSRSKLDAPKVDLLVKNCLLPLREYFKYFSQNSLPL

(SEQ ID NO: 120)
   CS
   *hTEAD₁₋₁₆₆*
*hTEAD₁₋₁₆₆-YR*[**TESv3**] 
MNHDQEFDPPKVYPPVPAEKRKPIRVLSLFDGIATGLLVLKDLGIQVDRYIASEVCEDSITV
GMVRHQGKIMYVGDVRSVTQKHIQEWGPFDLVIGGSPCNDLSIVNPARKGLYEGTGRLFFEF
YRLLHDARPKEGDDRPFFWLFENVVAMGVSDKRDISRFLESNPVMIDAKEVSAAHRARYFWG
NLPGMNRPLASTVNDKLELQECLEHGRIAKFSKVRTITTRSNSIKQGKDQHFPVFMNEKEDI
LWCTEMERVFGFPVHYTDVSNMSRLARQRLLGRSWSVPVIRHLFAPLKEYFACVSSGNSNAN
SRGPSFSSGLVPLSLRGSHMGPMEIYKTVSAWKRQPVRVLSLFRNIDKVLKSLGFLESGSGS
GGGTLKYVEDVTNVVRRDVEKWGPFDLVYGSTQPLGSSCDRCPGWYMFQFHRILQYALPRQE
SQRPFFWIFMDNLLLTEDDQETTTRFLQTEAVTLQDVRGRDYQNAMRVWSNIPGLKSKHAPL
TPKEEEYLQAQVRSRSKLDAPKVDLLVKNCLLPLREYFKYFSQNSLPLtMRPRLKNVDRSTA
QQLAVTVGNVTVIITDFKEKTRSSSTSSSTVTSSAGSEQQNQ

(SEQ ID NO: 121)
   *hTEAD₁₋₁₆₆*
   *Y-R*
CS- *hTEAD₁₋₁₆₆* - Y-R **[TESv4]** 
MLEPEKIIGATDSCGDLMFLMKWKDTDEADLVLAKEANVKCPQIVIAFYEERLTWHAYPENH
DQEFDPPKVYPPVPAEKRKPIRVLSLFDGIATGLLVLKDLGIQVDRYIASEVCEDSITVGMV
RHQGKIMYVGDVRSVTQKHIQEWGPFDLVIGGSPCNDLSIVNPARKGLYEGTGRLFFEFYRL
LHDARPKEGDDRPFFWLFENVVAMGVSDKRDISRFLESNPVMIDAKEVSAAHRARYFWGNLP
GMNRPLASTVNDKLELQECLEHGRIAKFSKVRTITTRSNSIKQGKDQHFPVFMNEKEDILWC
TEMERVFGFPVHYTDVSNMSRLARQRLLGRSWSVPVIRHLFAPLKEYFACVSSGNSNANSRG
PSFSSGLVPLSLRGSHMGPMEIYKTVSAWKRQPVRVLSLFRNIDKVLKSLGFLESGSGSGGG
TLKYVEDVTNVVRRDVEKWGPFDLVYGSTQPLGSSCDRCPGWYMFQFHRILQYALPRQESQR
PFFWIFMDNLLLTEDDQETTTRFLQTEAVTLQDVRGRDYQNAMRVWSNIPGLKSKHAPLTPK
EEEYLQAQVRSRSKLDAPKVDLLVKNCLLPLREYFKYFSQNSLPLtmrprlknvdrstaqql
avtvgnvtviitdfkektrssstssstvtssagseqqnq
 (SEQ ID NO: 122)
   CS
   *hTEAD₁₋₁₆₆*
   *y-r*
CS- hMYC₁₄₄₋₄₅₄ [**MESv2**] 
MLEPEKIIGATDSCGDLMFLMKWKDTDEADLVLAKEANVKCPQIVIAFYEERLTWHAYPEtI
IQDCMWSGFSAAAKLVSEKLASYQAARKDSGSPNPARGHSVCSTSSLYLQDLSAAASECIDP
SVVFPYPLNDSSSPKSCASQDSSAFSPSSDSLLSSTESSPQGSPEPLVLHEETPPTTSSDSE
EEQEDEEEIDVVSVEKRQAPGKRSESGSPSAGGHSKPPHSPLVLKRCHVSTHQHNYAAPPST
RKDYPAAKRVKLDSVRVLRQISNNRKCTSPRSSDTEENVKRRTHNVLERQRRNELKRSFFAL
RDQIPELENNEKAPKVVILKKATAYILSVQAEEQKLISEEDLLRKRREQLKHKLEQLRNSCA

(SEQ ID NO: 123)
   CS
   *hMYC₁₄₄₋₄₅₄*
YR- hMYC₁₄₄₋₄₅₄ [**MESv3**] 
MRPRLKNVDRSTAQQLAVTVGNVTVIITDFKEKTRSSSTSSSTVTSSAGSEQQNQtIIQDCM
WSGFSAAAKLVSEKLASYQAARKDSGSPNPARGHSVCSTSSLYLQDLSAAASECIDPSVVFP
YPLNDSSSPKSCASQDSSAFSPSSDSLLSSTESSPQGSPEPLVLHEETPPTTSSDSEEEQED
EEEIDVVSVEKRQAPGKRSESGSPSAGGHSKPPHSPLVLKRCHVSTHQHNYAAPPSTRKDYP
AAKRVKLDSVRVLRQISNNRKCTSPRSSDTEENVKRRTHNVLERQRRNELKRSFFALRDQIP
ELENNEKAPKVVILKKATAYILSVQAEEQKLISEEDLLRKRREQLKHKLEQLRNSCA

(SEQ ID NO: 124)
   YR
   *hMYC₁₄₄₋₄₅₄*
CS-YR- hMYC₁₄₄₋₄₅₄ [**MESv4**] 
MLEPEKIIGATDSCGDLMFLMKWKDTDEADLVLAKEANVKCPQIVIAFYEERLTWHAYPEtm
rprlknvdrstaqqlavtvgnvtviitdfkektrssstssstvtssagseqqnqtIIQDCMW
SGFSAAAKLVSEKLASYQAARKDSGSPNPARGHSVCSTSSLYLQDLSAAASECIDPSVVFPY
PLNDSSSPKSCASQDSSAFSPSSDSLLSSTESSPQGSPEPLVLHEETPPTTSSDSEEEQEDE
EEIDVVSVEKRQAPGKRSESGSPSAGGHSKPPHSPLVLKRCHVSTHQHNYAAPPSTRKDYPA
AKRVKLDSVRVLRQISNNRKCTSPRSSDTEENVKRRTHNVLERQRRNELKRSFFALRDQIPE
LENNEKAPKVVILKKATAYILSVQAEEQKLISEEDLLRKRREQLKHKLEQLRNSCA

(SEQ ID NO: 125)
   CS
   *yr*
   *hMYC₁₄₄₋₄₅₄*

Further exemplary nucleotide sequences encoding ESFs of the invention are:
CS*-hTEAD₁₋₁₆₆* 
ATGCTGGAACCAGAAAAGATCATTGGGGCAACAGATTCCTGTGGTGATTTAATGTTCCTAAT
GAAATGGAAAGACACAGATGAAGCTGACCTGGTTCTTGCAAAAGAAGCTAATGTGAAATGTC
CACAAATTGTGATAGCATTTTATGAAGAGAGACTGACATGGCATGCATATCCTGAGACCATT
GAGCCCAGCAGCTGGAGCGGCAGTGAGAGCCCTGCCGAAAACATGGAAAGGATGAGTGACTC
TGCAGATAAGCCAATTGACAATGATGCAGAAGGGGTCTGGAGCCCCGACATCGAGCAAAGCT
TTCAGGAGGCCCTGGCTATCTATCCACCATGTGGGAGGAGGAAAATCATCTTATCAGACGAA
GGCAAAATGTATGGTAGGAATGAATTGATAGCCAGATACATCAAACTCAGGACAGGCAAGAC
GAGGACCAGAAAACAGGTGTCTAGTCACATTCAGGTTCTTGCCAGAAGGAAATCTCGTGATT
TTCATTCCAAGCTAAAGGATCAGACTGCAAAGGATAAGGCCCTGCAGCACATGGCGGCCATG
TCCTCAGCCCAGATCGTCTCGGCCACTGCCATTCATAACAAGCTGGGGCTGCCTGGGATTCC
ACGCCCGACCTTCCCAGGGGCGCCGGGGTTCTGGCCGGGAATGATTCAAACAGGGCAGCCAG
GCGCGCCC

(SEQ ID NO: 126)
   CS
   *hTEAD₁₋₁₆₆*
*hTEAD₁₋₁₆₆-YR* 
ATGATTGAGCCCAGCAGCTGGAGCGGCAGTGAGAGCCCTGCCGAAAACATGGAAAGGATGAG
TGACTCTGCAGATAAGCCAATTGACAATGATGCAGAAGGGGTCTGGAGCCCCGACATCGAGC
AAAGCTTTCAGGAGGCCCTGGCTATCTATCCACCATGTGGGAGGAGGAAAATCATCTTATCA
GACGAAGGCAAAATGTATGGTAGGAATGAATTGATAGCCAGATACATCAAACTCAGGACAGG
CAAGACGAGGACCAGAAAACAGGTGTCTAGTCACATTCAGGTTCTTGCCAGAAGGAAATCTC
GTGATTTTCATTCCAAGCTAAAGGATCAGACTGCAAAGGATAAGGCCCTGCAGCACATGGCG
GCCATGTCCTCAGCCCAGATCGTCTCGGCCACTGCCATTCATAACAAGCTGGGGCTGCCTGG
GATTCCACGCCCGACCTTCCCAGGGGCGCCGGGGTTCTGGCCGGGAATGATTCAAACAGGGC
AGCCAaccATGAGGCCCCGGCTGAAAAACGTGGACAGGAGCACTGCACAGCAGTTGGCAGTA
ACTGTGGGCAACGTCACCGTCATTATCACAGACTTTAAGGAAAAGACTCGCTCCTCATCGAC
ATCCTCATCCACAGTGACCTCCAGTGCAGGGTCAGAACAGCAGAACCAGGGCGCGCCC

(SEQ ID NO: 127)
   *hTEAD₁₋₁₆₆*
   *Y-R*
CS- *hTEAD₁₋₁₆₆ -Y-R* 
ATGCTGGAACCAGAAAAGATCATTGGGGCAACAGATTCCTGTGGTGATTTAATGTTCCTAAT
GAAATGGAAAGACACAGATGAAGCTGACCTGGTTCTTGCAAAAGAAGCTAATGTGAAATGTC
CACAAATTGTGATAGCATTTTATGAAGAGAGACTGACATGGCATGCATATCCTGAGACCATT
GAGCCCAGCAGCTGGAGCGGCAGTGAGAGCCCTGCCGAAAACATGGAAAGGATGAGTGACTC
TGCAGATAAGCCAATTGACAATGATGCAGAAGGGGTCTGGAGCCCCGACATCGAGCAAAGCT
TTCAGGAGGCCCTGGCTATCTATCCACCATGTGGGAGGAGGAAAATCATCTTATCAGACGAA
GGCAAAATGTATGGTAGGAATGAATTGATAGCCAGATACATCAAACTCAGGACAGGCAAGAC
GAGGACCAGAAAACAGGTGTCTAGTCACATTCAGGTTCTTGCCAGAAGGAAATCTCGTGATT
TTCATTCCAAGCTAAAGGATCAGACTGCAAAGGATAAGGCCCTGCAGCACATGGCGGCCATG
TCCTCAGCCCAGATCGTCTCGGCCACTGCCATTCATAACAAGCTGGGGCTGCCTGGGATTCC
ACGCCCGACCTTCCCAGGGGCGCCGGGGTTCTGGCCGGGAATGATTCAAACAGGGCAGCCAA
CCatgaggccccggctgaaaaacgtggacaggagcactgcacagcagttggcagtaactgtg
ggcaacgtcaccgtcattatcacagactttaaggaaaagactcgctcctcatcgacatcctc
atccacagtgacctccagtgcagggtcagaacagcagaaccagGGCGCGCCC

(SEQ ID NO: 128)
   CS
   *hTEAD₁₋₁₆₆*
   *y-r*
CS- hMYC₁₄₄₋₄₅₄ 
ATGCTGGAACCAGAAAAGATCATTGGGGCAACAGATTCCTGTGGTGATTTAATGTTCCTAAT
GAAATGGAAAGACACAGATGAAGCTGACCTGGTTCTTGCAAAAGAAGCTAATGTGAAATGTC
CACAAATTGTGATAGCATTTTATGAAGAGAGACTGACATGGCATGCATATCCTGAGACCATC
ATCCAGGACTGTATGTGGAGCGGCTTCTCGGCCGCCGCCAAGCTCGTCTCAGAGAAGCTGGC
CTCCTACCAGGCTGCGCAAAGACAGCGGCAGCCCGAACCCCGCCCGCGGCCACAGCGTCT
GCTCCACCTCCAGCTTGTACCTGCAGGATCTGAGCGCCGCCGCCTCAGAGTGCATCGACCCC
TCGGTGGTCTTCCCCTACCCTCTCAACGACAGCAGCTCGCCCAAGTCCTGCGCCTCGCAAGA
CTCCAGCGCCTTCTCTCCGTCCTCGGATTCTCTGCTCTCCTCGACGGAGTCCTCCCCGCAGG
GCAGCCCCGAGCCCCTGGTGCTCCATGAGGAGACACCGCCCACCACCAGCAGCGACTCTGAG
GAGGAACAAGAAGATGAGGAAGAAATCGATGTTGTTTCTGTGGAAAAGAGGCAGGCTCCTGG
CAAAAGGTCAGAGTCTGGATCACCTTCTGCTGGAGGCCACAGCAAACCTCCTCACAGCCCAC
TGGTCCTCAAGAGGTGCCACGTCTCCACACATCAGCACAACTACGCAGCGCCTCCCTCCACT
CGGAAGGACTATCCTGCTGCCAAGAGGGTCAAGTTGGACAGTGTCAGAGTCCTGAGACAGAT
 
CAGCAACAACCGAAAATGCACCAGCCCCAGGTCCTCGGACACCGAGGAGAATGTCAAGAGGC
GAACACACAACGTCTTGGAGCGCCAGAGGAGGAACGAGCTAAAACGGAGCTTTTTGCCCTG
CGTGACCAGATCCCGGAGTTGGAAAACAATGAAAAGGCCCCCAAGGTAGTTATCCTTAAAAA
AGCCACAGCATACATCCTGTCCGTCCAAGCAGAGGAGCAAAAGCTCATTTCTGAAGAGGACT
TGTTGCGGAAACGACGAGAACAGTTGAAACACAAACTTGAACAGCTACGGAACTCTTGTGCG
GGCGCGCCC

(SEQ ID NO: 129)
   CS
   *hMYC₁₄₄₋₄₅₄*
YR-hMYC₁₄₄₋₄₅₄ 
ATGAGGCCCCGGCTGAAAAACGTGGACAGGAGCACTGCACAGCAGTTGGCAGTAACTGTGGG
CAACGTCACCGTCATTATCACAGACTTTAAGGAAAAGACTCGCTCCTCATCGACATCCTCAT
CCACAGTGACCTCCAGTGCAGGGTCAGAACAGCAGAACCAGACCATCATCCAGGACTGTATG
TGGAGCGGCTTCTCGGCCGCCGCCAAGCTCGTCTCAGAGAAGCTGGCCTCCTACCAGGCTGC
GCGCAAAGACAGCGGCAGCCCGAACCCCGCCCGCGGCCACAGCGTCTGCTCCACCTCCAGCT
TGTACCTGCAGGATCTGAGCGCCGCCGCCTCAGAGTGCATCGACCCCTCGGTGGTCTTCCCC
TACCCTCTCAACGACAGCAGCTCGCCCAAGTCCTGCGCCTCGCAAGACTCCAGCGCCTTCTC
TCCGTCCTCGGATTCTCTGCTCTCCTCGACGGAGTCCTCCCCGCAGGGCAGCCCCGAGCCCC
TGGTGCTCCATGAGGAGACACCGCCCACCACCAGCAGCGACTCTGAGGAGGAACAAGAAGAT
GAGGAAGAAATCGATGTTGTTTCTGTGGAAAAGAGGCAGGCTCCTGGCAAAAGGTCAGAGTC
TGGATCACCTTCTGCTGGAGGCCACAGCAAACCTCCTCACAGCCCACTGGTCCTCAAGAGGT
GCCACGTCTCCACACATCAGCACAACTACGCAGCGCCTCCCTCCACTCGGAAGGACTATCCT
GCTGCCAAGAGGGTCAAGTTGGACAGTGTCAGAGTCCTGAGACAGATCAGCAACAACCGAAA
ATGCACCAGCCCCAGGTCCTCGGACACCGAGGAGAATGTCAAGAGGCGAACACACAACGTCT
TGGAGCGCCAGAGGAGGAACGAGCTAAAACGGAGCTTTTTTGCCCTGCGTGACCAGATCCCG
GAGTTGGAAAACAATGAAAAGGCCCCCAAGGTAGTTATCCTTAAAAAAGCCACAGCATACAT
CCTGTCCGTCCAAGCAGAGGAGCAAAAGCTCATTTCTGAAGAGGACTTGTTGCGGAAACGAC
GAGAACAGTTGAAACACAAACTTGAACAGCTACGGAACTCTTGTGCGGGCGCGCCC

(SEQ ID NO: 130)
   YR
   *hMYC₁₄₄₋₄₅₄*
CS-YR-hMYC₁₄₄₋₄₅₄ 
ATGCTGGAACCAGAAAAGATCATTGGGGCAACAGATTCCTGTGGTGATTTAATGTTCCTAAT
GAAATGGAAAGACACAGATGAAGCTGACCTGGTTCTTGCAAAAGAAGCTAATGTGAAATGTC
CACAAATTGTGATAGCATTTTATGAAGAGAGACTGACATGGCATGCATATCCTGAGaccatg
aggccccggctgaaaaacgtggacaggagcactgcacagcagttggcagtaactgtgggcaa
cgtcaccgtcattatcacagactttaaggaaaagactcgctcctcatcgacatcctcatcca
cagtgacctccagtgcagggtcagaacagcagaaccagACCATCATCCAGGACTGTATGTGG
AGCGGCTTCTCGGCCGCCGCCAAGCTCGTCTCAGAGAAGCTGGCCTCCTACCAGGCTGCGCG
CAAAGACAGCGGCAGCCCGAACCCCGCCCGCGGCCACAGCGTCTGCTCCACCTCCAGCTTGT
ACCTGCAGGATCTGAGCGCCGCCGCCTCAGAGTGCATCGACCCCTCGGTGGTCTTCCCCTAC
CCTCTCAACGACAGCAGCTCGCCCAAGTCCTGCGCCTCGCAAGACTCCAGCGCCTTCTCTCC
GTCCTCGGATTCTCTGCTCTCCTCGACGGAGTCCTCCCCGCAGGGCAGCCCCGAGCCCCTGG
TGCTCCATGAGGAGACACCGCCCACCACCAGCAGCGACTCTGAGGAGGAACAAGAAGATGAG
GAAGAAATCGATGTTGTTTCTGTGGAAAAGAGGCAGGCTCCTGGCAAAAGGTCAGAGTCTGG
ATCACCTTCTGCTGGAGGCCACAGCAAACCTCCTCACAGCCCACTGGTCCTCAAGAGGTGCC
ACGTCTCCACACATCAGCACAACTACGCAGCGCCTCCCTCCACTCGGAAGGACTATCCTGCT
GCCAAGAGGGTCAAGTTGGACAGTGTCAGAGTCCTGAGACAGATCAGCAACAACCGAAAATG
CACCAGCCCCAGGTCCTCGGACACCGAGGAGAATGTCAAGAGGCGAACACACAACGTCTTGG
AGCGCCAGAGGAGGAACGAGCTAAAACGGAGCTTTTTTGCCCTGCGTGACCAGATCCCGGAG
TTGGAAAACAATGAAAAGGCCCCCAAGGTAGTTATCCTTAAAAAAGCCACAGCATACATCCT
GTCCGTCCAAGCAGAGGAGCAAAAGCTCATTTCTGAAGAGGACTTGTTGCGGAAACGACGAG
AACAGTTGAAACACAAACTTGAACAGCTACGGAACTCTTGTGCGGGCGCGCCC

(SEQ IDN NO: 131)
   CS
   *yr*
   *hMYC₁₄₄₋₄₅₄*

The ESF may, for example, comprise or consist of an amino acid sequence that has at least 70%, 80%, 90%, 95%, 96%, 97%, 98%, 99% or 100% identity to any one of SEQ ID NOs: 120-125, preferably wherein the amino acid sequence substantially retains the natural function of the protein represented by SEQ ID NO: 120-125, respectively.

The ESF may, for example, be encoded by a polynucleotide comprising or consisting of a nucleic acid sequence which encodes a protein that has at least 70%, 80%, 90%, 95%, 96%, 97%, 98%, 99% or 100% amino acid identity to any one of SEQ ID NOs: 120-125, preferably wherein the amino acid sequence substantially retains the natural function of the protein represented by SEQ ID NO: 120-125, respectively.

The ESF may, for example, be encoded by a polynucleotide comprising a nucleic acid sequence that has at least 70%, 80%, 90%, 95%, 96%, 97%, 98%, 99% or 100% nucleotide identity to any one of SEQ ID NOs: 126-131, or a fragment thereof, preferably wherein the encoded amino acid sequence substantially retains the natural function of the protein encoded by SEQ ID NO: 126-131, respectively.

### Tags

The polynucleotides of the invention may encode tags, such as polypeptide tags. Tags may be selected depending upon the intended purpose of the polynucleotide in question.

In one embodiment, the polynucleotide according to the invention further comprises a tag.

In one embodiment, the tag is a V5 tag.

The ESFs of the invention may have a polypeptide tag.

In one embodiment the polypeptide tag is a V5 tag.

### Example V5 Tag (SEQ ID NO: 132):GKPIPNPLLGLDST

In one embodiment, the polypeptide tag comprises or consists of SEQ ID NO: 132.

The polypeptide tag may be separated from the ESF sequence by a spacer.

In one embodiment the polypeptide tag is a C-terminal tag.

In one embodiment, the polypeptide tag has an N-terminal spacer sequence between the tag and the ESF.

### Example V5 Tag and spacer (SEQ ID NO: 133): gapGKPIPNPLLGLDST

It will be understood that such tags may not be advantageous in some settings but advantageous in others. For example, a tag may be useful for *in vitro* testing whilst it may not be desirable in polynucleotides intended for medical use.

In one embodiment, the ESF of the invention does not comprise a polypeptide tag, such as a V5 tag. In one embodiment, the invention provides a sequence obtained from any sequence described herein that includes a V5 tag by the deletion of said V5 tag (and optionally any spacer sequence between the V5 tag and the ESF).

The foregoing ESFs may advantageously be used in conjunction with any one of the miRNA target sequence cassettes of the invention. Further, the foregoing ESFs, whether comprised within a polynucleotide or cassette according to the invention or not, may advantageously be used for treating cancer according to the invention.

### Expression control sequences

The polynucleotide of the invention may comprise one or more expression control sequence. Suitably, the nucleic acid sequence encoding the ESF or transgene is operably linked to one or more expression control sequence.

As used herein an "expression control sequence" is any nucleotide sequence which controls expression of a transgene, e.g. to facilitate and/or increase expression in some cell types and/or decrease expression in other cell types.

The expression control sequence and the transgene (e.g. nucleic acid sequence encoding the ESF) may be in any suitable arrangement in the polynucleotide, providing that the expression control sequence is operably linked to the transgene (e.g. nucleic acid sequence encoding the ESF).

### Promoters

In some embodiments, the expression control sequence is a promoter.

Any suitable promoter may be used, the selection of which may be readily made by the skilled person. The promoter sequence may be constitutively active (i.e. operational in any host cell background), or alternatively may be active only in a specific host cell environment, thus allowing for targeted expression of the nucleotide of interest (e.g. the ESF) in a particular cell type (e.g. a tissue-specific promoter). The promoter may show inducible expression in response to presence of another factor, for example a factor present in a host cell. In any event, where the vector is administered for therapy, it is preferred that the promoter should be functional in the target cell (e.g. cancer cell) background.

In some embodiments, the polynucleotide further comprises a promoter operably linked to the transgene. In some embodiments, the polynucleotide further comprises a promoter operably linked to the nucleic acid sequence encoding the ESF.

In some embodiments, the promoter is a constitutive promoter. In some embodiments, the constitutive promoter is an Ef1a promoter.

An example sequence of an Ef1a promoter is:

CGATAAGCTTTGCAAAGATGGATAAAGTTTTAAACAGAGAGGAATCTTTGCAGCTAATGGAC
CTTCTAGGTCTTGAAAGGAGTGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG

TGGCGCGGGGTAAACTGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
C

(SEQ ID NO: 134)

In some embodiments, the promoter is a tissue-specific promoter, preferably a cancer cell-specific promoter.

In some embodiments, the promoter is a proliferating cell-specific promoter.

In some embodiments, the promoter is selected from the group consisting of a Mki67 promoter, a Ccnd1 promoter, a Ccnb2 promoter, a Ccna2 promoter, a Cdc25c promoter, a Cdc2 promoter, a Cks1 promoter, a PCNA promoter, a CDC6 promoter, a POLD1 promoter, a CSK1B promoter, a MCM2 promoter and a PLK1 promoter.

In some embodiments, the promoter is a Mki67 promoter.

An example sequence of a Mki67 promoter is:

acttcaggcttgtccttgagaccttgaggagagggtagacattggttctattgccccagaag
cggaattttacaacaccctactggaagaacactgatccaggaacaggaggagggagcatga
aaaaagaggacaggaaaacataggtactctatattcatctgactggcacctggcctcagata
accccaactggtgccccaggagccctatttaggatttgcaggtgcagtcttgcacaggtta
ttttaatcggcatttccccctgaaacagcaaagcattgcaaacttaaccgtgccgcagcaat
aactcttaagttatggtttcggaaaaaccgtgccactgcctcagtgttcattaagcagggag
actgtagaatgaattataatgatgtcccccagtttttaaaggacagtttgagggatactgaa
aaggcaggagaaacaaccttaggttgcgtatgaggtgttgcagaaacggcagaggaagggta
taaattttaaaaatagaaagcttctagcgttgagcaagatgcggcgttgtcggtgtttgatt
tctggagaggagcctacaggtagcttcctacaggtcgccacgctagttttgctgacactga
aaaagctgggaccctctaccctgcctattagagcagcagtggcccgcccaccagccccaccc
tgcccgccttccggagcttatctccgctcgggttccgcttctccagccagcttctggcccgg
ccccgcctaccggcctggcccctcccaccttccggctccacccccccccccccccccaggct
cgcagcctggccccacctctggacttccccaatcctctgtcgccctcgccattggtctctgg
ctggccgcctggcccaatcgcagcacttagcgccagaatttgaaccgccgttttggtttgaa

tcgggcgggctgcggcgggcgagccttggcgcggaaccgcctaggcggacagtcggggctgg
acggggcggcgggtggcgggtggcgggcacttgggacgcgggcggcgggagtgcgggtctgg
tcggggcggagcgaaggccgcgggtggccgtggtcggtcctccgcggctaaggagccgaggg
ctccgacgcgggctgcgcccggtgagcggcggccagagctaacttgcgctgactggaccagc
tgaggagcggcccggcgggcgactgcgagcttcaccgagaggcttctccgccctggtccgc
agtcccgacggccggcggacc

(SEQ ID NO: 135)

The promoter may, for example, comprise or consist of a nucleic acid sequence that has at least 70%, 80%, 90%, 95%, 96%, 97%, 98%, 99% or 100% sequence identity to any one of SEQ ID NOs: 134 or 135, preferably wherein the promoter substantially retains the natural function of the promoter of SEQ ID NO: 134 or 135, respectively.

In some embodiments, the promoter is an Ef1a promoter. Exemplary polynucleotide sequences comprising an EF1a promoter operably linked to a transgene encoding an ESF are as follows:
Ef1a::CS- *hTEAD₁₋₁₆₆* (SEQ ID NO: 158) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGACCATTGAGCCCAGCAGCTGGAGCGGCAGTGAGAGCCCTGCCGAAAACATGGAAA
GGATGAGTGACTCTGCAGATAAGCCAATTGACAATGATGCAGAAGGGGTCTGGAGCCCCGAC
ATCGAGCAAAGCTTTCAGGAGGCCCTGGCTATCTATCCACCATGTGGGAGGAGGAAAATCAT
CTTATCAGACGAAGGCAAAATGTATGGTAGGAATGAATTGATAGCCAGATACATCAAACTCA
 
GGACAGGCAAGACGAGGACCAGAAAACAGGTGTCTAGTCACATTCAGGTTCTTGCCAGAAGG
AAATCTCGTGATTTTCATTCCAAGCTAAAGGATCAGACTGCAAAGGATAAGGCCCTGCAGCA
CATGGCGGCCATGTCCTCAGCCCAGATCGTCTCGGCCACTGCCATTCATAACAAGCTGGGGC
TGCCTGGGATTCCACGCCCGACCTTCCCAGGGGCGCCGGGGTTCTGGCCGGGAATGATTCAA
ACAGGGCAGCC
   **Efla promoter**
   CS
   *hTEAD₁₋₁₆₆*
Ef1a::*hTEAD₁₋₁₆₆-YR* (SEQ ID NO: 159) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGATTGAGCCCAGCAGCTGGAGCGGCAGTGAGAGCCCTGCCGAAAACA
TGGAAAGGATGAGTGACTCTGCAGATAAGCCAATTGACAATGATGCAGAAGGGGTCTGGAGC
CCCGACATCGAGCAAAGCTTTCAGGAGGCCCTGGCTATCTATCCACCATGTGGGAGGAGGAA
AATCATCTTATCAGACGAAGGCAAAATGTATGGTAGGAATGAATTGATAGCCAGATACATCA
AACTCAGGACAGGCAAGACGAGGACCAGAAAACAGGTGTCTAGTCACATTCAGGTTCTTGCC
AGAAGGAAATCTCGTGATTTTCATTCCAAGCTAAAGGATCAGACTGCAAAGGATAAGGCCCT
GCAGCACATGGCGGCCATGTCCTCAGCCCAGATCGTCTCGGCCACTGCCATTCATAACAAGC
TGGGGCTGCCTGGGATTCCACGCCCGACCTTCCCAGGGGCGCCGGGGTTCTGGCCGGGAATG
ATTCAAACAGGGCAGCCAaccATGAGGCCCCGGCTGAAAAACGTGGACAGGAGCACTGCACA
GCAGTTGGCAGTAACTGTGGGCAACGTCACCGTCATTATCACAGACTTTAAGGAAAAGACTC
GCTCCTCATCGACATCCTCATCCACAGTGACCTCCAGTGCAGGGTCAGAACAGCAGAACCA
   **Efla promoter**
   *hTEAD₁₋₁₆₆*
   *Y-R*
Ef1a::CS- *hTEAD₁₋₁₆₆* - Y-R (SEQ ID NO: 160) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGACCATTGAGCCCAGCAGCTGGAGCGGCAGTGAGAGCCCTGCCGAAAACATGGAAA
GGATGAGTGACTCTGCAGATAAGCCAATTGACAATGATGCAGAAGGGGTCTGGAGCCCCGAC
ATCGAGCAAAGCTTTCAGGAGGCCCTGGCTATCTATCCACCATGTGGGAGGAGGAAAATCAT
CTTATCAGACGAAGGCAAAATGTATGGTAGGAATGAATTGATAGCCAGATACATCAAACTCA
GGACAGGCAAGACGAGGACCAGAAAACAGGTGTCTAGTCACATTCAGGTTCTTGCCAGAAGG
AAATCTCGTGATTTTCATTCCAAGCTAAAGGATCAGACTGCAAAGGATAAGGCCCTGCAGCA
CATGGCGGCCATGTCCTCAGCCCAGATCGTCTCGGCCACTGCCATTCATAACAAGCTGGGGC
TGCCTGGGATTCCACGCCCGACCTTCCCAGGGGCGCCGGGGTTCTGGCCGGGAATGATTCAA
ACAGGGCAGCCAACCatgaggccccggctgaaaaacgtggacaggagcactgcacagcagtt
ggcagtaactgtgggcaacgtcaccgtcattatcacagactttaaggaaaagactcgctcct
catcgacatcctcatccacagtgacctccagtgcagggtcagaacagcagaaccag
   **Ef1a** promoter
   CS
   *hTEAD₁₋₁₆₆*
   *y-r*
Ef1a::CS- hMYC₁₄₄₋₄₅₄ (SEQ ID NO: 161) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGACCATCATCCAGGACTGTATGTGGAGCGGCTTCTCGGCCGCCGCCAAGCTCGTCT
CAGAGAAGCTGGCCTCCTACCAGGCTGCGCGCAAAGACAGCGGCAGCCCGAACCCCGCCCGC
GGCCACAGCGTCTGCTCCACCTCCAGCTTGTACCTGCAGGATCTGAGCGCCGCCGCCTCAGA
GTGCATCGACCCCTCGGTGGTCTTCCCCTACCCTCTCAACGACAGCAGCTCGCCCAAGTCCT
GCGCCTCGCAAGACTCCAGCGCCTTCTCTCCGTCCTCGGATTCTCTGCTCTCCTCGACGGAG
TCCTCCCCGCAGGGCAGCCCCGAGCCCCTGGTGCTCCATGAGGAGACACCGCCCACCACCAG
CAGCGACTCTGAGGAGGAACAAGAAGATGAGGAAGAAATCGATGTTGTTTCTGTGGAAAAGA
GGCAGGCTCCTGGCAAAAGGTCAGAGTCTGGATCACCTTCTGCTGGAGGCCACAGCAAACCT
CCTCACAGCCCACTGGTCCTCAAGAGGTGCCACGTCTCCACACATCAGCACAACTACGCAGC
GCCTCCCTCCACTCGGAAGGACTATCCTGCTGCCAAGAGGGTCAAGTTGGACAGTGTCAGAG
TCCTGAGACAGATCAGCAACAACCGAAAATGCACCAGCCCCAGGTCCTCGGACACCGAGGAG
AATGTCAAGAGGCGAACACACAACGTCTTGGAGCGCCAGAGGAGGAACGAGCTAAAACGGAG
CTTTTTTGCCCTGCGTGACCAGATCCCGGAGTTGGAAAACAATGAAAAGGCCCCCAAGGTAG
TTATCCTTAAAAAAGCCACAGCATACATCCTGTCCGTCCAAGCAGAGGAGCAAAAGCTCATT
TCTGAAGAGGACTTGTTGCGGAAACGACGAGAACAGTTGAAACACAAACTTGAACAGCTACG
GAACTCTTGTGCG
   **Ef1a promoter**
   CS
   *hMYC₁₄₄₋₄₅₄*
Ef1a::YR- hMYC₁₄₄₋₄₅₄ (SEQ ID NO: 162) 
CGATAAGCTTTGCAAAGATGGATAAAGTTTTAAACAGAGAGGAATCTTTGCAGCTAATGGAC
CTTCTAGGTCTTGAAAGGAGTGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGAGGCCCCGGCTGAAAAACGTGGACAGGAGCACTGCACAGCAGTTGG
CAGTAACTGTGGGCAACGTCACCGTCATTATCACAGACTTTAAGGAAAAGACTCGCTCCTCA
TCGACATCCTCATCCACAGTGACCTCCAGTGCAGGGTCAGAACAGCAGAACCAGACCATCAT
CCAGGACTGTATGTGGAGCGGCTTCTCGGCCGCCGCCAAGCTCGTCTCAGAGAAGCTGGCCT
CCTACCAGGCTGCGCGCAAAGACAGCGGCAGCCCGAACCCCGCCCGCGGCCACAGCGTCTGC
TCCACCTCCAGCTTGTACCTGCAGGATCTGAGCGCCGCCGCCTCAGAGTGCATCGACCCCTC
GGTGGTCTTCCCCTACCCTCTCAACGACAGCAGCTCGCCCAAGTCCTGCGCCTCGCAAGACT
CCAGCGCCTTCTCTCCGTCCTCGGATTCTCTGCTCTCCTCGACGGAGTCCTCCCCGCAGGGC
AGCCCCGAGCCCCTGGTGCTCCATGAGGAGACACCGCCCACCACCAGCAGCGACTCTGAGGA
GGAACAAGAAGATGAGGAAGAAATCGATGTTGTTTCTGTGGAAAAGAGGCAGGCTCCTGGCA
AAAGGTCAGAGTCTGGATCACCTTCTGCTGGAGGCCACAGCAAACCTCCTCACAGCCCACTG
GTCCTCAAGAGGTGCCACGTCTCCACACATCAGCACAACTACGCAGCGCCTCCCTCCACTCG
GAAGGACTATCCTGCTGCCAAGAGGGTCAAGTTGGACAGTGTCAGAGTCCTGAGACAGATCA
GCAACAACCGAAAATGCACCAGCCCCAGGTCCTCGGACACCGAGGAGAATGTCAAGAGGCGA
ACACACAACGTCTTGGAGCGCCAGAGGAGGAACGAGCTAAAACGGAGCTTTTTTGCCCTGCG
TGACCAGATCCCGGAGTTGGAAAACAATGAAAAGGCCCCCAAGGTAGTTATCCTTAAAAAAG
CCACAGCATACATCCTGTCCGTCCAAGCAGAGGAGCAAAAGCTCATTTCTGAAGAGGACTTG
TTGCGGAAACGACGAGAACAGTTGAAACACAAACTTGAACAGCTACGGAACTCTTGTGCG
   **Ef1a promoter**
   YR
   *hMYC₁₄₄₋₄₅₄*
Ef1a::CS-YR- hMYC₁₄₄₋₄₅₄ (SEQ ID NO: 163) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
 
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGaccatgaggccccggctgaaaaacgtggacaggagcactgcacagcagttggcag
taactgtgggcaacgtcaccgtcattatcacagactttaaggaaaagactcgctcctcatcg
acatcctcatccacagtgacctccagtgcaggtcagaacagcagaaccagACCATCATCCA
GGACTGTATGTGGAGCGGCTTCTCGGCCGCCGCCAAGCTCGTCTCAGAGAAGCTGGCCTCCT
ACCAGGCTGCGCGCAAAGACAGCGGCAGCCCGAACCCCGCCCGCGGCCACAGCGTCTGCTCC
ACCTCCAGCTTGTACCTGCAGGATCTGAGCGCCGCCGCCTCAGAGTGCATCGACCCCTCGGT
GGTCTTCCCCTACCCTCTCAACGACAGCAGCTCGCCCAAGTCCTGCGCCTCGCAAGACTCCA
GCGCCTTCTCTCCGTCCTCGGATTCTCTGCTCTCCTCGACGGAGTCCTCCCCGCAGGGCAGC
CCCGAGCCCCTGGTGCTCCATGAGGAGACACCGCCCACCACCAGCAGCGACTCTGAGGAGGA
ACAAGAAGATGAGGAAGAAATCGATGTTGTTTCTGTGGAAAAGAGGCAGGCTCCTGGCAAAA
GGTCAGAGTCTGGATCACCTTCTGCTGGAGGCCACAGCAAACCTCCTCACAGCCCACTGGTC
CTCAAGAGGTGCCACGTCTCCACACATCAGCACAACTACGCAGCGCCTCCCTCCACTCGGAA
GGACTATCCTGCTGCCAAGAGGGTCAAGTTGGACAGTGTCAGAGTCCTGAGACAGATCAGCA
ACAACCGAAAATGCACCAGCCCCAGGTCCTCGGACACCGAGGAGAATGTCAAGAGGCGAACA
CACAACGTCTTGGAGCGCCAGAGGAGGAACGAGCTAAAACGGAGCTTTTTTGCCCTGCGTGA
CCAGATCCCGGAGTTGGAAAACAATGAAAAGGCCCCCAAGGTAGTTATCCTTAAAAAAGCCA
CAGCATACATCCTGTCCGTCCAAGCAGAGGAGCAAAAGCTCATTTCTGAAGAGGACTTGTTG
CGGAAACGACGAGAACAGTTGAAACACAAACTTGAACAGCTACGGAACTCTTGTGCG
   **Efla promoter**
   CS
   *yr*
   *hMYC₁₄₄₋₄₅₄*
Ef1a::KRAB-*hTEAD₁₋₁₆₆* -DNMT3A/L (SEQ ID NO: 178) 
CGATAAGCTTTGCAAAGATGGATAAAGTTTTAAACAGAGAGGAATCTTTGCAGCTAATGGAC
CTTCTAGGTCTTGAAAGGAGTGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CATGGATGCTAAGTCACTGACTGCCTGGTCCCGGACACTGGTGACCTTCAAGGATGTGTTTG
TGGACTTCACCAGGGAGGAGTGGAAGCTGCTGGACACTGCTCAGCAGATCCTGTACAGAAAT
GTGATGCTGGAGAACTATAAGAACCTGGTTTCCTTGGGTTATCAGCTTACTAAGCCAGATGT
GATCCTCCGGTTGGAGAAGGGAGAAGAGCCCTGGCTGGTGGAGAGAGAAATTCACCAAGAGA
CCCATCCTGATTCAGAGACTGCATTTGAAATCAAATCATCAGTTATTGAGCCAGCAGCTGG
AGCGGCAGTGAGAGCCCTGCCGAAAACATGGAAAGGATGAGTGACTCTGCAGATAAGCCAAT
TGACAATGATGCAGAAGGGGTCTGGAGCCCCGACATCGAGCAAAGCTTTCAGGAGGCCCTGG
CTATCTATCCACCATGTGGGAGGAGGAAAATCATCTTATCAGACGAAGGCAAAATGTATGGT
AGGAATGAATTGATAGCCAGATACATCAAACTCAGGACAGGCAAGACGAGGACCAGAAAACA
GGTGTCTAGTCACATTCAGGTTCTTGCCAGAAGGAAATCTCGTGATTTTCATTCCAAGCTAA
AGGATCAGACTGCAAAGGATAAGGCCCTGCAGCACATGGCGGCCATGTCCTCAGCCCAGATC
GTCTCGGCCACTGCCATTCATAACAAGCTGGGGCTGCCTGGGATTCCACGCCCGACCTTCCC
AGGGGCGCCGGGGTTCTGGCCGGGAATGATTCAAACAGGGCAGCCAAACCATGATCAAGAGT
TCGATCCTCCTAAAGTGTATCCCCCTGTCCCTGCCGAAAAGCGGAAACCGATCAGGGTTCTC
AGTCTCTTCGATGGAATTGCCACAGGCCTCTTGGTCCTTAAGGACCTGGGCATCCAAGTGGA
CCGCTACATCGCCTCAGAAGTATGTGAGGACAGCATCACAGTCGGCATGGTGCGCCACCAGG
GGAAAATAATGTACGTCGGCGACGTCAGATCAGTTACTCAGAAACATATTCAGGAATGGGGG
CCTTTTGATCTCGTAATTGGCGGCAGCCCCTGCAATGATCTGAGTATTGTGAACCCCGCCCG
GAAAGGTTTGTACGAAGGTACGGGCAGACTGTTCTTCGAGTTTTACAGACTGTTGCACGACG
CACGACCAAAGGAGGGTGACGATCGACCCTTCTTCTGGCTGTTCGAGAACGTGGTCGCTATG
GGCGTGTCTGACAAGCGGGACATTTCTAGATTCCTGGAGAGCAATCCAGTGATGATTGATGC
AAAGGAAGTATCCGCTGCCCACCGCGCCAGATACTTCTGGGGCAATCTGCCCGGCATGAATC
GACCCTTGGCAAGCACCGTGAATGATAAGTTGGAGTTGCAGGAGTGTCTGGAGCATGGCGG
ATCGCAAAGTTTAGCAAGGTGCGGACCATCACGACCCGAAGCAATTCAATTAAGCAGGAAA
GGACCAACATTTTCCAGTGTTTATGAACGAGAAAGAGGACATCCTGTGGTGCACAGAAATGG
AGCGGGTTTTCGGATTCCCCGTACATTATACTGATGTGTCCAATATGTCTAGGTTGGCTAGA
 
CAGAGGCTGTTGGACGCTCATGGAGTGTTCCTGTCATCCGCCACCTGTTCGCTCCTCTGAA
GGAGTATTTCGCCTGCGTTTCATCCGGGAATTCAAACGCAAACAGCAGAGGCCCATCCTTTT
CTTCCGGCCTGGTGCCACTTAGTCTGCGCGGCTCTCACATGGGACCTATGGAAATATACAAA
ACAGTGAGCGCCTGGAAGAGGCAGCCTGTGCGGGTCCTGAGTCTGTTCAGAAACATTGACAA
GGTGCTTAAGTCCCTTGGATTTCTGGAGTCTGGCAGCGGTTCTGGGGGTGGCACCCTTAAGT
ATGTGGAGGACGTGACAAACGTCGTGAGGAGAGATGTGGAGAAATGGGGGCCATTTGACCTG
GTATACGGGAGCACTCAACCTCTCGGTTCTTCTTGTGATCGCTGCCCAGGGTGGTATATGTT
TCAGTTCCATCGCATCCTGCAATACGCCCTTCCGCGGCAGGAGAGTCAGCGACCATTCTTCT
GGATATTCATGGACAATCTCCTGCTGACAGAGGACGACCAAGAGACTACGACTAGATTTCTT
CAGACCGAGGCCGTTACTCTCCAGGACGTTAGAGGTAGGGACTACCAGAATGCCATGCGAGT
GTGGAGTAACATCCCCGGACTCAAGTCAAAGCACGCACCCCTGACCCCCAAGGAAGAGGAAT
ACCTCCAGGCGCAGGTGAGGAGCCGCAGTAAGCTCGATGCTCCTAAAGTCGATTTGTTGGTG
AAGAATTGCTTGCTGCCCCTGAGAGAGTACTTTAAGTACTTTAGCCAGAACAGCTTGCCACT
G
   **Efla promoter**
   KRAB
   *hTEAD₁₋₁₆₆*
   **DNMT3a3L**
Ef1a::KRAB-DNMT3A/L-*hMYC₁₄₄₋₄₅₄* (SEQ ID NO: 179) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CATGGATGCTAAGTCACTGACTGCCTGGTCCCGGACACTGGTGACCTTCAAGGATGTGTTTG
TGGACTTCACCAGGGAGGAGTGGAAGCTGCTGGACACTGCTCAGCAGATCCTGTACAGAAAT
GTGATGCTGGAGAACTATAAGAACCTGGTTTCCTTGGGTTATCAGCTTACTAAGCCAGATGT
GATCCTCCGGTTGGAGAAGGGAGAAGAGCCCTGGCTGGTGGAGAGAGAAATTCACCAAGAGA
 
CCCATCCTGATTCAGAGACTGCATTTGAAATCAAATCATCAGTTAACCATGATCAAGAGTTC
GATCCTCCTAAAGTGTATCCCCCTGTCCCTGCCGAAAAGCGGAAACCGATCAGGGTTCTCAG
TCTCTTCGATGGAATTGCCACAGGCCTCTTGGTCCTTAAGGACCTGGGCATCCAAGTGGACC
GCTACATCGCCTCAGAAGTATGTGAGGACAGCATCACAGTCGGCATGGTGCGCCACCAGGGG
AAAATAATGTACGTCGGCGACGTCAGATCAGTTACTCAGAAACATATTCAGGAATGGGGGCC
TTTTGATCTCGTAATTGGCGGCAGCCCCTGCAATGATCTGAGTATTGTGAACCCCGCCCGGA
AAGGTTTGTACGAAGGTACGGGCAGACTGTTCTTCGAGTTTTACAGACTGTTGCACGACGCA
CGACCAAAGGAGGGTGACGATCGACCCTTCTTCTGGCTGTTCGAGAACGTGGTCGCTATGGG
CGTGTCTGACAAGCGGGACATTTCTAGATTCCTGGAGAGCAATCCAGTGATGATTGATGCAA
AGGAAGTATCCGCTGCCCACCGCGCCAGATACTTCTGGGGCAATCTGCCCGGCATGAATCGA
CCCTTGGCAAGCACCGTGAATGATAAGTTGGAGTTGCAGGAGTGTCTGGAGCATGGCGGAT
CGCAAAGTTTAGCAAGGTGCGGACCATCACGACCCGAAGCAATTCAATTAAGCAGGGAAAGG
ACCAACATTTTCCAGTGTTTATGAACGAGAAAGAGGACATCCTGTGGTGCACAGAAATGGAG
CGGGTTTTCGGATTCCCCGTACATTATACTGATGTGTCCAATATGTCTAGGTTGGCTAGACA
GAGGCTGTTGGGACGCTCATGGAGTGTTCCTGTCATCCGCCACCTGTTCGCTCCTCTGAAGG
AGTATTTCGCCTGCGTTTCATCCGGGAATTCAAACGCAAACAGCAGAGGCCCATCCTTTTCT
TCCGGCCTGGTGCCACTTAGTCTGCGCGGCTCTCACATGGGACCTATGGAAATATACAAAAC
AGTGAGCGCCTGGAAGAGGCAGCCTGTGCGGGTCCTGAGTCTGTTCAGAAACATTGACAAGG
TGCTTAAGTCCCTTGGATTTCTGGAGTCTGGCAGCGGTTCTGGGGGTGGCACCCTTAAGTAT
GTGGAGGACGTGACAAACGTCGTGAGGAGAGATGTGGAGAAATGGGGGCCATTTGACCTGGT
ATACGGGAGCACTCAACCTCTCGGTTCTTCTTGTGATCGCTGCCCAGGGTGGTATATGTTTC
AGTTCCATCGCATCCTGCAATACGCCCTTCCGCGGCAGGAGAGTCAGCGACCATTCTTCTGG
ATATTCATGGACAATCTCCTGCTGACAGAGGACGACCAAGAGACTACGACTAGATTTCTTCA
GACCGAGGCCGTTACTCTCCAGGACGTTAGAGGTAGGGACTACCAGAATGCCATGCGAGTGT
GGAGTAACATCCCCGGACTCAAGTCAAAGCACGCACCCCTGACCCCCAAGGAAGAGGAATAC
CTCCAGGCGCAGGTGAGGAGCCGCAGTAAGCTCGATGCTCCTAAAGTCGATTTGTTGGTGAA
GAATTGCTTGCTGCCCCTGAGAGAGTACTTTAAGTACTTTAGCCAGAACAGCTTGCCACTGA
TCATCCAGGACTGTATGTGGAGCGGCTTCTCGGCCGCCGCCAAGCTCGTCTCAGAGAAGCTG
GCCTCCTACCAGGCTGCGCGCAAAGACAGCGGCAGCCCGAACCCCGCCCGCGGCCACAGCGT
CTGCTCCACCTCCAGCTTGTACCTGCAGGATCTGAGCGCCGCCGCCTCAGAGTGCATCGACC
CCTCGGTGGTCTTCCCCTACCCTCTCAACGACAGCAGCTCGCCCAAGTCCTGCGCCTCGCAA
GACTCCAGCGCCTTCTCTCCGTCCTCGGATTCTCTGCTCTCCTCGACGGAGTCCTCCCCGCA
GGGCAGCCCCGAGCCCCTGGTGCTCCATGAGGAGACACCGCCCACCACCAGCAGCGACTCTG
AGGAGGAACAAGAAGATGAGGAAGAAATCGATGTTGTTTCTGTGGAAAAGAGGCAGGCTCCT
GGCAAAAGGTCAGAGTCTGGATCACCTTCTGCTGGAGGCCACAGCAAACCTCCTCACAGCCC
ACTGGTCCTCAAGAGGTGCCACGTCTCCACACATCAGCACAACTACGCAGCGCCTCCCTCCA
CTCGGAAGGACTATCCTGCTGCCAAGAGGGTCAAGTTGGACAGTGTCAGAGTCCTGAGACAG
ATCAGCAACAACCGAAAATGCACCAGCCCCAGGTCCTCGGACACCGAGGAGAATGTCAAGAG
GCGAACACACAACGTCTTGGAGCGCCAGAGGAGGAACGAGCTAAAACGGAGCTTTTTTGCCC
TGCGTGACCAGATCCCGGAGTTGGAAAACAATGAAAAGGCCCCCAAGGTAGTTATCCTTAAA
AAAGCCACAGCATACATCCTGTCCGTCCAAGCAGAGGAGCAAAAGCTCATTTCTGAAGAGGA
CTTGTTGCGGAAACGACGAGAACAGTTGAAACACAAACTTGAACAGCTACGGAACTCTTGTG
CG
   **Efla promoter**
   KRAB
   **DNMT3a3L**
   *hMYC₁₄₄₋₄₅₄*
Ef1a::KRAB- hSOX2₁₋₁₇₉-DNMT3A/L (SEQ ID NO: 180) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CATGGATGCTAAGTCACTGACTGCCTGGTCCCGGACACTGGTGACCTTCAAGGATGTGTTTG
TGGACTTCACCAGGGAGGAGTGGAAGCTGCTGGACACTGCTCAGCAGATCCTGTACAGAAAT
GTGATGCTGGAGAACTATAAGAACCTGGTTTCCTTGGGTTATCAGCTTACTAAGCCAGATGT
GATCCTCCGGTTGGAGAAGGGAGAAGAGCCCTGGCTGGTGGAGAGAGAAATTCACCAAGAGA
CCCATCCTGATTCAGAGACTGCATTTGAAATCAAATCATCAGTTATGTACAACATGATGGAG
ACGGAGCTGAAGCCGCCGGGCCCGCAGCAAACTTCGGGGGGCGGCGGCGGCAACTCCACCGC
GGCGGCGGCCGGCGGCAACCAGAAAAACAGCCCGGACCGCGTCAAGCGGCCCATGAATGCCT
TCATGGTGTGGTCCCGCGGGCAGCGGCGCAAGATGGCCCAGGAGAACCCCAAGATGCACAAC
TCGGAGATCAGCAAGCGCCTGGGCGCCGAGTGGAAACTTTTGTCGGAGACGGAGAAGCGGCC
GTTCATCGACGAGGCTAAGCGGCTGCGAGCGCTGCACATGAAGGAGCACCCGGATTATAAAT
ACCGGCCCCGGCGGAAAACCAAGACGCTCATGAAGAAGGATAAGTACACGCTGCCCGGCGGG
CTGCTGGCCCCCGGCGGCAATAGCATGGCGAGCGGGGTCGGGGTGGGCGCCGGCCTGGGCGC
GGGCGTGAACCAGCGCATGGACAGTTACGCGCACATGAACGGCTGGAGCAACGGCAGCTACA
GCATGATGCAGGACCAGCTGGGCAACCATGATCAAGAGTTCGATCCTCCTAAAGTGTATCCC
CCTGTCCCTGCCGAAAAGCGGAAACCGATCAGGGTTCTCAGTCTCTTCGATGGAATTGCCAC
AGGCCTCTTGGTCCTTAAGGACCTGGGCATCCAAGTGGACCGCTACATCGCCTCAGAAGTAT
GTGAGGACAGCATCACAGTCGGCATGGTGCGCCACCAGGGGAAAATAATGTACGTCGGCGAC
GTCAGATCAGTTACTCAGAAACATATTCAGGAATGGGGGCCTTTTGATCTCGTAATTGGCGG
CAGCCCCTGCAATGATCTGAGTATTGTGAACCCCGCCCGGAAAGGTTTGTACGAAGGTACGG
GCAGACTGTTCTTCGAGTTTTACAGACTGTTGCACGACGCACGACCAAAGGAGGGTGACGAT
CGACCCTTCTTCTGGCTGTTCGAGAACGTGGTCGCTATGGGCGTGTCTGACAAGCGGGACAT
TTCTAGATTCCTGGAGAGCAATCCAGTGATGATTGATGCAAAGGAAGTATCCGCTGCCCACC
GCGCCAGATACTTCTGGGGCAATCTGCCCGGCATGAATCGACCCTTGGCAAGCACCGTGAAT
GATAAGTTGGAGTTGCAGGAGTGTCTGGAGCATGGCGGATCGCAAAGTTTAGCAAGGTGCG
 
GACCATCACGACCCGAAGCAATTCAATTAAGCAGGGAAAGGACCAACATTTTCCAGTGTTTA
TGAACGAGAAAGAGGACATCCTGTGGTGCACAGAAATGGAGCGGGTTTTCGGATTCCCCGTA
CATTATACTGATGTGTCCAATATGTCTAGGTTGGCTAGACAGAGGCTGTTGGGACGCTCATG
GAGTGTTCCTGTCATCCGCCACCTGTTCGCTCCTCTGAAGGAGTATTTCGCCTGCGTTTCAT
CCGGGAATTCAAACGCAAACAGCAGAGGCCCATCCTTTTCTTCCGGCCTGGTGCCACTTAGT
CTGCGCGGCTCTCACATGGACCTATGGAAATATACAAAACAGTGAGCGCCTGGAAGAGGCA
GCCTGTGCGGGTCCTGAGTCTGTTCAGAAACATTGACAAGGTGCTTAAGTCCCTTGGATTTC
TGGAGTCTGGCAGCGGTTCTGGGGGTGGCACCCTTAAGTATGTGGAGGACGTGACAAACGTC
GTGAGGAGAGATGTGGAGAAATGGGGGCCATTTGACCTGGTATACGGAGCACTCAACCTCT
CGGTTCTTCTTGTGATCGCTGCCCAGGGTGGTATATGTTTCAGTTCCATCGCATCCTGCAAT
ACGCCCTTCCGCGGCAGGAGAGTCAGCGACCATTCTTCTGGATATTCATGGACAATCTCCTG
CTGACAGAGGACGACCAAGAGACTACGACTAGATTTCTTCAGACCGAGGCCGTTACTCTCCA
GGACGTTAGAGGTAGGGACTACCAGAATGCCATGCGAGTGTGGAGTAACATCCCCGGACTCA
AGTCAAAGCACGCACCCCTGACCCCCAAGGAAGAGGAATACCTCCAGGCGCAGGTGAGGAGC
CGCAGTAAGCTCGATGCTCCTAAAGTCGATTTGTTGGTGAAGAATTGCTTGCTGCCCCTGAG
AGAGTACTTTAAGTACTTTAGCCAGAACAGCTTGCCACTG
   **Efla promoter**
   KRAB
   *hSOX2₁₋₁₇₉*
   **DNMT3a3L**
Ef1a::CS-hSOX2₁₋₁₇₉ (SEQ ID NO: 181) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CATGCTGGAACCAGAAAAGATCATTGGGGCAACAGATTCCTGTGGTGATTTAATGTTCCTAA
TGAAATGGAAAGACACAGATGAAGCTGACCTGGTTCTTGCAAAAGAAGCTAATGTGAAATGT
 
CCACAAATTGTGATAGCATTTTATGAAGAGAGACTGACATGGCATGCATATCCTGAGATGTA
CAACATGATGGAGACGGAGCTGAAGCCGCCGGGCCCGCAGCAAACTTCGGGGGGCGGCGGCG
GCAACTCCACCGCGGCGGCGGCCGGCGGCAACCAGAAAAACAGCCCGGACCGCGTCAAGCGG
CCCATGAATGCCTTCATGGTGTGGTCCCGCGGGCAGCGGCGCAAGATGGCCCAGGAGAACCC
CAAGATGCACAACTCGGAGATCAGCAAGCGCCTGGGCGCCGAGTGGAAACTTTTGTCGGAGA
CGGAGAAGCGGCCGTTCATCGACGAGGCTAAGCGGCTGCGAGCGCTGCACATGAAGGAGCAC
CCGGATTATAAATACCGGCCCCGGCGGAAAACCAAGACGCTCATGAAGAAGGATAAGTACAC
GCTGCCCGGCGGGCTGCTGGCCCCCGGCGGCAATAGCATGGCGAGCGGGGTCGGGGTGGGCG
CCGGCCTGGGCGCGGGCGTGAACCAGCGCATGGACAGTTACGCGCACATGAACGGCTGGAGC
AACGGCAGCTACAGCATGATGCAGGACCAGCTGGGC
   **Efla promoter**
   CS
   *hSOX2₁₋₁₇₉*
Ef1a:: SOX2₁₋₁₇₉-Y-R (SEQ ID NO: 182) 
CGATAAGCTTTGCAAAGATGGATAAAGTTTTAAACAGAGAGGAATCTTTGCAGCTAATGGAC
CTTCTAGGTCTTGAAAGGAGTGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CATGTACAACATGATGGAGACGGAGCTGAAGCCGCCGGGCCCGCAGCAAACTTCGGGGGGCG
GCGGCGGCAACTCCACCGCGGCGGCGGCCGGCGGCAACCAGAAAAACAGCCCGGACCGCGTC
AAGCGGCCCATGAATGCCTTCATGGTGTGGTCCCGCGGGCAGCGGCGCAAGATGGCCCAGGA
GAACCCCAAGATGCACAACTCGGAGATCAGCAAGCGCCTGGGCGCCGAGTGGAAACTTTTGT
CGGAGACGGAGAAGCGGCCGTTCATCGACGAGGCTAAGCGGCTGCGAGCGCTGCACATGAAG
GAGCACCCGGATTATAAATACCGGCCCCGGCGGAAAACCAAGACGCTCATGAAGAAGGATAA
GTACACGCTGCCCGGCGGGCTGCTGGCCCCCGGCGGCAATAGCATGGCGAGCGGGGTCGGGG
TGGGCGCCGGCCTGGGCGCGGGCGTGAACCAGCGCATGGACAGTTACGCGCACATGAACGGC
TGGAGCAACGGCAGCTACAGCATGATGCAGGACCAGCTGGGCATGAGGCCCCGGCTGAAAAA
CGTGGACAGGAGCACTGCACAGCAGTTGGCAGTAACTGTGGGCAACGTCACCGTCATTATCA
 
CAGACTTTAAGGAAAAGACTCGCTCCTCATCGACATCCTCATCCACAGTGACCTCCAGTGCA
GGGTCAGAACAGCAGAACCAGGGCGCGCCCGGTAAGCCTATCCCTAACCCTCTCCTCGGTCT
CGATTCTACGTGA
   **Ef1a promoter**
   *hSOX2₁₋₁₇₉*
   Y-R

In one embodiment, the polynucleotide comprises a nucleotide sequence that has at least 90% sequence identity, such as at least 91%, 92%, 93%, 94%, 95%, 96%, 97%, 98%, 99%, or 100% sequence identity to any one of SEQ ID NOs: 158 - 163.

In one embodiment, the polynucleotide comprises a sequence according to any one of SEQ ID NOs: 158 - 163.

In one embodiment, the polynucleotide comprises a nucleotide sequence that has at least 90% sequence identity, such as at least 91%, 92%, 93%, 94%, 95%, 96%, 97%, 98%, 99%, or 100% sequence identity to any one of SEQ ID NOs: 178 - 182.

In one embodiment, the polynucleotide comprises a sequence according to any one of SEQ ID NOs: 178 - 182.

### miRNA target sequences

In some embodiments, the polynucleotide further comprises one or more miRNA target sequence. Suitably, the nucleic acid sequence encoding the transgene (e.g. ESF) is operably linked to the one or more miRNA target sequence.

MicroRNA (miRNA) genes are scattered across all human chromosomes, except for the Y chromosome. They can be either located in non-coding regions of the genome or within introns of protein-coding genes. Around 50% of miRNAs appear in clusters which are transcribed as polycistronic primary transcripts. Similar to protein-coding genes, miRNAs are usually transcribed from polymerase-II promoters, generating a so-called primary miRNA transcript (pri-miRNA). This pri-miRNA is then processed through a series of endonucleolytic cleavage steps, performed by two enzymes belonging to the RNAse Type III family, Drosha and Dicer. From the pri-miRNA, a stem loop of about 60 nucleotides in length, called miRNA precursor (pre-miRNA), is excised by a specific nuclear complex, composed of Drosha and DiGeorge syndrome critical region gene (DGCR8), which crops both strands near the base of the primary stem loop and leaves a 5' phosphate and a 2 bp long, 3' overhang. The pre-miRNA is then actively transported from the nucleus to the cytoplasm by RAN-GTP and Exportin. Then, Dicer performs a double strand cut at the end of the stem loop not defined by the Drosha cut, generating a 19-24 bp duplex, which is composed of the mature miRNA and the opposite strand of the duplex, called miRNA*. In agreement with the thermodynamic asymmetry rule, only one strand of the duplex is selectively loaded into the RNA-induced silencing complex (RISC), and accumulates as the mature microRNA. This strand is usually the one whose 5' end is less tightly paired to its complement, as was demonstrated by single-nucleotide mismatches introduced into the 5' end of each strand of siRNA duplexes. However, there are some miRNAs that support accumulation of both duplex strands to similar extent.

MicroRNAs trigger RNAi, very much like small interfering RNAs (siRNA) which are extensively used for experimental gene knockdown. The main difference between miRNA and siRNA is their biogenesis. Once loaded into RISC, the guide strand of the small RNA molecule interacts with mRNA target sequences preferentially found in the 3' untranslated region (3'UTR) of protein-coding genes. It has been shown that nucleotides 2-8 counted from the 5' end of the miRNA, the so-called seed sequence, are essential for triggering RNAi. If the whole guide strand sequence is perfectly complementary to the mRNA target, as is usually the case for siRNAs and plant miRNAs, the mRNA is endonucleolytically cleaved by involvement of the Argonaute (Ago) protein, also called "slicer" of the small RNA duplex into the RNA-induced silencing complex (RISC). DGRC (DiGeorge syndrome critical region gene 8) and TRBP (TAR (HIV) RNA binding protein 2) are double-stranded RNA-binding proteins that facilitate mature miRNA biogenesis by Drosha and Dicer RNase III enzymes, respectively. The guide strand of the miRNA duplex gets incorporated into the effector complex RISC, which recognises specific targets through imperfect base-pairing and induces post-transcriptional gene silencing. Several mechanisms have been proposed for this mode of regulation: miRNAs can induce the repression of translation initiation, mark target mRNAs for degradation by deadenylation, or sequester targets into the cytoplasmic P-body.

On the other hand, if only the seed is perfectly complementary to the target mRNA but the remaining bases show incomplete pairing, RNAi acts through multiple mechanisms leading to translational repression. Eukaryotic mRNA degradation mainly occurs through the shortening of the polyA tail at the 3' end of the mRNA, and de-capping at the 5' end, followed by 5'-3' exonuclease digestion and accumulation of the miRNA in discrete cytoplasmic areas, the so called P-bodies, enriched in components of the mRNA decay pathway.

Expression of the nucleic acid sequence encoding the transgene (e.g. ESF) may be regulated by one or more endogenous miRNAs using one or more corresponding miRNA target sequence. Using this method, one or more miRNAs endogenously expressed in a cell prevent or reduce transgene expression in that cell by binding to its corresponding miRNA target sequence positioned in the polynucleotide or vector.

The target sequence may be fully or partially complementary to the miRNA. The term "fully complementary", as used herein, may mean that the target sequence has a nucleic acid sequence which is 100% complementary to the sequence of the miRNA which recognises it. The term "partially complementary", as used herein, may mean that the target sequence is only in part complementary to the sequence of the miRNA which recognises it, whereby the partially complementary sequence is still recognised by the miRNA. In other words, a partially complementary target sequence in the context of the present invention is effective in recognising the corresponding miRNA and effecting prevention or reduction of transgene expression in cells expressing that miRNA. Suitably, a partially complementary miRNA target sequence may be fully complementary to the miRNA seed sequence.

Including more than one copy of a miRNA target sequence may increase the effectiveness of the system. Also, different miRNA target sequences can be included. For example, the protein-coding (e.g. ESF-coding) sequence may be operably linked to more than one miRNA target sequence, which may or may not be different. The miRNA target sequences may be in tandem, but other arrangements are envisaged. The polynucleotide may, for example, comprise 1, 2, 3, 4, 5, 6, 7 or 8 copies of the same or different miRNA target sequences. Suitably, the polynucleotide comprises 4 copies of each miRNA target sequence.

Copies of miRNA target sequences may be separated by a spacer sequence. The spacer sequence may comprise, for example, at least one, at least two, at least three, at least four or at least five nucleotide bases.

The one or more miRNA target sequence may, for example, suppress expression of the transgene (e.g. nucleic acid sequence encoding the ESF) in non-cancer cells. This may, for example, increase safety of a therapy using the ESF. Expression of the transgene (e.g. nucleic acid sequence encoding the ESF) in cancer cells may, for example, not be suppressed by the one or more miRNA target sequence.

The one or more miRNA target sequence may suppress transgene expression in one or more cells other than cancer cells, for example neurons, astrocytes and/or oligodendrocytes. In one embodiment, the one or more miRNA target sequence suppresses transgene expression in neurons. In one embodiment, the one or more miRNA target sequence suppresses transgene expression in astrocytes. In one embodiment, the one or more miRNA target sequence suppresses transgene expression in oligodendrocytes.

The term "suppress expression" as used herein may refer to a reduction of expression in the relevant cell type(s) of a transgene to which the one or more miRNA target sequence is operably linked as compared to transgene expression in the absence of the one or more miRNA target sequence, but under otherwise substantially identical conditions. In some embodiments, transgene expression is suppressed by at least 50%. In some embodiments, transgene expression is suppressed by at least 60%, 70%, 80%, 90% or 95%. In some embodiments, transgene expression is substantially prevented.

In some embodiments, the transgene encoding an ESF is operably linked to an Ef1a promoter and one or more miRNA target sequence. Exemplary polynucleotide sequences comprising a transgene encoding an ESF operably linked to an EF1a promoter and one or more miRNA target sequence are as follows:
Ef1a:: KRAB-hSOX2₁₋₁₇₉-DNMT3a3L-Tmir [SES-Tmir] (SEQ ID NO: 164) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGGATGCTAAGTCACTGACTGCCTGGTCCCGGACACTGGTGACCTTCA
AGGATGTGTTTGTGGACTTCACCAGGGAGGAGTGGAAGCTGCTGGACACTGCTCAGCAGATC
CTGTACAGAAATGTGATGCTGGAGAACTATAAGAACCTGGTTTCCTTGGGTTATCAGCTTAC
TAAGCCAGATGTGATCCTCCGGTTGGAGAAGGGAGAAGAGCCCTGGCTGGTGGAGAGAGAAA
TTCACCAAGAGACCCATCCTGATTCAGAGACTGCATTTGAAATCAAATCATCAGTTGGCAGC
GGCGCATGCatgtacaacatgatggagacggagctgaagccgccgggcccgcagcaaacttc
gggggcggcggcggcaactccaccgcggcggcggccggcggcaaccagaaaaacagcccgg
accgcgtcaagcggcccatgaatgccttcatggtgtggtcccgcgggcagcggcgcaagatg
gcccaggagaaccccaagatgcacaactcggagatcagcaagcgcctgggcgccgagtggaa
 
acttttgtcggagacggagaagcggccgttcatcgacgaggctaagcggctgcgagcgctgc
acatgaaggagcacccggattataaataccggccccggcggaaaccaagacgctcatgaag
aaggataagtacacgctgcccggcgggctgctggcccccggcggcaatagcatggcgagcgg
ggtcggggtgggcgccggcctgggcgcgggcgtgaaccagcgcatggacagttacgcgcaca
tgaacggctggagcaacggcagctacagcatgatgcaggaccagctgggcGCTAGCGGCAGC
GGCAACCATGATCAAGAGTTCGATCCTCCTAAAGTGTATCCCCCTGTCCCTGCCGAAAAGCG
GAAACCGATCAGGGTTCTCAGTCTCTTCGATGGAATTGCCACAGGCCTCTTGGTCCTTAAGG
ACCTGGGCATCCAAGTgGAcCGCTACATCGCCTCAGAAGTATGTGAgGACAGCATCACAGTC
GGCATGGTGCGCCACCAGGGGAAAATAATGTACGTCGGCGACGTCAGATCAGTTACTCAGAA
ACATATTCAGGAATGGGGGCCTTTTGATCTCGTAATTGGCGGCAGCCCCTGCAATGATCTGA
GTATTGTGAACCCCGCCCGGAAAGGTTTGTACGAAGGTACGGGCAGACTGTTCTTCGAGTTT
TACAGACTGTTGCACGACGCACGACCAAAGGAGGGTGAcGAtcGACCCTTCTTCTGGCTGTT
CGAGAACGTGGTCGCTATGGGCGTGTCTGACAAGCGGGACATTTCtAGATTCCTGGAGAGCA
ATCCAGTGATGATTGATGCAAAGGAAGTAtccGCTGCCCACCGCGCCAGATACTTCTGGGGC
AATCTGCCCGGCATGAATCGACCCTTGGCAAGCACCGTGAATGATAAGTTGGAGTTGCAGGA
GTGTCTgGAGCATGGGCGGATCGCAAAGTTTAGCAAGGTGCGGACCATCACGACCCGAAGCA
ATTCAATTAAGCAGGGAAAGGACCAACATTTTCCAGTGTTTATGAACGAGAAAGAGGAcATC
CTGTGGTGcACAGAAATGGAGCGGGTTTTCGGATTCCCCGTACATTATACTGATGTGTCCAA
TATGTCTAGGTTGGCTAGACAGAGGCTGtTGGGACGCTCATGGAGTGTTCCTGTCATCCGCC
ACCTGTTCGCTCCTCTGAAGGAGTATTTCGCcTGCGTtTCATCCGGgAATTCAAACGCAAAC
AGCAGAGGCCCATCCTTTTCTTCCGGCCTGGTgCCACTTAGTCTGCGCGGCTCTCACATGGG
ACCTATGGAAATATACAAAACAGTGAGCGCCTGGAAGAGGCAGCCTGTGCGGGTCCTGAGTC
TGTTCAGAAACATTGACAAGGTGCTTAAGTCCCTTGGATTTCTGGAGTCTGGCAGCGGTTCT
GGGGGTGgcACCCTTAAGTATGTGGAGGACGTGACAAACGTCGTGAGGAGAGATGTGGAGAA
ATGGGGGCCATTTGACCTGGTATACGGGAGCACTCAACCTCTCGGTTCTTCTTGTGATCGCT
GCCCAGGGTGGTATATGTTTCAGTTCCATCGcATCCTGCAATACGCCCTTCCGCGGCAGGAG
AGTCAGCGACCATTCTTCTGGATATTCATGGACAATCTCCTGCTGACAGAgGACGACCAAGA
GACTACGACTAGATTTCTTCAGACCGAGGCCGTTACTCTCCAGGACGTTAGAGGTAGGGACT
ACCAGAATGCCATGCGAGTGTGGAGTAACATCCCCGGACTCAAGTCAAAGCAcGCACCCCTG
ACCCCCAAGGAAGAGGAATACCTcCAGGCGCAGGTGAGGAGCCGCAGTAAGCTCGATGCTCC
TAAAGTCGATTTGTTGGTGAAGAATTGCTTGCTGCCCCTGAGAGAGTACTTTAAGTACTTTA
GCCAGAACAGCTTGCCACTGTGAatattgccttatttccgattattgccttatttcgcatta
ttgccttatttctcactattgccttatttccgatcccgggtttaaaccgatcaacaaaatc
actgatgctggacgatcaacaaaatcactgatgctggacgatcaacaaaatcactgatgctg
gatcaccaacaaaatcactgatgctggacgatgtttaaaccctgcaggcgatcagctatgcc
agcatcttgcccgatcagctatgccagcatcttgcccgatcagctatgccagcatcttgcct
caccagctatgccagcatcttgcccgatcctgcaggagatct
   **Efla promoter**
   KRAB
   *hSOX2₁₋₁₇₉*
   **DNMT3a3L**
   miR124 Target sequences
   miR338-3p Target sequences
   miR31 Target sequences
Ef1a::CS-hSOX2₁₋₁₇₉ -Tmir [SESv2-Tmir] (SEQ ID NO: 165) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGGCATGCATGTACAACATGATGGAGACGGAGCTGAAGCCGCCGGGCCCGCAGCAAA
CTTCGGGGGCGGCGGCGGCAACTCCACCGCGGCGGCGGCCGGCGGCAACCAGAAAAACAGC
CCGGACCGCGTCAAGCGGCCCATGAATGCCTTCATGGTGTGGTCCCGCGGGCAGCGGCGCAA
GATGGCCCAGGAGAACCCCAAGATGCACAACTCGGAGATCAGCAAGCGCCTGGGCGCCGAGT
GGAAACTTTTGTCGGAGACGGAGAAGCGGCCGTTCATCGACGAGGCTAAGCGGCTGCGAGCG
CTGCACATGAAGGAGCACCCGGATTATAAATACCGGCCCCGGCGGAAAACCAAGACGCTCAT
GAAGAAGGATAAGTACACGCTGCCCGGCGGGCTGCTGGCCCCCGGCGGCAATAGCATGGCGA
GCGGGGTCGGGGTGGGCGCCGGCCTGGGCGCGGGCGTGAACCAGCGCATGGACAGTTACGCG
CACATGAACGGCTGGAGCAACGGCAGCTACAGCATGATGCAGGACCAGCTGGGCTGAatatt
gccttatttccgattattgccttatttcgcattattgccttatttctcactattgccttatt
tccgatcccggggtttaaaccgatcaacaaaatcactgatgctggacgatcaacaaaatcac
tgatgctggacgatcaacaaaatcactgatgctggatcaccaacaaaatcactgatgctgga
cgatgtttaaaccctgcaggcgatcagctatgccagcatcttgcccgatcagctatgccagc
atcttgcccgatcagctatgccagcatcttgcctcaccagctatgccagcatcttgcccgat
cctgcaggagatct
   **Efla promoter**
   CS
   *hSOX2₁₋₁₇₉*
   miR124 Target sequences
   miR338-3p Target sequences
   miR31 Target sequences
Ef1a:: SOX2₁₋₁₇₉-Y-R-Tmir [SESv3-Tmir] (SEQ ID NO: 166) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCatgtacaacatgatggagacggagctgaagccgccgggcccgcagcaaa
cttcgggggcggcggcggcaactccaccgcggcggcggccggcggcaaccagaaaaacagc
ccggaccgcgtcaagcggcccatgaatgccttcatggtgtggtcccgcgggcagcggcgcaa
gatggcccaggagaaccccaagatgcacaactcggagatcagcaagcgcctgggcgccgagt
ggaaacttttgtcggagacggagaagcggccgttcatcgacgaggctaagcggctgcgagcg
ctgcacatgaaggagcacccggattataaataccggccccggcggaaaaccaagacgctcat
gaagaaggataagtacacgctgcccggcgggctgctggcccccggcggcaatagcatggcga
gcggggtcggggtgggcgccggcctgggcgcgggcgtgaaccagcgcatggacagttacgcg
cacatgaacggctggagcaacggcagctacagcatgatgcaggaccagctgggcgctagcGG
ATCCaacATGAGGCCCCGGCTGAAAAACGTGGACAGGAGCACTGCACAGCAGTTGGCAGTAA
CTGTGGGCAACGTCACCGTCATTATCACAGACTTTAAGGAAAAGACTCGCTCCTCATCGACA
TCCTCATCCACAGTGACCTCCAGTGCAGGGTCAGAACAGCAGAACCAGTGAatattgcctta
tttccgattattgccttatttcgcattattgccttatttctcactattgccttatttccgat
cccggggtttaaaccgatcaacaaaatcactgatgctggacgatcaacaaaatcactgatgc
tggacgatcaacaaaatcactgatgctggatcaccaacaaaatcactgatgctggacgatgt
ttaaaccctgcaggcgatcagctatgccagcatcttgcccgatcagctatgccagcatcttg
cccgatcagctatgccagcatcttgcctcaccagctatgccagcatcttgcccgatcctgca
ggagatct
   **Ef1a promoter**
   *hSOX2₁₋₁₇₉*
   Y-R
   miR124 Target sequences
   miR338-3p Target sequences
   miR31 Target sequences
Ef1a::KRAB-hTEAD₁₋₁₆₆- DNMT3a3L- Tmir [**TES-Tmir**](SEQ ID NO: 167) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGGATGCTAAGTCACTGACTGCCTGGTCCCGGACACTGGTGACCTTCA
AGGATGTGTTTGTGGACTTCACCAGGGAGGAGTGGAAGCTGCTGGACACTGCTCAGCAGATC
CTGTACAGAAATGTGATGCTGGAGAACTATAAGAACCTGGTTTCCTTGGGTTATCAGCTTAC
TAAGCCAGATGTGATCCTCCGGTTGGAGAAGGGAGAAGAGCCCTGGCTGGTGGAGAGAGAAA
TTCACCAAGAGACCCATCCTGATTCAGAGACTGCATTTGAAATCAAATCATCAGTTGGCAGC
GGCGCATGCATTGAGCCCAGCAGCTGGAGCGGCAGTGAGAGCCCTGCCGAAAACATGGAAAG
GATGAGTGACTCTGCAGATAAGCCAATTGACAATGATGCAGAAGGGGTCTGGAGCCCCGACA
TCGAGCAAAGCTTTCAGGAGGCCCTGGCTATCTATCCACCATGTGGGAGGAGGAAAATCATC
TTATCAGACGAAGGCAAAATGTATGGTAGGAATGAATTGATAGCCAGATACATCAAACTCAG
GACAGGCAAGACGAGGACCAGAAAACAGGTGTCTAGTCACATTCAGGTTCTTGCCAGAAGGA
AATCTCGTGATTTTCATTCCAAGCTAAAGGATCAGACTGCAAAGGATAAGGCCCTGCAGCAC
ATGGCGGCCATGTCCTCAGCCCAGATCGTCTCGGCCACTGCCATTCATAACAAGCTGGGGCT
GCCTGGGATTCCACGCCCGACCTTCCCAGGGGCGCCGGGGTTCTGGCCGGGAATGATTCAAA
CAGGGCAGCCAGCTAGCGGCAGCGGCAACCATGATCAAGAGTTCGATCCTCCTAAAGTGTAT
CCCCCTGTCCCTGCCGAAAAGCGGAAACCGATCAGGGTTCTCAGTCTCTTCGATGGAATTGC
CACAGGCCTCTTGGTCCTTAAGGACCTGGGCATCCAAGTgGAcCGCTACATCGCCTCAGAAG
TATGTGAgGACAGCATCACAGTCGGCATGGTGCGCCACCAGGGGAAAATAATGTACGTCGGC
 
GACGTCAGATCAGTTACTCAGAAACATATTCAGGAATGGGGGCCTTTTGATCTCGTAATTGG
CGGCAGCCCCTGCAATGATCTGAGTATTGTGAACCCCGCCCGGAAAGGTTTGTACGAAGGTA
CGGGCAGACTGTTCTTCGAGTTTTACAGACTGTTGCACGACGCACGACCAAAGGAGGGTGAc
GAtcGACCCTTCTTCTGGCTGTTCGAGAACGTGGTCGCTATGGGCGTGTCTGACAAGCGGGA
CATTTCtAGATTCCTGGAGAGCAATCCAGTGATGATTGATGCAAAGGAAGTAtccGCTGCCC
ACCGCGCCAGATACTTCTGGGGCAATCTGCCCGGCATGAATCGACCCTTGGCAAGCACCGTG
AATGATAAGTTGGAGTTGCAGGAGTGTCTgGAGCATGGCGGATCGCAAAGTTTAGCAAGGT
GCGGACCATCACGACCCGAAGCAATTCAATTAAGCAGGAAAGGACCAACATTTTCCAGTGT
TTATGAACGAGAAAGAGGAcATCCTGTGGTGcACAGAAATGGAGCGGGTTTTCGGATTCCCC
GTACATTATACTGATGTGTCCAATATGTCTAGGTTGGCTAGACAGAGGCTGtTGGGACGCTC
ATGGAGTGTTCCTGTCATCCGCCACCTGTTCGCTCCTCTGAAGGAGTATTTCGCcTGCGTtT
CATCCGGgAATTCAAACGCAAACAGCAGAGGCCCATCCTTTTCTTCCGGCCTGGTgCCACTT
AGTCTGCGCGGCTCTCACATGGGACCTATGGAAATATACAAAACAGTGAGCGCCTGGAAGAG
GCAGCCTGTGCGGGTCCTGAGTCTGTTCAGAAACATTGACAAGGTGCTTAAGTCCCTTGGAT
TTCTGGAGTCTGGCAGCGGTTCTGGGGGTGgcACCCTTAAGTATGTGGAGGACGTGACAAAC
GTCGTGAGGAGAGATGTGGAGAAATGGGGGCCATTTGACCTGGTATACGGGAGCACTCAACC
TCTCGGTTCTTCTTGTGATCGCTGCCCAGGGTGGTATATGTTTCAGTTCCATCGcATCCTGC
AATACGCCCTTCCGCGGCAGGAGAGTCAGCGACCATTCTTCTGGATATTCATGGACAATCTC
CTGCTGACAGAgGACGACCAAGAGACTACGACTAGATTTCTTCAGACCGAGGCCGTTACTCT
CCAGGACGTTAGAGGTAGGGACTACCAGAATGCCATGCGAGTGTGGAGTAACATCCCCGGAC
TCAAGTCAAAGCAcGCACCCCTGACCCCCAAGGAAGAGGAATACCTcCAGGCGCAGGTGAGG
AGCCGCAGTAAGCTCGATGCTCCTAAAGTCGATTTGTTGGTGAAGAATTGCTTGCTGCCCCT
GAGAGAGTACTTTAAGTACTTTAGCCAGAACAGCTTGCCACTGTGAatattgccttatttcc
gattattgccttatttcgcattattgccttatttctcactattgccttatttccgatcccgg
ggtttaaaccgatcaacaaaatcactgatgctggacgatcaacaaaatcactgatgctggac
gatcaacaaaatcactgatgctggatcaccaacaaaatcactgatgctggacgatgtttaaa
ccctgcaggcgatcagctatgccagcatcttgcccgatcagctatgccagcatcttgcccga
tcagctatgccagcatcttgcctcaccagctatgccagcatcttgcccgatcctgcaggaga
tct
   **Efla promoter**
   KRAB
   *hTEAD₁₋₁₆₆*
   **DNMT3a3L**
   miR124 Target sequences
   miR338-3p Target sequences
   miR31 Target sequences
Ef1a::CS- *hTEAD₁₋₁₆₆* -Tmir [**TESv2-Tmir**] (SEQ ID NO: 168) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
 
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGACCATTGAGCCCAGCAGCTGGAGCGGCAGTGAGAGCCCTGCCGAAAACATGGAAA
GGATGAGTGACTCTGCAGATAAGCCAATTGACAATGATGCAGAAGGGGTCTGGAGCCCCGAC
ATCGAGCAAAGCTTTCAGGAGGCCCTGGCTATCTATCCACCATGTGGGAGGAGGAAAATCAT
CTTATCAGACGAAGGCAAAATGTATGGTAGGAATGAATTGATAGCCAGATACATCAAACTCA
GGACAGGCAAGACGAGGACCAGAAAACAGGTGTCTAGTCACATTCAGGTTCTTGCCAGAAGG
AAATCTCGTGATTTTCATTCCAAGCTAAAGGATCAGACTGCAAAGGATAAGGCCCTGCAGCA
CATGGCGGCCATGTCCTCAGCCCAGATCGTCTCGGCCACTGCCATTCATAACAAGCTGGGGC
TGCCTGGGATTCCACGCCCGACCTTCCCAGGGGCGCCGGGGTTCTGGCCGGGAATGATTCAA
ACAGGGCAGCCATGAatattgccttatttccgattattgccttatttcgcattattgcctta
tttctcactattgccttatttccgatcccgggtttaaaccgatcaacaaaatcactgatgc
tggacgatcaacaaaatcactgatgctggacgatcaacaaaatcactgatgctggatcacca
acaaaatcactgatgctggacgatgtttaaaccctgcaggcgatcagctatgccagcatctt
gcccgatcagctatgccagcatcttgcccgatcagctatgccagcatcttgcctcaccagct
atgccagcatcttgcccgatcctgcaggagatct
   **Efla promoter**
   CS
   *hTEAD₁₋₁₆₆*
   miR124 Target sequences
   miR338-3p Target sequences
   miR31 Target sequences
Ef1a::*hTEAD₁₋₁₆₆*-*YR*-Tmir[**TESv3-Tmir**] (SEQ ID NO: 169) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
 
TGGCGCGGGGTAAACTGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGATTGAGCCCAGCAGCTGGAGCGGCAGTGAGAGCCCTGCCGAAAACA
TGGAAAGGATGAGTGACTCTGCAGATAAGCCAATTGACAATGATGCAGAAGGGGTCTGGAGC
CCCGACATCGAGCAAAGCTTTCAGGAGGCCCTGGCTATCTATCCACCATGTGGGAGGAGGAA
AATCATCTTATCAGACGAAGGCAAAATGTATGGTAGGAATGAATTGATAGCCAGATACATCA
AACTCAGGACAGGCAAGACGAGGACCAGAAAACAGGTGTCTAGTCACATTCAGGTTCTTGCC
AGAAGGAAATCTCGTGATTTTCATTCCAAGCTAAAGGATCAGACTGCAAAGGATAAGGCCCT
GCAGCACATGGCGGCCATGTCCTCAGCCCAGATCGTCTCGGCCACTGCCATTCATAACAAGC
TGGGGCTGCCTGGGATTCCACGCCCGACCTTCCCAGGGGCGCCGGGGTTCTGGCCGGGAATG
ATTCAAACAGGGCAGCCAaccATGAGGCCCGGCTGAAAAACGTGGACAGGAGCACTGCACA
GCAGTTGGCAGTAACTGTGGGCAACGTCACCGTCATTATCACAGACTTTAAGGAAAAGACTC
GCTCCTCATCGACATCCTCATCCACAGTGACCTCCAGTGCAGGGTCAGAACAGCAGAACCAG
TGAatattgccttatttccgattattgccttatttcgcattattgccttatttctcactatt
gccttatttccgatcccgggtttaaaccgatcaacaaaatcactgatgctggacgatcaac
aaaatcactgatgctggacgatcaacaaaatcactgatgctggatcaccaacaaaatcactg
atgctggacgatgtttaaaccctgcaggcgatcagctatgccagcatcttgcccgatcagct
atgccagcatcttgcccgatcagctatgccagcatcttgcctcaccagctatgccagcatct
tgcccgatcctgcaggagatct
   **Efla promoter**
   *hTEAD₁₋₁₆₆*
   *Y-R*
   miR124 Target sequences
   miR338-3p Target sequences
   miR31 Target sequences
Ef1a::CS- *hTEAD₁₋₁₆₆* - Y-R- Tmir [**TESv4-Tmir**] (SEQ ID NO: 170) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGACCATTGAGCCCAGCAGCTGGAGCGGCAGTGAGAGCCCTGCCGAAAACATGGAAA
GGATGAGTGACTCTGCAGATAAGCCAATTGACAATGATGCAGAAGGGGTCTGGAGCCCCGAC
ATCGAGCAAAGCTTTCAGGAGGCCCTGGCTATCTATCCACCATGTGGGAGGAGGAAAATCAT
CTTATCAGACGAAGGCAAAATGTATGGTAGGAATGAATTGATAGCCAGATACATCAAACTCA
GGACAGGCAAGACGAGGACCAGAAAACAGGTGTCTAGTCACATTCAGGTTCTTGCCAGAAGG
AAATCTCGTGATTTTCATTCCAAGCTAAAGGATCAGACTGCAAAGGATAAGGCCCTGCAGCA
CATGGCGGCCATGTCCTCAGCCCAGATCGTCTCGGCCACTGCCATTCATAACAAGCTGGGGC
TGCCTGGGATTCCACGCCCGACCTTCCCAGGGGCGCCGGGGTTCTGGCCGGGAATGATTCAA
ACAGGGCAGCCAACCatgaggccccggctgaaaaacgtggacaggagcactgcacagcagtt
ggcagtaactgtgggcaacgtcaccgtcattatcacagactttaaggaaaagactcgctcct
catcgacatcctcatccacagtgacctccagtgcagggtcagaacagcagaaccagTGAata
ttgccttatttccgattattgccttatttcgcattattgccttatttctcactattgcctta
tttccgatcccggggtttaaaccgatcaacaaaatcactgatgctggacgatcaacaaaatc
actgatgctggacgatcaacaaaatcactgatgctggatcaccaacaaaatcactgatgctg
gacgatgtttaaaccctgcaggcgatcagctatgccagcatcttgcccgatcagctatgcca
gcatcttgcccgatcagctatgccagcatcttgcctcaccagctatgccagcatcttgcccg
atcctgcaggagatct
   **Efla promoter**
   CS
   *hTEAD₁₋₁₆₆*
   *y-r*
   miR124 Target sequences
   miR338-3p Target sequences
   miR31 Target sequences
Ef1a::CS- hMYC₁₄₄₋₄₅₄ -Tmir [**MESv2-Tmir**] (SEQ ID NO: 171) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGACCATCATCCAGGACTGTATGTGGAGCGGCTTCTCGGCCGCCGCCAAGCTCGTCT
CAGAGAAGCTGGCCTCCTACCAGGCTGCGCGCAAAGACAGCGGCAGCCCGAACCCCGCCCGC
GGCCACAGCGTCTGCTCCACCTCCAGCTTGTACCTGCAGGATCTGAGCGCCGCCGCCTCAGA
GTGCATCGACCCCTCGGTGGTCTTCCCCTACCCTCTCAACGACAGCAGCTCGCCCAAGTCCT
GCGCCTCGCAAGACTCCAGCGCCTTCTCTCCGTCCTCGGATTCTCTGCTCTCCTCGACGGAG
TCCTCCCCGCAGGGCAGCCCCGAGCCCCTGGTGCTCCATGAGGAGACACCGCCCACCACCAG
CAGCGACTCTGAGGAGGAACAAGAAGATGAGGAAGAAATCGATGTTGTTTCTGTGGAAAAGA
GGCAGGCTCCTGGCAAAAGGTCAGAGTCTGGATCACCTTCTGCTGGAGGCCACAGCAAACCT
CCTCACAGCCCACTGGTCCTCAAGAGGTGCCACGTCTCCACACATCAGCACAACTACGCAGC
GCCTCCCTCCACTCGGAAGGACTATCCTGCTGCCAAGAGGGTCAAGTTGGACAGTGTCAGAG
TCCTGAGACAGATCAGCAACAACCGAAAATGCACCAGCCCCAGGTCCTCGGACACCGAGGAG
AATGTCAAGAGGCGAACACACAACGTCTTGGAGCGCCAGAGGAGGAACGAGCTAAAACGGAG
CTTTTTTGCCCTGCGTGACCAGATCCCGGAGTTGGAAAACAATGAAAAGGCCCCCAAGGTAG
TTATCCTTAAAAAAGCCACAGCATACATCCTGTCCGTCCAAGCAGAGGAGCAAAAGCTCATT
TCTGAAGAGGACTTGTTGCGGAAACGACGAGAACAGTTGAAACACAAACTTGAACAGCTACG
GAACTCTTGTGCGTGAatattgccttatttccgattattgccttatttcgcattattgcctt
atttctcactattgccttatttccgatcccggggtttaaaccgatcaacaaaatcactgatg
 
ctggacgatcaacaaaatcactgatgctggacgatcaacaaaatcactgatgctggatcacc
aacaaaatcactgatgctggacgatgtttaaaccctgcaggcgatcagctatgccagcatct
tgcccgatcagctatgccagcatcttgcccgatcagctatgccagcatcttgcctcaccagc
tatgccagcatcttgcccgatcctgcaggagatct
   **Ef1a promoter**
   CS
   *hMYC₁₄₄₋₄₅₄*
   miR124 Target sequences
   miR338-3p Target sequences
   miR31 Target sequences
Ef1a::YR- hMYC₁₄₄₋₄₅₄ -Tmir [MESv3-Tmir] (SEQ ID NO: 172) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGAGGCCCCGGCTGAAAAACGTGGACAGGAGCACTGCACAGCAGTTGG
CAGTAACTGTGGGCAACGTCACCGTCATTATCACAGACTTTAAGGAAAAGACTCGCTCCTCA
TCGACATCCTCATCCACAGTGACCTCCAGTGCAGGGTCAGAACAGCAGAACCAGACCATCAT
CCAGGACTGTATGTGGAGCGGCTTCTCGGCCGCCGCCAAGCTCGTCTCAGAGAAGCTGGCCT
CCTACCAGGCTGCGCGCAAAGACAGCGGCAGCCCGAACCCCGCCCGCGGCCACAGCGTCTGC
TCCACCTCCAGCTTGTACCTGCAGGATCTGAGCGCCGCCGCCTCAGAGTGCATCGACCCCTC
GGTGGTCTTCCCCTACCCTCTCAACGACAGCAGCTCGCCCAAGTCCTGCGCCTCGCAAGACT
CCAGCGCCTTCTCTCCGTCCTCGGATTCTCTGCTCTCCTCGACGGAGTCCTCCCCGCAGGGC
AGCCCCGAGCCCCTGGTGCTCCATGAGGAGACACCGCCCACCACCAGCAGCGACTCTGAGGA
GGAACAAGAAGATGAGGAAGAAATCGATGTTGTTTCTGTGGAAAAGAGGCAGGCTCCTGGCA
AAAGGTCAGAGTCTGGATCACCTTCTGCTGGAGGCCACAGCAAACCTCCTCACAGCCCACTG
 
GTCCTCAAGAGGTGCCACGTCTCCACACATCAGCACAACTACGCAGCGCCTCCCTCCACTCG
GAAGGACTATCCTGCTGCCAAGAGGGTCAAGTTGGACAGTGTCAGAGTCCTGAGACAGATCA
GCAACAACCGAAAATGCACCAGCCCCAGGTCCTCGGACACCGAGGAGAATGTCAAGAGGCGA
ACACACAACGTCTTGGAGCGCCAGAGGAGGAACGAGCTAAAACGGAGCTTTTTTGCCCTGCG
TGACCAGATCCCGGAGTTGGAAAACAATGAAAAGGCCCCCAAGGTAGTTATCCTTAAAAAAG
CCACAGCATACATCCTGTCCGTCCAAGCAGAGGAGCAAAAGCTCATTTCTGAAGAGGACTTG
TTGCGGAAACGACGAGAACAGTTGAAACACAAACTTGAACAGCTACGGAACTCTTGTGCGTG
Aatattgccttatttccgattattgccttatttcgcattattgccttatttctcactattgc
cttatttccgatcccgggtttaaaccgatcaacaaaatcactgatgctggacgatcaacaa
aatcactgatgctggacgatcaacaaaatcactgatgctggatcaccaacaaaatcactgat
gctggacgatgtttaaaccctgcaggcgatcagctatgccagcatcttgcccgatcagctat
gccagcatcttgcccgatcagctatgccagcatcttgcctcaccagctatgccagcatcttg
cccgatcctgcaggagatct
   **Ef1a promoter**
   YR
   *hMYC₁₄₄₋₄₅₄*
   miR124 Target sequences
   miR338-3p Target sequences
   miR31 Target sequences
Ef1a::CS-YR- hMYC₁₄₄₋₄₅₄ -Tmir [**MESv4-Tmir**] (SEQ ID NO: 173) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGCAACAGATTCCTGTGGTGATT
TAATGTTCCTAATGAAATGGAAAGACACAGATGAAGCTGACCTGGTTCTTGCAAAAGAAGCT
 
AATGTGAAATGTCCACAAATTGTGATAGCATTTTATGAAGAGAGACTGACATGGCATGCATA
TCCTGAGaccatgaggccccggctgaaaaacgtggacaggagcactgcacagcagttggcag
taactgtgggcaacgtcaccgtcattatcacagactttaaggaaaagactcgctcctcatcg
acatcctcatccacagtgacctccagtgcaggtcagaacagcagaaccagACCATCATCCA
GGACTGTATGTGGAGCGGCTTCTCGGCCGCCGCCAAGCTCGTCTCAGAGAAGCTGGCCTCCT
ACCAGGCTGCGCGCAAAGACAGCGGCAGCCCGAACCCCGCCCGCGGCCACAGCGTCTGCTCC
ACCTCCAGCTTGTACCTGCAGGATCTGAGCGCCGCCGCCTCAGAGTGCATCGACCCCTCGGT
GGTCTTCCCCTACCCTCTCAACGACAGCAGCTCGCCCAAGTCCTGCGCCTCGCAAGACTCCA
GCGCCTTCTCTCCGTCCTCGGATTCTCTGCTCTCCTCGACGGAGTCCTCCCCGCAGGGCAGC
CCCGAGCCCCTGGTGCTCCATGAGGAGACACCGCCCACCACCAGCAGCGACTCTGAGGAGGA
ACAAGAAGATGAGGAAGAAATCGATGTTGTTTCTGTGGAAAAGAGGCAGGCTCCTGGCAAAA
GGTCAGAGTCTGGATCACCTTCTGCTGGAGGCCACAGCAAACCTCCTCACAGCCCACTGGTC
CTCAAGAGGTGCCACGTCTCCACACATCAGCACAACTACGCAGCGCCTCCCTCCACTCGGAA
GGACTATCCTGCTGCCAAGAGGGTCAAGTTGGACAGTGTCAGAGTCCTGAGACAGATCAGCA
ACAACCGAAAATGCACCAGCCCCAGGTCCTCGGACACCGAGGAGAATGTCAAGAGGCGAACA
CACAACGTCTTGGAGCGCCAGAGGAGGAACGAGCTAAAACGGAGCTTTTTTGCCCTGCGTGA
CCAGATCCCGGAGTTGGAAAACAATGAAAAGGCCCCCAAGGTAGTTATCCTTAAAAAAGCCA
CAGCATACATCCTGTCCGTCCAAGCAGAGGAGCAAAAGCTCATTTCTGAAGAGGACTTGTTG
CGGAAACGACGAGAACAGTTGAAACACAAACTTGAACAGCTACGGAACTCTTGTGCGTGAat
attgccttatttccgattattgccttatttcgcattattgccttatttctcactattgcctt
atttccgatcccgggtttaaaccgatcaacaaaatcactgatgctggacgatcaacaaaat
cactgatgctggacgatcaacaaaatcactgatgctggatcaccaacaaaatcactgatgct
ggacgatgtttaaaccctgcaggcgatcagctatgccagcatcttgcccgatcagctatgcc
agcatcttgcccgatcagctatgccagcatcttgcctcaccagctatgccagcatcttgccc
gatcctgcaggagatct
   **Ef1a promoter**
   CS
   *yr*
   *hMYC₁₄₄₋₄₅₄*
   *V5*
   miR124 Target sequences
   miR338-3p Target sequences
   miR31 Target sequences
Ef1a::KRAB-DNMT3a3L-hMYC₁₄₄₋₄₅₄ -Tmir [**MES-Tmir**] (SEQ ID NO: 174) 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
 
GTTGGAAGTGGGTGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGGATGCTAAGTCACTGACTGCCTGGTCCCGGACACTGGTGACCTTCA
AGGATGTGTTTGTGGACTTCACCAGGGAGGAGTGGAAGCTGCTGGACACTGCTCAGCAGATC
CTGTACAGAAATGTGATGCTGGAGAACTATAAGAACCTGGTTTCCTTGGGTTATCAGCTTAC
TAAGCCAGATGTGATCCTCCGGTTGGAGAAGGGAGAAGAGCCCTGGCTGGTGGAGAGAGAAA
TTCACCAAGAGACCCATCCTGATTCAGAGACTGCATTTGAAATCAAATCATCAGTTGCTAGC
GGCAGCGGCAACCATGATCAAGAGTTCGATCCTCCTAAAGTGTATCCCCCTGTCCCTGCCGA
AAAGCGGAAACCGATCAGGGTTCTCAGTCTCTTCGATGGAATTGCCACAGGCCTCTTGGTCC
TTAAGGACCTGGGCATCCAAGTgGAcCGCTACATCGCCTCAGAAGTATGTGAgGACAGCATC
ACAGTCGGCATGGTGCGCCACCAGGGGAAAATAATGTACGTCGGCGACGTCAGATCAGTTAC
TCAGAAACATATTCAGGAATGGGGCCTTTTGATCTCGTAATTGGCGGCAGCCCCTGCAATG
ATCTGAGTATTGTGAACCCCGCCCGGAAAGGTTTGTACGAAGGTACGGGCAGACTGTTCTTC
GAGTTTTACAGACTGTTGCACGACGCACGACCAAAGGAGGGTGAcGAtcGACCCTTCTTCTG
GCTGTTCGAGAACGTGGTCGCTATGGGCGTGTCTGACAAGCGGGACATTTCtAGATTCCTGG
AGAGCAATCCAGTGATGATTGATGCAAAGGAAGTAtccGCTGCCCACCGCGCCAGATACTTC
TGGGGCAATCTGCCCGGCATGAATCGACCCTTGGCAAGCACCGTGAATGATAAGTTGGAGTT
GCAGGAGTGTCTgGAGCATGGCGGATCGCAAAGTTTAGCAAGGTGCGGACCATCACGACCC
GAAGCAATTCAATTAAGCAGGGAAAGGACCAACATTTTCCAGTGTTTATGAACGAGAAAGAG
GAcATCCTGTGGTGcACAGAAATGGAGCGGGTTTTCGGATTCCCCGTACATTATACTGATGT
GTCCAATATGTCTAGGTTGGCTAGACAGAGGCTGtTGGGACGCTCATGGAGTGTTCCTGTCA
TCCGCCACCTGTTCGCTCCTCTGAAGGAGTATTTCGCcTGCGTtTCATCCGGgAATTCAAAC
GCAAACAGCAGAGGCCCATCCTTTTCTTCCGGCCTGGTgCCACTTAGTCTGCGCGGCTCTCA
CATGGGACCTATGGAAATATACAAAACAGTGAGCGCCTGGAAGAGGCAGCCTGTGCGGGTCC
TGAGTCTGTTCAGAAACATTGACAAGGTGCTTAAGTCCCTTGGATTTCTGGAGTCTGGCAGC
GGTTCTGGGGGTGgcACCCTTAAGTATGTGGAGGACGTGACAAACGTCGTGAGGAGAGATGT
GGAGAAATGGGGGCCATTTGACCTGGTATACGGGAGCACTCAACCTCTCGGTTCTTCTTGTG
ATCGCTGCCCAGGGTGGTATATGTTTCAGTTCCATCGcATCCTGCAATACGCCCTTCCGCGG
CAGGAGAGTCAGCGACCATTCTTCTGGATATTCATGGACAATCTCCTGCTGACAGAgGACGA
CCAAGAGACTACGACTAGATTTCTTCAGACCGAGGCCGTTACTCTCCAGGACGTTAGAGGTA
GGGACTACCAGAATGCCATGCGAGTGTGGAGTAACATCCCCGGACTCAAGTCAAAGCAcGCA
CCCCTGACCCCCAAGGAAGAGGAATACCTcCAGGCGCAGGTGAGGAGCCGCAGTAAGCTCGA
TGCTCCTAAAGTCGATTTGTTGGTGAAGAATTGCTTGCTGCCCCTGAGAGAGTACTTTAAGT
ACTTTAGCCAGAACAGCTTGCCACTGGGCGCGCCCGCCGCTGCCATCATCCAGGACTGTATG
TGGAGCGGCTTCTCGGCCGCCGCCAAGCTCGTCTCAGAGAAGCTGGCCTCCTACCAGGCTGC
GCGCAAAGACAGCGGCAGCCCGAACCCCGCCCGCGGCCACAGCGTCTGCTCCACCTCCAGCT
 
TGTACCTGCAGGATCTGAGCGCCGCCTCAGAGTGCATCGACCCCTCGGTGGTCTTCCCC
TACCCTCTCAACGACAGCAGCTCGCCCAAGTCCTGCGCCTCGCAAGACTCCAGCGCCTTCTC
TCCGTCCTCGGATTCTCTGCTCTCCTCGACGGAGTCCTCCCCGCAGGGCAGCCCCGAGCCCC
TGGTGCTCCATGAGGAGACACCGCCCACCACCAGCAGCGACTCTGAGGAGGAACAAGAAGAT
GAGGAAGAAATCGATGTTGTTTCTGTGGAAAAGAGGCAGGCTCCTGGCAAAAGGTCAGAGTC
TGGATCACCTTCTGCTGGAGGCCACAGCAAACCTCCTCACAGCCCACTGGTCCTCAAGAGGT
GCCACGTCTCCACACATCAGCACAACTACGCAGCGCCTCCCTCCACTCGGAAGGACTATCCT
GCTGCCAAGAGGGTCAAGTTGGACAGTGTCAGAGTCCTGAGACAGATCAGCAACAACCGAAA
ATGCACCAGCCCCAGGTCCTCGGACACCGAGGAGAATGTCAAGAGGCGAACACACAACGTCT
TGGAGCGCCAGAGGAGGAACGAGCTAAAACGGAGCTTTTTTGCCCTGCGTGACCAGATCCCG
GAGTTGGAAAACAATGAAAAGGCCCCCAAGGTAGTTATCCTTAAAAAAGCCACAGCATACAT
CCTGTCCGTCCAAGCAGAGGAGCAAAAGCTCATTTCTGAAGAGGACTTGTTGCGGAAACGAC
GAGAACAGTTGAAACACAAACTTGAACAGCTACGGAACTCTTGTGCGTGAatattgccttat
ttccgattattgccttatttcgcattattgccttatttctcactattgccttatttccgatc
ccggggtttaaaccgatcaacaaaatcactgatgctggacgatcaacaaaatcactgatgct
ggacgatcaacaaaatcactgatgctggatcaccaacaaaatcactgatgctggacgatgtt
taaaccctgcaggcgatcagctatgccagcatcttgcccgatcagctatgccagcatcttgc
ccgatcagctatgccagcatcttgcctcaccagctatgccagcatcttgcccgatcctgcag
gagatct
   **Ef1a promoter**
   KRAB
   **DNMT3a3L**
   *hMYC₁₄₄₋₄₅₄*
   miR124 Target sequences
   miR338-3p Target sequences
   miR31 Target sequences

In one embodiment, the polynucleotide comprises a nucleotide sequence that has at least 90% sequence identity, such as at least 91%, 92%, 93%, 94%, 95%, 96%, 97%, 98%, 99%, or 100% sequence identity to any one of SEQ ID NOs: 164 - 174.

In one embodiment, the polynucleotide comprises a sequence according to any one of SEQ ID NOs: 164 - 174.

In one embodiment, the polynucleotide comprises a nucleotide sequence that has at least 90% sequence identity, such as at least 91%, 92%, 93%, 94%, 95%, 96%, 97%, 98%, 99%, or 100% sequence identity to any one of SEQ ID NOs: 168 - 173.

In one embodiment, the polynucleotide comprises a sequence according to any one of SEQ ID NOs: 168 - 173.

### Exemplary constructs

The polynucleotide may, for example, comprise or consist of a nucleic acid sequence that has at least 70%, 80%, 90%, 95%, 96%, 97%, 98%, 99% or 100% sequence identity to any one of SEQ ID NOs: 114 - 119, 126 - 131, and 136 - 174 or a fragment thereof, preferably wherein the polynucleotide substantially retains the natural function of the polynucleotide of SEQ ID NO: 114 - 119, 126 - 131, or 136 - 174, respectively.

### Target gene transcription and expression

The ESF of the invention may be used in a method that represses transcription and/or expression of at least one target gene. Suitably, the target gene is an endogenous gene.

The at least one target gene transcription and/or expression may be repressed by epigenetic editing.

The level of transcription or expression of a target gene may be decreased by, for example, at least 30%, 40%, 50%, 60%, 70%, 80%, 90%, 95%, 99% or 100% compared to the level of transcription or expression in the absence of the ESF.

The at least one target gene may be silenced. By "silencing a target gene", it is to be understood that transcription and/or expression of the target gene is reduced to an extent sufficient to achieve a desired effect. The reduced expression may be sufficient to achieve a therapeutically relevant effect, such as the prevention or treatment of a disease, such as cancer. For example, a target gene is preferably repressed to an extent that there is either no transcription and/or expression of the target gene, or the residual level of transcription and/or expression of the target gene is sufficiently low to ameliorate or prevent the disease state.

Methods of analysing transcription or expression of a gene are well known in the art. Methods for determining the transcription of a gene are known in the art and include reverse transcription PCR and Northern blot-based approaches. Methods for determining the expression of a gene are known in the art and include Western blot-based or flow cytometry approaches.

Preferably, the repression of the target gene occurs following transient delivery or expression of the ESF of the invention to or in a cell.

By "transient expression", it is to be understood that the expression of the ESF is not stable over a prolonged period of time. Preferably, a polynucleotide encoding the ESF does not integrate into the host genome. More specifically, transient expression may be expression which is substantially lost within 20 weeks following introduction of the polynucleotide encoding the ESF into the cell. Preferably, expression is substantially lost within 12, 6, 4 or 2 weeks following introduction of the polynucleotide encoding the ESF into the cell.

Similarly, by "transient delivery", it is to be understood that the ESF substantially does not remain in the cell (i.e. is substantially lost by the cell) over a prolonged period of time. More specifically, transient delivery may result in the ESF being substantially lost by the cell within 20 weeks following introduction of the ESF into the cell. Preferably, the ESF is substantially lost within 12, 6, 4 or 2 weeks following introduction of the ESF into the cell.

In some embodiments, the ESF is delivered transiently. Transient delivery may result in permanent change

Preferably, the at least one target gene is repressed or silenced permanently. By "permanent repression" or "permanent silencing" of a target gene, it is to be understood that transcription or expression of the target gene is reduced (e.g. reduced by at least 60%, at least 70%, at least 80%, at least 90% or 100%) compared to the level of transcription or expression in the absence of the ESF for at least 2 months, 6 months, 1 year, 2 year or the entire lifetime of the cell/organism. Preferably, a permanently repressed or silenced target gene remains repressed or silenced for the remainder of the cell's life.

In some embodiments, the ESF is stably expressed.

### Proteins

The term "protein" as used herein includes single-chain polypeptide molecules as well as multiple-polypeptide complexes where individual constituent polypeptides are linked by covalent or non-covalent means. The terms "polypeptide" and "peptide" as used herein refer to a polymer in which the monomers are amino acids and are joined together through peptide or disulfide bonds.

### Protein transduction

As an alternative to the delivery of polynucleotides to cells, the ESF of the invention may be delivered to cells by protein transduction.

Protein transduction may be via vector delivery (Cai et al. (2014) Elife 3: e01911; Maetzig et al. (2012) Curr. Gene Ther. 12: 389-409). Vector delivery involves the engineering of viral particles (e.g. lentiviral particles) to comprise the proteins to be delivered to a cell. Accordingly, when the engineered viral particles enter a cell as part of their natural life cycle, the proteins comprised in the particles are carried into the cell.

Protein transduction may be via protein delivery (Gaj et al. (2012) Nat. Methods 9: 805-7). Protein delivery may be achieved, for example, by utilising a vehicle (e.g. nanoparticles, such as liposomes) or even by administering the protein itself directly to a cell.

In some embodiments, the ESF is comprised in a nanoparticle. In some embodiments, the nanoparticle is a polymeric nanoparticle, inorganic nanoparticle or lipid nanoparticle. In some embodiments, the nanoparticle is a liposome.

The nanoparticle may be targeted to a specific cell type(s) (e.g. cancer cells) using one or more ligand displayed on its surface.

### Polynucleotides

Polynucleotides of the invention may comprise DNA or RNA. They may be single-stranded or double-stranded. It will be understood by a skilled person that numerous different polynucleotides can encode the same polypeptide as a result of the degeneracy of the genetic code. In addition, it is to be understood that the skilled person may, using routine techniques, make nucleotide substitutions that do not affect the polypeptide sequence encoded by the polynucleotides of the invention to reflect the codon usage of any particular host organism in which the polypeptides of the invention are to be expressed.

Transgenes and coding sequences of the invention, such as sequences disclosed herein, may also include a stop codon, for example TGA, at the 3' end of the transgene or coding sequence.

The polynucleotides may be modified by any method available in the art. Such modifications may be carried out in order to enhance the *in vivo* activity or lifespan of the polynucleotides of the invention.

Polynucleotides such as DNA polynucleotides may be produced recombinantly, synthetically or by any means available to the skilled person. They may also be cloned by standard techniques.

Longer polynucleotides will generally be produced using recombinant means, for example using polymerase chain reaction (PCR) cloning techniques. This may involve making a pair of primers (e.g. of about 15 to 30 nucleotides) flanking the target sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture with an agarose gel) and recovering the amplified DNA. The primers may be designed to contain suitable restriction enzyme recognition sites so that the amplified DNA can be cloned into a suitable vector.

### Vectors

A vector is a tool that allows or facilitates the transfer of an entity from one environment to another. In accordance with the invention, and by way of example, some vectors used in recombinant nucleic acid techniques allow entities, such as a segment of nucleic acid (e.g. a heterologous DNA segment, such as a heterologous cDNA segment), to be transferred into a target cell. The vector may serve the purpose of maintaining the heterologous nucleic acid (DNA or RNA) within the cell, facilitating the replication of the vector comprising a segment of nucleic acid or facilitating the expression of the protein encoded by a segment of nucleic acid. Vectors may be non-viral or viral. Examples of vectors used in recombinant nucleic acid techniques include, but are not limited to, plasmids, mRNA molecules (e.g. *in vitro* transcribed mRNAs), chromosomes, artificial chromosomes and viruses. The vector may also be, for example, a naked nucleic acid (e.g. DNA). In its simplest form, the vector may itself be a nucleotide of interest.

The vectors used in the invention may be, for example, plasmid, mRNA or virus vectors and may include a promoter for the expression of a polynucleotide and optionally a regulator of the promoter.

Vectors comprising polynucleotides used in the invention may be introduced into cells using a variety of techniques known in the art, such as transfection, transformation and transduction. Several such techniques are known in the art, for example infection with recombinant viral vectors, such as retroviral, lentiviral (e.g. integration-defective lentiviral), adenoviral, adeno-associated viral, baculoviral and herpes simplex viral vectors; direct injection of nucleic acids and biolistic transformation.

Non-viral delivery systems include but are not limited to DNA transfection methods. Here, transfection includes a process using a non-viral vector to deliver a gene to a target cell. Typical transfection methods include electroporation, DNA biolistics, lipid-mediated transfection, compacted DNA-mediated transfection, liposomes, immunoliposomes, lipofectin, cationic agent-mediated transfection, cationic facial amphiphiles (CFAs) (Nat. Biotechnol. (1996) 14: 556) and combinations thereof.

Transfection of cells with mRNA vectors can be achieved, for example, using nanoparticles, such as liposomes.

In some embodiments, the vector (e.g. mRNA vector) is comprised in a nanoparticle. In some embodiments, the nanoparticle is a polymeric nanoparticle, inorganic nanoparticle or lipid nanoparticle. In some embodiments, the nanoparticle is a liposome.

The nanoparticle may be targeted to a specific cell type(s) (e.g. cancer cells) using one or more ligand displayed on its surface.

### Viral vectors

In preferred embodiments, the vector is a viral vector. The viral vector may be in the form of a viral vector particle.

The viral vector may be, for example, a retroviral, lentiviral, adeno-associated viral (AAV) or adenoviral vector.

In some embodiments, the vector is a lentiviral vector. In some embodiments, the vector is an AAV vector. In some embodiments, the vector is an AAV vector particle.

### Retroviral and lentiviral vectors

A retroviral vector may be derived from or may be derivable from any suitable retrovirus. A large number of different retroviruses have been identified. Examples include murine leukaemia virus (MLV), human T-cell leukaemia virus (HTLV), mouse mammary tumour virus (MMTV), Rous sarcoma virus (RSV), Fujinami sarcoma virus (FuSV), Moloney murine leukaemia virus (Mo-MLV), FBR murine osteosarcoma virus (FBR MSV), Moloney murine sarcoma virus (Mo-MSV), Abelson murine leukaemia virus (A-MLV), avian myelocytomatosis virus-29 (MC29) and avian erythroblastosis virus (AEV). A detailed list of retroviruses may be found in Coffin et al. (1997) Retroviruses, Cold Spring Harbour Laboratory Press, 758-63.

Retroviruses may be broadly divided into two categories, "simple" and "complex". Retroviruses may be even further divided into seven groups. Five of these groups represent retroviruses with oncogenic potential. The remaining two groups are the lentiviruses and the spumaviruses. A review of these retroviruses is presented in Coffin et al. (1997) Retroviruses, Cold Spring Harbour Laboratory Press, 758-63.

The basic structure of retrovirus and lentivirus genomes share many common features such as a 5' LTR and a 3' LTR. Between or within these are located a packaging signal to enable the genome to be packaged, a primer binding site, integration sites to enable integration into a host cell genome, and gag, pol and env genes encoding the packaging components - these are polypeptides required for the assembly of viral particles. Lentiviruses have additional features, such as rev and RRE sequences in HIV, which enable the efficient export of RNA transcripts of the integrated provirus from the nucleus to the cytoplasm of an infected target cell.

In the provirus, these genes are flanked at both ends by regions called long terminal repeats (LTRs). The LTRs are responsible for proviral integration and transcription. LTRs also serve as enhancer-promoter sequences and can control the expression of the viral genes.

The LTRs themselves are identical sequences that can be divided into three elements: U3, R and U5. U3 is derived from the sequence unique to the 3' end of the RNA. R is derived from a sequence repeated at both ends of the RNA. U5 is derived from the sequence unique to the 5' end of the RNA. The sizes of the three elements can vary considerably among different retroviruses.

In a defective retroviral vector genome gag, pol and env may be absent or not functional.

In a typical retroviral vector, at least part of one or more protein coding regions essential for replication may be removed from the virus. This makes the viral vector replication-defective.

Lentivirus vectors are part of the larger group of retroviral vectors. A detailed list of lentiviruses may be found in Coffin et al. (1997) Retroviruses, Cold Spring Harbour Laboratory Press, 758-63. Lentiviruses can be divided into primate and non-primate groups. Examples of primate lentiviruses include but are not limited to human immunodeficiency virus (HIV), the causative agent of human acquired immunodeficiency syndrome (AIDS); and simian immunodeficiency virus (SIV). Examples of non-primate lentiviruses include the prototype "slow virus" visna/maedi virus (VMV), as well as the related caprine arthritisencephalitis virus (CAEV), equine infectious anaemia virus (EIAV), and the more recently described feline immunodeficiency virus (FIV) and bovine immunodeficiency virus (BIV).

The lentivirus family differs from retroviruses in that lentiviruses have the capability to infect both dividing and non-dividing cells (Lewis et al. (1992) EMBO J. 11: 3053-8; Lewis et al. (1994) J. Virol. 68: 510-6). In contrast, other retroviruses, such as MLV, are unable to infect non-dividing or slowly dividing cells such as those that make up, for example, muscle, brain, lung and liver tissue.

A lentiviral vector, as used herein, is a vector which comprises at least one component part derivable from a lentivirus. Preferably, that component part is involved in the biological mechanisms by which the vector infects cells, expresses genes or is replicated.

The lentiviral vector may be a "primate" vector. The lentiviral vector may be a "non-primate" vector (i.e. derived from a virus which does not primarily infect primates, especially humans).

Examples of non-primate lentiviruses may be any member of the family of lentiviridae which does not naturally infect a primate.

Preferably, the viral vector used in the present invention has a minimal viral genome.

By "minimal viral genome" it is to be understood that the viral vector has been manipulated so as to remove the non-essential elements and to retain the essential elements in order to provide the required functionality to infect, transduce and deliver a nucleotide sequence of interest to a target host cell. Further details of this strategy can be found in WO 1998/017815.

Preferably, the plasmid vector used to produce the viral genome within a host cell/packaging cell will have sufficient lentiviral genetic information to allow packaging of an RNA genome, in the presence of packaging components, into a viral particle which is capable of infecting a target cell, but is incapable of independent replication to produce infectious viral particles within the final target cell. Preferably, the vector lacks a functional gag-pol and/or env gene and/or other genes essential for replication.

However, the plasmid vector used to produce the viral genome within a host cell/packaging cell will also include transcriptional regulatory control sequences operably linked to the lentiviral genome to direct transcription of the genome in a host cell/packaging cell. These regulatory sequences may be the natural sequences associated with the transcribed viral sequence (i.e. the 5' U3 region), or they may be a heterologous promoter, such as another viral promoter (e.g. the CMV promoter).

The vectors may be self-inactivating (SIN) vectors in which the viral enhancer and promoter sequences have been deleted. SIN vectors can be generated and transduce non-dividing cells *in vivo* with an efficacy similar to that of wild-type vectors. The transcriptional inactivation of the long terminal repeat (LTR) in the SIN provirus should prevent mobilisation by replication-competent virus. This should also enable the regulated expression of genes from internal promoters by eliminating any cis-acting effects of the LTR.

The vectors may be integration-defective. Integration defective lentiviral vectors (IDLVs) can be produced, for example, either by packaging the vector with catalytically inactive integrase (such as an HIV integrase bearing the D64V mutation in the catalytic site) or by modifying or deleting essential att sequences from the vector LTR, or by a combination of the above.

### Adeno-associated viral (AAV) vectors

Adeno-associated virus (AAV) is an attractive vector system for use in the invention as it has a high frequency of integration. Furthermore, AAVs can spread through the brain tissue due to their small size and reduced binding to cell membranes.

AAV has a broad host range for infectivity. Details concerning the generation and use of AAV vectors are described in US Patent No. 5139941 and US Patent No. 4797368.

Recombinant AAV vectors have been used successfully for *in vitro* and *in vivo* transduction of marker genes and genes involved in human diseases.

In some embodiments the vector is an AAV2 vector. In some embodiments the vector is an AAV5 vector.

In some embodiments the vector is an AAV9 vector.

The viral vectors may be modified or mutant viral vectors. Such vectors may be vectors modified to possess certain desirable properties. For example, the AAV2 vector HBKO (or AAV2-HBKO) is a modified AAV2 that is incapable of binding to the heparin sulfate proteoglycan receptor (Naidoo et al. (2018) Mol Ther 26: 2418-2430).

In one embodiment, the vector is a modified AAV2 vector.

In one embodiment, the vector is an AAV2-HBKO vector.

Modified viral vectors may comprise proteins that confer, for example, altered cell tropism, and have been described, for example, in WO2021155137 and WO2015168666.

In one embodiment, the vector is a modified AAV5 vector.

In one embodiment, the vector comprises a capsid with one or more mutations in one or more capsid proteins.

In one embodiment, the vector comprises a capsid with one or more mutations in VP1.

In one embodiment, the vector is an AAV vector, preferably an AAV5 vector, that comprises a capsid protein (e.g. a VP1 protein) comprising: (a) a G at the position corresponding to amino acid 194; (b) an R at the position corresponding to amino acid 474; (c) an R at the position corresponding to amino acid 564; and/or (d) an R at the position corresponding to amino acid 573, wherein the amino acids are numbered with reference to VP1 of AAV5.

### Variants, derivatives, analogues, homologues, and fragments

In addition to the specific proteins and polynucleotides mentioned herein, the invention also encompasses the use of variants, derivatives, analogues, homologues and fragments thereof.

In the context of the invention, a variant of any given sequence is a sequence in which the specific sequence of residues (whether amino acid or nucleic acid residues) has been modified in such a manner that the polypeptide or polynucleotide in question substantially retains at least one of its endogenous functions. A variant sequence can be obtained by addition, deletion, substitution, modification, replacement and/or variation of at least one residue present in the naturally-occurring protein.

The term "derivative" as used herein in relation to proteins or polypeptides of the invention includes any substitution of, variation of, modification of, replacement of, deletion of and/or addition of one (or more) amino acid residues from or to the sequence providing that the resultant protein or polypeptide substantially retains at least one of its endogenous functions.

The term "analogue" as used herein in relation to polypeptides or polynucleotides includes any mimetic, that is, a chemical compound that possesses at least one of the endogenous functions of the polypeptides or polynucleotides which it mimics.

Typically, amino acid substitutions may be made, for example from 1, 2 or 3 to 10 or 20 substitutions provided that the modified sequence substantially retains the required activity or ability. Amino acid substitutions may include the use of non-naturally occurring analogues.

Proteins used in the invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Conservative substitutions may be made, for example according to the table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R H |
| AROMA TIC | | F W Y |

The term "homologue" as used herein means an entity having a certain homology with the wild type amino acid sequence or the wild type nucleotide sequence. The term "homology" can be equated with "identity".

A homologous sequence may include an amino acid sequence which may be at least 50%, 55%, 65%, 75%, 85% or 90% identical, preferably at least 95% or 97% or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

A homologous sequence may include a nucleotide sequence which may be at least 50%, 55%, 65%, 75%, 85% or 90% identical, preferably at least 95% or 97% or 99% identical to the subject sequence. Although homology can also be considered in terms of similarity, in the context of the present invention it is preferred to express homology in terms of sequence identity.

Preferably, reference to a sequence which has a percent identity to any one of the SEQ ID NOs disclosed herein refers to a sequence which has the stated percent identity over the entire length of the SEQ ID NO referred to.

Homology comparisons can be conducted by eye or, more usually, with the aid of readily available sequence comparison programs. These commercially available computer programs can calculate percentage homology or identity between two or more sequences.

Percentage homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the nucleotide sequence may cause the following codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum percentage homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al. (1984) Nucleic Acids Res. 12: 387). Examples of other software that can perform sequence comparisons include, but are not limited to, the BLAST package, FASTA (Atschul et al. (1990) J. Mol. Biol. 403-410) and the GENEWORKS suite of comparison tools. Both BLAST and FASTA are available for offline and online searching. However, for some applications, it is preferred to use the GCG Bestfit program. Another tool, called BLAST 2 Sequences is also available for comparing protein and nucleotide sequences (see FEMS Microbiol. Lett. (1999) 174: 247-50; FEMS Microbiol. Lett. (1999) 177: 187-8).

Although the final percentage homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see the user manual for further details). For some applications, it is preferred to use the public default values for the GCG package, or in the case of other software, the default matrix, such as BLOSUM62.

Once the software has produced an optimal alignment, it is possible to calculate percentage homology, preferably percentage sequence identity. The software typically does this as part of the sequence comparison and generates a numerical result.

"Fragments" are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants may be prepared using standard recombinant DNA techniques such as sitedirected mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

### Codon optimisation

The polynucleotides used in the invention may be codon-optimised. Codon optimisation has previously been described in WO 1999/41397 and WO 2001/79518. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available.

### Compositions

The polynucleotides, proteins, vectors, nanoparticles and cells of the invention may be formulated for administration to subjects with a pharmaceutically-acceptable carrier, diluent or excipient. Suitable carriers and diluents include isotonic saline solutions, for example phosphate-buffered saline, and potentially contain human serum albumin.

Materials used to formulate a pharmaceutical composition should be non-toxic and should not interfere with the efficacy of the active ingredient. The precise nature of the carrier or other material may be determined by the skilled person according to the route of administration.

The pharmaceutical composition is typically in liquid form. Liquid pharmaceutical compositions generally include a liquid carrier such as water, petroleum, animal or vegetable oils, mineral oil or synthetic oil. Physiological saline solution, magnesium chloride, dextrose or other saccharide solution, or glycols such as ethylene glycol, propylene glycol or polyethylene glycol may be included. In some cases, a surfactant, such as pluronic acid (PF68) 0.001% may be used. In some cases, serum albumin may be used in the composition.

For injection, the active ingredient may be in the form of an aqueous solution which is pyrogen-free, and has suitable pH, isotonicity and stability. The skilled person is well able to prepare suitable solutions using, for example, isotonic vehicles such as Sodium Chloride Injection, Ringer's Injection or Lactated Ringer's Injection. Preservatives, stabilisers, buffers, antioxidants and/or other additives may be included as required.

For delayed release, the medicament may be included in a pharmaceutical composition which is formulated for slow release, such as in microcapsules formed from biocompatible polymers or in liposomal carrier systems according to methods known in the art.

Handling of the cell therapy products is preferably performed in compliance with FACT-JACIE International Standards for cellular therapy.

### Methods of treatment

In one aspect, the invention provides the polynucleotide, vector, protein, nanoparticle, cell, composition or pharmaceutical composition of the invention for use in therapy.

In another aspect, the invention provides the ESF, polynucleotide, vector, cell or composition of the invention for use in the treatment of cancer.

In some embodiments, the cancer is glioma, gliobastoma, medulloblastoma, astrocytoma, neuroblastomas, ependymoma, meningioma, retinoblastoma, rhabdomyosarcoma, lung cancer, prostate cancer, breast cancer, liver cancer, pancreatic cancer, bladder cancer, oropharyngeal cancer or kidney cancer. In some embodiments, the cancer is a brain tumour. In some embodiments, the cancer is gliobastoma multiforme.

In some embodiments, the treatment reduces tumour size.

In some embodiments, the treatment is as an adjuvant therapy, optionally in combination with surgery.

All references herein to treatment include curative, palliative and prophylactic treatment. The treatment of mammals, particularly humans, is preferred. Both human and veterinary treatments are within the scope of the invention.

In some embodiments, the method of treatment provides the polynucleotide, vector, ESF, protein, nanoparticle, cell, composition or pharmaceutical composition of the invention to a tumor.

In some embodiments, the method of treatment provides the polynucleotide, vector, ESF, protein, nanoparticle, cell, composition or pharmaceutical composition of the invention to the brain of a subject.

### Administration

In some embodiments, the polynucleotide, vector, ESF, protein, nanoparticle, cell, composition or pharmaceutical composition is administered to a subject locally.

In some embodiments, the polynucleotide, vector, ESF, protein, nanoparticle, cell, composition or pharmaceutical composition is administered to a subject's brain.

In preferred embodiments, the polynucleotide, vector, ESF, protein, nanoparticle, cell, composition or pharmaceutical composition is administered to a tumor.

In some embodiments, the polynucleotide, vector, ESF, protein, nanoparticle, cell, composition or pharmaceutical composition is administered to a subject systemically, for example intravenously.

In some embodiments, the polynucleotide, vector, ESF, protein, nanoparticle, cell, composition or pharmaceutical composition is administered to a subject locally.

The term "systemic delivery" or "systemic administration" as used herein means that the agent of the invention is administered into the circulatory system, for example to achieve broad distribution of the agent. In contrast, topical or local administration restricts the delivery of the agent to a localised area, e.g. a tumor.

### Dosage

The skilled person can readily determine an appropriate dose of an agent of the invention to administer to a subject. Typically, a physician will determine the actual dosage that will be most suitable for an individual patient, which will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. There can of course be individual instances where higher or lower dosage ranges are merited, and such are within the scope of the invention.

### Subject

The term "subject" as used herein refers to either a human or non-human animal.

Examples of non-human animals include vertebrates, for example mammals, such as non-human primates (particularly higher primates), dogs, rodents (e.g. mice, rats or guinea pigs), pigs and cats. The non-human animal may be a companion animal.

Preferably, the subject is a human.

The skilled person will understand that they can combine all features of the invention disclosed herein without departing from the scope of the invention as disclosed.

Preferred features and embodiments of the invention will now be described by way of nonlimiting examples.

The practice of the present invention will employ, unless otherwise indicated, conventional techniques of chemistry, biochemistry, molecular biology, microbiology and immunology, which are within the capabilities of a person of ordinary skill in the art. Such techniques are explained in the literature. See, for example, Sambrook, J., Fritsch, E.F. and Maniatis, T. (1989) Molecular Cloning: A Laboratory Manual, 2nd Edition, Cold Spring Harbor Laboratory Press; Ausubel, F.M. et al. (1995 and periodic supplements) Current Protocols in Molecular Biology, Ch. 9, 13 and 16, John Wiley & Sons; Roe, B., Crabtree, J. and Kahn, A. (1996) DNA Isolation and Sequencing: Essential Techniques, John Wiley & Sons; Polak, J.M. and McGee, J.O'D. (1990) In Situ Hybridization: Principles and Practice, Oxford University Press; Gait, M.J. (1984) Oligonucleotide Synthesis: A Practical Approach, IRL Press; and Lilley, D.M. and Dahlberg, J.E. (1992) Methods in Enzymology: DNA Structures Part A: Synthesis and Physical Analysis of DNA, Academic Press. Each of these general texts is herein incorporated by reference.

All publications mentioned in the above specification are herein incorporated by reference. Various modifications and variations of the disclosed polypeptides, polynucleotides, vectors, cells, compositions, uses and methods of the invention will be apparent to the skilled person without departing from the scope and spirit of the invention. Although the invention has been disclosed in connection with specific preferred embodiments, it should be understood that the invention as claimed should not be unduly limited to such specific embodiments. Indeed, various modifications of the disclosed modes for carrying out the invention, which are obvious to the skilled person are intended to be within the scope of the following claims.

### EXAMPLES

### EXAMPLE A

### RESULTS

We sought to build a set of epigenetic silencer factors by adding the Kruppel-associated box (KRAB) domain and/or the catalytic domain of DNA methyltransferase DNMT3A together with its cofactor DNMT3L to the full length Sox2 or Sox2 lacking its C-terminal transcriptional activation domain (Fig. 1a).

The KRAB domain (from the zinc finger protein ZNF10) recruits different epigenetic complexes that are able to both induce repressive chromatin modification (e.g. H3K9me3) and remove active marks (e.g. H3K4ac), while the DNMT domains coordinate *de novo* DNA methylation, thus preventing altogether genic transcription (Amabile et al. (2016) Cell 167: 219-232.e14). Only the factor composed of the N-terminal and DNA binding regions of SOX2 plus the KRAB and the DNMT domains, respectively at the 5' and 3' terminals (referred to as SES), rapidly killed glioma cell lines (e.g. U87, U251 and SNB19) *in vitro* (Fig. 1b-f). Accordingly, genes associated with cell proliferation and *bona fide* SOX2 targets (CDK1, CDC6, *CCND1,* etc.) were strongly repressed in SES-treated cancer cells (Fig. 1g). Thus, removal of the SOX2 transcriptional activation domain was critical to obtain a synthetic factor capable of repressing cancer cell proliferation. Interestingly, proliferation of cell lines from prostatic, liver and pancreatic cancers were largely unaffected by SES treatment suggesting a cancer-specific SES efficacy (Fig. 1h). We extended the *in vitro* analysis to patient-derived glioblastoma multiforme cancer stem cells (GBM CSCs) of both classical and mesenchymal subtypes that better preserve primary tumor features. Both lines of GBM CSCs displayed a strong proliferative loss and high cell death after SES lentiviral (LV) transduction (Fig. 2a). To determine SES specificity, we mutated two residues in the HMG-box domain (arginine at position 74 and leucine at position 97 were replaced by two prolines) that have been described as important for Sox2 binding the DNA (Fig. 1e). Expression of SES(R74P/L97P) failed to arrest CSC growth, indicating that SES activity relies on its DNA binding activity (Fig. 2a). Additionally, clonogenic analysis showed that SES-treated CSCs exhibited a diminished self-renewal capacity with impairment in forming tumor spheres and sustaining their growth (Fig. 2b).

Next, we set out to assess global SES transcriptional output and its genome-wide occupancy. SES-treated GBM CSCs exhibited massive transcriptional changes with general upregulation of apoptosis-related genes and silencing of genes encoding for proliferative and cancer-promoting factors in at least two different glioma cell lines (Fig. 3a-c). Interestingly, using a list of putative SOX2 targets inferred with computational methods (Janky (2014) et al. PLoS Comput Biol. 10: e1003731), we discovered that they were largely downregulated in SES-expressing cells (Fig. 3d). Comparative ChIP-seq experiments to determine SOX2 and SES genome-wide binding on CSCs revealed that SES maintained the ability to bind to the majority of the SOX2 genomic sites (Fig. 3e). Given that SES includes the catalytic domains of the DNMT3A/L *de novo* DNA methyltransferases, we used MeDIP-seq to profile the methylated DNA. Notably, SOX2-bound regions presented a significant increase in the methylation state in SES transduced cells (Fig. 2f).

We then asked whether SES expression in vivo could exert any anti-tumoral activity. Initially, we performed subcutaneous xenografts in NSG immunodeficient mice using CSCs previously transduced with lentiviruses expressing either GFP (mock) or SES. Importantly, heterotopic transplants grew only from grafted mock cells, while SES transduced cells were unable to sustain tumor growth (Fig. 4a-c), suggesting that this factor is toxic for glioma cells and prevents tumor formation. No SES⁺ cells were ever identified even when some scar-like epithelial mass could be isolated within the injection site (Fig. 4c). Similar results were obtained inducing subcutaneous tumoral growth with glioma cell lines (U87, SNB19, U251) (Fig. 4d). Next, orthotopic intracranial xenografts were carried out with GFP or SES expressing U87 cancer cells. 4 weeks after brain transplantation, mock GFP⁺ grafted cells generated large tumoral masses extending throughout the striatum up to the cerebral cortex that were rich in proliferating cells (Fig. 5a-e). Accordingly, all the mice transplanted with mock U87 cells succumbed within 30 days from the grafting (Fig. 5f). Conversely, grafted SES⁺ U87 cells completely lost their tumor-initiating capacity and no tumoral masses could be retrieved from the transplanted mice that exhibited an unaltered survival curve (Fig. 5b-g). Similar results were obtained with SES-treated GBM CSCs that were able to produce very small tumoral masses 6 weeks after brain grafting (Fig. 5h). To test GBM CSC tumorigenicity in a full humanized model system, we developed brain organoids by 3D differentiation of human iPSCs (Lancaster et al. (2013) Nature 501: 373-9) and assembled them together with GBM CSC-derived spheroids using Matrigel embedding (Linkous et al. (2019) Cell Rep. 26: 3203-3211.e5; Goranci-Buzhala et al. (2020) Cell Rep. 31: 107738). In these assembloids (GBM-cortex organoids), mock GFP⁺ CSCs succeed over time to infiltrate and diffuse within the brain cortical-like tissue (Fig. 6a). By contrast, in SES-treated organoids the GBM part remained very small failing to grow over time and delaminating cells in the organoid territory (Fig. 6b). These results strongly imply that SES expression in GBM cancer cells restrains their growth and survival erasing their *in vivo* tumor-initiating capacity. Following these findings that validate the SES anti-tumor activity based on *in vitro* transduction of SES in cancer cells before grafting, we moved to an approach of SES viral transduction *in vivo* to curb the development of an already growing GBM mass in the brain parenchyma. Intracranial transplanted U87 cancer cells were left to proliferate and form a tumoral mass for 4 days before injecting a mock (GFP) or SES-expressing lentivirus (Fig. 7a,b). 4 weeks after lentiviral gene transfer mock transduced tumors developed large masses diffused throughout the striatum (Fig. 7b,c). By contrast, in situ SES expression was sufficient to strongly reduce the tumor mass and significantly extend the survival rate of the affected animals (Fig. 7b-d). Notably, V5 immunohistochemistry in 4 weeks transplanted brains showed that SES+ cells were not present in the tumor mass but only in the surrounding brain parenchymal tissue (Fig. 7e). This finding indicates that cancer cells transduced with the SES lentivirus were lost over time and that the remaining tumor tissue at 4 weeks from transplantation was composed exclusively by SES⁻ cells.

Then, we challenged our approach by treating tumors generated from patient-derived CSCs. Either a mock (GFP) or SES expressing lentivirus were injected *in situ* (7 days after the transplant of the CSCs) and the tumor growth was followed by weekly MRI T1 scanning up to the histological analysis after 6 weeks (Fig. 7f). SES-treated tumors are smaller compared to the mock treated group both at the MRI screening and at the endpoint (Fig. 7g-j). These data suggested that *in situ* viral treatment with SES is able to reduce the mass of patient derived GBM in mice.

SOX2 is a pivotal factor in stem cells and neural progenitors, but it is strongly downregulated during neuronal differentiation. Thus, SES should have a minor impact on mature brain neurons in adulthood. To determine the effect of SES on brain cells, primary mouse cortical neuronal cultures were treated with the SES LV and survival, morphology and gene expression were assessed two weeks later. Mock and SES expressing neurons displayed similar morphology with no sign of cell sufferance and with comparable low numbers of PI⁺ dying cells (Fig. 8a). Notably, transcriptomes of mock and SES transduced cultures were substantially comparable with only Sox2 and 15 other genes differentially expressed between the two neuronal populations (Fig. 8b). Similar results were collected using human iPSC-derived neuronal cultures where SES treatment did not alter survival and morphology of MAP2⁺ neurons (Fig. 8c,d). This analysis could last no more than 2 weeks given the short survival time of primary neurons in vitro. Thus, we moved in vivo and transduced SES in the hippocampus of C57BL/6 adult mice and assessed behavioral performance at long term (Fig. 9a,b). 4 weeks after stereotactic injections of mock (GFP) and SES LVs both mock and SES viral vectors were equally present in the hippocampi post-mortem (Fig. 9c). Similar results were obtained with the evaluation of transgenes' mRNA (Fig. 9c). Of note, the same level of cell death was observed followed both GFP and SES injection (Fig. 9d). Before sacrifice, mice were evaluated in the spontaneous alternation, Radial maze and Morris water maze tests to assess their exploratory behavior and cognitive function related to spatial learning and memory (Fig. 9e-g). Both groups of animals performed equally well in these tasks suggesting that SES expression did not elicit significant functional alterations in hippocampal neurons.

We then conceived a strategy to restrict SES expression to cancer cells after brain viral inoculation. We isolated a KI67 promoter (Zambon (2010) Cytometry A 77: 564-70) that was inserted in the LV cassette (SES v1.1) to drive SES expression exclusively in proliferative cells (Fig. 10a). Firstly, we confirmed that our strategy did not affect either GFP (when it is placed downstream of the KI67 promoter) or SES expression levels in cancer cells and, in the case of SES, triggered a significant cell loss in the transduced cancer cells *in vitro* (Fig. 10b). Next, mouse primary neuronal cultures constituted by both neurons and glial cells, were transfected with either EF1a-GFP (constitutive) or pKI67-GFP LVs and investigated for the presence of fluorescent protein 1 week later. Interestingly, the great majority of neurons (MAP2⁺) infected with pKI67-GFP were found to be GFP⁻ indicating that its transcription was strongly reduced to undetectable levels, while constitutive GFP was expressed in all the cell of the dish (Fig. 10c). Curiously, the few GFP⁺ cells in the pKI67-GFP transduced cultures were also Ki67⁺ and, thus, in active cell-cycle that likely correspond to young proliferative astrocytes (red arrows in Fig. 10c). Thus, this strategy resulted in effective silencing of SES expression in brain post mitotic cells without compromising the SES transgene activation in cancer cells.

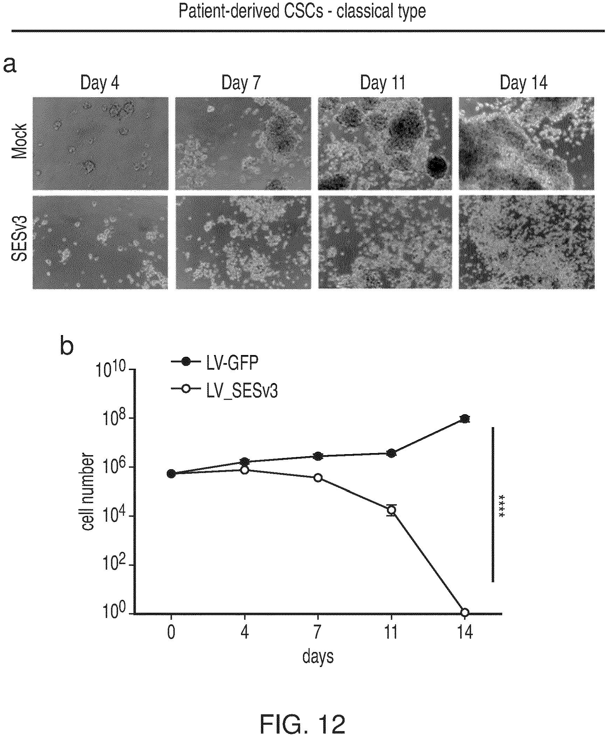

Then we generated additional variants of SES using alternative repressor domains such as the chromo shadow domain from CBX5 protein (inserted at the 5') (SESv2) or the YAF2-RYBP domain from RYBP protein (inserted at the 3') (SESv3) that replaced the KRAB and DNMT3A/L catalytic domains (Fig. 11a). Cancer cells transduced with either SESv2 or v3 showed a rapid proliferative loss and diffuse cell death which caused a premature termination of the cultures within two weeks from the initial treatment (Fig. 11b-c). Patient-derived GBM CSCs of classical subtype, that better preserve primary tumor features, also displayed a strong proliferative loss after SESv3 lentiviral (LV) transduction (Fig. 12a-b).

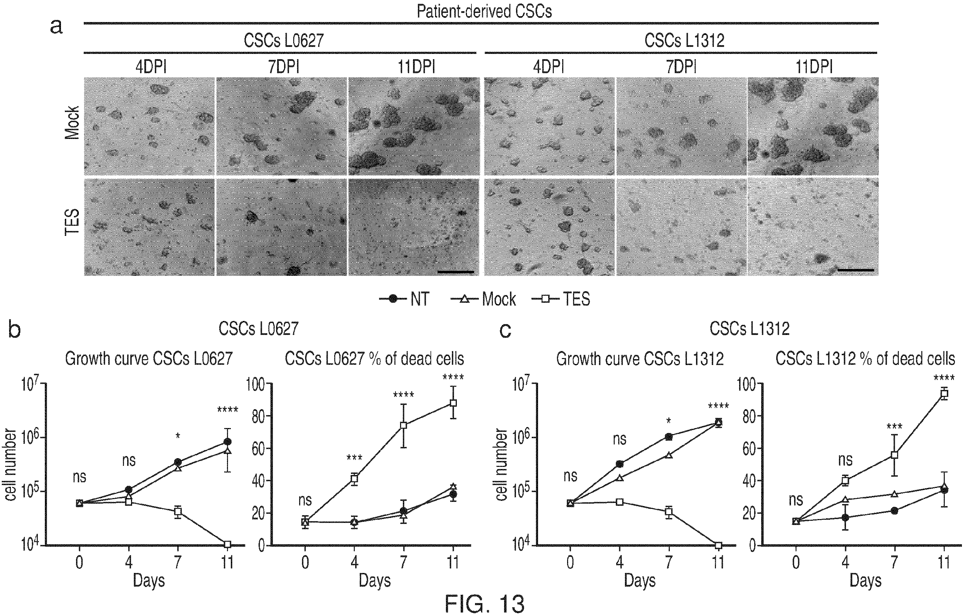

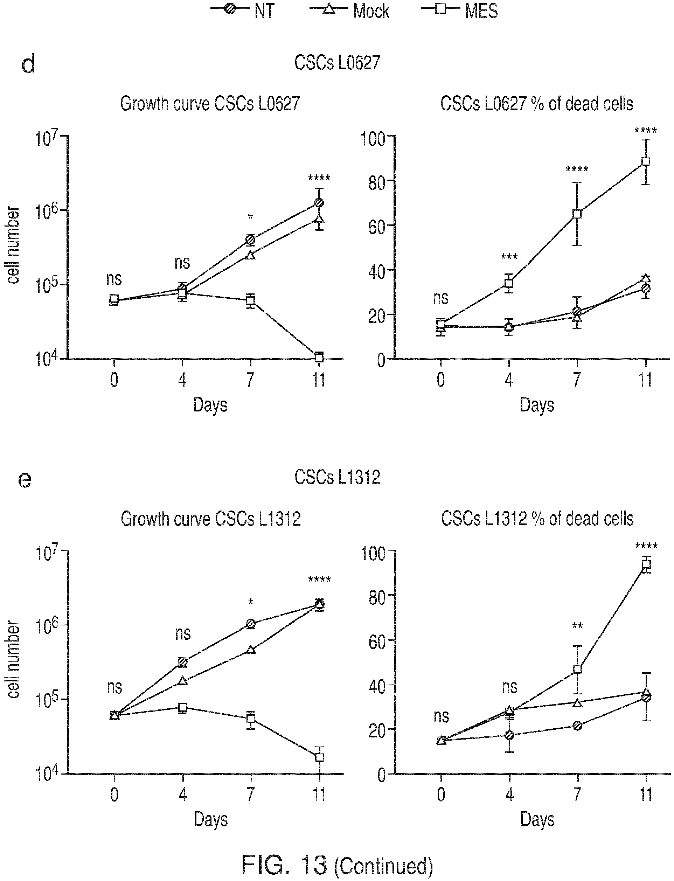

These results obtained with SES indicate that reconfiguration of activatory TFs into epigenetic silencer factors (ESFs) can be generalized to build more epigenetic regulators by engineering other activatory cancer associated TFs. With this goal, we applied the same rational design to TEAD1 and c-MYC, two other transcriptional activators with crucial oncogenic activity, thereby, generating the TES and MES factors, respectively (Fig. 11d). As for SES, ESFs were generated by removing the transcriptional activation domain and adding the KRAB and DNMT3A/L catalytic domain to the remaining parts of the two TFs (Fig. 11d). In case of c-MYC the repressor domains were inserted at the 5' to avoid spatial hindrance at the 3'-end which directly interacts with MAX for the formation of heterodimers (Fig. 11d). Cancer cells transduced with either TES or MES showed a rapid proliferative loss and diffuse cell death which caused a premature termination of the cultures within two weeks from the initial treatment (Fig. 11e-f and Fig. 13).

We then assessed TES/MES repression of tumor development in vivo. Both TES and MES pre-infected CSCs displayed decreased tumorigenic potential when xenotransplanted subcutaneously in NSG mice (Fig. 14). TES/MES pre-infected CSCs also showed limited tumor growth in NSG brain (orthotopic transplantation) compared to mock infected CSCs (Fig. 15). Notably, in situ TES expression was sufficient to strongly reduce the growth of a tumor mass formed by transplantation of naïve CSCs a week before the injection of the virus (Fig. 16).

Thus, the ESF design can be applied to different oncogenic TFs generating a family of synthetic factors with strong anti-tumor activity. ESFs represent a new class of rationally designed factors with pervasive and long-lasting epigenetic functions that can remodel entire transcriptional pathways with precision and efficacy. Targeted ESF expression can suppress cancer development and represent new gene-based therapeutics for glioblastoma and other cancers.

### DISCUSSION

Activity of developmental TFs is mainly restricted during morphogenesis executing a prominent role in stem cell identity, cell lineage commitment and differentiation. However, these TFs can be re-activated or hijacked by the cancer genetic program to propel tumor development and progression. It is estimated that about 20% of all the known oncogenic proteins are represented by TFs with critical importance for acquiring malignant cell dedifferentiation, proliferation, and migration. Despite their pervasive role in tumors, interfering with their functions in a translational perspective has proven challenging. In fact, stable and complete gene silencing by various genetic tools or small molecules have been difficult to achieve in cancer cells. Moreover, the cancer genetic program has been repeatedly shown to overcome the inactivation of single genes by reconfiguring the transcriptional network to promote cancer resistance and recurrence. Herein, we designed an epigenetic repressor (SES) by reconfiguring SOX2 TF by rational assembling of transcriptional and epigenetic negative regulators of gene transcription. This design is modular and versatile and can be in principle applied to other activatory oncogenic TFs as we showed for TEAD1 and c-MYC. Importantly, SES-dependent *de novo* DNA methylation in SOX2 target genes triggered by DNMTA/L catalytic domains promotes the widespread and stable silencing of the SOX2 downstream network. These wide transcriptional changes inhibited cell proliferation in cancer cells that failed to cope with these alterations, eventually dying. Here, we show that these domains can reconfigure the transcriptional activity of endogenous TFs while preserving their chromatin occupancy and target selectivity. We also showed that different configuration of SES, by means of using different epigenetically active protein domains (e.g. Chromo Shadow domain from CBX5 and YAF2-RYBP domain from RYBP), can elicit the same functional activity.

SOX2 expression is fundamental to control self-renewal and a malignant phenotype in GBM cancer cells as well as in many stem cells including pluripotent and neural types. Importantly, SOX2 has a primary role in promoting tumor development in many other malignancies other than GBM including medulloblastoma and lung, prostate, breast cancers.

Thus, the use of SES or other ESFs can be expanded for the treatment of other cancers. Tumor targeting of ESFs by viral-mediated delivery can be in principle effective on cancers confined into those solid tissues which can be efficiently targeted by viral transduction *in vivo.* On this view, cancers in liver, lung, breast and kidney represent plausible targets for this approach since delivery routes and viral strains are known to obtain wide and high tissue transduction efficiency. Likewise, the same approach can be proposed to treat metastatic masses in the same organs.

Herein, SES was directly injected in the tumoral mass repressing its development. Similar approaches can be useful in a clinical setting to treat glioblastoma whose surgical resection is impractical due to unattainable locations within the brain or the close proximity to vital brain regions. Moreover, SES can be delivered in the brain parenchyma surrounding the resected primary tumor as adjuvant therapy in order to target the remaining cancer cells and restrain subsequent tumor recurrence.

Herein, glioblastoma treatment with ESFs was carried out through lentiviral transduction by local injections into the affected tissue. However, alternative therapeutic viruses could be similarly employed as, in particular, strains of adeno-associated viruses (AAVs) that can spread through the brain tissue due to their small size and reduced binding to cell membranes. Maximizing viral spreading in the brain parenchyma will increase the targeting efficiency of cancer cells scattered in the tissue providing a better protection from tumor recurrence. Moreover, non-viral vehicles such as nanoparticles or liposomes can be used to deliver SES mRNA or protein to obtain an acute transgene expression which can be still sufficient to inhibit cancer cells while strongly enhancing the overall safety profile of the procedure.

We showed that SES expression is not harmful to neuronal cultures and in murine brain, and we further elaborated a strategy to restrict its activation only to proliferative cells, strongly enriched in cancers and rarely present in brain parenchyma. The system described here proved effective in expressing the viral transgene mainly in cancer cells, but not in brain post-mitotic cells.

Altogether, we assembled an epigenetic repressor which operates as a dominant negative version of the oncogenic SOX2 TF and is able to bind and stably repress the SOX2 transcriptional network. Targeted viral delivery of SES in glioblastoma is sufficient to inhibit tumor development by blocking cell proliferation and inducing cell death. Given its wide applicability to other oncogenic TFs and the high efficiency of targeting cancer cells by viral transduction, this approach provides the opportunity to repress glioblastoma and other deadly cancers.

### MATERIAL AND METHODS

### Constructs

KRAB-hSOX2: the full length human *SOX2* gene was fused with the KRAB repressor domain (from the gene *ZNF10* encoding for a Zinc finger protein, aa 1-97) at its N-terminus while a V5 tag was fused at the C-terminus of the SOX2. The transgene was used in a lentiviral construct with Ef1a as a promoter.

KRAB-hSOX2-D3A&L: the full length human SOX2 gene was fused with the KRAB repressor domain (from the gene *ZNF10* encoding for a Zinc finger protein, aa 1-97) at its N-terminus while the functional domains of DNMT3A (aa 388-689) and 3L (aa 206-421) were fused at the C-terminus of the SOX2, a V5 tag was fused at the end of the last domain, at the C-terminus of the new chimeric transgene. The transgene was used in a lentiviral construct with Ef1a as a promoter.

KRAB-hSOX2₁₋₁₇₉: the initial part of *SOX2* gene, coding for aa 1-179 (thus excluding the SOX2 activator domain) was fused with the KRAB repressor domain (from the gene *ZNF10* encoding for a Zinc finger protein, aa 1-97) at its N-terminus while a V5 tag was fused at the C-terminus of the SOX2. The transgene was used in a lentiviral construct with Ef1a as a promoter.

hSOX2₁₋₁₇₉-D3A&L: the initial part of SOX2 gene, coding for the aa 1-179 (thus excluding the SOX2 activator domain) was fused with the functional domains of DNMT3A (aa 388-689) and 3L (aa 206-421), with a V5 tag fused at the C-terminus of the SOX2. The transgene was used in a lentiviral construct with Ef1a as promoter.

SES v1: the initial part of *SOX2* gene, coding for aa 1-179 (thus excluding the SOX2 activator domain) was fused with the KRAB repressor domain (from the gene *ZNF10* encoding for a Zinc finger protein, aa 1-97) at its N-terminus while the functional domains of DNMT3A (aa 388-689) and 3L (aa 206-421) were fused at the C-terminus of the SOX2 portion, a V5 tag was fused at the end of the last domain, at the C-terminus of the new chimeric transgene. The transgene was used in a lentiviral construct with Ef1a as a promoter.

SES (R74P/L97P): the SESv1 was mutagenized at residues 74 and 97 of the initial part of SOX2 gene (arginine at position 74 and leucine at position 97 to prolines).

SES v1.1: The transgene was the same as the SES version 1, however the Ef1a promoter was replaced with the proximal promoter of the murine *Mki67* gene (-1263 to -1 related to *Mki67* atg).

SES v2: the initial part of *SOX2* gene, coding for aa 1-179 (thus excluding the SOX2 activator domain) was fused with the Chromo Shadow (CS) repressor domain (from the gene *CBX5,* aa 121-179) at its N-terminus while a V5 tag was fused at the C-terminus of the SOX2.

SES v3: the initial part of *SOX2* gene, coding for aa 1-179 (thus excluding the SOX2 activator domain) was fused with the YAF2-RYBP (Y-R) repressor domain (from the gene *RYBP,* aa 145-189) at its C-terminus, and a V5 tag was fused at the C-terminus of the Y-R.

TES: the initial part of *TEAD1* gene, coding for the aa 1-166 (thus excluding the TEAD1 activator domain) was fused with the KRAB repressor domain (from the gene *ZNF10* encoding for a Zinc finger protein, aa 1-97) at its N-terminus while the functional domains of DNMT3A (aa 388-689) and 3L (aa 206-421) were fused at the C-terminus of the TEAD1 portion, a V5 tag was fused at the end of the last domain, at the C-terminus of the new chimeric transgene. The transgene was used in a lentiviral construct with Ef1a as a promoter.

MES: the C-terminal part of *MYC* gene, coding for the aa 144-454 (thus excluding the MYC activator domain) was fused with the KRAB repressor domain (from the gene *ZNF10* encoding for a Zinc finger protein, aa 1-97) and DNMT3A (aa 388-689) and 3L (aa 206-421) in tandem at the N-terminus of the MYC portion while a V5 tag was fused at the C-terminus. The transgene was used in a lentiviral construct with Ef1a as a promoter.

### Lentivirus production

Replication-incompetent, VSVg-coated lentiviral particles were packaged in 293 T cells. Cells were transfected with 30 µg of vector and packaging constructs, according to a conventional CaCl₂ transfection protocol. After 30 hrs, medium was collected, filtered through 0.44 µm cellulose acetate and centrifuged at 20000 rpm for 2 hrs at 20°C in order to concentrate the virus.

### Cell culture

U-87, U-251 and SNB-19 (human glioblastoma cell lines), HeLa (human cervix cancer cell line), DU145 (human prostatic cancer cell line) and HepG2 (human liver carcinoma cell line) were cultured in plastic-adherence conditions in DMEM medium (Dulbecco's Modified Eagle's Medium - high glucose, Sigma-Aldrich) containing 10% fetal bovine serum (FBS, Sigma-Aldrich), 1% Pen/Strept (Sigma-Aldrich), 2 mM Glutamine (Sigma-Aldrich), 1% non-essential amino acids (MEM NEAA, ThermoFisher Scientific), 1% sodium pyruvate solution (Sigma-Aldrich) and passaged twice a week using Trypsin-EDTA solution (Sigma-Aldrich).

BxPC3 (human pancreatic cancer cell line) were cultured in plastic-adherence conditions in RPMI-1640 (Sigma-Aldrich) containing 10% FBS, 1% Pen/Strept, 2 mM Glutamine. All the cell lines were passaged twice a week using Trypsin-EDTA solution (Sigma-Aldrich).

Cancer stem cells (CSCs) from classical (L0627) and mesenchymal (1312) glioblastoma tumors were maintained in spheres in suspension cultures in DMEM/F12 (Sigma-Aldrich) supplemented with Hormon Mix (DMEM/F12, 0.6% Glucose (Sigma-Aldrich) (30% in phosphate buffer (PBS) (Euroclone)), Insulin (Sigma-Aldrich) 250 µg/ml, putrescine powder (Sigma-Aldrich) 97 µg/ml, apotransferrin powder (Sigma Aldrich), sodium selenite 0.3 µM, progesterone 0.2 µM), 1% Pen/Strept, 2 mM Glutamine, 0.66% Glucose (30% in phosphate buffer salt (PBS) (Euroclone)), and heparin (4 mg/ml, Sigma-Aldrich); bFGF (20 ng/ml, ThermoFisher Scientific) and EGF (20 ng/ml, ThermoFisher Scientific) were added freshly to culture medium. Sphere cultures were passaged once a week by mechanical dissociation of the sphere to single cell suspension.

All the cultures were kept in humidified atmosphere of 5% CO₂ at 37°C under atmospheric oxygen conditions.

### Growth curve analysis

5×10^5 of cancer cell lines were seeded in adherent condition in a 6 multi-well plate at day 0; at day 1 cultures were infected with lentiviral vectors and at day 3, cells were detached, live cells were stained with Trypan Blue Solution (0.4%, ThermoFisher Scientific) and counted using Countess^{™} II Automated Cell Counter (ThermoFisher Scientific); after this passage, 3×10^5 cells were seeded again. This was repeated for 3 time points every 3-4 days; the experiment was repeated 3 times for each time point. Brightfield representative pictures were taken at each time-point.

25×10^4 CSCs were seeded in single cell suspension condition in a 24 multi-well plate at day 0; at day 1 cultures were infected with lentiviral vectors (expressing either SES or GFP). At day 4, day 7 day 10, and day 13 CSC spheres were dissociated to single cell suspension and live cells were stained with Trypan Blue Solution and counted as previously described. Live cell number and the % of dead cells were reported on graphs for each time point; the experiment was repeated 3 times for each time-point. Bright-field representative pictures were taken at each time-point.

### Western blot analysis

Cells were homogenized in RIPA buffer (50 mM Tris pH 7.5, 150 mM NaCl, 1 mM EDTA, SDS (0.1% for cells, 1% for 3D cultures), 1% Triton X-100, Roche Complete EDTA-free Protease Inhibitor Cocktail, Roche PhosSTOP EASYpack) and Western blot analysis was performed incubating primary antibodies overnight at 4°C in blocking solution composed of 5% BSA (Sigma-Aldrich) or 5% Non-Fat Dry Milk in PBS-TWEEN 0.1% (Sigma-Aldrich) according to antibody datasheet. The primary antibodies utilized were as follows: anti-V5 (mouse, 1:1000, ThermoFisher Scientific, R96025), anti-SOX2 (Clone # 245610, mouse, 1:500, R&D system, MAB2018), anti-Histone H3 (rabbit, 1:2000, Abcam, ab1791). Band densitometry relative to control was calculated using Fiji software (NIH, USA), normalized on housekeeping (H3).

### Clonogenic assay

25×10^4 CSCs were seeded in single cell suspension condition in a 24 multi-well plate at day 0; at day 1 cultures were or were not infected with lentiviral vectors (expressing either SES or GFP). At day 6 spheres were dissociated to single cell suspension and live cells were counted as previously described and 25×10^4 CSCs were seeded again, letting cells grow and form spheres until day 10. Bright-field images were taken at day 6 and day 10 and the resulting number of sphere was counted for each condition (not infected, GFP-infected or SES-infected); sphere diameter was measured and the % of sphere having a diameter < 100 mm was reported on the graph for each condition and time-point. The experiment was repeated 3 times for each time point.

### RNA isolation and real-time RT-qPCR

RNA was extracted using the TRI Reagent isolation system (Sigma-Aldrich) according to the manufacturer's instructions. For quantitative RT-PCR (qRT-PCR), one microgram of RNA was reverse transcribed using the ImProm-II Reverse Transcription System (Promega). Thereafter qRT-PCR was performed in triplicate with custom designed oligos using the CFX96 Real-Time PCR Detection System (Bio-Rad, USA) with the Titan HotTaq EvaGreen qPCR Mix (BIOATLAS). Obtained cDNA was diluted 1:10 and was amplified in a 16 µl reaction mixture containing 2 µl of diluted cDNA, 1× Titan Hot Taq EvaGreen qPCR Mix (Bioatlas, Estonia) and 0.4 mM of each primer. Analysis of relative expression was performed using the ΔΔCt method, using 18S rRNA as a housekeeping gene and CFX Manager software (Bio-Rad, USA).

### RNA-sequencing

RNA libraries were generated starting from 1 mg of total RNA (deriving from U87, SNB19 and murine hippocampi) the quality of which was assessed by using a TapeStation instrument (Agilent). To avoid over-representation of 30 ends, only high-quality RNA with a RNA Integrity Number (RIN) R 8 was used. RNA was processed according to the TruSeq Stranded mRNA Library Prep Kit protocol. The libraries were sequenced on an Illumina HiSeq 3000 with 76 bp stranded reads using Illumina TruSeq technology. Image processing and base call were performed using the Illumina Real Time Analysis Software. Fastq files were aligned to hg19 or mm10 human or mouse reference genomes by using the splice junction map- per TopHat. Differential gene expression and Functional enrichment analyses were performed with DESeq2 and GSEA, respectively.

### ChIP-sequencing

Chromatin was isolated from SNB19. Cells were plated in adherent condition using Matrigel coated 15 mm plates at a density of 6×10^6 per plate. When the plates reached 90% of confluence, cells were fixed by adding formaldehyde directly to the cell culture media to reach a final concentration of 1%, and then were incubated for 10 min at RT. The reaction was quenched adding glycine to a final concentration of 125 mM and incubated 5 min at RT. Medium was then removed and cells were washed 3 times with cold, sterile PBS+protease inhibitor, then cells were gently scraped and collected for centrifugation at 4°C for 50 at 1200 rpm. For ChIP experiments, collected cell pellets were lysed in lysis buffer (50 mM Tris-HCI pH 8, 0.1% SDS, 10 mM EDTA pH 8, 1 mM phenylmethylsulfonyl fluoride (PMSF, Sigma # P7626), protease inhibitor cocktail (Roche #04693159001)) and chromatin was sonicated with a Branson D250 sonifier (4 cycles of 30 s, 20% amplitude), to reach an average fragment size of 0.1-0.5 kb. Following quantification, 100 mg of sonicated chromatin was used in each immunoprecipitation and incubated overnight at 4° C with 4 mg of V5 antibody (mouse, 1:5, ThermoFisher Scientific, R96025).

ChIP-seq libraries were produced using 5 ng of each immunoprecipitated and purified DNA. End repair of DNA fragments was achieved by sequential 15 min incubations at 12°C and 25°C with 0.15 U/ml T4 PNK (NEB #M0201L), 0.04 U/ml T4 POL (NEB #M0203L) and 0.1 mM dNTPs (NEB #N0446S). A-base addition was performed by an incubation with 0.25 U/ml of Klenow fragment (NEB # M0212L) and 167 mM dATP (NEB N0440S) for 30 min at 30°C. Adaptor ligation was achieved by using the Quick ligation kit (NEB #M2200L) and performing an incubation of 15 min at 25°C. DNA fragments were finally amplified for 14 cycles, by using the PfuUltra II Fusion HS DNA Pol kit (Agilent #600674). DNA purification steps after each enzymatic reaction were performed using Agencourt AMPure XP SPRI beads (Beckman #A63882). The obtained libraries were quality controlled at an Agilent Bioanalyzer (Agilent Technologies #G2943CA) before sequencing with Illumina HiSeq 2000. Sequencing read quality was assessed by using fastQC (https://www.bioinformatics.babraham.ac.uk/projects/fastqc/) and total reads were aligned to the human genome (hg19) using Bowtie2 version 2.2.3 (http://bowtie-bio.sourceforge.net/ bowtie2/index.shtml). Only uniquely mapped reads were used in the subsequent analyses, with an average mappability > 96% of initial total reads. Normalized BigWig tracks of ChIP-seq experiments were generated with bedtools 2.24.0 (https://bedtools.readthedocs.io/en/latest/) and the bedGraphTo- BigWig program (https://www.encodeproject.org/software/bedgraphtobigwig/) and visualized in the UCSC Genome Browser (http://genome.ucsc.edu/). In order to find the regions of ChIP-seq enrichment over background, we used SICER V1.1 (https:// home.gwu.edu/$wpeng/Software.html) (window size = 200; gap size = 200; FDR < 0.01 parameters for all ChIP-seq data, FDR 0.01 parameters were used). Density plots (±10 kb) were generated with the ngsplot 2.47 (https:// github.com/shenlab-sinai/ngsplot) command ngs.plot.r and eventually re-plotted with GraphPad Prism.

### MeDIP sequencing

1 µg of purified genomic DNA (gDNA) was used with qiAMP DNA mini kit (Qiagen, cat. 51304). In brief, for methylated DNA immune precipitation and purification MagMeDIPseq kit was used (Diagenode, cod. C02010040). First, gDNA was sonicated to obtain a fragment size between 150-300 bp, then, it was de-natured to ssDNA and immune-precipitated using α-metyl-cytosine antibody provided by the kit. The next day, immunoprecipitated DNA and Input were purified and eluted. Library preparation was performed using NEBNext Ultra II kit for Illumina (cod.E7645), following the manufacturer's instructions. Each library was dual indexed using NEBNext Multiplex Oligos for Illumina (cod.E6440) and sequenced at 30 million pair-end depth with Illumina HiSeq 2000. First Adaptor trimming was performed using Trimmomatic (http://www.usadellab.org/cms/?page=trimmomatic). Trimmed reads were then aligned to reference Hg19 genome using Bowtie2 (http://bowtiebio.sourceforge.net/bowtie2/index.shtml). To obtain Coverage tracks bamCoverage was used (https://deeptools.readthedocs.io/en/develop/content/tools/bamCoverage.html), BAM files were converted to BigWigs using CPM normalization and effective genome size parameters with a bin size of 10. Peaks were called using Macs2 (https://github.com/macs3-project/MACS). Normal peak calling mode was used, in paired end mode with a q-value set at 0.05. Differential peaks were called with Macs2, using the BedGraph obtained comparing BAM files of treated against control condition. Afterwards, differential peaks were intersected with normal peaks for each condition in order to filter differential peaks, actually present in at least one of the two conditions. Density plots (±10 kb) were generated with the ngsplot 2.47 (https://github.com/shenlab-sinai/ngsplot) command ngs.plot.r and eventually re-plotted with GraphPad Prism.

### Xenografts

GBM lines or Cancer stem cells L0627 were seeded in 6-well dishes and infected with 10 µl LV-EIF1α-SES/TES/MES or 5 µl LV-EIF1α-GFP each well for 48 hours, as described in the infection section.

*Heterotopic xenografts.* The infected cells were counted and resuspended 3×10^6 cells in 100 µl of Matrigel (Matrigel growth factor reduced, Corning). By using 1 ml syringes previously cooled at 20°C, GFP-infected cells were subcutaneously injected into the left flank of NOD-SCID mice (NOD.cg-Prkdc scid II2rg tm1Wjl/SzJ), whereas SES/TES/MES-infected cells were subcutaneously injected into the right flank of the same animal. Mice were sacrificed at 1-3 months after injections (according with growth rate) and subcutaneous growing tumors were extracted and fixed in 4% PFA for at least 24 hours. Tumor samples were sized and kept overnight in Sucrose 30% in PBS and then embedded in O.C.T. for cryopreservation. Histological slides were cut in 50-µm sections on a cryostat (CM1850 UV, Leica). Subsequently, the sections were processed for immunofluorescence or mounted on gelatin-coated glass slides and processed for Nissl staining.

*Intracranial xenograft.* The infected cells were counted and resuspended 3×10^5 cells in 3 µl PBS 1X and then were unilaterally injected in the striatum of NOD-SCID mice (AP +0.5; ML ±1.8; DV -3.3 from skull). Mice were sacrificed upon observation of general condition or after 40 days from the U87 injection or after 3-5 weeks of CSC injection; following anesthesia, mice were transcardially perfused with 4% PFA in PBS, then brains were removed from the skull and kept in the same solution for overnight fixation. After fixation, brains were kept overnight in Sucrose 30% in PBS and then embedded in O.C.T. for cryopreservation. The samples were cut coronally in 50-µm sections on a cryostat (CM1850 UV, Leica). Subsequently, the sections were processed for immunofluorescence or mounted on gelatin-coated glass slides and processed for Nissl staining.

*In vivo treatment* (*with U87*)*.* U87 orthotopic xenograft were induced as described before but with 75000 naïve cells. After 4 days the mice, randomly divided in two groups were injected at the same topological coordinates with LV carrying either GF or SES. A cohort of animals was sacrificed after 26 days from the LV injection and another cohort was left alive for performing survival rate and sacrificed upon observation of general condition or at 90 days after the first surgery; following anesthesia, mice were transcardially perfused with 4% PFA in PBS, then brains were removed from the skull and kept in the same solution for overnight fixation. After fixation, brains were kept overnight in Sucrose 30% in PBS and then embedded in O.C.T. for cryopreservation. The samples were cut coronally in 50-µm sections on a cryostat (CM1850 UV, Leica). Subsequently, the sections were processed for immunofluorescence or mounted on gelatin-coated glass slides and processed for Nissl staining.

*In vivo treatment* (*with* CSCs). Classical naïve CSCs orthotopic xenograft were induced as described previously (3×10^5 cells). After 7 days the mice, randomly divided in two groups, were injected at the same topological coordinates with LV carrying either GFP or TES and sacrificed after 3 weeks from the LV injection; following anesthesia, mice were transcardially perfused with 4% PFA in PBS, then brains were removed from the skull and kept in the same solution for O/N fixation. After fixation, brains were kept O/N in Sucrose 30% in PBS and then embedded in O.C.T. for cryopreservation. The samples were cut coronally in 50-µm sections on a cryostat (CM1850 UV, Leica). Subsequently, the sections were processed for immunofluorescence or mounted on gelatin-coated glass slides and processed for Nissl staining.

### In vivo SES delivery in WT animals

Hippocampi of Wt C57BL/6 animals were injected with LV carrying either GFP or SES (2 injections per hippocampus AP -2.8, ML +/- 3, DV -3.5; -2.5., 0.8 µl each). After 1 month from the surgery the animals were tested for behavioral tasks and sacrificed for molecular and histological analyses.

### Immunostaining

Cells were seeded on glass coverslips (for CSCs previously coated with Matrigel to allow cell adhesion) and they were fixed for 20 minutes on ice in 4% paraformaldehyde (PFA, Sigma), solution in phosphate-buffered saline (PBS, Euroclone). Then they were washed twice with PBS and were permeabilized for 30' in blocking solution, containing 0.2% Triton X-100 (SigmaAldrich) and 5% donkey serum (Euroclone), and incubated overnight at 4°C with the primary antibodies diluted in blocking solution. The primary antibodies utilized were as follows: anti-V5 (mouse, 1:500, ThermoFisher Scientific, R96025), anti-GFP (chicken, 1:1000, Thermo Fisher Scientific, A10262), anti-MAP2 (chicken, 1:1000, Abcam, ab92434), anti-phospho-Histone H3 (Ser10, rabbit, 1:200, Sigma-Aldrich, 06-570), anti-Cleaved Caspase-3 (Asp175, rabbit, 1:200, Cell Signaling Technology, 9661), anti-Ki-67 (Clone SP6, rabbit, 1:500, Immunological Sciences, MAB-90948), anti-Human Nuclei (mouse, 1:500, Millipore, MAB1281). The next day, cells were washed 3 times with PBS for 5 minutes and incubated for 1 hour at room temperature with Hoechst 33342 (ThermoFischer Scientific) and with secondary antibodies (ThermoFisher Scientific) in blocking solution. Brain sections were blocked in 10% donkey serum and 0.2% Triton X-100 for 1 hr at RT. Incubation with primary antibodies was performed at 4°C overnight. Secondary antibodies were applied to sections for 2 hrs at RT in blocking solution containing Hoechst 33342. Finally, slices were washed and mounted in Fluorescent Mounting Medium (Dako Cytomation). Images were acquired with epifluorescence microscope Nikon DS-Qi2 and analyzed with Fiji software.

### Nissl staining

Brain sections were rinsed in distilled H₂O for 1 min, then stained in 0.1% Cresyl Violet solution boiled at 50°C for 7 min. After, they were first rinsed in distilled H₂O for 3 min and then washed in 70% to 100% ethanol serial dilutions for 1 min. Finally, they were cleared in xylene for 2 hours and mounted with mounting solution (Eukitt, Sigma Aldrich).

### MRI acquisition

MRI was performed on a small animal-dedicated 7 T scanner (30/70 BioSpec; Bruker, Ettlingen, Germany). The animal protocol included high resolution T2 sequence. Analysis of the tumor volume was performed using MIPAV software (https://mipav.cit.nih.gov).

### GBM-cortex organoids

For cerebral organoid generation WT iPSCs at 70-80% confluence were detached by Accutase solution incubation at 37°C for 10 minutes in order to obtain a single cell suspension. Cells were centrifuged, counted and a total of 9000 cells were then plated into each well of an ultra-low-attachment 96-well plate (Corning), in medium containing DMEM/F12, 20% KnockOut^{™} Serum Replacement (KSR, Thermo Fisher Scientific), 2 mM Glutamine, 1% Pen/Strept, 1% non-essential amino acids, 50 nM β-mercaptoethanol (ThermoFisher Scientific) and 4 ng/ml bFGF. After seeding, plates were briefly centrifuged to allow single EB formation inside each well; ROCK inhibitor Y27632 (50 uM) was included in the first 24 hours. EBs were maintained in 96-well plates for 6 days, then transferred by firmly pipetting (with a cut end of a P200 tip) medium in the well up and down to ultra-low-attachment 24-well plates (Corning), in neural induction medium containing DMEM/F12, 1X N-2 supplement, 1% non-essential amino acids, 2 mM Glutamine and 1 µg/ml heparin (Sigma-Aldrich). On day 10, EBs were embedded in Matrigel (Matrigel growth factor reduced, Corning) together with CSC spheres (previously infected with either RFP only or SES+RFP) in the same droplet of Matrigel to allow fusion, and then gel at 37°C for 30-60 minutes. Embedded EBs-CSCs were subsequently cultured in neural maturation medium containing 50% DMEM/F12, 50% Neurobasal A, 0.5X N-2 supplement, 0.5X B-27 supplement without vitamin A, 2mM Glutamine, 2.5 ng/ml human insulin, 1% non-essential amino acids, and 25 nM β-mercaptoethanol. Droplets were cultured in stationary condition in 6 cm suspension dishes for 4 days, followed by transfer to an orbital shaker (Orbit^{™} LS Low Speed Orbital Shaker) rotating continuously at 60 rpm; here 0.5X B-27 supplement with vitamin A was substituted in the neural maturation medium.

### Behavioral testing

Animals were housed at a constant 23°C in a 12 h light/dark cycle (lights off at 19:00), with food and water available *ad libitum.* We analyzed WT C57BL/6 mice, both males and females, at adult stage (ranging from 2 to 4 months of age) (all tests) infected 4 weeks before in their hippocampi with either GFP (mock) or SES. The sessions were recorded with the video tracking software Ethovision XT (Noldus).

*Spontaneous alternation test.* To test exploratory behavior and cognitive function related to spatial learning and memory the mice were inserted in a 4-arm maze and video recorded for 10 minutes to evaluate: total number of the entries in all arms, percentage of entries in each arm, and the consecution of the arm entries. This latter allows to identify pattern of behavior as (see also Fig 9e): Spontaneous Alternation Performance (SAP), a score index in which the visit of the 4 different arms without repetition is scored as 1 while at least one repetition in a string of 4 entrance is scored 0; Alternate Arm Return, a score index in which while at least one repetition in a string of 3 entrance is scored 1; and Sam Arm Return (SAR), a score index in which two consecutive entries in the same arm is as scored 1.

*Radial maze test.* The eight-arm radial maze consisted of eight identical arms extending radially from an octagonal platform. It was elevated 80 cm above the floor and surrounded by external cues. A cup containing food was placed at the end of each arm. The protocol was divided into distinct phases: Day 1 - Habituation at the apparatus for 10 min (without food at the end of the arms). Day 2 - Food deprivation until when the animals had arrived at the 80%-85% of their initial weight; during the experiment the mice had to maintain this weight. Day 3 - Training: put the food in half and at the end of each arm. Release the mouse in the center of the arena, it must eat two of the eight pellets placed at the end of the arms. Day 4-13 (experimental days 1-10 in the Fig 10f) - Test: The pellets are placed only at the end of the eight arms. The mouse is released in the center of the arena to calculate (i) the time taken to eat the eight pellets and (ii) the percentage of the incorrect choices (the mouse chooses an empty arm) on the total entries. The maze was cleaned with water and 70% ethanol before the next mouse was placed on the apparatus.

*Morris water maze test.* The mice were inserted in a circular pool with a platform that allows them to escape the water (max length of each trial 120"). The release site can be in a different quadrant of the pool (see protocol in Fig 9g) with the position of the platform that was the same for the first 3 days and the reverse for the last 2 days of the protocol. The time to complete each trial and the time spent in the platform zone and in the opposite quadrant was quantified.

### Neurons from iPSCs

WT iPSCs were maintained in feeder-free conditions in mTeSR1 (Stem Cell Technologies) supplemented with Pen/Strept and seeded on human embryonic stem cell (HESC)-qualified Matrigel (Corning)-coated six-well plates; cells were fed daily and passaged in cell clumps weekly using Accutase solution (Sigma-Aldrich). At differentiation day -2, 90% confluent iPSC cultures were infected with the lentiviral vector TetO-Ngn2-T2A-Puro in mTeSR1 medium supplemented with Doxycycline (2 µg/ml, Sigma-Aldrich), overnight. The next day, the medium was replaced with fresh mTeSR1 medium supplemented with antibiotic selection (Puromycin 1 µg/ml, Sigma-Aldrich) and Doxycycline; Doxycycline was maintained for all the experiment. At day 0 medium was replaced with differentiation medium "mTeSR1 + LSBX". Differentiation medium was replaced daily according to the following scheme: Day 0,1: mTeSR1 + LSBX; Day 2,3: mTeSR1 + LSBX + PSD; Day 4,5: 2/3 mTeSR1 + 1/3 N-2 medium + LSX + PSD; Day 6,7: 1/3 mTeSR1 + 2/3 N-2 medium + PSD. At day 8 cells were detached by Accutase solution incubation at 37°C for 20 minutes in order to obtain a single cell suspension. Cells were centrifuged, counted and seeded at a density of 55000 cells/cm² onto Poly-L-Lysine/Laminin/Fibronectin coated plates or coverslip in neuronal maturation medium supplemented with ROCK inhibitor Y27632 (10 uM, Selleckchem) for the first 24 hours. Culture medium was replaced the next day to remove ROCK inhibitor, and then half of the medium was replaced with fresh Neuronal maturation medium twice a week.

LSBX: LDN193189 (Stemgent, 250 nm), SB431542 (Sigma-Aldrich, 10 µM) XAV939 (Sigma-Aldrich, 5 µM). PSD: PD0325901 (Sigma-Aldrich, 8 µM), SU5402 (Sigma-Aldrich, 10 µM), DAPT (Sigma-Aldrich, 10 µM). N-2 medium: DMEM/F12 with B-27 supplement (0.5X, ThermoFisher Scientific) and N-2 supplement (0.5X, ThermoFisher Scientific). Neuronal maturation medium: Neurobasal A (ThermoFisher Scientific) supplemented with 1X B-27 supplement, 2 mM Glutamine, 1% Pen/Strept, BDNF (Peprotech, 20 ng/ml), Ascorbic acid (Sigma-Aldrich, 100 nM), Laminin (1 µg/µl), DAPT (10 µM), dbcAMP (Selleckchem, 250 µM).

### Primary murine neuronal culture

Primary cultures of mouse embryonic cortical neurons were prepared from E17.5 C57BL/6 Wild-Type mice. Briefly, after dissection, cortices were enzymatically digested with 0.025% trypsin (GIBCO) in Hank's balanced salt solution (HBSS) (Euroclone) for 20 min at 37°C. Successively, HBSS with trypsin was removed and the hippocampi were washed with plating medium (Neurobasal A medium supplemented with 1X B-27 supplement, 3.3 mM glucose, 2 mM glutamine and 1% penicillin/streptomycin) and mechanically dissociated with a P1000-pipette to obtain a homogeneous cell suspension. Cells were then plated on poly-L-lysine (PLL) (0.1 mg/ml) coated glass coverslips.

### EXAMPLE B

### MATERIAL AND METHODS

### Constructs

TES v2: the initial part of TEAD1 gene, coding for the amino acids (aa) 1-166 (thus excluding the TEAD1 activator domain) was fused with the Chromo Shadow (CS) repressor domain (from the gene *CBX5,* encoding aa 121-179) at its N-terminus while the V5 tag was fused at the end of the last domain, at the C-terminus of the new chimeric transgene. The transgene was used in a lentiviral construct with Ef1a as a promoter.

### TES v2 (SEQ ID NO: 136):

Ef1a::CS- *hTEAD₁₋₁₆₆* -V5 [**TESv2**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGACCATTGAGCCCAGCAGCTGGAGCGGCAGTGAGAGCCCTGCCGAAAACATGGAAA
GGATGAGTGACTCTGCAGATAAGCCAATTGACAATGATGCAGAAGGGGTCTGGAGCCCCGAC
ATCGAGCAAAGCTTTCAGGAGGCCCTGGCTATCTATCCACCATGTGGGAGGAGGAAAATCAT
CTTATCAGACGAAGGCAAAATGTATGGTAGGAATGAATTGATAGCCAGATACATCAAACTCA
GGACAGGCAAGACGAGGACCAGAAAACAGGTGTCTAGTCACATTCAGGTTCTTGCCAGAAGG
AAATCTCGTGATTTTCATTCCAAGCTAAAGGATCAGACTGCAAAGGATAAGGCCCTGCAGCA
CATGGCGGCCATGTCCTCAGCCCAGATCGTCTCGGCCACTGCCATTCATAACAAGCTGGGGC
TGCCTGGGATTCCACGCCCGACCTTCCCAGGGGCGCCGGGGTTCTGGCCGGGAATGATTCAA
 
ACAGGGCAGCCAGGCGCGCCCGGTAAGCCTATCCCTAACCCTCTCCTCGGTCTCGATTCTAC
GTGA
**Ef1a promoter**
CS
*hTEAD₁₋₁₆₆*
*V5*

TES v3: the initial part of TEAD1 gene, coding for the aa 1-166 (thus excluding the TEAD1 activator domain) was fused with the YAF2-RYBP (Y-R) repressor domain (from the gene RYBP, aa 145-189) at its C-terminus, and a V5 tag was fused at the C-terminus of the Y-R. The transgene was used in a lentiviral construct with Ef1a as a promoter.

### TES v3 (SEQ ID NO: 137):

Ef1a::*hTEAD₁₋₁₆₆-YR*-V5 [**TESv3**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGATTGAGCCCAGCAGCTGGAGCGGCAGTGAGAGCCCTGCCGAAAACA
TGGAAAGGATGAGTGACTCTGCAGATAAGCCAATTGACAATGATGCAGAAGGGGTCTGGAGC
CCCGACATCGAGCAAAGCTTTCAGGAGGCCCTGGCTATCTATCCACCATGTGGGAGGAGGAA
AATCATCTTATCAGACGAAGGCAAAATGTATGGTAGGAATGAATTGATAGCCAGATACATCA
AACTCAGGACAGGCAAGACGAGGACCAGAAAACAGGTGTCTAGTCACATTCAGGTTCTTGCC
AGAAGGAAATCTCGTGATTTTCATTCCAAGCTAAAGGATCAGACTGCAAAGGATAAGGCCCT
GCAGCACATGGCGGCCATGTCCTCAGCCCAGATCGTCTCGGCCACTGCCATTCATAACAAGC
TGGGGCTGCCTGGGATTCCACGCCCGACCTTCCCAGGGGCGCCGGGGTTCTGGCCGGGAATG
 
ATTCAAACAGGGCAGCCAaccATGAGGCCCCGGCTGAAAAACGTGGACAGGAGCACTGCACA
GCAGTTGGCAGTAACTGTGGGCAACGTCACCGTCATTATCACAGACTTTAAGGAAAAGACTC
GCTCCTCATCGACATCCTCATCCACAGTGACCTCCAGTGCAGGGTCAGAACAGCAGAACCAG
GGCGCGCCCGGTAAGCCTATCCCTAACCCTCTCCTCGGTCTCGATTCTACGTGA
**Ef1a promoter**
*hTEAD₁₋₁₆₆*
*Y-R*
*V5*

TES v4: TES v2 was fused with the YAF2-RYBP (Y-R) repressor domain (from the gene RYBP, aa 145-189) at its C-terminus, and a V5 tag was fused at the C-terminus of the Y-R. The transgene was used in a lentiviral construct with Ef1a as a promoter.

### TES v4 (SEQ ID NO: 138):

Ef1a::CS- *hTEAD₁₋₁₆₆* - Y-R -V5 [**TESv4**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGACCATTGAGCCCAGCAGCTGGAGCGGCAGTGAGAGCCCTGCCGAAAACATGGAAA
GGATGAGTGACTCTGCAGATAAGCCAATTGACAATGATGCAGAAGGGGTCTGGAGCCCCGAC
ATCGAGCAAAGCTTTCAGGAGGCCCTGGCTATCTATCCACCATGTGGGAGGAGGAAAATCAT
CTTATCAGACGAAGGCAAAATGTATGGTAGGAATGAATTGATAGCCAGATACATCAAACTCA
 
GGACAGGCAAGACGAGGACCAGAAAACAGGTGTCTAGTCACATTCAGGTTCTTGCCAGAAGG
AAATCTCGTGATTTTCATTCCAAGCTAAAGGATCAGACTGCAAAGGATAAGGCCCTGCAGCA
CATGGCGGCCATGTCCTCAGCCCAGATCGTCTCGGCCACTGCCATTCATAACAAGCTGGGGC
TGCCTGGGATTCCACGCCCGACCTTCCCAGGGGCGCCGGGGTTCTGGCCGGGAATGATTCAA
ACAGGGCAGCCAACCatgaggccccggctgaaaaacgtggacaggagcactgcacagcagtt
ggcagtaactgtgggcaacgtcaccgtcattatcacagactttaaggaaaagactcgctcct
catcgacatcctcatccacagtgacctccagtgcaggtcagaacagcagaaccagGGCGCG
CCCGGTAAGCCTATCCCTAACCCTCTCCTCGGTCTCGATTCTACGTGA
**Ef1a promoter**
CS
*hTEAD₁₋₁₆₆*
*y*-*r*
*V5*

MES v2: the C-terminal part of MYC gene, coding for the aa 144-454 (thus excluding the MYC activator domain) was fused with the Chromo Shadow (CS) repressor domain (from the gene *CBX5,* aa 121-179) at its N-terminus, and a V5 tag was fused at the C-terminus of the new transgene. The transgene was used in a lentiviral construct with Ef1a as a promoter.

### MES v2 (SEQ ID NO: 139):

Ef1a: : CS- hMYC₁₄₄₋₄₅₄ -V5 [**MESv2**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
 
CAACGGATCCACCATGCTGGAACCAGAAAAGATCATTGGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGACCATCATCCAGGACTGTATGTGGAGCGGCTTCTCGGCCGCCGCCAAGCTCGTCT
CAGAGAAGCTGGCCTCCTACCAGGCTGCGCGCAAAGACAGCGGCAGCCCGAACCCCGCCCGC
GGCCACAGCGTCTGCTCCACCTCCAGCTTGTACCTGCAGGATCTGAGCGCCGCCGCCTCAGA
GTGCATCGACCCCTCGGTGGTCTTCCCCTACCCTCTCAACGACAGCAGCTCGCCCAAGTCCT
GCGCCTCGCAAGACTCCAGCGCCTTCTCTCCGTCCTCGGATTCTCTGCTCTCCTCGACGGAG
TCCTCCCCGCAGGGCAGCCCCGAGCCCCTGGTGCTCCATGAGGAGACACCGCCCACCACCAG
CAGCGACTCTGAGGAGGAACAAGAAGATGAGGAAGAAATCGATGTTGTTTCTGTGGAAAAGA
GGCAGGCTCCTGGCAAAAGGTCAGAGTCTGGATCACCTTCTGCTGGAGGCCACAGCAAACCT
CCTCACAGCCCACTGGTCCTCAAGAGGTGCCACGTCTCCACACATCAGCACAACTACGCAGC
GCCTCCCTCCACTCGGAAGGACTATCCTGCTGCCAAGAGGGTCAAGTTGGACAGTGTCAGAG
TCCTGAGACAGATCAGCAACAACCGAAAATGCACCAGCCCCAGGTCCTCGGACACCGAGGAG
AATGTCAAGAGGCGAACACACAACGTCTTGGAGCGCCAGAGGAGGAACGAGCTAAAACGGAG
CTTTTTTGCCCTGCGTGACCAGATCCCGGAGTTGGAAAACAATGAAAAGGCCCCCAAGGTAG
TTATCCTTAAAAAAGCCACAGCATACATCCTGTCCGTCCAAGCAGAGGAGCAAAAGCTCATT
TCTGAAGAGGACTTGTTGCGGAAACGACGAGAACAGTTGAAACACAAACTTGAACAGCTACG
GAACTCTTGTGCGGGCGCGCCCGGTAAGCCTATCCCTAACCCTCTCCTCGGTCTCGATTCTA
CGTGA
**Ef1a promoter**
CS
*hMYC₁₄₄₋₄₅₄*
*V5*

MES v3: the C-terminal part of MYC gene, coding for the aa 144-454 (thus excluding the MYC activator domain) was fused with the YAF2-RYBP (Y-R) repressor domain (from the gene RYBP, aa 145-189) at its N-terminus, while a V5 tag was fused at the C-terminus of the new transgene. The transgene was used in a lentiviral construct with Ef1a as a promoter.

### MES v3 (SEQ ID NO: 140):

Ef1a: : YR- hMYC₁₄₄₋₄₅₄ -V5 [**MESv3**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
 
TGGGGCCGCGGCGGCGACGGGGCCCGTGCGTCCCAGCGCACATGTTCGGCGAGGCGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGAGGCCCCGGCTGAAAAACGTGGACAGGAGCACTGCACAGCAGTTGG
CAGTAACTGTGGGCAACGTCACCGTCATTATCACAGACTTTAAGGAAAAGACTCGCTCCTCA
TCGACATCCTCATCCACAGTGACCTCCAGTGCAGGGTCAGAACAGCAGAACCAGACCATCAT
CCAGGACTGTATGTGGAGCGGCTTCTCGGCCGCCGCCAAGCTCGTCTCAGAGAAGCTGGCCT
CCTACCAGGCTGCGCGCAAAGACAGCGGCAGCCCGAACCCCGCCCGCGGCCACAGCGTCTGC
TCCACCTCCAGCTTGTACCTGCAGGATCTGAGCGCCGCCGCCTCAGAGTGCATCGACCCCTC
GGTGGTCTTCCCCTACCCTCTCAACGACAGCAGCTCGCCCAAGTCCTGCGCCTCGCAAGACT
CCAGCGCCTTCTCTCCGTCCTCGGATTCTCTGCTCTCCTCGACGGAGTCCTCCCCGCAGGGC
AGCCCCGAGCCCCTGGTGCTCCATGAGGAGACACCGCCCACCACCAGCAGCGACTCTGAGGA
GGAACAAGAAGATGAGGAAGAAATCGATGTTGTTTCTGTGGAAAAGAGGCAGGCTCCTGGCA
AAAGGTCAGAGTCTGGATCACCTTCTGCTGGAGGCCACAGCAAACCTCCTCACAGCCCACTG
GTCCTCAAGAGGTGCCACGTCTCCACACATCAGCACAACTACGCAGCGCCTCCCTCCACTCG
GAAGGACTATCCTGCTGCCAAGAGGGTCAAGTTGGACAGTGTCAGAGTCCTGAGACAGATCA
GCAACAACCGAAAATGCACCAGCCCCAGGTCCTCGGACACCGAGGAGAATGTCAAGAGGCGA
ACACACAACGTCTTGGAGCGCCAGAGGAGGAACGAGCTAAAACGGAGCTTTTTTGCCCTGCG
TGACCAGATCCCGGAGTTGGAAAACAATGAAAAGGCCCCCAAGGTAGTTATCCTTAAAAAAG
CCACAGCATACATCCTGTCCGTCCAAGCAGAGGAGCAAAAGCTCATTTCTGAAGAGGACTTG
TTGCGGAAACGACGAGAACAGTTGAAACACAAACTTGAACAGCTACGGAACTCTTGTGCGGG
CGCGCCCGGTAAGCCTATCCCTAACCCTCTCCTCGGTCTCGATTCTACGTGA
**Ef1a promoter**
YR
*hMYC₁₄₄₋₄₅₄*
*V5*

MES v4: the C-terminal part of MYC gene, coding for the aa 144-454 (thus excluding the MYC activator domain) was fused with the Chromo Shadow (CS) repressor domain (from the gene *CBX5,* aa 121-179) and YAF2-RYBP (Y-R) repressor domain (from the gene RYBP, aa 145-189) in tandem at its N-terminus, and a V5 tag was fused at the C-terminus of the new transgene. The transgene was used in a lentiviral construct with Ef1a as a promoter.

### MES v4 (SEQ ID NO: 141):

Ef1a: : CS-YR- hMYC₁₄₄₋₄₅₄ -V5 [**MESv4**] 
CGATAAGCTTTGCAAAGATGGATAAAGTTTTAAACAGAGAGGAATCTTTGCAGCTAATGGAC
CTTCTAGGTCTTGAAAGGAGTGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGaccatgaggccccggctgaaaaacgtggacaggagcactgcacagcagttggcag
taactgtgggcaacgtcaccgtcattatcacagactttaaggaaaagactcgctcctcatcg
acatcctcatccacagtgacctccagtgcagggtcagaacagcagaaccagACCATCATCCA
GGACTGTATGTGGAGCGGCTTCTCGGCCGCCGCCAAGCTCGTCTCAGAGAAGCTGGCCTCCT
ACCAGGCTGCGCGCAAAGACAGCGGCAGCCCGAACCCCGCCCGCGGCCACAGCGTCTGCTCC
ACCTCCAGCTTGTACCTGCAGGATCTGAGCGCCGCCGCCTCAGAGTGCATCGACCCCTCGGT
GGTCTTCCCCTACCCTCTCAACGACAGCAGCTCGCCCAAGTCCTGCGCCTCGCAAGACTCCA
GCGCCTTCTCTCCGTCCTCGGATTCTCTGCTCTCCTCGACGGAGTCCTCCCCGCAGGGCAGC
CCCGAGCCCCTGGTGCTCCATGAGGAGACACCGCCCACCACCAGCAGCGACTCTGAGGAGGA
ACAAGAAGATGAGGAAGAAATCGATGTTGTTTCTGTGGAAAAGAGGCAGGCTCCTGGCAAAA
GGTCAGAGTCTGGATCACCTTCTGCTGGAGGCCACAGCAAACCTCCTCACAGCCCACTGGTC
CTCAAGAGGTGCCACGTCTCCACACATCAGCACAACTACGCAGCGCCTCCCTCCACTCGGAA
GGACTATCCTGCTGCCAAGAGGGTCAAGTTGGACAGTGTCAGAGTCCTGAGACAGATCAGCA
ACAACCGAAAATGCACCAGCCCCAGGTCCTCGGACACCGAGGAGAATGTCAAGAGGCGAACA
CACAACGTCTTGGAGCGCCAGAGGAGGAACGAGCTAAAACGGAGCTTTTTTGCCCTGCGTGA
CCAGATCCCGGAGTTGGAAAACAATGAAAAGGCCCCCAAGGTAGTTATCCTTAAAAAAGCCA
CAGCATACATCCTGTCCGTCCAAGCAGAGGAGCAAAAGCTCATTTCTGAAGAGGACTTGTTG
CGGAAACGACGAGAACAGTTGAAACACAAACTTGAACAGCTACGGAACTCTTGTGCGGGCGC
GCCCGGTAAGCCTATCCCTAACCCTCTCCTCGGTCTCGATTCTACGTGA
**Ef1a promoter**
CS
*yr*
*hMYC₁₄₄₋₄₅₄*
*V5*

ESF-Tmir: Tmir cassette, consisting of four target sequences in tandem for both miR124 (atattgccttatttc [SEQ ID NO: 1]), MiR338-3p (caacaaaatcactgatgctgga [SEQ ID NO: 2]), and miR31 (cagctatgccagcatcttgcc [SEQ ID NO: 3]) was generated by synthesis and cloned into the 3' UTR of the ESF.

A triple miRNA Target sequence (Tmir) is shown below as SEQ ID NO: 4: 
atattgccttatttccgattattgccttatttcgcattattgccttatttctcactattgccttattt
ccgatcccggggtttaaaccgatcaacaaaatcactgatgctggacgatcaacaaaatcactgatgct
ggacgatcaacaaaatcactgatgctggatcaccaacaaaatcactgatgctggacgatgtttaaacc
ctgcaggcgatcagctatgccagcatcttgcccgatcagctatgccagcatcttgcccgatcagctat
gccagcatcttgcctcaccagctatgccagcatcttgcccgatcctgcaggagatct
miR124 Target sequences
miR338-3p Target sequences
miR31 Target sequences

Tmir cassettes comprising ESFs are set out in the following sequences.

### SES-Tmir (SEQ ID NO: 142):

Ef1a:: KRAB-hSOX2₁₋₁₇₉-DNMT3a3L-V5-Tmir [**SES-Tmir**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
 
TTTTTTTTCTTCCATTTCAGGTGTCGTGAGGAATTCTGCAGTCGATCGACGGTACCGCGGGCC
CAACGGATCCACCATGGATGCTAAGTCACTGACTGCCTGGTCCCGGACACTGGTGACCTTCA
AGGATGTGTTTGTGGACTTCACCAGGGAGGAGTGGAAGCTGCTGGACACTGCTCAGCAGATC
CTGTACAGAAATGTGATGCTGGAGAACTATAAGAACCTGGTTTCCTTGGGTTATCAGCTTAC
TAAGCCAGATGTGATCCTCCGGTTGGAGAAGGGAGAAGAGCCCTGGCTGGTGGAGAGAGAAA
TTCACCAAGAGACCCATCCTGATTCAGAGACTGCATTTGAAATCAAATCATCAGTTGGCAGC
GGCGCATGCatgtacaacatgatggagacggagctgaagccgccgggcccgcagcaaacttc
gggggcggcggcggcaactccaccgcggcggcggccggcggcaaccagaaaaacagcccgg
accgcgtcaagcggcccatgaatgccttcatggtgtggtcccgcgggcagcggcgcaagatg
gcccaggagaaccccaagatgcacaactcggagatcagcaagcgcctgggcgccgagtggaa
acttttgtcggagacggagaagcggccgttcatcgacgaggctaagcggctgcgagcgctgc
acatgaaggagcacccggattataaataccggccccggcggaaaaccaagacgctcatgaag
aaggataagtacacgctgcccggcgggctgctggcccccggcggcaatagcatggcgagcgg
ggtcggggtgggcgccggcctgggcgcgggcgtgaaccagcgcatggacagttacgcgcaca
tgaacggctggagcaacggcagctacagcatgatgcaggaccagctgggcGCTAGCGGCAGC
GGCAACCATGATCAAGAGTTCGATCCTCCTAAAGTGTATCCCCCTGTCCCTGCCGAAAAGCG
GAAACCGATCAGGGTTCTCAGTCTCTTCGATGGAATTGCCACAGGCCTCTTGGTCCTTAAGG
ACCTGGGCATCCAAGTgGAcCGCTACATCGCCTCAGAAGTATGTGAgGACAGCATCACAGTC
GGCATGGTGCGCCACCAGGGGAAAATAATGTACGTCGGCGACGTCAGATCAGTTACTCAGAA
ACATATTCAGGAATGGGGGCCTTTTGATCTCGTAATTGGCGGCAGCCCCTGCAATGATCTGA
GTATTGTGAACCCCGCCCGGAAAGGTTTGTACGAAGGTACGGGCAGACTGTTCTTCGAGTTT
TACAGACTGTTGCACGACGCACGACCAAAGGAGGGTGAcGAtcGACCCTTCTTCTGGCTGTT
CGAGAACGTGGTCGCTATGGGCGTGTCTGACAAGCGGGACATTTCtAGATTCCTGGAGAGCA
ATCCAGTGATGATTGATGCAAAGGAAGTAtccGCTGCCCACCGCGCCAGATACTTCTGGGGC
AATCTGCCCGGCATGAATCGACCCTTGGCAAGCACCGTGAATGATAAGTTGGAGTTGCAGGA
GTGTCTgGAGCATGGCGGATCGCAAAGTTTAGCAAGGTGCGGACCATCACGACCCGAAGCA
ATTCAATTAAGCAGGGAAAGGACCAACATTTTCCAGTGTTTATGAACGAGAAAGAGGAcATC
CTGTGGTGcACAGAAATGGAGCGGGTTTTCGGATTCCCCGTACATTATACTGATGTGTCCAA
TATGTCTAGGTTGGCTAGACAGAGGCTGtTGGGACGCTCATGGAGTGTTCCTGTCATCCGCC
ACCTGTTCGCTCCTCTGAAGGAGTATTTCGCcTGCGTtTCATCCGGgAATTCAAACGCAAAC
AGCAGAGGCCCATCCTTTTCTTCCGGCCTGGTgCCACTTAGTCTGCGCGGCTCTCACATGGG
ACCTATGGAAATATACAAAACAGTGAGCGCCTGGAAGAGGCAGCCTGTGCGGGTCCTGAGTC
TGTTCAGAAACATTGACAAGGTGCTTAAGTCCCTTGGATTTCTGGAGTCTGGCAGCGGTTCT
GGGGGTGgcACCCTTAAGTATGTGGAGGACGTGACAAACGTCGTGAGGAGAGATGTGGAGAA
ATGGGGGCCATTTGACCTGGTATACGGGAGCACTCAACCTCTCGGTTCTTCTTGTGATCGCT
GCCCAGGGTGGTATATGTTTCAGTTCCATCGcATCCTGCAATACGCCCTTCCGCGGCAGGAG
AGTCAGCGACCATTCTTCTGGATATTCATGGACAATCTCCTGCTGACAGAgGACGACCAAGA
GACTACGACTAGATTTCTTCAGACCGAGGCCGTTACTCTCCAGGACGTTAGAGGTAGGGACT
ACCAGAATGCCATGCGAGTGTGGAGTAACATCCCCGGACTCAAGTCAAAGCAcGCACCCCTG
ACCCCCAAGGAAGAGGAATACCTcCAGGCGCAGGTGAGGAGCCGCAGTAAGCTCGATGCTCC
TAAAGTCGATTTGTTGGTGAAGAATTGCTTGCTGCCCCTGAGAGAGTACTTTAAGTACTTTA
GCCAGAACAGCTTGCCACTGGGCGCGCCCGGTAAGCCTATCCCTAACCCTCTCCTCGGTCTC
GATTCTACGTGAattaattaagaattcgacccagctttcttgtacaaagtggttgatatcca
gcacagtggcggccgctcgagtctagagggcccgcggttcgaaggtaagcctatccctaacc
ctctcctcggtctcgattctacgcgtaccggtgtcgactctagataaagcatcaagcttatc
gcgataccgtcgacaatcaacctctggattacaaaatttgtgaaagattgactggtattctt
aactatgttgctccttttacgctatgtggatacgctgctttaatgcctttgtatcatgctat
tgcttcccgtatggctttcattttctcctccttgtataaatcctggttgctgtctctttatg
 
aggagttgtggcccgttgtcaggcaacgtggcgtggtgtgcactgtgtttgctgacgcaacc
cccactggttggggcattgccaccacctgtcagctcctttccgggactttcgctttccccct
ccctattgccacggcggaactcatcgccgcctgccttgcccgctgctggacaggggctcggc
tgttgggcactgacaattccgtggtgttgtcggggaagctgacgtcctttccatggctgctc
gcctgtgttgccacctggattctgcgcgggacgtccttctgctacgtcccttcggccctcaa
tccagcggaccttccttcccgcggcctgctgccggctctgcggcctcttccgcgtcttcgcc
ttcgccctcagacgagtcggatctccctttgggccgcctccccgcctggaatgatatcctcg
agcccgggcgatatattgccttatttccgattattgccttatttcgcattattgccttattt
ctcactattgccttatttccgatcccgggtttaaaccgatcaacaaaatcactgatgctgg
acgatcaacaaaatcactgatgctggacgatcaacaaaatcactgatgctggatcaccaaca
aaatcactgatgctggacgatgtttaaaccctgcaggcgatcagctatgccagcatcttgcc
cgatcagctatgccagcatcttgcccgatcagctatgccagcatcttgcctcaccagctatg
ccagcatcttgcccgatcctgcaggagatct
**Ef1a promoter**
KRAB
*hSOX2₁₋₁₇₉*
**DNMT3a3L**
*V5*
miR124 Target sequences
miR338-3p Target sequences
miR31 Target sequences

### SES (SEQ ID NO: 155):

Ef1a:: KRAB-hSOX2₁₋₁₇₉-DNMT3a3L-V5 [**SES**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
 
CTCGAGCTTTTGGAGTACGTCGTCTTTAGGTTGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGGATGCTAAGTCACTGACTGCCTGGTCCCGGACACTGGTGACCTTCA
AGGATGTGTTTGTGGACTTCACCAGGGAGGAGTGGAAGCTGCTGGACACTGCTCAGCAGATC
CTGTACAGAAATGTGATGCTGGAGAACTATAAGAACCTGGTTTCCTTGGGTTATCAGCTTAC
TAAGCCAGATGTGATCCTCCGGTTGGAGAAGGGAGAAGAGCCCTGGCTGGTGGAGAGAGAAA
TTCACCAAGAGACCCATCCTGATTCAGAGACTGCATTTGAAATCAAATCATCAGTTGGCAGC
GGCGCATGCatgtacaacatgatggagacggagctgaagccgccgggcccgcagcaaacttc
gggggcggcggcggcaactccaccgcggcggcggccggcggcaaccagaaaaacagcccgg
accgcgtcaagcggcccatgaatgccttcatggtgtggtcccgcgggcagcggcgcaagatg
gcccaggagaaccccaagatgcacaactcggagatcagcaagcgcctgggcgccgagtggaa
acttttgtcggagacggagaagcggccgttcatcgacgaggctaagcggctgcgagcgctgc
acatgaaggagcacccggattataaataccggccccggcggaaaaccaagacgctcatgaag
aaggataagtacacgctgcccggcgggctgctggcccccggcggcaatagcatggcgagcgg
ggtcggggtgggcgccggcctgggcgcgggcgtgaaccagcgcatggacagttacgcgcaca
tgaacggctggagcaacggcagctacagcatgatgcaggaccagctgggcGCTAGCGGCAGC
GGCAACCATGATCAAGAGTTCGATCCTCCTAAAGTGTATCCCCCTGTCCCTGCCGAAAAGCG
GAAACCGATCAGGGTTCTCAGTCTCTTCGATGGAATTGCCACAGGCCTCTTGGTCCTTAAGG
ACCTGGGCATCCAAGTgGAcCGCTACATCGCCTCAGAAGTATGTGAgGACAGCATCACAGTC
GGCATGGTGCGCCACCAGGGGAAAATAATGTACGTCGGCGACGTCAGATCAGTTACTCAGAA
ACATATTCAGGAATGGGGGCCTTTTGATCTCGTAATTGGCGGCAGCCCCTGCAATGATCTGA
GTATTGTGAACCCCGCCCGGAAAGGTTTGTACGAAGGTACGGGCAGACTGTTCTTCGAGTTT
TACAGACTGTTGCACGACGCACGACCAAAGGAGGGTGAcGAtcGACCCTTCTTCTGGCTGTT
CGAGAACGTGGTCGCTATGGGCGTGTCTGACAAGCGGACATTTCtAGATTCCTGGAGAGCA
ATCCAGTGATGATTGATGCAAAGGAAGTAtccGCTGCCCACCGCGCCAGATACTTCTGGGGC
AATCTGCCCGGCATGAATCGACCCTTGGCAAGCACCGTGAATGATAAGTTGGAGTTGCAGGA
GTGTCTgGAGCATGGCGGATCGCAAAGTTTAGCAAGGTGCGGACCATCACGACCCGAAGCA
ATTCAATTAAGCAGGGAAAGGACCAACATTTTCCAGTGTTTATGAACGAGAAAGAGGAcATC
CTGTGGTGcACAGAAATGGAGCGGGTTTTCGGATTCCCCGTACATTATACTGATGTGTCCAA
TATGTCTAGGTTGGCTAGACAGAGGCTGtTGGGACGCTCATGGAGTGTTCCTGTCATCCGCC
ACCTGTTCGCTCCTCTGAAGGAGTATTTCGCcTGCGTtTCATCCGGgAATTCAAACGCAAAC
AGCAGAGGCCCATCCTTTTCTTCCGGCCTGGTgCCACTTAGTCTGCGCGGCTCTCACATGGG
ACCTATGGAAATATACAAAACAGTGAGCGCCTGGAAGAGGCAGCCTGTGCGGGTCCTGAGTC
TGTTCAGAAACATTGACAAGGTGCTTAAGTCCCTTGGATTTCTGGAGTCTGGCAGCGGTTCT
GGGGGTGgcACCCTTAAGTATGTGGAGGACGTGACAAACGTCGTGAGGAGAGATGTGGAGAA
ATGGGGGCCATTTGACCTGGTATACGGGAGCACTCAACCTCTCGGTTCTTCTTGTGATCGCT
GCCCAGGGTGGTATATGTTTCAGTTCCATCGcATCCTGCAATACGCCCTTCCGCGGCAGGAG
AGTCAGCGACCATTCTTCTGGATATTCATGGACAATCTCCTGCTGACAGAgGACGACCAAGA
GACTACGACTAGATTTCTTCAGACCGAGGCCGTTACTCTCCAGGACGTTAGAGGTAGGGACT
ACCAGAATGCCATGCGAGTGTGGAGTAACATCCCCGGACTCAAGTCAAAGCAcGCACCCCTG
ACCCCCAAGGAAGAGGAATACCTcCAGGCGCAGGTGAGGAGCCGCAGTAAGCTCGATGCTCC
TAAAGTCGATTTGTTGGTGAAGAATTGCTTGCTGCCCCTGAGAGAGTACTTTAAGTACTTTA
GCCAGAACAGCTTGCCACTGGGCGCGCCCGGTAAGCCTATCCCTAACCCTCTCCTCGGTCTC
GATTCTACGTGAattaattaagaattcgacccagctttcttgtacaaagtggttgatatcca
gcacagtggcggccgctcgagtctagagggcccgcggttcgaaggtaagcctatccctaacc
ctctcctcggtctcgattctacgcgtaccggtgtcgactctagataaagcatcaagcttatc
 
gcgataccgtcgacaatcaacctctggattacaaaatttgtgaaagattgactggtattctt
aactatgttgctccttttacgctatgtggatacgctgctttaatgcctttgtatcatgctat
tgcttcccgtatggctttcattttctcctccttgtataaatcctggttgctgtctctttatg
aggagttgtggcccgttgtcaggcaacgtggcgtggtgtgcactgtgtttgctgacgcaacc
cccactggttggggcattgccaccacctgtcagctcctttccgggactttcgctttccccct
ccctattgccacggcggaactcatcgccgcctgccttgcccgctgctggacaggggctcggc
tgttgggcactgacaattccgtggtgttgtcggggaagctgacgtcctttccatggctgctc
gcctgtgttgccacctggattctgcgcgggacgtccttctgctacgtcccttcggccctcaa
tccagcggaccttccttcccgcggcctgctgccggctctgcggcctcttccgcgtcttcgcc
ttcgccctcagacgagtcggatctccctttgggccgcctccccgcctggaatgatatcctcg
agcccgggcgat
**Ef1a promoter**
KRAB
*hSOX2₁₋₁₇₉*
**DNMT3a3L**
*V5*

### SESv2-Tmir (SEQ ID NO: 143):

Ef1a: : CS-hSOX2₁₋₁₇₉ -V5-Tmir [**SESv2-Tmir**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
 
TCCTGAGGCATGCatgtacaacatgatggagacggagctgaagccgccgggcccgcagcaaa
cttcgggggcggcggcggcaactccaccgcggcggccggcggcaaccagaaaaacagc
ccggaccgcgtcaagcggcccatgaatgccttcatggtgtggtcccgcgggcagcggcgcaa
gatggcccaggagaaccccaagatgcacaactcggagatcagcaagcgcctgggcgccgagt
ggaaacttttgtcggagacggagaagcggccgttcatcgacgaggctaagcggctgcgagcg
ctgcacatgaaggagcacccggattataaataccggccccggcggaaaaccaagacgctcat
gaagaaggataagtacacgctgcccggcgggctgctggcccccggcggcaatagcatggcga
gcggggtcggggtgggcgccggcctgggcgcgggcgtgaaccagcgcatggacagttacgcg
cacatgaacggctggagcaacggcagctacagcatgatgcaggaccagctgggcGCTAGCGG
CAGCGGCGGCGCGCCCGGTAAGCCTATCCCTAACCCTCTCCTCGGTCTCGATTCTACGTGAa
ttaattaagaattcgacccagctttcttgtacaaagtggttgatatccagcacagtggcggc
cgctcgagtctagagggcccgcggttcgaaggtaagcctatccctaaccctctcctcggtct
cgattctacgcgtaccggtgtcgactctagataaagcatcaagcttatcgcgataccgtcga
caatcaacctctggattacaaaatttgtgaaagattgactggtattcttaactatgttgctc
cttttacgctatgtggatacgctgctttaatgcctttgtatcatgctattgcttcccgtatg
gctttcattttctcctccttgtataaatcctggttgctgtctctttatgaggagttgtggcc
cgttgtcaggcaacgtggcgtggtgtgcactgtgtttgctgacgcaacccccactggttggg
gcattgccaccacctgtcagctcctttccgggactttcgctttccccctccctattgccacg
gcggaactcatcgccgcctgccttgcccgctgctggacaggggctcggctgttgggcactga
caattccgtggtgttgtcggggaagctgacgtcctttccatggctgctcgcctgtgttgcca
cctggattctgcgcgggacgtccttctgctacgtcccttcggccctcaatccagcggacctt
ccttcccgcggcctgctgccggctctgcggcctcttccgcgtcttcgccttcgccctcagac
gagtcggatctccctttgggccgcctccccgcctggaatgatatcctcgagcccgggcgata
tattgccttatttccgattattgccttatttcgcattattgccttatttctcactattgcct
tatttccgatcccgggtttaaaccgatcaacaaaatcactgatgctggacgatcaacaaaa
tcactgatgctggacgatcaacaaaatcactgatgctggatcaccaacaaaatcactgatgc
tggacgatgtttaaaccctgcaggcgatcagctatgccagcatcttgcccgatcagctatgc
cagcatcttgcccgatcagctatgccagcatcttgcctcaccagctatgccagcatcttgcc
cgatcctgcaggagatct
**Ef1a promoter**
CS
*hSOX2₁₋₁₇₉*
*V5*
miR124 Target sequences
miR338-3p Target sequences
miR31 Target sequences

### SESv2 (SEQ ID NO: 156):

Ef1a: :CS-hSOX2₁₋₁₇₉ -V5 [**SESv2**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
 
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGGCATGCatgtacaacatgatggagacggagctgaagccgccgggcccgcagcaaa
cttcgggggcggcggcggcaactccaccgcggcggcggccggcggcaaccagaaaaacagc
ccggaccgcgtcaagcggcccatgaatgccttcatggtgtggtcccgcgggcagcggcgcaa
gatggcccaggagaaccccaagatgcacaactcggagatcagcaagcgcctgggcgccgagt
ggaaacttttgtcggagacggagaagcggccgttcatcgacgaggctaagcggctgcgagcg
ctgcacatgaaggagcacccggattataaataccggccccggcggaaaaccaagacgctcat
gaagaaggataagtacacgctgcccggcgggctgctggcccccggcggcaatagcatggcga
gcggggtcggggtgggcgccggcctgggcgcgggcgtgaaccagcgcatggacagttacgcg
cacatgaacggctggagcaacggcagctacagcatgatgcaggaccagctgggcGCTAGCGG
CAGCGGCGGCGCGCCCGGTAAGCCTATCCCTAACCCTCTCCTCGGTCTCGATTCTACGTGAa
ttaattaagaattcgacccagctttcttgtacaaagtggttgatatccagcacagtggcggc
cgctcgagtctagagggcccgcggttcgaaggtaagcctatccctaaccctctcctcggtct
cgattctacgcgtaccggtgtcgactctagataaagcatcaagcttatcgcgataccgtcga
caatcaacctctggattacaaaatttgtgaaagattgactggtattcttaactatgttgctc
cttttacgctatgtggatacgctgctttaatgcctttgtatcatgctattgcttcccgtatg
gctttcattttctcctccttgtataaatcctggttgctgtctctttatgaggagttgtggcc
cgttgtcaggcaacgtggcgtggtgtgcactgtgtttgctgacgcaacccccactggttggg
gcattgccaccacctgtcagctcctttccgggactttcgctttccccctccctattgccacg
gcggaactcatcgccgcctgccttgcccgctgctggacaggggctcggctgttgggcactga
caattccgtggtgttgtcggggaagctgacgtcctttccatggctgctcgcctgtgttgcca
cctggattctgcgcgggacgtccttctgctacgtcccttcggccctcaatccagcggacctt
ccttcccgcggcctgctgccggctctgcggcctcttccgcgtcttcgccttcgccctcagac
gagtcggatctccctttgggccgcctccccgcctggaatgatatcctcgagcccgggcgat
**Ef1a promoter**
CS
*hSOX2₁₋₁₇₉*
*V5*

### SESv3-Tmir (SEQ ID NO: 144):

Ef1a:: SOX2₁₋₁₇₉-Y-R-V5-Tmir [**SESv3-Tmir**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCatgtacaacatgatggagacggagctgaagccgccgggcccgcagcaaa
cttcgggggcggcggcggcaactccaccgcggcggcggccggcggcaaccagaaaaacagc
ccggaccgcgtcaagcggcccatgaatgccttcatggtgtggtcccgcgggcagcggcgcaa
gatggcccaggagaaccccaagatgcacaactcggagatcagcaagcgcctgggcgccgagt
ggaaacttttgtcggagacggagaagcggccgttcatcgacgaggctaagcggctgcgagcg
ctgcacatgaaggagcacccggattataaataccggccccggcggaaaaccaagacgctcat
gaagaaggataagtacacgctgcccggcgggctgctggcccccggcggcaatagcatggcga
gcggggtcggggtgggcgccggcctgggcgcgggcgtgaaccagcgcatggacagttacgcg
cacatgaacggctggagcaacggcagctacagcatgatgcaggaccagctgggcgctagcGG
ATCCaacATGAGGCCCCGGCTGAAAAACGTGGACAGGAGCACTGCACAGCAGTTGGCAGTAA
CTGTGGGCAACGTCACCGTCATTATCACAGACTTTAAGGAAAGACTCGCTCCTCATCGACA
TCCTCATCCACAGTGACCTCCAGTGCAGGGTCAGAACAGCAGAACCAGGGCGCGCCCGGTAA
GCCTATCCCTAACCCTCTCCTCGGTCTCGATTCTACGTGAattaattaagaattcgacccag
ctttcttgtacaaagtggttgatatccagcacagtggcggccgctcgagtctagagggcccg
cggttcgaaggtaagcctatccctaaccctctcctcggtctcgattctacgcgtaccggtgt
cgactctagataaagcatcaagcttatcgcgataccgtcgacaatcaacctctggattacaa
aatttgtgaaagattgactggtattcttaactatgttgctccttttacgctatgtggatacg
ctgctttaatgcctttgtatcatgctattgcttcccgtatggctttcattttctcctccttg
tataaatcctggttgctgtctctttatgaggagttgtggcccgttgtcaggcaacgtggcgt
ggtgtgcactgtgtttgctgacgcaacccccactggttggggcattgccaccacctgtcagc
 
tcctttccgggactttcgctttccccctccctattgccacggcggaactcatcgccgcctgc
cttgcccgctgctggacaggggctcggctgttgggcactgacaattccgtggtgttgtcggg
gaagctgacgtcctttccatggctgctcgcctgtgttgccacctggattctgcgcgggacgt
ccttctgctacgtcccttcggccctcaatccagcggaccttccttcccgcggcctgctgccg
gctctgcggcctcttccgcgtcttcgccttcgccctcagacgagtcggatctccctttgggc
cgcctccccgcctggaatgatatcctcgagcccggcgatatattgccttatttccgattat
tgccttatttcgcattattgccttatttctcactattgccttatttccgatcccgggttta
aaccgatcaacaaaatcactgatgctggacgatcaacaaaatcactgatgctggacgatcaa
caaaatcactgatgctggatcaccaacaaaatcactgatgctggacgatgtttaaaccctgc
aggcgatcagctatgccagcatcttgcccgatcagctatgccagcatcttgcccgatcagct
atgccagcatcttgcctcaccagctatgccagcatcttgcccgatcctgcaggagatct
**Ef1a promoter**
*hSOX2₁₋₁₇₉*
Y-R
*V5*
miR124 Target sequences
miR338-3p Target sequences
miR31 Target sequences

### SESv3 (SEQ ID NO: 157):

Ef1a:: SOX2₁₋₁₇₉-Y-R-V5 [**SESv3**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
 
CAACGGATCCACCatgtacaacatgatggagacggagctgaagccgccgggcccgcagcaa
cttcgggggcggcggcggcaactccaccgcggcggccggcggcaaccagaaaaacagc
ccggaccgcgtcaagcggcccatgaatgccttcatggtgtggtcccgcgggcagcggcgcaa
gatggcccaggagaaccccaagatgcacaactcggagatcagcaagcgcctgggcgccgagt
ggaaacttttgtcggagacggagaagcggccgttcatcgacgaggctaagcggctgcgagcg
ctgcacatgaaggagcacccggattataaataccggccccggcggaaaaccaagacgctcat
gaagaaggataagtacacgctgcccggcgggctgctggcccccggcggcaatagcatggcga
gcggggtcggggtgggcgccggcctgggcgcgggcgtgaaccagcgcatggacagttacgcg
cacatgaacggctggagcaacggcagctacagcatgatgcaggaccagctgggcgctagcGG
ATCCaacATGAGGCCCCGGCTGAAAAACGTGGACAGGAGCACTGCACAGCAGTTGGCAGTAA
CTGTGGGCAACGTCACCGTCATTATCACAGACTTTAAGGAAAAGACTCGCTCCTCATCGACA
TCCTCATCCACAGTGACCTCCAGTGCAGGGTCAGAACAGCAGAACCAGGGCGCGCCCGGTAA
GCCTATCCCTAACCCTCTCCTCGGTCTCGATTCTACGTGAattaattaagaattcgacccag
ctttcttgtacaaagtggttgatatccagcacagtggcggccgctcgagtctagagggcccg
cggttcgaaggtaagcctatccctaaccctctcctcggtctcgattctacgcgtaccggtgt
cgactctagataaagcatcaagcttatcgcgataccgtcgacaatcaacctctggattacaa
aatttgtgaaagattgactggtattcttaactatgttgctccttttacgctatgtggatacg
ctgctttaatgcctttgtatcatgctattgcttcccgtatggctttcattttctcctccttg
tataaatcctggttgctgtctctttatgaggagttgtggcccgttgtcaggcaacgtggcgt
ggtgtgcactgtgtttgctgacgcaacccccactggttggggcattgccaccacctgtcagc
tcctttccgggactttcgctttccccctccctattgccacggcggaactcatcgccgcctgc
cttgcccgctgctggacaggggctcggctgttgggcactgacaattccgtggtgttgtcggg
gaagctgacgtcctttccatggctgctcgcctgtgttgccacctggattctgcgcgggacgt
ccttctgctacgtcccttcggccctcaatccagcggaccttccttcccgcggcctgctgccg
gctctgcggcctcttccgcgtcttcgccttcgccctcagacgagtcggatctccctttgggc
cgcctccccgcctggaatgatatcctcgagcccggcgat
**Ef1a promoter**
*hSOX2₁₋₁₇₉*
Y-R
*V5*

### TES-Tmir (SEQ ID NO: 145):

Ef1a: :KRAB-hTEAD₁₋₁₆₆- DNMT3a3L-V5-Tmir [**TES-Tmir**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
 
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGGATGCTAAGTCACTGACTGCCTGGTCCCGGACACTGGTGACCTTCA
AGGATGTGTTTGTGGACTTCACCAGGGAGGAGTGGAAGCTGCTGGACACTGCTCAGCAGATC
CTGTACAGAAATGTGATGCTGGAGAACTATAAGAACCTGGTTTCCTTGGGTTATCAGCTTAC
TAAGCCAGATGTGATCCTCCGGTTGGAGAAGGGAGAAGAGCCCTGGCTGGTGGAGAGAGAAA
TTCACCAAGAGACCCATCCTGATTCAGAGACTGCATTTGAAATCAAATCATCAGTTGGCAGC
GGCGCATGCATTGAGCCCAGCAGCTGGAGCGGCAGTGAGAGCCCTGCCGAAAACATGGAAAG
GATGAGTGACTCTGCAGATAAGCCAATTGACAATGATGCAGAAGGGGTCTGGAGCCCCGACA
TCGAGCAAAGCTTTCAGGAGGCCCTGGCTATCTATCCACCATGTGGGAGGAGGAAAATCATC
TTATCAGACGAAGGCAAAATGTATGGTAGGAATGAATTGATAGCCAGATACATCAAACTCAG
GACAGGCAAGACGAGGACCAGAAAACAGGTGTCTAGTCACATTCAGGTTCTTGCCAGAAGGA
AATCTCGTGATTTTCATTCCAAGCTAAAGGATCAGACTGCAAAGGATAAGGCCCTGCAGCAC
ATGGCGGCCATGTCCTCAGCCCAGATCGTCTCGGCCACTGCCATTCATAACAAGCTGGGGCT
GCCTGGGATTCCACGCCCGACCTTCCCAGGGGCGCCGGGGTTCTGGCCGGGAATGATTCAAA
CAGGGCAGCCAGCTAGCGGCAGCGGCAACCATGATCAAGAGTTCGATCCTCCTAAAGTGTAT
CCCCCTGTCCCTGCCGAAAAGCGGAAACCGATCAGGGTTCTCAGTCTCTTCGATGGAATTGC
CACAGGCCTCTTGGTCCTTAAGGACCTGGGCATCCAAGTgGAcCGCTACATCGCCTCAGAAG
TATGTGAgGACAGCATCACAGTCGGCATGGTGCGCCACCAGGGGAAAATAATGTACGTCGGC
GACGTCAGATCAGTTACTCAGAAACATATTCAGGAATGGGGGCCTTTTGATCTCGTAATTGG
CGGCAGCCCCTGCAATGATCTGAGTATTGTGAACCCCGCCCGGAAAGGTTTGTACGAAGGTA
CGGGCAGACTGTTCTTCGAGTTTTACAGACTGTTGCACGACGCACGACCAAAGGAGGGTGAc
GAtcGACCCTTCTTCTGGCTGTTCGAGAACGTGGTCGCTATGGGCGTGTCTGACAAGCGGGA
CATTTCtAGATTCCTGGAGAGCAATCCAGTGATGATTGATGCAAAGGAAGTAtccGCTGCCC
ACCGCGCCAGATACTTCTGGGGCAATCTGCCCGGCATGAATCGACCCTTGGCAAGCACCGTG
AATGATAAGTTGGAGTTGCAGGAGTGTCTgGAGCATGGGCGGATCGCAAAGTTTAGCAAGGT
GCGGACCATCACGACCCGAAGCAATTCAATTAAGCAGGGAAAGGACCAACATTTTCCAGTGT
TTATGAACGAGAAAGAGGAcATCCTGTGGTGcACAGAAATGGAGCGGGTTTTCGGATTCCCC
GTACATTATACTGATGTGTCCAATATGTCTAGGTTGGCTAGACAGAGGCTGtTGGGACGCTC
ATGGAGTGTTCCTGTCATCCGCCACCTGTTCGCTCCTCTGAAGGAGTATTTCGCcTGCGTTtT
CATCCGGgAATTCAAACGCAAACAGCAGAGGCCCATCCTTTTCTTCCGGCCTGGTgCCACTT
AGTCTGCGCGGCTCTCACATGGGACCTATGGAAATATACAAAACAGTGAGCGCCTGGAAGAG
GCAGCCTGTGCGGGTCCTGAGTCTGTTCAGAAACATTGACAAGGTGCTTAAGTCCCTTGGAT
TTCTGGAGTCTGGCAGCGGTTCTGGGGGTGgcACCCTTAAGTATGTGGAGGACGTGACAAAC
GTCGTGAGGAGAGATGTGGAGAAATGGGGGCCATTTGACCTGGTATACGGGAGCACTCAACC
TCTCGGTTCTTCTTGTGATCGCTGCCCAGGGTGGTATATGTTTCAGTTCCATCGcATCCTGC
AATACGCCCTTCCGCGGCAGGAGAGTCAGCGACCATTCTTCTGGATATTCATGGACAATCTC
CTGCTGACAGAgGACGACCAAGAGACTACGACTAGATTTCTTCAGACCGAGGCCGTTACTCT
CCAGGACGTTAGAGGTAGGGACTACCAGAATGCCATGCGAGTGTGGAGTAACATCCCCGGAC
TCAAGTCAAAGCAcGCACCCCTGACCCCCAAGGAAGAGGAATACCTcCAGGCGCAGGTGAGG
 
AGCCGCAGTAAGCTCGATGCTCCTAAAGTCGATTTGTTGGTGAAGAATTGCTTGCTGCCCCT
GAGAGAGTACTTTAAGTACTTTAGCCAGAACAGCTTGCCACTGGGCGCGCCCGGTAAGCCTA
TCCCTAACCCTCTCCTCGGTCTCGATTCTACGTGAattaattaagaattcgacccagctttc
ttgtacaaagtggttgatatccagcacagtggcggccgctcgagtctagagggcccgcggtt
cgaaggtaagcctatccctaaccctctcctcggtctcgattctacgcgtaccggtgtcgact
ctagataaagcatcaagcttatcgcgataccgtcgacaatcaacctctggattacaaaattt
gtgaaagattgactggtattcttaactatgttgctccttttacgctatgtggatacgctgct
ttaatgcctttgtatcatgctattgcttcccgtatggctttcattttctcctccttgtataa
atcctggttgctgtctctttatgaggagttgtggcccgttgtcaggcaacgtggcgtggtgt
gcactgtgtttgctgacgcaacccccactggttggggcattgccaccacctgtcagctcctt
tccgggactttcgctttccccctccctattgccacggcggaactcatcgccgcctgccttgc
ccgctgctggacaggggctcggctgttgggcactgacaattccgtggtgttgtcggggaagc
tgacgtcctttccatggctgctcgcctgtgttgccacctggattctgcgcgggacgtccttc
tgctacgtcccttcggccctcaatccagcggaccttccttcccgcggcctgctgccggctct
gcggcctcttccgcgtcttcgccttcgccctcagacgagtcggatctccctttgggccgcct
ccccgcctggaatgatatcctcgagcccgggcgatatattgccttatttccgattattgcct
tatttcgcattattgccttatttctcactattgccttatttccgatcccgggtttaaaccg
atcaacaaaatcactgatgctggacgatcaacaaaatcactgatgctggacgatcaacaaaa
tcactgatgctggatcaccaacaaaatcactgatgctggacgatgtttaaaccctgcaggcg
atcagctatgccagcatcttgcccgatcagctatgccagcatcttgcccgatcagctatgcc
agcatcttgccctcaccagctatgccagcatcttgcccgatcctgcaggagatct
**Ef1a promoter**
KRAB
*hTEAD₁₋₁₆₆*
**DNMT3a3L**
*V5*
miR124 Target sequences
miR338-3p Target sequences
miR31 Target sequences

### TES (SEQ ID NO: 153):

Ef1a: :KRAB-hTEAD₁₋₁₆₆- DNMT3a3L-V5 [**TES**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
 
GTCTCGCTGCTTTCGATAAGTCTCTAGCCATTTAAAATTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGGATGCTAAGTCACTGACTGCCTGGTCCCGGACACTGGTGACCTTCA
AGGATGTGTTTGTGGACTTCACCAGGGAGGAGTGGAAGCTGCTGGACACTGCTCAGCAGATC
CTGTACAGAAATGTGATGCTGGAGAACTATAAGAACCTGGTTTCCTTGGGTTATCAGCTTAC
TAAGCCAGATGTGATCCTCCGGTTGGAGAAGGGAGAAGAGCCCTGGCTGGTGGAGAGAGAAA
TTCACCAAGAGACCCATCCTGATTCAGAGACTGCATTTGAAATCAAATCATCAGTTGGCAGC
GGCGCATGCATTGAGCCCAGCAGCTGGAGCGGCAGTGAGAGCCCTGCCGAAAACATGGAAAG
GATGAGTGACTCTGCAGATAAGCCAATTGACAATGATGCAGAAGGGGTCTGGAGCCCCGACA
TCGAGCAAAGCTTTCAGGAGGCCCTGGCTATCTATCCACCATGTGGGAGGAGGAAAATCATC
TTATCAGACGAAGGCAAAATGTATGGTAGGAATGAATTGATAGCCAGATACATCAAACTCAG
GACAGGCAAGACGAGGACCAGAAAACAGGTGTCTAGTCACATTCAGGTTCTTGCCAGAAGGA
AATCTCGTGATTTTCATTCCAAGCTAAAGGATCAGACTGCAAAGGATAAGGCCCTGCAGCAC
ATGGCGGCCATGTCCTCAGCCCAGATCGTCTCGGCCACTGCCATTCATAACAAGCTGGGGCT
GCCTGGGATTCCACGCCCGACCTTCCCAGGGGCGCCGGGGTTCTGGCCGGGAATGATTCAAA
CAGGGCAGCCAGCTAGCGGCAGCGGCAACCATGATCAAGAGTTCGATCCTCCTAAAGTGTAT
CCCCCTGTCCCTGCCGAAAAGCGGAAACCGATCAGGGTTCTCAGTCTCTTCGATGGAATTGC
CACAGGCCTCTTGGTCCTTAAGGACCTGGGCATCCAAGTgGAcCGCTACATCGCCTCAGAAG
TATGTGAgGACAGCATCACAGTCGGCATGGTGCGCCACCAGGGGAAAATAATGTACGTCGGC
GACGTCAGATCAGTTACTCAGAAACATATTCAGGAATGGGGGCCTTTTGATCTCGTAATTGG
CGGCAGCCCCTGCAATGATCTGAGTATTGTGAACCCCGCCCGGAAAGGTTTGTACGAAGGTA
CGGGCAGACTGTTCTTCGAGTTTTACAGACTGTTGCACGACGCACGACCAAAGGAGGGTGAc
GAtcGACCCTTCTTCTGGCTGTTCGAGAACGTGGTCGCTATGGGCGTGTCTGACAAGCGGGA
CATTTCtAGATTCCTGGAGAGCAATCCAGTGATGATTGATGCAAAGGAAGTAtccGCTGCCC
ACCGCGCCAGATACTTCTGGGGCAATCTGCCCGGCATGAATCGACCCTTGGCAAGCACCGTG
AATGATAAGTTGGAGTTGCAGGAGTGTCTgGAGCATGGGCGGATCGCAAAGTTTAGCAAGGT
GCGGACCATCACGACCCGAAGCAATTCAATTAAGCAGGGAAAGGACCAACATTTTCCAGTGT
TTATGAACGAGAAAGAGGAcATCCTGTGGTGcACAGAAATGGAGCGGGTTTTCGGATTCCCC
GTACATTATACTGATGTGTCCAATATGTCTAGGTTGGCTAGACAGAGGCTGtTGGGACGCTC
ATGGAGTGTTCCTGTCATCCGCCACCTGTTCGCTCCTCTGAAGGAGTATTTCGCcTGCGTtT
CATCCGGgAATTCAAACGCAAACAGCAGAGGCCCATCCTTTTCTTCCGGCCTGGTgCCACTT
AGTCTGCGCGGCTCTCACATGGACCTATGGAAATATACAAAACAGTGAGCGCCTGGAAGAG
GCAGCCTGTGCGGGTCCTGAGTCTGTTCAGAAACATTGACAAGGTGCTTAAGTCCCTTGGAT
TTCTGGAGTCTGGCAGCGGTTCTGGGGGTGgcACCCTTAAGTATGTGGAGGACGTGACAAAC
GTCGTGAGGAGAGATGTGGAGAAATGGGGGCCATTTGACCTGGTATACGGGAGCACTCAACC
TCTCGGTTCTTCTTGTGATCGCTGCCCAGGGTGGTATATGTTTCAGTTCCATCGcATCCTGC
AATACGCCCTTCCGCGGCAGGAGAGTCAGCGACCATTCTTCTGGATATTCATGGACAATCTC
CTGCTGACAGAgGACGACCAAGAGACTACGACTAGATTTCTTCAGACCGAGGCCGTTACTCT
 
CCAGGACGTTAGAGGTAGGGACTACCAGAATGCCATGCGAGTGTGGAGTAACATCCCCGGAC
TCAAGTCAAAGCAcGCACCCCTGACCCCCAAGGAAGAGGAATACCTcCAGGCGCAGGTGAGG
AGCCGCAGTAAGCTCGATGCTCCTAAAGTCGATTTGTTGGTGAAGAATTGCTTGCTGCCCCT
GAGAGAGTACTTTAAGTACTTTAGCCAGAACAGCTTGCCACTGGGCGCGCCCGGTAAGCCTA
TCCCTAACCCTCTCCTCGGTCTCGATTCTACGTGAattaattaagaattcgacccagctttc
ttgtacaaagtggttgatatccagcacagtggcggccgctcgagtctagagggcccgcggtt
cgaaggtaagcctatccctaaccctctcctcggtctcgattctacgcgtaccggtgtcgact
ctagataaagcatcaagcttatcgcgataccgtcgacaatcaacctctggattacaaaattt
gtgaaagattgactggtattcttaactatgttgctccttttacgctatgtggatacgctgct
ttaatgcctttgtatcatgctattgcttcccgtatggctttcattttctcctccttgtataa
atcctggttgctgtctctttatgaggagttgtggcccgttgtcaggcaacgtggcgtggtgt
gcactgtgtttgctgacgcaacccccactggttggggcattgccaccacctgtcagctcctt
tccgggactttcgctttccccctccctattgccacggcggaactcatcgccgcctgccttgc
ccgctgctggacaggggctcggctgttgggcactgacaattccgtggtgttgtcggggaagc
tgacgtcctttccatggctgctcgcctgtgttgccacctggattctgcgcgggacgtccttc
tgctacgtcccttcggccctcaatccagcggaccttccttcccgcggcctgctgccggctct
gcggcctcttccgcgtcttcgccttcgccctcagacgagtcggatctccctttgggccgcct
ccccgcctggaatgatatcctcgagcccgggcgat
**Ef1a promoter**
KRAB
*hTEAD₁₋₁₆₆*
**DNMT3a3L**
*V5*

### TESv2-Tmir (SEQ ID NO: 146):

Ef1a::CS- *hTEAD₁₋₁₆₆* -V5 -Tmir [**TESv2-Tmir**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
 
CTCGAGCTTTTGGAGTACGTCGTCTTTAGGTTGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGACCATTGAGCCCAGCAGCTGGAGCGGCAGTGAGAGCCCTGCCGAAAACATGGAAA
GGATGAGTGACTCTGCAGATAAGCCAATTGACAATGATGCAGAAGGGGTCTGGAGCCCCGAC
ATCGAGCAAAGCTTTCAGGAGGCCCTGGCTATCTATCCACCATGTGGGAGGAGGAAAATCAT
CTTATCAGACGAAGGCAAAATGTATGGTAGGAATGAATTGATAGCCAGATACATCAAACTCA
GGACAGGCAAGACGAGGACCAGAAAACAGGTGTCTAGTCACATTCAGGTTCTTGCCAGAAGG
AAATCTCGTGATTTTCATTCCAAGCTAAAGGATCAGACTGCAAAGGATAAGGCCCTGCAGCA
CATGGCGGCCATGTCCTCAGCCCAGATCGTCTCGGCCACTGCCATTCATAACAAGCTGGGGC
TGCCTGGGATTCCACGCCCGACCTTCCCAGGGGCGCCGGGGTTCTGGCCGGGAATGATTCAA
ACAGGGCAGCCAGGCGCGCCCGGTAAGCCTATCCCTAACCCTCTCCTCGGTCTCGATTCTAC
GTGAattaattaagaattcgacccagctttcttgtacaaagtggttgatatccagcacagtg
gcggccgctcgagtctagagggcccgcggttcgaaggtaagcctatccctaaccctctcctc
ggtctcgattctacgcgtaccggtgtcgactctagataaagcatcaagcttatcgcgatacc
gtcgacaatcaacctctggattacaaaatttgtgaaagattgactggtattcttaactatgt
tgctccttttacgctatgtggatacgctgctttaatgcctttgtatcatgctattgcttccc
gtatggctttcattttctcctccttgtataaatcctggttgctgtctctttatgaggagttg
tggcccgttgtcaggcaacgtggcgtggtgtgcactgtgtttgctgacgcaacccccactgg
ttggggcattgccaccacctgtcagctcctttccgggactttcgctttccccctccctattg
ccacggcggaactcatcgccgcctgccttgcccgctgctggacaggggctcggctgttgggc
actgacaattccgtggtgttgtcggggaagctgacgtcctttccatggctgctcgcctgtgt
tgccacctggattctgcgcgggacgtccttctgctacgtcccttcggccctcaatccagcgg
accttccttcccgcggcctgctgccggctctgcggcctcttccgcgtcttcgccttcgccct
cagacgagtcggatctccctttgggccgcctccccgcctggaatgatatcctcgagcccggg
cgatatattgccttatttccgattattgccttatttcgcattattgccttatttctcactat
tgccttatttccgatcccgggtttaaaccgatcaacaaaatcactgatgctggacgatcaa
caaaatcactgatgctggacgatcaacaaaatcactgatgctggatcaccaacaaaatcact
gatgctggacgatgtttaaaccctgcaggcgatcagctatgccagcatcttgcccgatcagc
tatgccagcatcttgcccgatcagctatgccagcatcttgcctcaccagctatgccagcatc
ttgccccgatcctgcaggagatct
**Ef1a promoter**
CS
*hTEAD₁₋₁₆₆*
*V5*
miR124 Target sequences
miR338-3p Target sequences
miR31 Target sequences

### TESv3-Tmir (SEQ ID NO: 147):

Ef1a: :*hTEAD₁₋₁₆₆-YR*-V5-Tmir [**TESv3-Tmir**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGATTGAGCCCAGCAGCTGGAGCGGCAGTGAGAGCCCTGCCGAAAACA
TGGAAAGGATGAGTGACTCTGCAGATAAGCCAATTGACAATGATGCAGAAGGGGTCTGGAGC
CCCGACATCGAGCAAAGCTTTCAGGAGGCCCTGGCTATCTATCCACCATGTGGGAGGAGGAA
AATCATCTTATCAGACGAAGGCAAAATGTATGGTAGGAATGAATTGATAGCCAGATACATCA
AACTCAGGACAGGCAAGACGAGGACCAGAAAACAGGTGTCTAGTCACATTCAGGTTCTTGCC
AGAAGGAAATCTCGTGATTTCATTCCAAGCTAAAGGATCAGACTGCAAAGGATAAGGCCCT
GCAGCACATGGCGGCCATGTCCTCAGCCCAGATCGTCTCGGCCACTGCCATTCATAACAAGC
TGGGGCTGCCTGGGATTCCACGCCCGACCTTCCCAGGGGCGCCGGGGTTCTGGCCGGAATG
ATTCAAACAGGGCAGCCAaccATGAGGCCCCGGCTGAAAAACGTGGACAGGAGCACTGCACA
GCAGTTGGCAGTAACTGTGGGCAACGTCACCGTCATTATCACAGACTTTAAGGAAAAGACTC
GCTCCTCATCGACATCCTCATCCACAGTGACCTCCAGTGCAGGGTCAGAACAGCAGAACCAG
GGCGCGCCCGGTAAGCCTATCCCTAACCCTCTCCTCGGTCTCGATTCTACGTGAattaatta
agaattcgacccagctttcttgtacaaagtggttgatatccagcacagtggcggccgctcga
gtctagagggcccgcggttcgaaggtaagcctatccctaaccctctcctcggtctcgattct
acgcgtaccggtgtcgactctagataaagcatcaagcttatcgcgataccgtcgacaatcaa
cctctggattacaaaatttgtgaaagattgactggtattcttaactatgttgctccttttac
gctatgtggatacgctgctttaatgcctttgtatcatgctattgcttcccgtatggctttca
ttttctcctccttgtataaatcctggttgctgtctctttatgaggagttgtggcccgttgtc
aggcaacgtggcgtggtgtgcactgtgtttgctgacgcaacccccactggttggggcattgc
caccacctgtcagctcctttccgggactttcgctttccccctccctattgccacggcggaac
tcatcgccgcctgccttgcccgctgctggacaggggctcggctgttgggcactgacaattcc
gtggtgttgtcggggaagctgacgtcctttccatggctgctcgcctgtgttgccacctggat
tctgcgcgggacgtccttctgctacgtcccttcggccctcaatccagcggaccttccttccc
 
gcggcctgctgccggctctgcggcctcttccgcgtcttcgccttcgccctcagacgagtcgg
atctccctttgggccgcctccccgcctggaatgatatcctcgagcccgggcgatatattgcc
ttatttccgattattgccttatttcgcattattgccttatttctcactattgccttatttcc
gatcccggggtttaaaccgatcaacaaaatcactgatgctggacgatcaacaaaatcactga
tgctggacgatcaacaaaatcactgatgctggatcaccaacaaaatcactgatgctggacga
tgtttaaaccctgcaggcgatcagctatgccagcatcttgcccgatcagctatgccagcatc
ttgcccgatcagctatgccagcatcttgcctcaccagctatgccagcatcttgcccgatcct
gcaggagatct
**Ef1a promoter**
*hTEAD₁₋₁₆₆*
*Y-R*
*V5*
miR124 Target sequences
miR338-3p Target sequences
miR31 Target sequences

### TESv4-Tmir (SEQ ID NO: 148):

Ef1a::CS- *hTEAD₁₋₁₆₆* - Y-R-V5-Tmir [**TESv4-Tmir**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
 
TCCTGAGACCATTGAGCCCAGCAGCTGGAGCGGCAGTGAGAGCCCTGCCGAAAACATGGAAA
GGATGAGTGACTCTGCAGATAAGCCAATTGACAATGATGCAGAAGGGGTCTGGAGCCCCGAC
ATCGAGCAAAGCTTTCAGGAGGCCCTGGCTATCTATCCACCATGTGGGAGGAGGAAAATCAT
CTTATCAGACGAAGGCAAAATGTATGGTAGGAATGAATTGATAGCCAGATACATCAAACTCA
GGACAGGCAAGACGAGGACCAGAAAACAGGTGTCTAGTCACATTCAGGTTCTTGCCAGAAGG
AAATCTCGTGATTTTCATTCCAAGCTAAAGGATCAGACTGCAAAGGATAAGGCCCTGCAGCA
CATGGCGGCCATGTCCTCAGCCCAGATCGTCTCGGCCACTGCCATTCATAACAAGCTGGGGC
TGCCTGGGATTCCACGCCCGACCTTCCCAGGGGCGCCGGGGTTCTGGCCGGAATGATTCAA
ACAGGGCAGCCAACCatgaggccccggctgaaaaacgtggacaggagcactgcacagcagtt
ggcagtaactgtgggcaacgtcaccgtcattatcacagactttaaggaaaagactcgctcct
catcgacatcctcatccacagtgacctccagtgcaggtcagaacagcagaaccagGGCGCG
CCCGGTAAGCCTATCCCTAACCCTCTCCTCGGTCTCGATTCTACGTGAattaattaagaatt
cgacccagctttcttgtacaaagtggttgatatccagcacagtggcggccgctcgagtctag
agggcccgcggttcgaaggtaagcctatccctaaccctctcctcggtctcgattctacgcgt
accggtgtcgactctagataaagcatcaagcttatcgcgataccgtcgacaatcaacctctg
gattacaaaatttgtgaaagattgactggtattcttaactatgttgctccttttacgctatg
tggatacgctgctttaatgcctttgtatcatgctattgcttcccgtatggctttcattttct
cctccttgtataaatcctggttgctgtctctttatgaggagttgtggcccgttgtcaggcaa
cgtggcgtggtgtgcactgtgtttgctgacgcaacccccactggttggggcattgccaccac
ctgtcagctcctttccgggactttcgctttccccctccctattgccacggcggaactcatcg
ccgcctgccttgcccgctgctggacaggggctcggctgttgggcactgacaattccgtggtg
ttgtcggggaagctgacgtcctttccatggctgctcgcctgtgttgccacctggattctgcg
cgggacgtccttctgctacgtcccttcggccctcaatccagcggaccttccttcccgcggcc
tgctgccggctctgcggcctcttccgcgtcttcgccttcgccctcagacgagtcggatctcc
ctttgggccgcctccccgcctggaatgatatcctcgagcccgggcgatatattgccttattt
ccgattattgccttatttcgcattattgccttatttctcactattgccttatttccgatccc
ggggtttaaaccgatcaacaaaatcactgatgctggacgatcaacaaaatcactgatgctgg
acgatcaacaaaatcactgatgctggatcaccaacaaaatcactgatgctggacgatgttta
aaccctgcaggcgatcagctatgccagcatcttgcccgatcagctatgccagcatcttgccc
gatcagctatgccagcatcttgcctcaccagctatgccagcatcttgcccgatcctgcagga
gatct
 **Ef1a promoter**
CS
*hTEAD₁₋₁₆₆*
*y*-*r*
*V5*
miR124 Target sequences
miR338-3p Target sequences
miR31 Target sequences

### MES-Tmir (SEQ ID NO: 149):

Ef1a::KRAB-DNMT3a3L-hMYC₁₄₄₋₄₅₄-V5 -Tmir [**MES-Tmir**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGGATGCTAAGTCACTGACTGCCTGGTCCCGGACACTGGTGACCTTCA
AGGATGTGTTTGTGGACTTCACCAGGGAGGAGTGGAAGCTGCTGGACACTGCTCAGCAGATC
CTGTACAGAAATGTGATGCTGGAGAACTATAAGAACCTGGTTTCCTTGGGTTATCAGCTTAC
TAAGCCAGATGTGATCCTCCGGTTGGAGAAGGGAGAAGAGCCCTGGCTGGTGGAGAGAGAAA
TTCACCAAGAGACCCATCCTGATTCAGAGACTGCATTTGAAATCAAATCATCAGTTGCTAGC
GGCAGCGGCAACCATGATCAAGAGTTCGATCCTCCTAAAGTGTATCCCCCTGTCCCTGCCGA
AAAGCGGAAACCGATCAGGGTTCTCAGTCTCTTCGATGGAATTGCCACAGGCCTCTTGGTCC
TTAAGGACCTGGGCATCCAAGTgGAcCGCTACATCGCCTCAGAAGTATGTGAgGACAGCATC
ACAGTCGGCATGGTGCGCCACCAGGGGAAAATAATGTACGTCGGCGACGTCAGATCAGTTAC
TCAGAAACATATTCAGGAATGGGGGCCTTTTGATCTCGTAATTGGCGGCAGCCCCTGCAATG
ATCTGAGTATTGTGAACCCCGCCCGGAAAGGTTTGTACGAAGGTACGGCAGACTGTTCTTC
GAGTTTTACAGACTGTTGCACGACGCACGACCAAAGGAGGGTGAcGAtcGACCCTTCTTCTG
GCTGTTCGAGAACGTGGTCGCTATGGGCGTGTCTGACAAGCGGGACATTTCtAGATTCCTGG
AGAGCAATCCAGTGATGATTGATGCAAAGGAAGTAtccGCTGCCCACCGCGCCAGATACTTC
TGGGGCAATCTGCCCGGCATGAATCGACCCTTGGCAAGCACCGTGAATGATAAGTTGGAGTT
GCAGGAGTGTCTgGAGCATGGCGGATCGCAAAGTTTAGCAAGGTGCGGACCATCACGACCC
GAAGCAATTCAATTAAGCAGGGAAAGGACCAACATTTTCCAGTGTTTATGAACGAGAAAGAG
GAcATCCTGTGGTGcACAGAAATGGAGCGGGTTTTCGGATTCCCCGTACATTATACTGATGT
GTCCAATATGTCTAGGTTGGCTAGACAGAGGCTGtTGGGACGCTCATGGAGTGTTCCTGTCA
TCCGCCACCTGTTCGCTCCTCTGAAGGAGTATTTCGCcTGCGTtTCATCCGGgAATTCAAAC
GCAAACAGCAGAGGCCCATCCTTTTCTTCCGGCCTGGTgCCACTTAGTCTGCGCGGCTCTCA
CATGGGACCTATGGAAATATACAAAACAGTGAGCGCCTGGAAGAGGCAGCCTGTGCGGGTCC
TGAGTCTGTTCAGAAACATTGACAAGGTGCTTAAGTCCCTTGGATTTCTGGAGTCTGGCAGC
GGTTCTGGGGGTGgcACCCTTAAGTATGTGGAGGACGTGACAAACGTCGTGAGGAGAGATGT
GGAGAAATGGGGGCCATTTGACCTGGTATACGGGAGCACTCAACCTCTCGGTTCTTCTTGTG
ATCGCTGCCCAGGGTGGTATATGTTTCAGTTCCATCGcATCCTGCAATACGCCCTTCCGCGG
CAGGAGAGTCAGCGACCATTCTTCTGGATATTCATGGACAATCTCCTGCTGACAGAgGACGA
 
CCAAGAGACTACGACTAGATTTCTTCAGACCGAGGCCGTTACTCTCCAGGACGTTAGAGGTA
GGGACTACCAGAATGCCATGCGAGTGTGGAGTAACATCCCCGGACTCAAGTCAAAGCAcGCA
CCCCTGACCCCCAAGGAAGAGGAATACCTcCAGGCGCAGGTGAGGAGCCGCAGTAAGCTCGA
TGCTCCTAAAGTCGATTTGTTGGTGAAGAATTGCTTGCTGCCCCTGAGAGAGTACTTTAAGT
ACTTTAGCCAGAACAGCTTGCCACTGGGCGCGCCCGCCGCTGCCATCATCCAGGACTGTATG
TGGAGCGGCTTCTCGGCCGCCGCCAAGCTCGTCTCAGAGAAGCTGGCCTCCTACCAGGCTGC
GCGCAAAGACAGCGGCAGCCCGAACCCCGCCCGCGGCCACAGCGTCTGCTCCACCTCCAGCT
TGTACCTGCAGGATCTGAGCGCCGCCGCCTCAGAGTGCATCGACCCCTCGGTGGTCTTCCCC
TACCCTCTCAACGACAGCAGCTCGCCCAAGTCCTGCGCCTCGCAAGACTCCAGCGCCTTCTC
TCCGTCCTCGGATTCTCTGCTCTCCTCGACGGAGTCCTCCCCGCAGGGCAGCCCCGAGCCCC
TGGTGCTCCATGAGGAGACACCGCCCACCACCAGCAGCGACTCTGAGGAGGAACAAGAAGAT
GAGGAAGAAATCGATGTTGTTTCTGTGGAAAAGAGGCAGGCTCCTGGCAAAAGGTCAGAGTC
TGGATCACCTTCTGCTGGAGGCCACAGCAAACCTCCTCACAGCCCACTGGTCCTCAAGAGGT
GCCACGTCTCCACACATCAGCACAACTACGCAGCGCCTCCCTCCACTCGGAAGGACTATCCT
GCTGCCAAGAGGGTCAAGTTGGACAGTGTCAGAGTCCTGAGACAGATCAGCAACAACCGAAA
ATGCACCAGCCCCAGGTCCTCGGACACCGAGGAGAATGTCAAGAGGCGAACACACAACGTCT
TGGAGCGCCAGAGGAGGAACGAGCTAAAACGGAGCTTTTTTGCCCTGCGTGACCAGATCCCG
GAGTTGGAAAACAATGAAAAGGCCCCCAAGGTAGTTATCCTTAAAAAAGCCACAGCATACAT
CCTGTCCGTCCAAGCAGAGGAGCAAAAGCTCATTTCTGAAGAGGACTTGTTGCGGAAACGAC
GAGAACAGTTGAAACACAAACTTGAACAGCTACGGAACTCTTGTGCGGCTAGCGGCGCGCCC
GGTAAGCCTATCCCTAACCCTCTCCTCGGTCTCGATTCTACGTGAattaattaagaattcga
cccagctttcttgtacaaagtggttgatatccagcacagtggcggccgctcgagtctagagg
gcccgcggttcgaaggtaagcctatccctaaccctctcctcggtctcgattctacgcgtacc
ggtgtcgactctagataaagcatcaagcttatcgcgataccgtcgacaatcaacctctggat
tacaaaatttgtgaaagattgactggtattcttaactatgttgctccttttacgctatgtgg
atacgctgctttaatgcctttgtatcatgctattgcttcccgtatggctttcattttctcct
ccttgtataaatcctggttgctgtctctttatgaggagttgtggcccgttgtcaggcaacgt
ggcgtggtgtgcactgtgtttgctgacgcaacccccactggttggggcattgccaccacctg
tcagctcctttccgggactttcgctttccccctccctattgccacggcggaactcatcgccg
cctgccttgcccgctgctggacaggggctcggctgttgggcactgacaattccgtggtgttg
tcggggaagctgacgtcctttccatggctgctcgcctgtgttgccacctggattctgcgcgg
gacgtccttctgctacgtcccttcggccctcaatccagcggaccttccttcccgcggcctgc
tgccggctctgcggcctcttccgcgtcttcgccttcgccctcagacgagtcggatctccctt
tgggccgcctccccgcctggaatgatatcctcgagcccgggcgatatattgccttatttccg
attattgccttatttcgcattattgccttatttctcactattgccttatttccgatcccggg
gtttaaaccgatcaacaaaatcactgatgctggacgatcaacaaaatcactgatgctggacg
atcaacaaaatcactgatgctggatcaccaacaaaatcactgatgctggacgatgtttaaac
cctgcaggcgatcagctatgccagcatcttgcccgatcagctatgccagcatcttgcccgat
cagctatgccagcatcttgcctcaccagctatgccagcatcttgcccgatcctgcaggagat
ct
**Ef1a promoter**
KRAB
**DNMT3a3L**
*hMYC₁₄₄₋₄₅₄*
*V5*
miR124 Target sequences
miR338-3p Target sequences
miR31 Target sequences

### MES (SEQ ID NO: 154):

Ef1a: :KRAB-DNMT3a3L-hMYC₁₄₄₋₄₅₄-V5 [**MES**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGGATGCTAAGTCACTGACTGCCTGGTCCCGGACACTGGTGACCTTCA
AGGATGTGTTTGTGGACTTCACCAGGGAGGAGTGGAAGCTGCTGGACACTGCTCAGCAGATC
CTGTACAGAAATGTGATGCTGGAGAACTATAAGAACCTGGTTTCCTTGGGTTATCAGCTTAC
TAAGCCAGATGTGATCCTCCGGTTGGAGAAGGGAGAAGAGCCCTGGCTGGTGGAGAGAGAAA
TTCACCAAGAGACCCATCCTGATTCAGAGACTGCATTTGAAATCAAATCATCAGTTGCTAGC
GGCAGCGGCAACCATGATCAAGAGTTCGATCCTCCTAAAGTGTATCCCCCTGTCCCTGCCGA
AAAGCGGAAACCGATCAGGGTTCTCAGTCTCTTCGATGGAATTGCCACAGGCCTCTTGGTCC
TTAAGGACCTGGGCATCCAAGTgGAcCGCTACATCGCCTCAGAAGTATGTGAgGACAGCATC
ACAGTCGGCATGGTGCGCCACCAGGGGAAAATAATGTACGTCGGCGACGTCAGATCAGTTAC
TCAGAAACATATTCAGGAATGGGGCCTTTTGATCTCGTAATTGGCGGCAGCCCCTGCAATG
ATCTGAGTATTGTGAACCCCGCCCGGAAAGGTTTGTACGAAGGTACGGGCAGACTGTTCTTC
GAGTTTTACAGACTGTTGCACGACGCACGACCAAAGGAGGGTGAcGAtcGACCCTTCTTCTG
GCTGTTCGAGAACGTGGTCGCTATGGGCGTGTCTGACAAGCGGGACATTTCtAGATTCCTGG
AGAGCAATCCAGTGATGATTGATGCAAAGGAAGTAtccGCTGCCCACCGCGCCAGATACTTC
TGGGGCAATCTGCCCGGCATGAATCGACCCTTGGCAAGCACCGTGAATGATAAGTTGGAGTT
GCAGGAGTGTCTgGAGCATGGCGGATCGCAAAGTTTAGCAAGGTGCGGACCATCACGACCC
GAAGCAATTCAATTAAGCAGGGAAAGGACCAACATTTTCCAGTGTTTATGAACGAGAAAGAG
GAcATCCTGTGGTGcACAGAAATGGAGCGGGTTTTCGGATTCCCCGTACATTATACTGATGT
 
GTCCAATATGTCTAGGTTGGCTAGACAGAGGCTGtTGGACGCTCATGGAGTGTTCCTGTCA
TCCGCCACCTGTTCGCTCCTCTGAAGGAGTATTTCGCcTGCGTtTCATCCGGgAATTCAAAC
GCAAACAGCAGAGGCCCATCCTTTTCTTCCGGCCTGGTgCCACTTAGTCTGCGCGGCTCTCA
CATGGGACCTATGGAAATATACAAAACAGTGAGCGCCTGGAAGAGGCAGCCTGTGCGGGTCC
TGAGTCTGTTCAGAAACATTGACAAGGTGCTTAAGTCCCTTGGATTTCTGGAGTCTGGCAGC
GGTTCTGGGGGTGgcACCCTTAAGTATGTGGAGGACGTGACAAACGTCGTGAGGAGAGATGT
GGAGAAATGGGGGCCATTTGACCTGGTATACGGAGCACTCAACCTCTCGGTTCTTCTTGTG
ATCGCTGCCCAGGGTGGTATATGTTTCAGTTCCATCGcATCCTGCAATACGCCCTTCCGCGG
CAGGAGAGTCAGCGACCATTCTTCTGGATATTCATGGACAATCTCCTGCTGACAGAgGACGA
CCAAGAGACTACGACTAGATTTCTTCAGACCGAGGCCGTTACTCTCCAGGACGTTAGAGGTA
GGGACTACCAGAATGCCATGCGAGTGTGGAGTAACATCCCCGGACTCAAGTCAAAGCAcGCA
CCCCTGACCCCCAAGGAAGAGGAATACCTcCAGGCGCAGGTGAGGAGCCGCAGTAAGCTCGA
TGCTCCTAAAGTCGATTTGTTGGTGAAGAATTGCTTGCTGCCCCTGAGAGAGTACTTTAAGT
ACTTTAGCCAGAACAGCTTGCCACTGGGCGCGCCCGCCGCTGCCATCATCCAGGACTGTATG
TGGAGCGGCTTCTCGGCCGCCGCCAAGCTCGTCTCAGAGAAGCTGGCCTCCTACCAGGCTGC
GCGCAAAGACAGCGGCAGCCCGAACCCCGCCCGCGGCCACAGCGTCTGCTCCACCTCCAGCT
TGTACCTGCAGGATCTGAGCGCCGCCGCCTCAGAGTGCATCGACCCCTCGGTGGTCTTCCCC
TACCCTCTCAACGACAGCAGCTCGCCCAAGTCCTGCGCCTCGCAAGACTCCAGCGCCTTCTC
TCCGTCCTCGGATTCTCTGCTCTCCTCGACGGAGTCCTCCCCGCAGGGCAGCCCCGAGCCCC
TGGTGCTCCATGAGGAGACACCGCCCACCACCAGCAGCGACTCTGAGGAGGAACAAGAAGAT
GAGGAAGAAATCGATGTTGTTTCTGTGGAAAAGAGGCAGGCTCCTGGCAAAAGGTCAGAGTC
TGGATCACCTTCTGCTGGAGGCCACAGCAAACCTCCTCACAGCCCACTGGTCCTCAAGAGGT
GCCACGTCTCCACACATCAGCACAACTACGCAGCGCCTCCCTCCACTCGGAAGGACTATCCT
GCTGCCAAGAGGGTCAAGTTGGACAGTGTCAGAGTCCTGAGACAGATCAGCAACAACCGAAA
ATGCACCAGCCCCAGGTCCTCGGACACCGAGGAGAATGTCAAGAGGCGAACACACAACGTCT
TGGAGCGCCAGAGGAGGAACGAGCTAAAACGGAGCTTTTTTGCCCTGCGTGACCAGATCCCG
GAGTTGGAAAACAATGAAAAGGCCCCCAAGGTAGTTATCCTTAAAAAAGCCACAGCATACAT
CCTGTCCGTCCAAGCAGAGGAGCAAAAGCTCATTTCTGAAGAGGACTTGTTGCGGAAACGAC
GAGAACAGTTGAAACACAAACTTGAACAGCTACGGAACTCTTGTGCGGCTAGCGGCGCGCCC
GGTAAGCCTATCCCTAACCCTCTCCTCGGTCTCGATTCTACGTGAattaattaagaattcga
cccagctttcttgtacaaagtggttgatatccagcacagtggcggccgctcgagtctagagg
gcccgcggttcgaaggtaagcctatccctaaccctctcctcggtctcgattctacgcgtacc
ggtgtcgactctagataaagcatcaagcttatcgcgataccgtcgacaatcaacctctggat
tacaaaatttgtgaaagattgactggtattcttaactatgttgctccttttacgctatgtgg
atacgctgctttaatgcctttgtatcatgctattgcttcccgtatggctttcattttctcct
ccttgtataaatcctggttgctgtctctttatgaggagttgtggcccgttgtcaggcaacgt
ggcgtggtgtgcactgtgtttgctgacgcaacccccactggttggggcattgccaccacctg
tcagctcctttccgggactttcgctttccccctccctattgccacggcggaactcatcgccg
cctgccttgcccgctgctggacaggggctcggctgttgggcactgacaattccgtggtgttg
tcggggaagctgacgtcctttccatggctgctcgcctgtgttgccacctggattctgcgcgg
gacgtccttctgctacgtcccttcggccctcaatccagcggaccttccttcccgcggcctgc
tgccggctctgcggcctcttccgcgtcttcgccttcgccctcagacgagtcggatctccctt
tgggccgcctccccgcctggaatgatatcctcgagcccgggcgat
**Ef1a promoter**
KRAB
**DNMT3a3L**
*hMYC₁₄₄₋₄₅₄*
*V5*

### MESv2-Tmir (SEQ ID NO: 150):

Ef1a: :CS- hMYC₁₄₄₋₄₅₄ -V5-Tmir [**MESv2-Tmir**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGACCATCATCCAGGACTGTATGTGGAGCGGCTTCTCGGCCGCCGCCAAGCTCGTCT
CAGAGAAGCTGGCCTCCTACCAGGCTGCGCGCAAAGACAGCGGCAGCCCGAACCCCGCCCGC
GGCCACAGCGTCTGCTCCACCTCCAGCTTGTACCTGCAGGATCTGAGCGCCGCCGCCTCAGA
GTGCATCGACCCCTCGGTGGTCTTCCCCTACCCTCTCAACGACAGCAGCTCGCCCAAGTCCT
GCGCCTCGCAAGACTCCAGCGCCTTCTCTCCGTCCTCGGATTCTCTGCTCTCCTCGACGGAG
TCCTCCCCGCAGGGCAGCCCCGAGCCCCTGGTGCTCCATGAGGAGACACCGCCCACCACCAG
CAGCGACTCTGAGGAGGAACAAGAAGATGAGGAAGAAATCGATGTTGTTTCTGTGGAAAAGA
GGCAGGCTCCTGGCAAAAGGTCAGAGTCTGGATCACCTTCTGCTGGAGGCCACAGCAAACCT
CCTCACAGCCCACTGGTCCTCAAGAGGTGCCACGTCTCCACACATCAGCACAACTACGCAGC
GCCTCCCTCCACTCGGAAGGACTATCCTGCTGCCAAGAGGGTCAAGTTGGACAGTGTCAGAG
TCCTGAGACAGATCAGCAACAACCGAAAATGCACCAGCCCCAGGTCCTCGGACACCGAGGAG
AATGTCAAGAGGCGAACACACAACGTCTTGGAGCGCCAGAGGAGGAACGAGCTAAAACGGAG
CTTTTTTGCCCTGCGTGACCAGATCCCGGAGTTGGAAAACAATGAAAAGGCCCCCAAGGTAG
TTATCCTTAAAAAAGCCACAGCATACATCCTGTCCGTCCAAGCAGAGGAGCAAAAGCTCATT
TCTGAAGAGGACTTGTTGCGGAAACGACGAGAACAGTTGAAACACAAACTTGAACAGCTACG
GAACTCTTGTGCGGGCGCGCCCGGTAAGCCTATCCCTAACCCTCTCCTCGGTCTCGATTCTA
CGTGAattaattaagaattcgacccagctttcttgtacaaagtggttgatatccagcacagt
 
ggcggccgctcgagtctagagggcccgcggttcgaaggtaagcctatccctaaccctctcct
cggtctcgattctacgcgtaccggtgtcgactctagataaagcatcaagcttatcgcgatac
cgtcgacaatcaacctctggattacaaaatttgtgaaagattgactggtattcttaactatg
ttgctccttttacgctatgtggatacgctgctttaatgcctttgtatcatgctattgcttcc
cgtatggctttcattttctcctccttgtataaatcctggttgctgtctctttatgaggagtt
gtggcccgttgtcaggcaacgtggcgtggtgtgcactgtgtttgctgacgcaacccccactg
gttggggcattgccaccacctgtcagctcctttccgggactttcgctttccccctccctatt
gccacggcggaactcatcgccgcctgccttgcccgctgctggacaggggctcggctgttggg
cactgacaattccgtggtgttgtcggggaagctgacgtcctttccatggctgctcgcctgtg
ttgccacctggattctgcgcgggacgtccttctgctacgtcccttcggccctcaatccagcg
gaccttccttcccgcggcctgctgccggctctgcggcctcttccgcgtcttcgccttcgccc
tcagacgagtcggatctccctttgggccgcctccccgcctggaatgatatcctcgagcccgg
gcgatatattgccttatttccgattattgccttatttcgcattattgccttatttctcacta
ttgccttatttccgatcccgggtttaaaccgatcaacaaaatcactgatgctggacgatca
acaaaatcactgatgctggacgatcaacaaaatcactgatgctggatcaccaacaaaatcac
tgatgctggacgatgtttaaaccctgcaggcgatcagctatgccagcatcttgcccgatcag
ctatgccagcatcttgcccgatcagctatgccagcatcttgcctcaccagctatgccagcat
cttgccccgatcctgcaggagatct
**Ef1a promoter**
CS
*hMYC₁₄₄₋₄₅₄*
*V5*
miR124 Target sequences
miR338-3p Target sequences
miR31 Target sequences

### MESv3-Tmir (SEQ ID NO: 151):

Ef1a: :YR- hMYC₁₄₄₋₄₅₄ -V5 -Tmir [**MESv3-Tmir**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
 
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGAGGCCCCGGCTGAAAAACGTGGACAGGAGCACTGCACAGCAGTTGG
CAGTAACTGTGGGCAACGTCACCGTCATTATCACAGACTTTAAGGAAAGACTCGCTCCTCA
TCGACATCCTCATCCACAGTGACCTCCAGTGCAGGGTCAGAACAGCAGAACCAGACCATCAT
CCAGGACTGTATGTGGAGCGGCTTCTCGGCCGCCGCCAAGCTCGTCTCAGAGAAGCTGGCCT
CCTACCAGGCTGCGCGCAAAGACAGCGGCAGCCCGAACCCCGCCCGCGGCCACAGCGTCTGC
TCCACCTCCAGCTTGTACCTGCAGGATCTGAGCGCCGCCGCCTCAGAGTGCATCGACCCCTC
GGTGGTCTTCCCCTACCCTCTCAACGACAGCAGCTCGCCCAAGTCCTGCGCCTCGCAAGACT
CCAGCGCCTTCTCTCCGTCCTCGGATTCTCTGCTCTCCTCGACGGAGTCCTCCCCGCAGGGC
AGCCCCGAGCCCCTGGTGCTCCATGAGGAGACACCGCCCACCACCAGCAGCGACTCTGAGGA
GGAACAAGAAGATGAGGAAGAAATCGATGTTGTTTCTGTGGAAAAGAGGCAGGCTCCTGGCA
AAAGGTCAGAGTCTGGATCACCTTCTGCTGGAGGCCACAGCAAACCTCCTCACAGCCCACTG
GTCCTCAAGAGGTGCCACGTCTCCACACATCAGCACAACTACGCAGCGCCTCCCTCCACTCG
GAAGGACTATCCTGCTGCCAAGAGGGTCAAGTTGGACAGTGTCAGAGTCCTGAGACAGATCA
GCAACAACCGAAAATGCACCAGCCCCAGGTCCTCGGACACCGAGGAGAATGTCAAGAGGCGA
ACACACAACGTCTTGGAGCGCCAGAGGAGGAACGAGCTAAAACGGAGCTTTTTTGCCCTGCG
TGACCAGATCCCGGAGTTGGAAAACAATGAAAAGGCCCCCAAGGTAGTTATCCTTAAAAAAG
CCACAGCATACATCCTGTCCGTCCAAGCAGAGGAGCAAAAGCTCATTTCTGAAGAGGACTTG
TTGCGGAAACGACGAGAACAGTTGAAACACAAACTTGAACAGCTACGGAACTCTTGTGCGGG
CGCGCCCGGTAAGCCTATCCCTAACCCTCTCCTCGGTCTCGATTCTACGTGAattaattaag
aattcgacccagctttcttgtacaaagtggttgatatccagcacagtggcggccgctcgagt
ctagagggcccgcggttcgaaggtaagcctatccctaaccctctcctcggtctcgattctac
gcgtaccggtgtcgactctagataaagcatcaagcttatcgcgataccgtcgacaatcaacc
tctggattacaaaatttgtgaaagattgactggtattcttaactatgttgctccttttacgc
tatgtggatacgctgctttaatgcctttgtatcatgctattgcttcccgtatggctttcatt
ttctcctccttgtataaatcctggttgctgtctctttatgaggagttgtggcccgttgtcag
gcaacgtggcgtggtgtgcactgtgtttgctgacgcaacccccactggttggggcattgcca
ccacctgtcagctcctttccgggactttcgctttccccctccctattgccacggcggaactc
atcgccgcctgccttgcccgctgctggacaggggctcggctgttgggcactgacaattccgt
ggtgttgtcggggaagctgacgtcctttccatggctgctcgcctgtgttgccacctggattc
tgcgcgggacgtccttctgctacgtcccttcggccctcaatccagcggaccttccttcccgc
ggcctgctgccggctctgcggcctcttccgcgtcttcgccttcgccctcagacgagtcggat
ctccctttgggccgcctccccgcctggaatgatatcctcgagcccgggcgatatattgcctt
atttccgattattgccttatttcgcattattgccttatttctcactattgccttatttccga
tcccggggtttaaaccgatcaacaaaatcactgatgctggacgatcaacaaaatcactgatg
ctggacgatcaacaaaatcactgatgctggatcaccaacaaaatcactgatgctggacgatg
tttaaaccctgcaggcgatcagctatgccagcatcttgcccgatcagctatgccagcatctt
gcccgatcagctatgccagcatcttgcctcaccagctatgccagcatcttgcccgatcctgc
aggagatct
**Ef1a promoter**
YR
*hMYC₁₄₄₋₄₅₄*
*V5*
miR124 Target sequences
miR338-3p Target sequences
miR31 Target sequences

### MESv4-Tmir (SEQ ID NO: 152):

Ef1a: :CS-YR- hMYC₁₄₄₋₄₅₄ -V5 -Tmir [**MESv4-Tmir**] 
CGATAAGCTTTGCAAAGATGGATAAAGTTTTAAACAGAGAGGAATCTTTGCAGCTAATGGAC
CTTCTAGGTCTTGAAAGGAGTGGGAATTGGCTCCGGTGCCCGTCAGTGGGCAGAGCGCACAT
CGCCCACAGTCCCCGAGAAGTTGGGGGGAGGGGTCGGCAATTGAACCGGTGCCTAGAGAAGG
TGGCGCGGGGTAAACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGCAACGGGTTTGCCG
CCAGAACACAGGTAAGTGCCGTGTGTGGTTCCCGCGGGCCTGGCCTCTTTACGGGTTATGGC
CCTTGCGTGCCTTGAATTACTTCCACTGGCTGCAGTACGTGATTCTTGATCCCGAGCTTCGG
GTTGGAAGTGGGTGGGAGAGTTCGAGGCCTTGCGCTTAAGGAGCCCCTTCGCCTCGTGCTTG
AGTTGAGGCCTGGCCTGGGCGCTGGGGCCGCCGCGTGCGAATCTGGTGGCACCTTCGCGCCT
GTCTCGCTGCTTTCGATAAGTCTCTAGCCATTTAAAATTTTTGATGACCTGCTGCGACGCTT
TTTTTCTGGCAAGATAGTCTTGTAAATGCGGGCCAAGATCTGCACACTGGTATTTCGGTTTT
TGGGGCCGCGGGCGGCGACGGGGCCCGTGCGTCCCAGCGCACATGTTCGGCGAGGCGGGGCC
TGCGAGCGCGGCCACCGAGAATCGGACGGGGGTAGTCTCAAGCTGGCCGGCCTGCTCTGGTG
CCTGGCCTCGCGCCGCCGTGTATCGCCCCGCCCTGGGCGGCAAGGCTGGCCCGGTCGGCACC
AGTTGCGTGAGCGGAAAGATGGCCGCTTCCCGGCCCTGCTGCAGGGAGCTCAAAATGGAGGA
CGCGGCGCTCGGGAGAGCGGGCGGGTGAGTCACCCACACAAAGGAAAAGGGCCTTTCCGTCC
TCAGCCGTCGCTTCATGTGACTCCACGGAGTACCGGGCGCCGTCCAGGCACCTCGATTAGTT
CTCGAGCTTTTGGAGTACGTCGTCTTTAGGTTGGGGGGAGGGGTTTTATGCGATGGAGTTTC
CCCACACTGAGTGGGTGGAGACTGAAGTTAGGCCAGCTTGGCACTTGATGTAATTCTCCTTG
GAATTTGCCCTTTTTGAGTTTGGATCTTGGTTCATTCTCAAGCCTCAGACAGTGGTTCAAAG
TTTTTTTCTTCCATTTCAGGTGTCGTGAGGAATTCTGCAGTCGATCGACGGTACCGCGGGCC
CAACGGATCCACCATGCTGGAACCAGAAAAGATCATTGGGGCAACAGATTCCTGTGGTGATT
TAATGTTCCTAATGAAATGGAAAGACACAGATGAAGCTGACCTGGTTCTTGCAAAAGAAGCT
AATGTGAAATGTCCACAAATTGTGATAGCATTTTATGAAGAGAGACTGACATGGCATGCATA
TCCTGAGaccatgaggccccggctgaaaaacgtggacaggagcactgcacagcagttggcag
taactgtgggcaacgtcaccgtcattatcacagactttaaggaaaagactcgctcctcatcg
acatcctcatccacagtgacctccagtgcagggtcagaacagcagaaccagACCATCATCCA
GGACTGTATGTGGAGCGGCTTCTCGGCCGCCGCCAAGCTCGTCTCAGAGAAGCTGGCCTCCT
ACCAGGCTGCGCGCAAAGACAGCGGCAGCCCGAACCCCGCCCGCGGCCACAGCGTCTGCTCC
ACCTCCAGCTTGTACCTGCAGGATCTGAGCGCCGCCGCCTCAGAGTGCATCGACCCCTCGGT
GGTCTTCCCCTACCCTCTCAACGACAGCAGCTCGCCCAAGTCCTGCGCCTCGCAAGACTCCA
GCGCCTTCTCTCCGTCCTCGGATTCTCTGCTCTCCTCGACGGAGTCCTCCCCGCAGGGCAGC
CCCGAGCCCCTGGTGCTCCATGAGGAGACACCGCCCACCACCAGCAGCGACTCTGAGGAGGA
ACAAGAAGATGAGGAAGAAATCGATGTTGTTTCTGTGGAAAAGAGGCAGGCTCCTGGCAAAA
GGTCAGAGTCTGGATCACCTTCTGCTGGAGGCCACAGCAAACCTCCTCACAGCCCACTGGTC
CTCAAGAGGTGCCACGTCTCCACACATCAGCACAACTACGCAGCGCCTCCCTCCACTCGGAA
 
GGACTATCCTGCTGCCAAGAGGGTCAAGTTGGACAGTGTCAGAGTCCTGAGACAGATCAGCA
ACAACCGAAAATGCACCAGCCCCAGGTCCTCGGACACCGAGGAGAATGTCAAGAGGCGAACA
CACAACGTCTTGGAGCGCCAGAGGAGGAACGAGCTAAAACGGAGCTTTTTTGCCCTGCGTGA
CCAGATCCCGGAGTTGGAAAACAATGAAAAGGCCCCCAAGGTAGTTATCCTTAAAAAAGCCA
CAGCATACATCCTGTCCGTCCAAGCAGAGGAGCAAAAGCTCATTTCTGAAGAGGACTTGTTG
CGGAAACGACGAGAACAGTTGAAACACAAACTTGAACAGCTACGGAACTCTTGTGCGGGCGC
GCCCGGTAAGCCTATCCCTAACCCTCTCCTCGGTCTCGATTCTACGTGAattaattaagaat
tcgacccagctttcttgtacaaagtggttgatatccagcacagtggcggccgctcgagtcta
gagggcccgcggttcgaaggtaagcctatccctaaccctctcctcggtctcgattctacgcg
taccggtgtcgactctagataaagcatcaagcttatcgcgataccgtcgacaatcaacctct
ggattacaaaatttgtgaaagattgactggtattcttaactatgttgctccttttacgctat
gtggatacgctgctttaatgcctttgtatcatgctattgcttcccgtatggctttcattttc
tcctccttgtataaatcctggttgctgtctctttatgaggagttgtggcccgttgtcaggca
acgtggcgtggtgtgcactgtgtttgctgacgcaacccccactggttggggcattgccacca
cctgtcagctcctttccgggactttcgctttccccctccctattgccacggcggaactcatc
gccgcctgccttgcccgctgctggacaggggctcggctgttgggcactgacaattccgtggt
gttgtcggggaagctgacgtcctttccatggctgctcgcctgtgttgccacctggattctgc
gcgggacgtccttctgctacgtcccttcggccctcaatccagcggaccttccttcccgcggc
ctgctgccggctctgcggcctcttccgcgtcttcgccttcgccctcagacgagtcggatctc
cctttgggccgcctccccgcctggaatgatatcctcgagcccgggcgatatattgccttatt
tccgattattgccttatttcgcattattgccttatttctcactattgccttatttccgatcc
cggggtttaaaccgatcaacaaaatcactgatgctggacgatcaacaaaatcactgatgctg
gacgatcaacaaaatcactgatgctggatcaccaacaaaatcactgatgctggacgatgttt
aaaccctgcaggcgatcagctatgccagcatcttgcccgatcagctatgccagcatcttgcc
cgatcagctatgccagcatcttgcctcaccagctatgccagcatcttgcccgatcctgcagg
agatct
**Ef1a promoter**
CS
*yr*
*hMYC₁₄₄₋₄₅₄*
*V5*
miR124 Target sequences
miR338-3p Target sequences
miR31 Target sequences

### Cell culture

U-251 and CT26 cells were cultured in plastic-adherence conditions in DMEM medium (Dulbecco's Modified Eagle's Medium - high glucose, Sigma-Aldrich) containing 10% fetal bovine serum (FBS, Sigma-Aldrich), 1% Pen/Strept (Sigma-Aldrich), 2 mM Glutamine (Sigma-Aldrich), 1% non-essential amino acids (MEM NEAA, ThermoFisher Scientific), 1% sodium pyruvate solution (Sigma-Aldrich) and passaged twice a week using Trypsin-EDTA solution (Sigma-Aldrich).

BxPC-3 and CFPAC-1 were cultured in plastic-adherence conditions in RPMI-1640 (Sigma-Aldrich) containing 10% FBS, 1% Pen/Strept, 2 mM Glutamine. All the cell lines were passaged twice a week using Trypsin-EDTA solution (Sigma-Aldrich).

Cancer stem cells (CSCs) glioblastoma tumors were maintained in plastic-adherence conditions in DMEM/F12 (Sigma-Aldrich) supplemented with Hormon Mix (DMEM/F12, 0.6% Glucose (Sigma-Aldrich) (30% in phosphate buffer (PBS) (Euroclone)), Insulin (Sigma-Aldrich) 250 µg/ml, putrescine powder (Sigma-Aldrich) 97 µg/ml, apotransferrin powder (Sigma Aldrich), sodium selenite 0.3 µM, progesterone 0.2 µM), 1% Pen/Strept, 2 mM Glutamine, 0.66% Glucose (30% in phosphate buffer salt (PBS) (Euroclone)), and heparin (4 mg/ml, Sigma-Aldrich); bFGF (20 ng/ml, ThermoFisher Scientific) and EGF (20 ng/ml, ThermoFisher Scientific) were added freshly to culture medium..

All the cultures were kept in humidified atmosphere of 5% CO2 at 37°C under atmospheric oxygen conditions.

### Growth curve analysis

1.5×10⁵ of cells were seeded in adherent condition in a 6 multi-well plate at day 0; at day 1 cultures were infected with lentiviral vectors and at day 3, cells were detached, live cells were stained with Trypan Blue Solution (0.4%, ThermoFisher Scientific) and counted using Countess^{™} II Automated Cell Counter (ThermoFisher Scientific); after this passage, 1.5×10^5 cells were seeded again. This was repeated for 3-4 time points every 3-4 days; the experiment was repeated 3 times for each time point.

### Primary murine neuronal culture

Primary cultures of mouse embryonic cortical neurons were prepared from E17.5 C57BL/6 Wild-Type mice. Briefly, after dissection, cortices were enzymatically digested with 0.025% trypsin (GIBCO) in Hank's balanced salt solution (HBSS) (Euroclone) for 20 min at 37°C. Successively, HBSS with trypsin was removed and the hippocampi were washed with plating medium (Neurobasal A medium supplemented with 1X B-27 supplement, 3.3 mM glucose, 2 mM glutamine and 1% penicillin/streptomycin) and mechanically dissociated with a P1000-pipette to obtain a homogeneous cell suspension. Cells were then plated on poly-L-lysine (PLL) (0.1 mg/ml) coated glass coverslips

### Immunostaining

Cells were seeded on glass coverslips and they were fixed for 20 minutes on ice in 4% paraformaldehyde (PFA, Sigma), solution in phosphate-buffered saline (PBS, Euroclone). Then they were washed twice with PBS and were permeabilized for 30' in blocking solution, containing 0.2% Triton X-100 (SigmaAldrich) and 5% donkey serum (Euroclone), and incubated overnight at 4°C with the primary antibodies diluted in blocking solution. The primary antibodies utilized were as follows: anti-V5 (mouse, 1:500, ThermoFisher Scientific, R96025), anti-GFP (chicken, 1:1000, Thermo Fisher Scientific, A10262), and anti-MAP2 (chicken, 1:1000, Abcam, ab92434). The next day, cells were washed 3 times with PBS for 5 minutes and incubated for 1 hour at room temperature with Hoechst 33342 (ThermoFischer Scientific) and with secondary antibodies (ThermoFisher Scientific) in blocking solution. Finally, slides were washed and mounted in Fluorescent Mounting Medium (Dako Cytomation). Images were acquired with epifluorescence microscope Nikon DS-Qi2 and analyzed with Fiji software.

### AA V production and infection

Replication-incompetent, recombinant viral particles were produced in 293 T cells by polyethylenimine (PEI) (Polyscience) co-transfection of three different plasmids: transgenecontaining plasmid, packaging plasmid for rep and cap genes and pHelper (Agilent) for the three adenoviral helper genes. The cells and supernatant were harvested at 120 hr. Cells were lysed in hypertonic buffer (40 mM Tris, 500 mM NaCl, 2 mM MgCl2, pH = 8) containing 100 U/ml Salt Active Nuclease (SAN, Arcticzymes) for 1 hr at 37°C, whereas the viral particles present in the supernatant were concentrated by precipitation with 8% PEG8000 (Polyethylene glycol 8000, Sigma-Aldrich) and then added to supernatant for an additional incubation of 30 min at 37°C. To clarify the lysate cellular debris were separated by centrifugation (4000 g, 30 min). The viral phase was isolated by iodixanol step gradient (15%, 25%, 40%, 60% Optiprep, Sigma-Aldrich) in the 40% fraction and concentrated in PBS (Phosphate Buffer Saline) with 100K cut-off concentrator (Amicon Ultra15, MERCK-Millipore). Virus titers were determined using AAVpro Titration Kit Ver2 (TaKaRa).

2,5×10^4 CSCs L0627 were infected with adenoviral vectors expressing GFP (5 µl/well) and seeded in a 24 multi-wells plate on glass coverslips previously coated with Matrigel. After 4 days, cells were fixed and used for immunostaining studies.

### EXAMPLE B1

The inventors sought to build a set of ESF variants by using TEAD1 and c-MYC, two transcriptional activators with oncogenic activity. ESFs were generated by rational design, wherein the transcriptional activation domains of the relevant transcription factors (TFs) were removed and alternative suppressor domains, namely the chromo shadow (CS) domain from CBX5 protein and the YAF2-RYBP (Y-R) domain from RYBP protein, were added thereto. TEAD Epigenetic Silencers (TES) were generated by fusing: the CS at the N-terminus of the amino-acids 1-166 of human TEAD (TESv2); the Y-R at the C-terminus of the amino-acids 1-166 of human TEAD (TESv3); the CS at the N-terminus and the Y-R at the C-terminus of the amino-acids 1-166 of human TEAD (TESv4) (Fig. 17a). MYC Epigenetic Silencers (MES) were generated by fusing either the CS, or the Y-R or both at the N-terminus of the amino-acids 144-454 of human MYC, to avoid spatial hindrance at the protein's C-terminus, which directly interacts with MAX for the formation of heterodimers (MESv2-4) (Fig. 17b).

### EXAMPLE B2

ESF should not have any appreciable and harmful effect on heathy brain cells, which do not express the oncogenes of interest and are not proliferative cells. In such healthy cells, ESF induced decrease of some key cell-cycle genes may be detrimental to the cells. However, it is possible that ESF-dependent chromatin changes could alter neuronal performance *in vivo* over longer periods of time, e.g., if used for treatment in humans. Thus, the inventors have devised a strategy to restrict ESF expression to cancer cells after viral inoculation of the brain. Said strategy is based on a microRNA (miRNAs) detargeting system, which enables the silencing of a transgene by the inclusion of binding/target sites (TS) of miRNAs endogenously expressed in specific cell types in which expression is not desired. To this end, a cassette was generated in which the 3'-UTR downstream to the transgene, e.g., the ESF cDNA, comprised a cluster of 4x TS for each of miRNA-124, -338-3p and -31, which are specifically expressed in neurons, astrocytes and oligodendrocytes, respectively. Thus, exogenous transgene expression, e.g., SES expression as demonstrated in Fig. 18, should be silenced in all of the foregoing cell types through miRNA-dependent post-transcriptional silencing and block of protein translation (Fig. 18a). Notably, miRNA-124, -338-3p and -31, are not expressed in GBM, as demonstrated by GFP-Tmir and SES-Tmir lentiviruses (LVs), which exhibited unaffected transgene expression in cancer cells (Fig. 18b). The data herein further demonstrate that the presence of Tmir did not jeopardize SES activity, as the construct induced a significant cell loss in the transduced cancer cells *in vitro* (Fig. 18b, bottom right). The specificity of the miRNA detargeting system is demonstrated in Fig. 18c wherein the data show that, when used in primary cortical culture from mouse, no cells expressed the SES, as intended and due to the miRNA detargeting (Fig. 18c).

### EXAMPLE B3

It is desirable that ESFs be used with a system of delivery that allows easy and broad distribution of the vector over the brain area that may contain residual tumor cells after surgery. This would reduce the likelihood of tumor recurrence, which in GBM is a significant problem. To this end, the inventors tested the use of adeno-associated viruses (AAVs) as shuttling vectors for the constructs of the invention. Recombinant serotypes 2 and 5 performed better, as compared to serotype 9, for transgene delivery, as assessed by GFP expression, within patient derived cancer stem cells (Fig. 19).

### EXAMPLE B4

Since SOX2, TEAD1 and MYC are widely express in many cancer types, it is envisaged that there is the possibility to expand the usage of ESFs disclosed herein to other cancer types, especially where these types can be efficiently targeted by viral transduction *in vivo.* For instance, here the inventors show that SES is able to reduce the growth of a murine cell line derived from liver metastasis of colon adenocarcinomas (CT26) (Fig. 20a). Moreover, TES is also efficient in decreasing the growth of human pancreatic ductal adenocarcinoma (PDAC) cell lines, including BxPC-3 and CFPAC-1 (Fig. 20b and 20c).

### DISCUSSION

The activity of developmental transcription factors (TFs) is mainly restricted during morphogenesis executing a prominent role in stem cell identity, cell lineage commitment and differentiation. However, these TFs can be re-activated or hijacked by the cancer genetic program to propel tumor development and progression. It is estimated that about 20% of all the known oncogenic proteins are represented by TFs with critical importance for acquiring malignant cell dedifferentiation, proliferation, and migration. Despite their pervasive role in tumors, interfering with their functions in a translational perspective has proven challenging. In fact, stable and complete gene silencing by various genetic tools or small molecules have been difficult to achieve in cancer cells. Moreover, the cancer genetic program has been repeatedly shown to overcome the inactivation of single genes by reconfiguring the transcriptional network to promote cancer resistance and recurrence. Herein, we designed additional version of ESFs by reconfiguring SOX2, TEAD1, and MYC TFs by rational assembly of transcriptional and epigenetic negative regulators of gene transcription as well as an miRNA based cassette for detargeting ESF expression, or the expression of any relevant transgene, from brains resident cells.

The inventors herein demonstrate that configurations of ESFs that utilize different epigenetically active protein domains (e.g. Chromo Shadow domain from CBX5 and YAF2-RYBP domain from RYBP), can elicit the same functional activity of those harboring KRAB and DNMT3A/L domains. Moreover, detargeting the expression of the exogenous ESF from healthy brain cells, e.g., neurons, astrocytes and oligodendrocytes, by using miRNA target sequences within the 3' UTR was demonstrated, thus affording improved safety and specificity. By utilizing target sequences of miRNAs that are highly expressed in brain cells, but not in tumor cells, the inventors provide polynucleotides and transgene expression cassettes with high specificity, e.g., for use as an anti-GBM treatment.

The inventors herein also provide alternative therapeutic viruses to lentiviruses, that could be similarly used. Particularly useful are strains of adeno-associated viruses (AAVs) that can spread through the brain tissue due to their small size and which exhibit reduced binding to cell membranes. Maximizing viral spreading in the brain parenchyma will increase the targeting efficiency of cancer cells scattered in the tissue, providing a better protection from tumor recurrence. Here, the inventors demonstrate that AAV2 diffuses in brain parenchyma when injected directly in the organ and is able to infect the GBM CSCs with high efficiency, *in vitro.*

The inventors further provide evidence that ESFs may act as effective anti-cancer agents/ treatments in a variety of cancers that share a common vulnerability due to the important role of the chosen TFs in promoting tumor development. In particular, the inventors herein demonstrate that SES is able to reduce the proliferation of cells derived from liver metastasis of colon adenocarcinoma in mice and that TES is effective in blocking the growth of cell lines of human PDAC.

Taken together, the disclosures herein provide a set of epigenetic repressors which operate as a dominant negative version of the oncogenic TFs, SOX2, TEAD1, and MYC and are able to bind and stably repress their respective transcriptional networks. Targeted viral delivery of these ESFs in glioblastoma cells is sufficient to inhibit tumor development by blocking cell proliferation and inducing cell death. Specificity and thus safety, is afforded by an assembly of miRNA target sequences which are able to detarget, i.e., inhibit, the expression of the ESFs from specific brain cells, whilst the ESF is expressed in tumor cells. Given the wide applicability to other oncogenic TFs and the high efficiency of targeting cancer cells by viral transduction, using viral vectors that include AAV, this approach provides the opportunity to repress glioblastoma and other deadly and hard to treat cancers.

### EMBODIMENTS

Various preferred features and embodiments of the present invention will now be described with reference to the following numbered paragraphs (paras).
1. A polynucleotide comprising at least one miR-124 target sequence, and/or at least one miR-338-3p target sequence, and/or at least one miR-31 target sequence, wherein the miRNA target sequences are operably linked to a transgene.
2. The polynucleotide of para 1, wherein the number of copies of each of the miRNA target sequences is independently selected from the group consisting of: one, two, three, and four.
3. The polynucleotide of para 1 or para 2, wherein the polynucleotide comprises four miR-124 target sequences, four miR-338-3p target sequences and four miR-31 target sequences, wherein the miRNA target sequences are operably linked to the transgene.
4. The polynucleotide of any one of paras 1 to 3, wherein:
   (a) the miR-124 target sequence comprises or consists of a nucleotide sequence that has at least 90% sequence identity to SEQ ID NO: 1;
   (b) the miR-338-3p target sequence comprises or consists of a nucleotide sequence that has at least 90% sequence identity to SEQ ID NO: 2; and/or
   (c) the miR-31 target sequence comprises or consists of a nucleotide sequence that has at least 90% sequence identity to SEQ ID NO: 3.
5. The polynucleotide of any one of paras 1 to 4, wherein the miRNA target sequences are located after the transgene in the 5' to 3' direction.
6. The polynucleotide of any one of paras 1 to 5, wherein the miRNA target sequences or clusters of copies of the miRNA target sequences are, from 5' to 3', arranged in the order: miR-124 target sequence(s), miR-338-3p target sequence(s), and miR-31 target sequence(s).
7. The polynucleotide of any one of paras 1 to 6, wherein the miRNA target sequences are separated by spacer sequences.
8. The polynucleotide of any one of paras 1 to 7, wherein the polynucleotide comprises a nucleotide sequence that has at least 90% sequence identity to SEQ ID NO: 4.
9. The polynucleotide of any one of paras 1 to 8, wherein the transgene encodes an epigenetic silencer factor (ESF) comprising a transcription factor DNA-binding domain operably linked to at least one epigenetic effector domain, wherein the transcription factor is an oncogenic transcription factor or a cancer-associated transcription factor.
10. The polynucleotide of any one of para 9, wherein the transcription factor is selected from the group consisting of SOX2, MYC, MYCN, TEAD1, TEAD2, TEAD3, TEAD4, FOXA1, FOXA2, ELK1, ELK3, ELK4, SRF, FOXM1, FOXC1, FOXC2, TWIST1, SALL4, ELF1, HIF1A, SOX9, SOX12, SOX18, ETS1, PAX3, PAX8, GLI1, GLI2, GLI3, ETV1, ETV2, ETV3, RUNX1, RUNX2, RUNX3, MAFB, TFAP2C and E2F1.
11. The polynucleotide of para 9 or para 10, wherein the transcription factor is SOX2, TEAD1 or MYC.
12. The polynucleotide of any one of paras 9 to 11, wherein the epigenetic effector domain is selected from the group consisting of a KRAB domain, a DNMT3A domain, a DNMT3L domain, a ZIM3-KRAB (Z-KRAB) domain, a Chromo Shadow (CS) domain, a YAF2-RYBP (Y-R) domain, an Engrailed Repressor (En-R) domain, a MeCP2 domain, a GLI3RD domain and a MAD1RD domain.
13. The polynucleotide of any one of paras 9 to 12, wherein the ESF comprises: (a) a CS domain; (b) a Y-R domain; (c) a CS domain and a Y-R domain; (d) a KRAB domain; and/or (e) a DNMT3A domain and a DNMT3L domain.
14. The polynucleotide of any one of paras 9 to 13, wherein the ESF comprises:
   (a) a KRAB domain, a SOX2 DNA-binding domain, a DNMT3A domain, and a DNMT3L domain;
   (b) a Chromo Shadow (CS) domain and a SOX2 DNA-binding domain;
   (c) a SOX2 DNA-binding domain, and a YAF2-RYBP (Y-R) domain;
   (d) a KRAB domain, a TEAD1 DNA-binding domain, a DNMT3A domain, and a DNMT3L domain;
   (e) a KRAB domain, a DNMT3A domain, a DNMT3L domain, and a MYC DNA-binding domain;
   (f) a Chromo Shadow (CS) domain, and a TEAD1 DNA-binding domain;
   (g) a TEAD1 DNA-binding domain, and a YAF2-RYBP (Y-R) domain;
   (h) a Chromo Shadow (CS) domain, a TEAD1 DNA-binding domain, and a YAF2-RYBP (Y-R) domain;
   (i) a Chromo Shadow (CS) domain, and a MYC DNA-binding domain;
   (j) a YAF2-RYBP (Y-R) domain, and a MYC DNA-binding domain; or
   (k) a Chromo Shadow (CS) domain, a YAF2-RYBP (Y-R) domain, and a MYC DNA-binding domain.
15. The polynucleotide of any one of paras 9 to 14, wherein the polynucleotide comprises a nucleotide sequence that has at least 90% sequence identity to any one of SEQ ID NOs: 114 - 119 and 126 - 131.
16. The polynucleotide of any one of paras 1 to 15, wherein the polynucleotide further comprises a promoter operably linked to the transgene, optionally wherein the promoter is a tissue-specific promoter or a constitutive promoter, optionally a cancer cell-specific promoter or a proliferating cell-specific promoter.
17. The polynucleotide of para 16, wherein the promoter is an Ef1a promoter or a Mki67 promoter.
18. A vector comprising the polynucleotide of any one of paras 1 to 17, optionally wherein the vector is a viral vector, optionally wherein the vector is a lentiviral vector or adeno-associated viral (AAV) vector.
19. A protein encoded by the polynucleotide of any one of paras 1 to 17, or the vector of para 18.
20. An epigenetic silencer factor (ESF) comprising a transcription factor DNA-binding domain operably linked to at least one epigenetic effector domain, wherein the transcription factor is an oncogenic transcription factor or a cancer-associated transcription factor, wherein the ESF comprises:
   (a) a Chromo Shadow (CS) domain, and a TEAD1 DNA-binding domain;
   (b) a TEAD1 DNA-binding domain, and a YAF2-RYBP (Y-R) domain;
   (c) a Chromo Shadow (CS) domain, a TEAD1 DNA-binding domain, and a YAF2-RYBP (Y-R) domain;
   (d) a Chromo Shadow (CS) domain, and a MYC DNA-binding domain;
   (e) a YAF2-RYBP (Y-R) domain, and a MYC DNA-binding domain; or
   (f) a Chromo Shadow (CS) domain, a YAF2-RYBP (Y-R) domain, and a MYC DNA-binding domain.
21. A nanoparticle comprising the polynucleotide of any one of paras 1 to 17, the vector of para 18, the protein of para 19 or the ESF of para 20.
22. A cell comprising the polynucleotide of any one of paras 1 to 17, the vector of para 18, the protein of para 19, the ESF of para 20, or the nanoparticle of para 21.
23. A composition comprising the polynucleotide of any one of paras 1 to 17, the vector of para 18, the protein of para 19, the ESF of para 20, the nanoparticle of para 21, or the cell of para 22.
24. The polynucleotide of any one of paras 1 to 17, the vector of para 18, the protein of para 19, the ESF of para 20, the nanoparticle of para 21, the cell of para 22, or the composition of para 23 for use in therapy.
25. The polynucleotide of any one of paras 1 to 17, the vector of para 18, the protein of para 19, the ESF of para 20, the nanoparticle of para 21, the cell of para 22, or the composition of para 23 for use in the treatment of cancer.
26. Use of the polynucleotide of any one of paras 1 to 17, the vector of para 18, the protein of para 19, the ESF of para 20, the nanoparticle of para 21, the cell of para 22, or the composition of para 23 for decreasing transcription and/or expression of at least one target gene in a cell.
27. A method of decreasing transcription and/or expression of at least one target gene in a cell, the method comprising introducing polynucleotide of any one of paras 1 to 17, the vector of para 18, the protein of para 19, the ESF of para 20, the nanoparticle of para 21, or the composition of para 23 into the cell.
28. An epigenetic silencer factor (ESF), or polynucleotide encoding therefor, for use in the treatment of cancer, wherein the ESF comprises a transcription factor DNA-binding domain operably linked to at least one epigenetic effector domain, wherein the transcription factor is an oncogenic transcription factor or a cancer-associated transcription factor, wherein the cancer is selected from the group consisting of: glioma, gliobastoma, medulloblastoma, astrocytoma, neuroblastomas, ependymoma, meningioma, retinoblastoma, rhabdomyosarcoma, lung cancer, prostate cancer, breast cancer, liver cancer, pancreatic cancer (e.g. human pancreatic ductal adenocarcinoma), bladder cancer, oropharyngeal cancer, kidney cancer, colon cancer (e.g. colon adenocarcinoma), or a metastasis of any of the foregoing.

## Claims

1. A polynucleotide comprising at least one miR-124 target sequence, and/or at least one miR-338-3p target sequence, and/or at least one miR-31 target sequence, wherein the miRNA target sequences are operably linked to a transgene.

2. The polynucleotide of claim 1, wherein the polynucleotide comprises at least one miR-124 target sequence, at least one miR-338-3p target sequence and at least one miR-31 target sequence, wherein the miRNA target sequences are operably linked to a transgene

3. The polynucleotide of claim 1 or claim 2, wherein:
(a) the miR-124 target sequence comprises or consists of a nucleotide sequence that has at least 90% sequence identity to SEQ ID NO: 1;
(b) the miR-338-3p target sequence comprises or consists of a nucleotide sequence that has at least 90% sequence identity to SEQ ID NO: 2; and/or
(c) the miR-31 target sequence comprises or consists of a nucleotide sequence that has at least 90% sequence identity to SEQ ID NO: 3.

4. The polynucleotide of any one of claims 1 to 3, wherein the polynucleotide comprises a nucleotide sequence that has at least 90% sequence identity to SEQ ID NO: 4.

5. The polynucleotide of any one of claims 1 to 4, wherein the transgene encodes an epigenetic silencer factor (ESF) comprising a transcription factor DNA-binding domain operably linked to at least one epigenetic effector domain, wherein the transcription factor is an oncogenic transcription factor or a cancer-associated transcription factor.

6. The polynucleotide of any one of claim 5, wherein the transcription factor is selected from the group consisting of SOX2, MYC, MYCN, TEAD1, TEAD2, TEAD3, TEAD4, FOXA1, FOXA2, ELK1, ELK3, ELK4, SRF, FOXM1, FOXC1, FOXC2, TWIST1, SALL4, ELF1, HIF1A, SOX9, SOX12, SOX18, ETS1, PAX3, PAX8, GLI1, GLI2, GLI3, ETV1, ETV2, ETV3, RUNX1, RUNX2, RUNX3, MAFB, TFAP2C and E2F1.

7. The polynucleotide of claim 5 or claim 6, wherein the epigenetic effector domain is selected from the group consisting of a KRAB domain, a DNMT3A domain, a DNMT3L domain, a ZIM3-KRAB (Z-KRAB) domain, a Chromo Shadow (CS) domain, a YAF2-RYBP (Y-R) domain, an Engrailed Repressor (En-R) domain, a MeCP2 domain, a GLI3RD domain and a MAD1RD domain.

8. The polynucleotide of any one of claims 1 to 7, wherein the polynucleotide further comprises a promoter operably linked to the transgene, optionally wherein the promoter is a tissue-specific promoter or a constitutive promoter, optionally a cancer cell-specific promoter or a proliferating cell-specific promoter.

9. The polynucleotide of claim 8, wherein the promoter is an Ef1a promoter or a Mki67 promoter.

10. A vector comprising the polynucleotide of any one of claims 1 to 9, optionally wherein the vector is a viral vector, optionally wherein the vector is a lentiviral vector or adeno-associated viral (AAV) vector.

11. An epigenetic silencer factor (ESF) comprising a transcription factor DNA-binding domain operably linked to at least one epigenetic effector domain, wherein the transcription factor is an oncogenic transcription factor or a cancer-associated transcription factor, wherein the ESF comprises:
(a) a Chromo Shadow (CS) domain, and a TEAD1 DNA-binding domain;
(b) a TEAD1 DNA-binding domain, and a YAF2-RYBP (Y-R) domain;
(c) a Chromo Shadow (CS) domain, a TEAD1 DNA-binding domain, and a YAF2-RYBP (Y-R) domain;
(d) a Chromo Shadow (CS) domain, and a MYC DNA-binding domain;
(e) a YAF2-RYBP (Y-R) domain, and a MYC DNA-binding domain; or
(f) a Chromo Shadow (CS) domain, a YAF2-RYBP (Y-R) domain, and a MYC DNA-binding domain.

12. A nanoparticle comprising the polynucleotide of any one of claims 1 to 9, the vector of claim 10, or the ESF of claim 11.

13. A cell comprising the polynucleotide of any one of claims 1 to 9, the vector of claim 10, the ESF of claim 11, or the nanoparticle of claim 12.

14. The polynucleotide of any one of claims 1 to 9, the vector of claim 10, the ESF of claim 11, the nanoparticle of claim 12, or the cell of claim 13 for use in therapy.

15. An epigenetic silencer factor (ESF), or polynucleotide encoding therefor, for use in the treatment of cancer, wherein the ESF comprises a transcription factor DNA-binding domain operably linked to at least one epigenetic effector domain, wherein the transcription factor is an oncogenic transcription factor or a cancer-associated transcription factor, wherein the cancer is selected from the group consisting of: glioma, gliobastoma, medulloblastoma, astrocytoma, neuroblastomas, ependymoma, meningioma, retinoblastoma, rhabdomyosarcoma, lung cancer, prostate cancer, breast cancer, liver cancer, pancreatic cancer (e.g. human pancreatic ductal adenocarcinoma), bladder cancer, oropharyngeal cancer, kidney cancer, colon cancer (e.g. colon adenocarcinoma), or a metastasis of any of the foregoing.
